(19) Europäisches Patentamt / European Patent Office / Office européen des brevets

(11) **EP 3 950 061 A1**

(12) **EUROPEAN PATENT APPLICATION**
published in accordance with Art. 153(4) EPC


(43) Date of publication:
**09.02.2022 Bulletin 2022/06**

(21) Application number: **20777744.2**

(22) Date of filing: **24.03.2020**

(51) International Patent Classification (IPC):
***A61P 35/00*** *(2006.01)* ***C07K 16/30*** *(2006.01)*
***A61K 47/68*** *(2017.01)* ***C12N 15/13*** *(2006.01)*
***A61K 31/551*** *(2006.01)*

(52) Cooperative Patent Classification (CPC):
**A61K 31/551; A61K 47/68; A61P 35/00; C07K 16/30**

(86) International application number:
**PCT/JP2020/012883**

(87) International publication number:
**WO 2020/196474 (01.10.2020 Gazette 2020/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2019 JP 2019057041**



(71) Applicant: **Daiichi Sankyo Company, Limited**
**Tokyo 103-8426 (JP)**

(72) Inventors:
- **TODA Narihiro**
  **Tokyo 103-8426 (JP)**
- **OTA Yusuke**
  **Tokyo 103-8426 (JP)**
- **DOI Fuminao**
  **Tokyo 103-8426 (JP)**
- **MEGURO Masaki**
  **Tokyo 103-8426 (JP)**
- **HAYAKAWA Ichiro**
  **Tokyo 103-8426 (JP)**
- **ASHIDA Shinji**
  **Tokyo 103-8426 (JP)**
- **MASUDA Takeshi**
  **Tokyo 103-8426 (JP)**
- **NAKADA Takashi**
  **Tokyo 103-8426 (JP)**

(74) Representative: **Wallace, Sheila Jane**
**Marks & Clerk LLP**
**15 Fetter Lane**
**London EC4A 1BW (GB)**


(54) **ANTIBODY-PYRROLOBENZODIAZEPINE DERIVATIVE CONJUGATE**


(57) A novel antibody-pyrrolobenzodiazepine (PBD) derivative conjugate, a medicine having therapeutic effect against tumor with the antibody-drug conjugate, and a method for treating a tumor by using the antibody-drug conjugate or medicine.


[Figure 1]

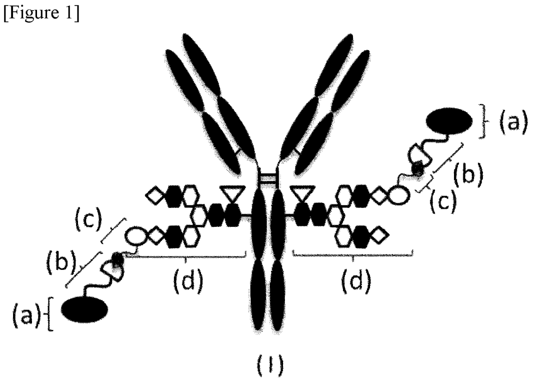


EP 3 950 061 A1

## Description

Technical Field

The present invention relates to an antibody-drug conjugate useful as an antitumor drug, the antibody-drug conjugate having an antibody capable of targeting tumor cells and a pyrrolobenzodiazepine derivative that are conjugated to each other via a linker structure moiety.

Background Art

Antibody-drug conjugates (ADCs), which are used for treatment of cancer and so on, have a drug with cytotoxic activity conjugated to an antibody, for example, that binds to an antigen expressed on the surface of cancer cells and is capable of cellular internalization of the antigen through the binding. ADCs can effectively deliver the drug to cancer cells, and are thus expected to cause accumulation of the drug within the cancer cells and to kill the cancer cells.

A useful example of drugs to be used for ADCs is pyrrolobenzodiazepine (PBD). PBD exhibits cytotoxicity by binding to, for example, the PuGPu sequence in the DNA minor groove. Anthramycin, a naturally-occurring PBD, was first discovered in 1965, and since this discovery various naturally-occurring PBDs and analog PBDs thereof have been discovered (Non Patent Literatures 1 to 4).

The general structural formula of PBDs is represented by the following formula:

[Formula 1]

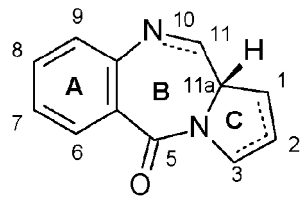

Known are PBDs different in the number of, types of, and sites of substituents in the A and C ring parts, and those different in degree of unsaturation in the B and C ring parts.

PBDs are known to come to have dramatically enhanced cytotoxicity through formation of a dimer structure (Non Patent Literatures 5, 6), and various ADCs with a dimer PBD have been reported (Patent Literatures 1 to 15). However, a PBD having a spiro ring at its C2-position and an ADC thereof have not known.

Human CLDN6 (claudin-6, hereinafter expressed as hCLDN6), a member of claudin (CLDN) family proteins, is a four-transmembrane protein consisting of 220 amino acid residues. Previous studies have suggested that hCLDN6 is overexpressed in some cancers, and is an attractive cancer therapeutic target (Non Patent Literatures 7 to 9). CLDN family proteins are incorporated into cells by endocytosis, and some of the family proteins have been reported to have short turnover time (Non Patent Literature 10), and hence CLDN family proteins are considered to be suitable as the target of antibody-drug conjugates (ADCs).

From such information suggesting the relation to cancer, monoclonal antibodies capable of specifically recognizing hCLDN6 have been discovered (Patent Literatures 16, 17), and ADCs having monomethyl auristatin E (MMAE) or maytansinoid (DM1), which are tubulin polymerization inhibitors, conjugated to a CLDN6-specific monoclonal antibody have been reported (Non Patent Literature 11).

On the other hand, antibodies capable of recognizing multiple members of the CLDN family are considered to allow a wider range of application of treatment, and in view of this an ADC having a pyrrolobenzodiazepine (PBD) with potent cytocidal effect conjugated to an antibody capable of recognizing CLDN6 and CLDN9 (Patent Literature 18) has been disclosed (Patent Literature 19).

However, the intensities of activity of the ADCs are still insufficient, and there exist unmet medical needs for use of hCLDN6 as a therapeutic target.

Citation List

Patent Literature

Patent Literature 1: WO 2013/173496
Patent Literature 2: WO 2014/130879
Patent Literature 3: WO 2017/004330
Patent Literature 4: WO 2017/004025
Patent Literature 5: WO 2017/020972
Patent Literature 6: WO 2016/036804
Patent Literature 7: WO 2015/095124
Patent Literature 8: WO 2015/052322
Patent Literature 9: WO 2015/052534
Patent Literature 10: WO 2016/115191
Patent Literature 11: WO 2015/052321
Patent Literature 12: WO 2015/031693
Patent Literature 13: WO 2011/130613
Patent Literature 14: WO 2005/040170
Patent Literature 15: WO 2017/137556
Patent Literature 16: WO 2009/087978
Patent Literature 17: WO 2011/057788
Patent Literature 18: WO 2015/069794
Patent Literature 19: WO 2017/096163

Non Patent Literature

Non Patent Literature 1: Julia Mantaj, et. al., Angewandte Chemie Internationl Edition 2016, 55, 2-29
Non Patent Literature 2: Dyeison Antonow. et. al., Chemical Reviews 2010, 111, 2815-2864
Non Patent Literature 3: In Antibiotics III. Springer Verlag, New York, pp.3-11
Non Patent Literature 4: Accounts of Chemical Research 1986, 19, 230
Non Patent Literature 5: Journal of the American Chemical Society 1992,114, 4939
Non Patent Literature 6: Journal of Organic Chemistry 1996, 61, 8141
Non Patent Literature 7: BMC Cancer, 2006, 6, 186
Non Patent Literature 8: Histopatholody, 2012, 61, 1043-1056
Non Patent Literature 9: Int J Cancer, 2014, 135, 2206-2214
Non Patent Literature 10: J Membrane Biol, 2004, 199, 29-38
Non Patent Literature 11: 14th Annu Meet Cancer Immunother (CIMT) (May 10-12, Mainz) 2016, Abst 185

SUMMARY OF INVENTION

Problems to be resolved by the Invention

The present invention provides a novel antibody-pyrrolobenzodiazepine (PBD) derivative conjugate and a novel pyrrolobenzodiazepine (PBD) derivative.
In addition, the present invention provides a pharmaceutical composition containing the antibody-PBD derivative conjugate with antitumor activity.
Further, the present invention provides a method for treating cancer by using the antibody-PBD derivative conjugate.

Means of solving the Problems

The present inventors diligently examined to find that a novel antibody-pyrrolobenzodiazepine (PBD) derivative conjugate has strong antitumor activity, and that the absolute steric configuration of the hydroxy group at the 11'-position of the pyrrolobenzodiazepine derivative in the conjugate is S-configuration. The present invention was completed on the basis of the findings.
Specifically, the present invention relates to the following.
[1] An antibody-drug conjugate represented by the following formula:

[Formula 2]

$$Ab\left[(N297\ glycan)\left[L - D\right]_{m^1}\right]_2$$

wherein

$m^1$ represents an integer of 1 or 2;
D is any one selected from the following group:

[Formula 3]

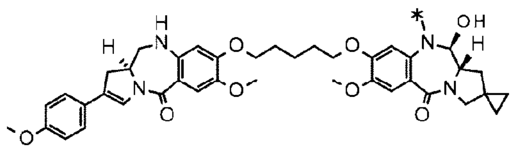

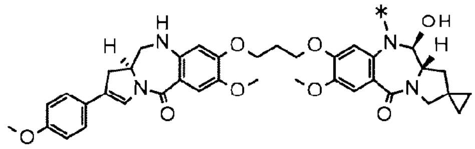

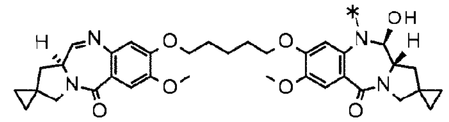

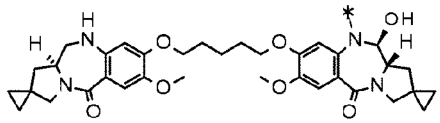

wherein

each asterisk * represents bonding to L;
L is a linker linking a glycan and D, the glycan bonding to Asn297 of Ab (N297 glycan);
the N297 glycan is optionally remodeled; and
Ab represents an antibody or a functional fragment of the antibody.

[2] The antibody-drug conjugate according to [1], wherein

L is represented by -Lb-La-Lp-NH-B-$CH_2$-O(C=O)-*, the asterisk * representing bonding to D;
B is a 1,4-phenyl group, a 2,5-pyridyl group, a 3,6-pyridyl group, a 2,5-pyrimidyl group, or a 2,5-thienyl group;
Lp represents any one selected from the following group:

- GGVA-, -GG-(D-)VA-, -VA-, -GGFG-, -GGPI-, -GGVCit-, -GGVK-, and - GGPL-;

La represents any one selected from the following group:

- C(=O)-$CH_2CH_2$-C(=O)-,-C(=O)-$(CH_2CH_2)_2$-C(=O)-,
- C(=O)-$CH_2CH_2$-C(=O)-NH-$(CH_2CH_2)_2$-C(=O)-,
- C(=O)-$CH_2CH_2$-C(=O)-NH-$(CH_2CH_2O)_2$-$CH_2$-C(=O)-,
- C(=O)-$CH_2CH_2$-NH-C(=O)-$(CH_2CH_2O)_4$-$CH_2CH_2$-C(=O)-, -$CH_2$-OC(=O)-, and -OC(=O)-; and

Lb is represented by the following formula:

[Formula 4]

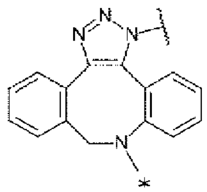

or

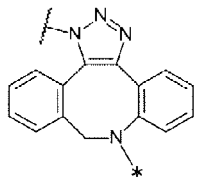

[Formula 5]

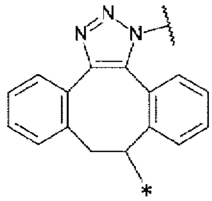

or

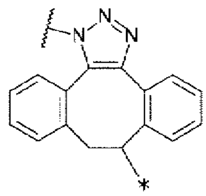

[Formula 6]

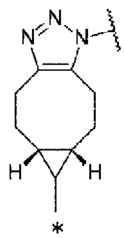

or

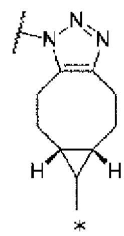

wherein, in each structural formula for Lb shown above,
each asterisk * represents bonding to La, and each wavy line represents bonding to N297 glycan or remodeled N297 glycan.

[3] The antibody-drug conjugate according to [1] or [2], wherein
L represents any one selected from the following group:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-GG-(D-)VA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGPI-NH-B-$CH_2$-OC(=O)-,
- $Z^1$-C(=O)-$CH_2CH_2$-C(=O)-GGFG-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-GGVK-NH-B-$CH_2$-OC(=O)-,
- $Z^1$-C(=O)-$CH_2CH_2$-C(=O)-GGPL-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)-,
- $Z^1$-C(=O)-$CH_2CH_2$-NH-C(=O)-$(CH_2CH_2O)_4$-$CH_2CH_2$-C(=O)-VA-NH-B-$CH_2$-OC(=O)-,
- $Z^2$-OC(=O)-GGVA-NH-B-$CH_2$-OC(=O)-, and
- $Z^3$-$CH_2$-OC(=O)-GGVA-NH-B-$CH_2$-OC(=O)-, wherein

B represents a 1,4-phenyl group,
$Z^1$ represents the following structural formula:

[Formula 7]

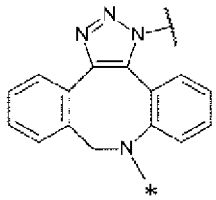

or

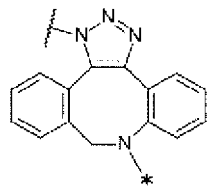

$Z^2$ represents the following structural formula:

[Formula 8]

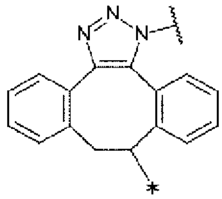

or

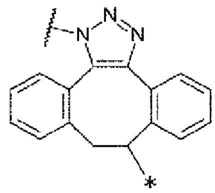

$Z^3$ represents the following structural formula:

[Formula 9]

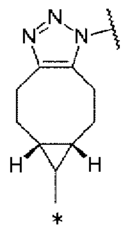

or

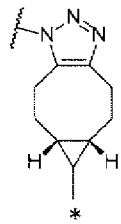

wherein, in each structural formula for $Z^1$, $Z^2$, and $Z^3$,
each asterisk * represents bonding to neighboring C(=O), OC(=O), or $CH_2$, and each wavy line represents bonding to N297 glycan or remodeled N297 glycan.

[4] The antibody-drug conjugate according to [3], wherein
L represents any one selected from the following group:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)-, and
- $Z^1$-C(=O)-$CH_2CH_2$-NH-C(=O)-$(CH_2CH_2O)_4$-$CH_2CH_2$-C(=O)-VA-NH-B-$CH_2$-OC(=O)-, wherein

B is a 1,4-phenyl group,
$Z^1$ represents the following structural formula:

[Formula 10]

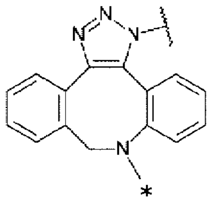

or

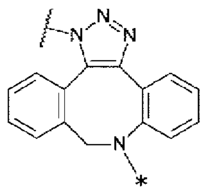

wherein, in the structural formula for $Z^1$, each asterisk * represents bonding to C(=O) neighboring to $Z^1$, and each wavy line represents bonding to N297 glycan or remodeled N297 glycan.

[5] The antibody-drug conjugate according to any one of [1] to [4], wherein the N297 glycan is a remodeled glycan.

[6] The antibody-drug conjugate according to any one of [1] to [5], wherein the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 11]

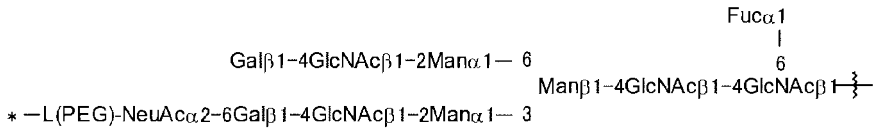

[N297-(Fuc)MSG1]

[Formula 12]

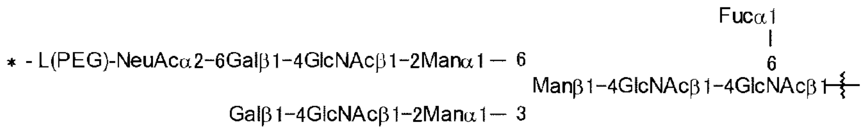

[N297-(Fuc)MSG2]

[Formula 13]

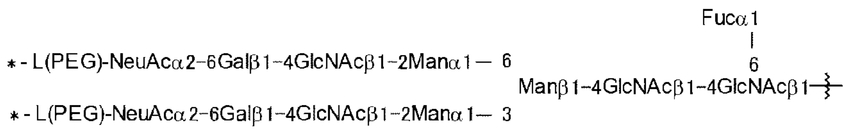

[N297-(Fuc)SG]

wherein

each wavy line represents bonding to Asn297 of the antibody,
L(PEG) in the N297 glycan represents *-$(CH_2CH_2\text{-}O)n^5\text{-}CH_2CH_2\text{-}NH$-, wherein $n^5$ represents an integer of 2 to 5, the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring of $Z^1$ in L.

[7] The antibody-drug conjugate according to [6], wherein $n^5$ is 3.

[8] An antibody drug conjugate represented by the following formula:

[Formula 14]

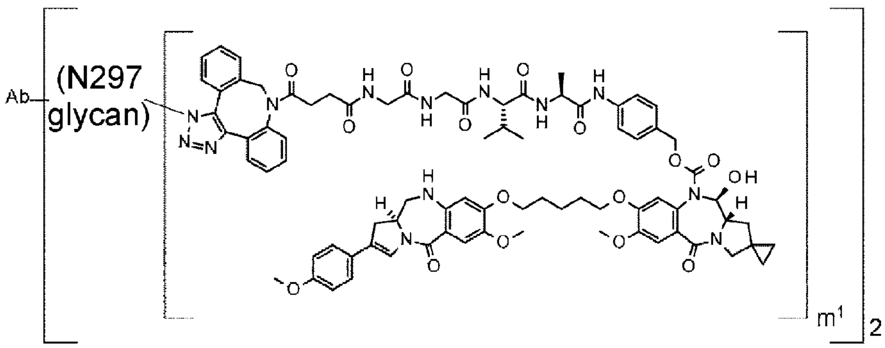

or

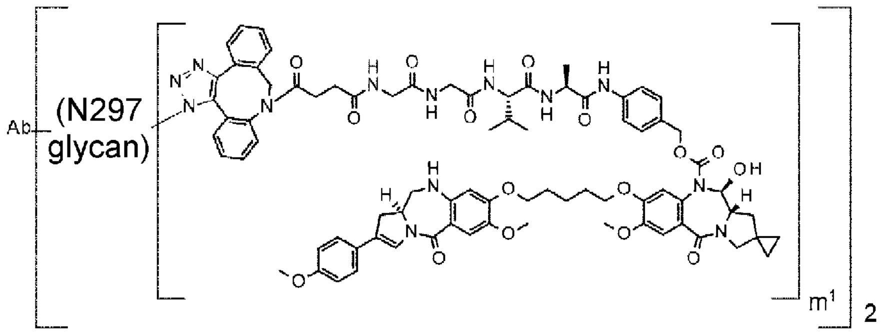

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 15]

Fucα1
|
Galβ1-4GlcNAcβ1-2Manα1— 6 6
Manβ1-4GlcNAcβ1-4GlcNAcβ1-
* —L(PEG)-NeuAcα2-6Galβ1-4GlcNAcβ1-2Manα1— 3

[N297-(Fuc)MSG1]

[Formula 16]

Fucα1
|
* - L(PEG)-NeuAcα2-6Galβ1-4GlcNAcβ1-2Manα1— 6 6
Manβ1-4GlcNAcβ1-4GlcNAcβ1-
Galβ1-4GlcNAcβ1-2Manα1— 3

[N297-(Fuc)MSG2]

[Formula 17]

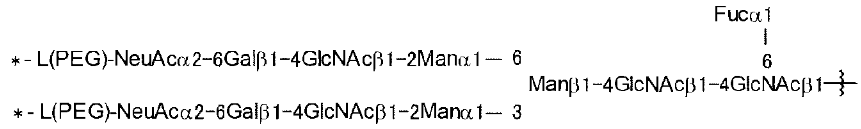

[N297-(Fuc)SG]

wherein

each wavy line represents bonding to Asn297 of the antibody,
L(PEG) in the N297 glycan represents *-$(CH_2CH_2$-$O)_3$-$CH_2CH_2$-NH-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

[9] An antibody drug conjugate represented by the following formula:

[Formula 18]

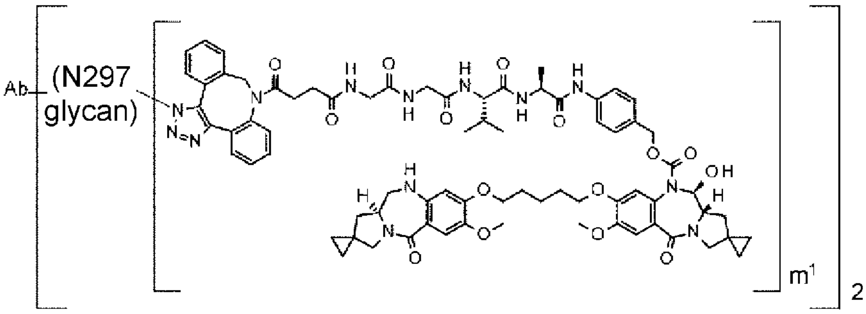

or

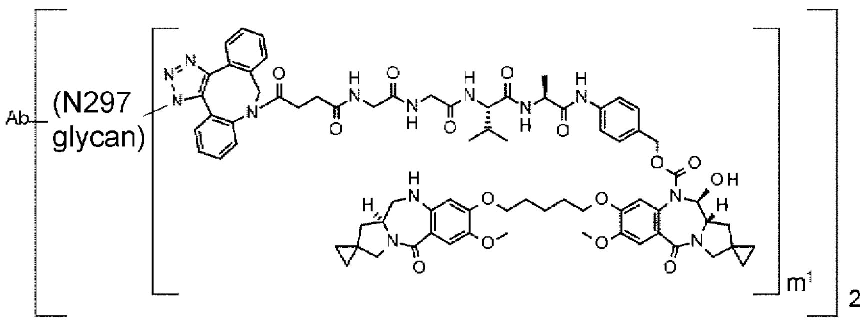

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 19]

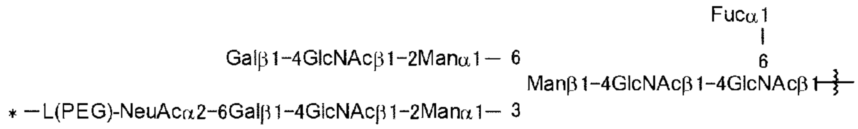

[N297-(Fuc)MSG1]

[Formula 20]

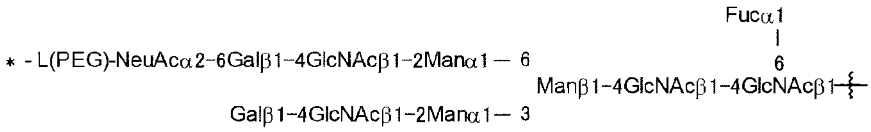

[N297-(Fuc)MSG2]

[Formula 21]

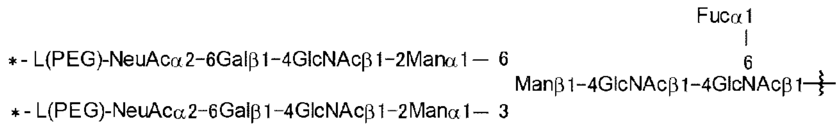

[N297-(Fuc)SG]

wherein

each wavy line represents bonding to Asn297 of the antibody,
L(PEG) in the N297 glycan represents *-$(CH_2CH_2\text{-}O)_3$-$CH_2CH_2$-NH-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

[10] An antibody drug conjugate represented by the following formula:

[Formula 22]

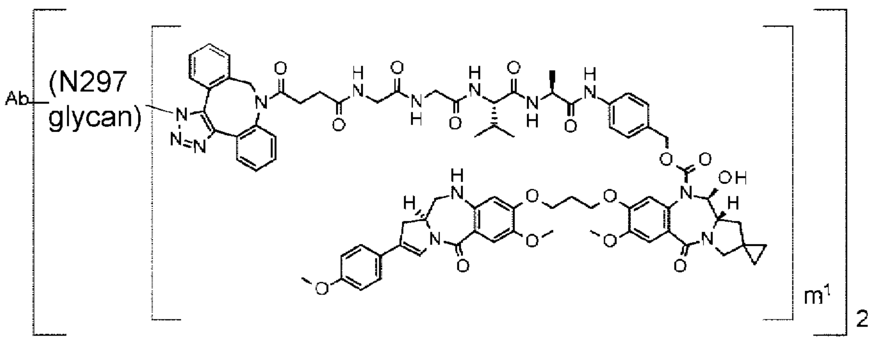

or

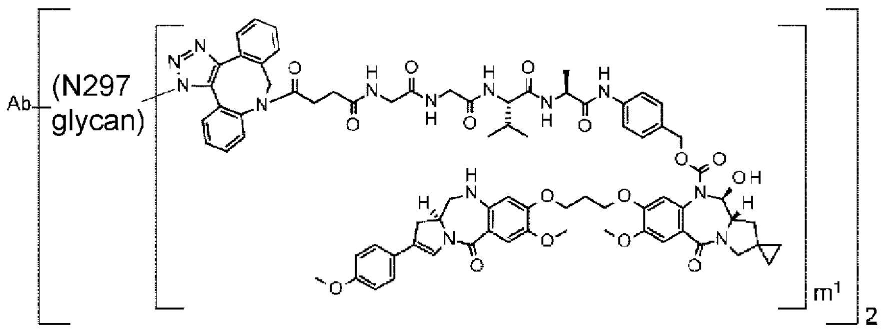

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 23]

Fucα1
|
6
Galβ1-4GlcNAcβ1-2Manα1— 6
Manβ1-4GlcNAcβ1-4GlcNAcβ1—
* —L(PEG)-NeuAcα2-6Galβ1-4GlcNAcβ1-2Manα1— 3

[N297-(Fuc)MSG1]

[Formula 24]

Fucα1
|
6
* - L(PEG)-NeuAcα2-6Galβ1-4GlcNAcβ1-2Manα1 — 6
Manβ1-4GlcNAcβ1-4GlcNAcβ1—
Galβ1-4GlcNAcβ1-2Manα1— 3

[N297-(Fuc)MSG2]

[Formula 25]

Fucα1
|
6
* - L(PEG)-NeuAcα2-6Galβ1-4GlcNAcβ1-2Manα1 — 6
Manβ1-4GlcNAcβ1-4GlcNAcβ1—
* - L(PEG)-NeuAcα2-6Galβ1-4GlcNAcβ1-2Manα1— 3

[N297-(Fuc)SG]

wherein

each wavy line represents bonding to Asn297 of the antibody,
L(PEG) in the N297 glycan represents *-$(CH_2CH_2\text{-}O)_3\text{-}CH_2CH_2\text{-}NH\text{-}$,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

[11] An antibody drug conjugate represented by the following formula:

[Formula 26]

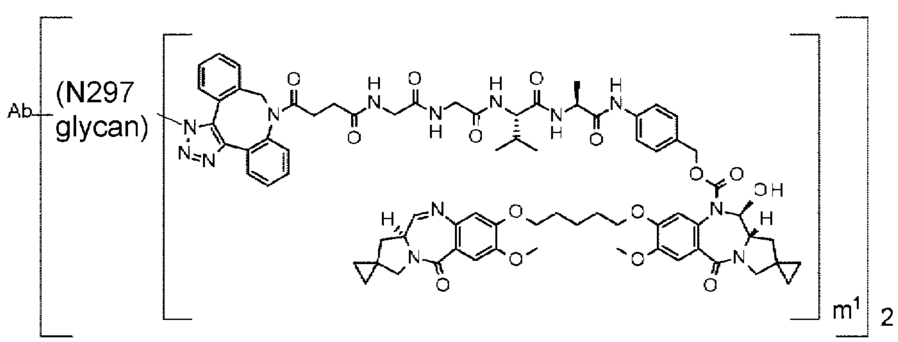

or

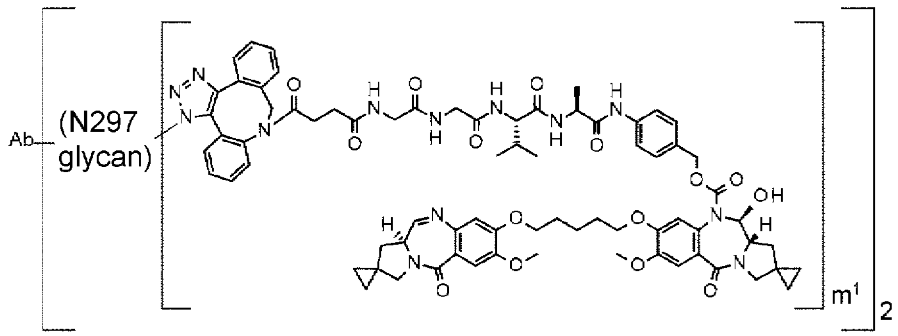

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 27]

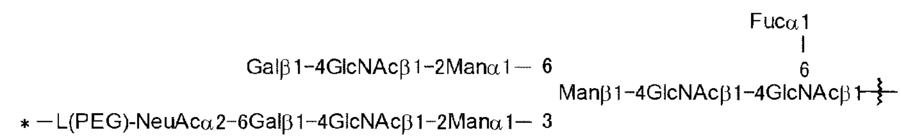

[N297-(Fuc)MSG1]

[Formula 28]

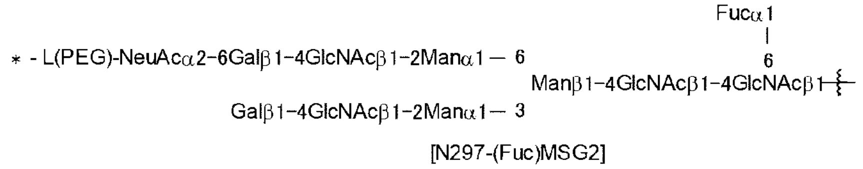

[N297-(Fuc)MSG2]

[Formula 29]

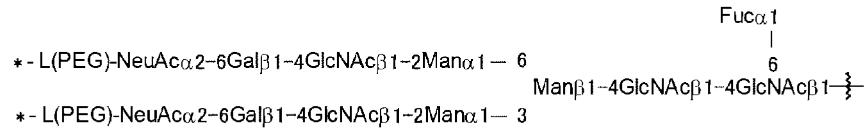

[N297-(Fuc)SG]

wherein

each wavy line represents bonding to Asn297 of the antibody,
L(PEG) in the N297 glycan represents *-$(CH_2CH_2\text{-}O)_3$-$CH_2CH_2$-NH-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the formula.

[12] The antibody-drug conjugate according to any one of [1] to [11], comprising an antibody having internalization activity.
[13] The antibody-drug conjugate according to any one of [1] to [12], wherein the antibody is an antibody having a characteristic of binding to an antigen expressed on a tumor cell and being incorporated and internalizing in the tumor cell.
[14] The antibody-drug conjugate according to any one of [1] to [13], wherein the antibody has antitumor effect.
[15] The antibody-drug conjugate according to any one of [1] to [14], wherein the antibody is an anti-CLDN6 antibody, an anti-CLDN9 antibody, an anti-CLDN6/CLDN9 antibody, an anti-HER2 antibody, an anti-HER3 antibody, an anti-DLL3 antibody, an anti-FAP antibody, an anti-CDH11 antibody, an anti-A33 antibody, an anti-CanAg antibody, an anti-CD 19 antibody, an anti-CD20 antibody, an anti-CD22 antibody, an anti-CD25 antibody, an anti-CD30 antibody, an anti-CD33 antibody, an anti-CD37 antibody, an anti-CD56 antibody, an anti-CD70 antibody, an anti-CD98 antibody, an anti-B7-H3 antibody, an anti-TROP2 antibody, an anti-CEA antibody, an anti-Cripto antibody, an anti-EphA2 antibody, an anti-FGFR2 antibody, an anti-G250 antibody, an anti-MUC1 antibody, an anti-GPNMB antibody, an anti-Integrin antibody, an anti-PSMA antibody, an anti-Tenascin-C antibody, an anti-SLC44A4 antibody, an anti-Mesothelin antibody, an anti-EGFR antibody, an anti-5T4 antibody, an anti-LRRC15 antibody, an anti-DR5 antibody, an anti-CDH3 antibody, an anti-PDPN antibody, or an anti-CD123 antibody.
[16] The antibody-drug conjugate according to any one of [1] to [15], wherein the antibody specifically binds to CLDN6 and/or CLDN9.
[17] The antibody-drug conjugate according to [16], the antibody comprising a heavy chain comprising CDRH1, CDRH2, and CDRH3 and a light chain comprising CDRL1, CDRL2, and CDRL3 as described in any one of the following (a) and (b):

(a) CDRH1 consisting of an amino acid sequence represented by SEQ ID NO: 9, CDRH2 consisting of an amino acid sequence represented by SEQ ID NO: 10, and CDRH3 consisting of an amino acid sequence represented by SEQ ID NO: 11, and CDRL1 consisting of an amino acid sequence represented by SEQ ID NO: 5, CDRL2 consisting of an amino acid sequence represented by SEQ ID NO: 6, and CDRL3 consisting of an amino acid sequence represented by SEQ ID NO: 7 or an amino acid sequence having one or two amino acid substitutions in the amino acid sequence represented by SEQ ID NO: 7; and
(b) CDRH1 consisting of an amino acid sequence represented by SEQ ID NO: 15, CDRH2 consisting of an amino acid sequence represented by SEQ ID NO: 16, and CDRH3 consisting of an amino acid sequence represented by SEQ ID NO: 17, and CDRL1 consisting of an amino acid sequence represented by SEQ ID NO: 12, CDRL2 consisting of an amino acid sequence represented by SEQ ID NO: 13, and CDRL3 consisting of an amino acid sequence represented by SEQ ID NO: 14.

[18] A antibody-drug conjugate according to [17], the antibody comprising a heavy chain comprising CDRH1, CDRH2, and CDRH3 and a light chain comprising CDRL1, CDRL2, and CDRL3 as described in any one of the following (a) and (b):

(a) CDRH1 consisting of an amino acid sequence represented by SEQ ID NO: 9, CDRH2 consisting of an amino acid sequence represented by SEQ ID NO: 10, and CDRH3 consisting of an amino acid sequence represented by SEQ ID NO: 11, and CDRL1 consisting of an amino acid sequence represented by SEQ ID NO: 5, CDRL2 consisting

of an amino acid sequence represented by SEQ ID NO: 6, and CDRL3 consisting of an amino acid sequence represented by SEQ ID NO: 7 or an amino acid sequence represented by SEQ ID NO: 8; and
(b) CDRH1 consisting of an amino acid sequence represented by SEQ ID NO: 15, CDRH2 consisting of an amino acid sequence represented by SEQ ID NO: 16, and CDRH3 consisting of an amino acid sequence represented by SEQ ID NO: 17, and CDRL1 consisting of an amino acid sequence represented by SEQ ID NO: 12, CDRL2 consisting of an amino acid sequence represented by SEQ ID NO: 13, and CDRL3 consisting of an amino acid sequence represented by SEQ ID NO: 14.

[19] The antibody-drug conjugate according to any one of [16] to [18], the antibody comprising a heavy chain variable region and a light chain variable region as described in any one of the following (a) and (b):

(a) a heavy chain variable region consisting of an amino acid sequence represented by SEQ ID NO: 21 and a light chain variable region consisting of an amino acid sequence represented by SEQ ID NO: 19; and
(b) a heavy chain variable region consisting of an amino acid sequence represented by SEQ ID NO: 25 and a light chain variable region consisting of an amino acid sequence represented by SEQ ID NO: 23.

[20] The antibody-drug conjugate according to any one of [16] to [19], comprising a heavy chain variable region consisting of an amino acid sequence selected from the group consisting of the following (a) to (e) and a light chain variable region consisting of an amino acid sequence selected from the group consisting of the following (f) to (k):

(a) an amino acid sequence represented by SEQ ID NO: 54;
(b) an amino acid sequence represented by SEQ ID NO: 58;
(c) an amino acid sequence represented by SEQ ID NO: 62;
(d) an amino acid sequence with a homology of at least 95% or higher to a sequence of a framework region excluding CDR sequences in any of the sequences (a) to (c);
(e) an amino acid sequence having one to several amino acid deletions, substitutions, or additions in a sequence of a framework region excluding CDR sequences in any of the sequences (a) to (c);
(f) an amino acid sequence represented by SEQ ID NO: 38;
(g) an amino acid sequence represented by SEQ ID NO: 42;
(h) an amino acid sequence represented by SEQ ID NO: 46;
(i) an amino acid sequence represented by SEQ ID NO: 50;
(j) an amino acid sequence with a homology of at least 95% or higher to a sequence of a framework region excluding CDR sequences in any of the sequences (f) to (i); and
(k) an amino acid sequence having one to several amino acid deletions, substitutions, or additions in a sequence of a framework region excluding CDR sequences in any of the sequences (f) to (i).

[21] The antibody-drug conjugate according to [20], the antibody comprising a heavy chain variable region and a light chain variable region selected from the group consisting of the following (a) to (e):

(a) a heavy chain variable region consisting of an amino acid sequence represented by SEQ ID NO: 54 and a light chain variable region consisting of an amino acid sequence represented by SEQ ID NO: 38;
(b) a heavy chain variable region consisting of an amino acid sequence represented by SEQ ID NO: 58 and a light chain variable region consisting of an amino acid sequence represented by SEQ ID NO: 42;
(c) a heavy chain variable region consisting of an amino acid sequence represented by SEQ ID NO: 54 and a light chain variable region consisting of an amino acid sequence represented by SEQ ID NO: 46;
(d) a heavy chain variable region consisting of an amino acid sequence represented by SEQ ID NO: 58 and a light chain variable region consisting of a light chain variable region consisting of an amino acid sequence represented by SEQ ID NO: 50; and
(e) a heavy chain variable region consisting of an amino acid sequence represented by SEQ ID NO: 62 and a light chain variable region consisting of an amino acid sequence represented by SEQ ID NO: 46.

[22] The antibody-drug conjugate according to any one of [16] to [21], wherein the antibody is a chimeric antibody.
[23] The antibody-drug conjugate according to any one of [16] to [21], wherein the antibody is a humanized antibody.
[24] The antibody-drug conjugate to any one of [16] to [23], wherein the antibody comprises a heavy chain constant region of human IgG1, human IgG2, or human IgG4.
[25] The antibody-drug conjugate according to [23] or [24], comprising a heavy chain and a light chain selected from the group consisting of the following (a) to (e):

(a) a heavy chain consisting of an amino acid sequence consisting of amino acid residues 20 to 471 of SEQ ID NO: 52 and a light chain consisting of an amino acid sequence consisting of amino acid residues 21 to 234 of SEQ ID NO: 36;
(b) a heavy chain consisting of an amino acid sequence consisting of amino acid residues 20 to 471 of SEQ ID NO: 56 and a light chain consisting of an amino acid sequence consisting of amino acid residues 21 to 234 of SEQ ID NO: 40;
(c) a heavy chain consisting of an amino acid sequence consisting of amino acid residues 20 to 471 of SEQ ID NO: 52 and a light chain consisting of an amino acid sequence consisting of amino acid residues 21 to 234 of SEQ ID NO: 44;
(d) a heavy chain consisting of an amino acid sequence consisting of amino acid residues 20 to 471 of SEQ ID NO: 56 and a light chain consisting of an amino acid sequence consisting of amino acid residues 21 to 234 of SEQ ID NO: 48; and
(e) a heavy chain consisting of an amino acid sequence consisting of amino acid residues 20 to 471 of SEQ ID NO: 60 and a light chain consisting of an amino acid sequence consisting of amino acid residues 21 to 234 of SEQ ID NO: 44.

[26] The antibody-drug conjugate according to [16], wherein the antibody competes with the antibody according to any one of [17] to [21] and [25] for binding to CLDN6 and/or CLDN9, or binds to a site of CLDN6 and/or CLDN9 recognizable to the antibody according to any one of [17] to [21] and [25].
[27] The antibody-drug conjugate according to any one of [1] to [15], wherein the antibody specifically binds to HER2.
[28] The antibody-drug conjugate according to any one of [27], having activities or activity of antibody-dependent cellular cytotoxicity (ADCC) and/or complement-dependent cytotoxicity (CDC).
[29] The antibody-drug conjugate according to [27], wherein the heavy chain constant region of the antibody is a heavy chain constant region of human IgG1, and comprises a mutation that causes lowering of activities or activity of ADCC and/or CDC.
[30] The antibody-drug conjugate according to [29], wherein the heavy chain constant region of the antibody is a heavy chain constant region of human IgG1, and leucine at the 234- and 235-positions specified by EU Index numbering in the heavy chain constant region is substituted with alanine.
[31] The antibody-drug conjugate according to [27] or [28], being a humanized monoclonal antibody comprising a heavy chain consisting of an amino acid sequence represented by SEQ ID NO: 65 and a light chain consisting of an amino acid sequence represented by SEQ ID NO: 64.
[32] The antibody-drug conjugate according to any one of [27], [29], and [30], being an antibody comprising a heavy chain variable region consisting of an amino acid sequence consisting of amino acid residues 20 to 139 of SEQ ID NO: 75 and a light chain variable region consisting of an amino acid sequence consisting of amino acid residues 21 to 127 of SEQ ID NO: 73.
[33] The antibody-drug conjugate according to any one of [27], [29], [30], and [32], being a humanized monoclonal antibody comprising a heavy chain consisting of an amino acid sequence consisting of amino acid residues 20 to 469 of SEQ ID NO: 75 and a light chain consisting of an amino acid sequence consisting of amino acid residues 21 to 234 of SEQ ID NO: 73.
[34] The antibody-drug conjugate according to any one of [16] to [33], wherein the antibody comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.
[35] The antibody-drug conjugate according to [34], wherein one or several amino acid residues are deleted at the carboxyl terminus of a heavy chain of the antibody.
[36] The antibody-drug conjugate according to [34] or [35], wherein one amino acid residue is deleted at the carboxyl terminus of each of the two heavy chains of the antibody.
[37] The antibody-drug conjugate according to any one of [34] to [36], wherein a proline residue at the carboxyl terminus of a heavy chain is further amidated.
[38] The antibody-drug conjugate according to any one of [1] to [37], wherein the antibody is a glycan-remodeled antibody obtained through the following steps of:

i) culturing a host cell containing a polynucleotide encoding the antibody according to any one of [12] to [37] and collecting a targeted antibody from the culture obtained;
ii) treating the antibody obtained in step i) with hydrolase to produce a (Fucα1,6)GlcNAc-antibody; and
iii) reacting the (Fucα1,6)GlcNAc-antibody with a glycan donor molecule in the presence of transglycosidase, the

glycan donor molecule obtained by introducing a PEG linker having an azide group to the carbonyl group of carboxylic acid at the 2-position of a sialic acid in MSG (9) or SG (10) and oxazolinating the reducing terminal.

[39] The antibody-drug conjugate according to [38], further comprising the step of purifying the (Fucα1,6)GlcNAc-antibody through purification of a reaction solution in step ii) with a hydroxyapatite column.
[40] A compound, a salt of the compound, or a hydrate of the compound or the salt, wherein the compound is any one represented by the following formula:

[Formula 30]

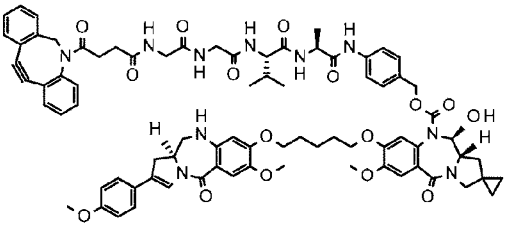
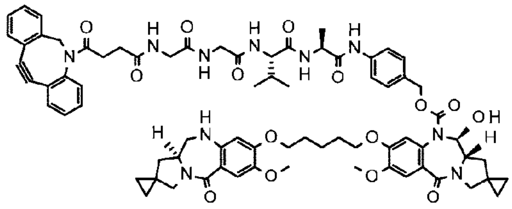
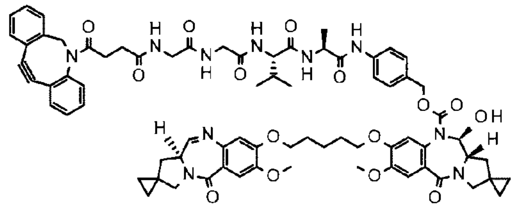
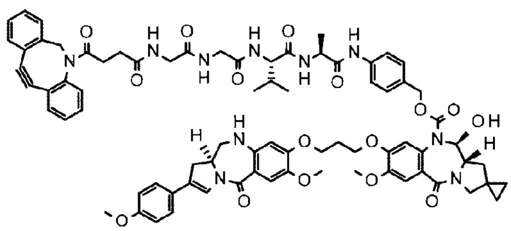

[41] A method for producing the antibody-drug conjugate according to any one of [1] to [39], the method comprising the step of reacting the glycan-remodeled antibody according to [38] or [39] and the compound according to [40], a salt of the compound, or a hydrate of the compound or the salt.
[42] An antibody-drug conjugate obtained by using the method according to [41].
[43] The antibody-drug conjugate according to any one of [1] to [39] and [42], wherein the N297 glycan is N297-(Fuc)MSG1.
[44] The antibody-drug conjugate according to any one of [1] to [39], [42], and [43], wherein $m^1$ is an integer of 1.
[45] The antibody-drug conjugate according to any one of [1] to [39] and [42] to [44], wherein the average number of conjugated drug molecules per antibody molecule in the antibody-drug conjugate is 1 to 3 or 3 to 5.
[46] A pharmaceutical composition comprising the antibody-drug conjugate according to any one of [1] to [39] and [42] to [45], a salt of the antibody-drug conjugate, or a hydrate of the antibody-drug conjugate or the salt.
[47] The pharmaceutical composition according to [46], being an antitumor drug.
[48] A method for treating a tumor, wherein the antibody-drug conjugate according to any one of [1] to [39] and [42] to [45], a salt of the antibody-drug conjugate, or a hydrate of the antibody-drug conjugate or the salt is administered to an individual.
[49] A pharmaceutical composition, wherein a pharmaceutical composition comprising the antibody-drug conjugate according to any one of [1] to [39] and [42] to
[45], a salt of the antibody-drug conjugate, or a hydrate of the antibody-drug conjugate or the salt, and at least one antitumor drug are administered to an individual simultaneously, separately, or consecutively.

Advantageous Effects of Invention

The novel antibody-pyrrolobenzodiazepine (PBD) derivative conjugate provided by the present invention is superior in antitumor activity and safety, and hence useful as an antitumor agent. The PBD derivative of the present invention has antitumor activity, and thus is useful as a drug for the conjugate. In addition, the antibody of the present invention recognizes an antigen expressed on tumor cells or binds to the antigen, and hence is useful as an antibody for the conjugate.

BRIEF DESCRIPTION OF DRAWINGS

[Figure 1] Figure 1 is a schematic diagram of the antibody-drug conjugate of the present invention (the molecule of (I)). (a) indicates drug D, (b) indicates linker L, (c) indicates $N_3$-L(PEG)-, and (d) indicates N297 glycan (open ellipse: NeuAc(Sia), open hexagon: Man, filled hexagon: GlcNAc, open diamond: Gal, open inverted triangle: Fuc). (b) and (c) are combined together to form a triazole ring by reaction between the azide group (filled teardrop shape) of (c) and the spacer (open semicircle) of (b). The Y-shaped diagram represents antibody Ab. For convenience, in this schematic diagram, N297 glycan is indicated as N297-(Fuc)MSG and the diagram shows an embodiment wherein any one of two branches in each of N297 glycans has a sialic acid to which a PEG linker having an azide group ($N_3$-L(PEG)-) bonds while the other branch has no sialic acid at the non-reducing terminal (i.e. N297-(Fuc)MSG); however, another embodiment wherein each of two branches of N297 glycan has a sialic acid to which a PEG linker having an azide group bonds at the non-reducing terminal (i.e. N297-(Fuc)SG) is also acceptable. Unless otherwise stated, such a manner of illustration is applied throughout the present specification.

[Figure 2] Figure 2 is schematic diagrams illustrating the structures of a (Fucα1,6)GlcNAc-antibody (the molecule of A in (II) of Figure 2), which is a production intermediate of the antibody-drug conjugate of the present invention, and an MSG-type glycan-remodeled antibody (the molecule of (III) in B of Figure 2). In each of the diagrams, the Y-shaped diagram represents antibody Ab as in Figure 1. In A in Figure 2, (e) indicates N297 glycan consisting only of GlcNAc at the 6-position connected to 1-positions of Fuc via an α glycosidic bond. In B in Figure 2, (d) indicates the same N297 glycan as in Figure 1, and (f) indicates a structure of a PEG linker portion having an azide group, specifically, an azide group to be bonded to liker L at the end. The bonding mode of the PEG linker having an azide group is as described for Figure 1.

[Figure 3] Figure 3 is a schematic diagram for the step of producing an MSG-type glycan-remodeled antibody from an antibody produced in an animal cell. As in Figure 2, molecules (II) and (III) in this Figure represent an (Fucα1,6)GlcNAc-antibody and an MSG-type glycan-remodeled antibody, respectively. Molecule (IV) is an antibody produced in an animal cell, and is a mixture of molecules with heterogeneous N297 glycan moieties. Figure 3A illustrates the step of producing homogeneous (Fucα1,6)GlcNAc-antibody (II) by treating heterogeneous N297 glycan moieties of (IV) with hydrolase such as EndoS. Figure 3B illustrates the step of producing the MSG-type glycan-remodeled antibody of (III) by subjecting GlcNAc of N297 glycan in antibody (II) to transglycosidase such as an EndoS D233Q/Q303L variant to transglycosylate the glycan of an MSG-type glycan donor molecule. The MSG-type glycan donor molecule used here has a sialic acid at the non-reducing terminal of MSG modified with a PEG linker having an azide group. Thus, resulting MSG-type N297 glycan-remodeled antibody also has a sialic acid at the non-reducing terminal modified in the same manner as described for Figure 2B. For convenience, Figure 3B shows MSG as a donor molecule. However, a glycan-remodeled antibody in which a linker molecule having an azide group bonds to each non-reducing terminal of N297 glycan also can be synthesized as the glycan-remodeled antibody of (III) by using SG (10) as a glycan donor.

[Figure 4] Figure 4 shows the effects of the anti-HER2 antibody-drug conjugates ADC2 and ADC1 on subcutaneously transplanted NCI-N87 cells, a human gastric cancer cell line.

[Figure 5] Figure 5 shows the effects of the anti-HER2 antibody-drug conjugate ADC7, trastuzumab, and the anti-LPS antibody-drug conjugate ADC13 on subcutaneously transplanted NCI-N87 cells, a human gastric cancer cell line.

[Figure 6] Figure 6 shows the effects of the anti-HER2 antibody-drug conjugate ADC7, the anti-LPS antibody-drug conjugate ADC13, and trastuzumab-tesirine (Reference Example 1) on subcutaneously transplanted KPL-4 cells, a human breast cancer cell line.

[Figure 7] Figure 7 shows the effects of the anti-HER2 antibody-drug conjugate ADC7 and trastuzumab-tesirine on subcutaneously transplanted JIMT-1 cells, a human breast cancer cell line.

[Figure 8] Figure 8 shows the effects of the anti-CLDN6 antibody-drug conjugate ADC8 and an anti-CLDN6 antibody(H1L1)-tesirine on subcutaneously transplanted OV-90 cells, a human ovarian cancer cell line.

[Figure 9] Figure 9 shows the effects of the anti-CLDN6 antibody-drug conjugate ADC8 and an anti-CLDN6 antibody(H1L1)-tesirine on subcutaneously transplanted NIH:OVCAR-3 cells, a human ovarian cancer cell line.

[Figure 10] Figure 10 shows the effects of the anti-TROP2 antibody-drug conjugate ADC11 and the anti-LPS antibody-drug conjugate ADC13 on subcutaneously transplanted FaDu cells, a human head-and-neck cancer cell line.

[Figure 11] Figure 11 shows the full-length amino acid sequence of human CLDN6 (SEQ ID NO: 1) and the nucleotide sequence of full-length cDNA for human CLDN6 (SEQ ID NO: 2).

[Figure 12] Figure 12 shows the full-length amino acid sequence of human CLDN9 (SEQ ID NO: 3) and the nucleotide sequence of full-length cDNA for human CLDN9 (SEQ ID NO: 4).

[Figure 13] Figure 13 shows the amino acid sequences of CDRL1 to 3 of a B1 antibody light chain (SEQ ID NOs: 5 to 7).

[Figure 14] Figure 14 shows the amino acid sequence of CDRL3 of the humanized B1 antibody light chain L4 (SEQ

ID NO: 8).

[Figure 15] Figure 15 shows the amino acid sequences of CDRH1 to 3 of a B1 antibody heavy chain (SEQ ID NOs: 9 to 11).

[Figure 16] Figure 16 shows the amino acid sequences of CDRL1 to 3 of a C7 antibody light chain (SEQ ID NOs: 12 to 14).

[Figure 17] Figure 17 shows the amino acid sequences of CDRH1 to 3 of a C7 antibody heavy chain (SEQ ID NOs: 15 to 17).

[Figure 18] Figure 18 shows the nucleotide sequence of cDNA encoding the variable region of a B1 antibody light chain (SEQ ID NO: 18) and the amino acid sequence of the variable region of a B1 antibody light chain (SEQ ID NO: 19). Each underline in the amino acid sequence indicates a CDR sequence.

[Figure 19] Figure 19 shows the nucleotide sequence of cDNA encoding the variable region of a B1 antibody heavy chain (SEQ ID NO: 20) and the amino acid sequence of the variable region of a B1 antibody heavy chain (SEQ ID NO: 21). Each underline in the amino acid sequence indicates a CDR sequence.

[Figure 20] Figure 20 shows the nucleotide sequence of cDNA encoding the variable region of a C7 antibody light chain (SEQ ID NO: 22) and the amino acid sequence of the variable region of a C7 antibody light chain (SEQ ID NO: 23). Each underline in the amino acid sequence indicates a CDR sequence.

[Figure 21] Figure 21 shows the nucleotide sequence of cDNA encoding the variable region of a C7 antibody heavy chain (SEQ ID NO: 24) and the amino acid sequence of the variable region of a C7 antibody heavy chain (SEQ ID NO: 25). Each underline in the amino acid sequence indicates a CDR sequence.

[Figure 22] Figure 22 shows the amino acid sequence of a chB1 light chain (SEQ ID NO: 28) and a DNA fragment including a DNA sequence encoding the amino acid sequence of a chB1 light chain (SEQ ID NO: 29). Each underline in the amino acid sequence indicates a CDR sequence.

[Figure 23] Figure 23 shows the amino acid sequence of the variable region of a chB1 light chain (SEQ ID NO: 30) and the nucleotide sequence encoding a chB1 light chain variable region (SEQ ID NO: 31). Each underline in the amino acid sequence indicates a CDR sequence.

[Figure 24] Figure 24 shows the amino acid sequence of a chB1 heavy chain (SEQ ID NO: 32) and the nucleotide sequence encoding a chB1 heavy chain (SEQ ID NO: 33). Each underline in the amino acid sequence indicates a CDR sequence.

[Figure 25] Figure 25 shows the amino acid sequence of the variable region of a chB1 heavy chain (SEQ ID NO: 34) and the nucleotide sequence encoding a variable region of a chB1 heavy chain (SEQ ID NO: 35). Each underline in the amino acid sequence indicates a CDR sequence.

[Figure 26] Figure 26 shows the amino acid sequence of the humanized antibody light chain hL1 (SEQ ID NO: 36) and the nucleotide sequence encoding the humanized antibody light chain hL1 (SEQ ID NO: 37). Each underline in the amino acid sequence indicates a CDR sequence.

[Figure 27] Figure 27 shows the amino acid sequence of the variable region of the humanized antibody light chain hL1 (SEQ ID NO: 38) and the nucleotide sequence encoding the variable region of the humanized antibody light chain hL1 (SEQ ID NO: 39). Each underline in the amino acid sequence indicates a CDR sequence.

[Figure 28] Figure 28 shows the amino acid sequence of the humanized antibody light chain hL2 (SEQ ID NO: 40) and the nucleotide sequence encoding the humanized antibody light chain hL2 (SEQ ID NO: 41). Each underline in the amino acid sequence indicates a CDR sequence.

[Figure 29] Figure 29 shows the amino acid sequence of the variable region of the humanized antibody light chain hL2 (SEQ ID NO: 42) and the nucleotide sequence encoding the variable region of the humanized antibody light chain hL2 (SEQ ID NO: 43).

[Figure 30] Figure 30 shows the amino acid sequence of the humanized antibody light chain hL3 (SEQ ID NO: 44) and the nucleotide sequence encoding the humanized antibody light chain hL3 (SEQ ID NO: 45). Each underline in the amino acid sequence indicates a CDR sequence.

[Figure 31] Figure 31 shows the amino acid sequence of the variable region of the humanized antibody light chain hL3 (SEQ ID NO: 46) and the nucleotide sequence encoding the variable region of the humanized antibody light chain hL3 (SEQ ID NO: 47). Each underline in the amino acid sequence indicates a CDR sequence.

[Figure 32] Figure 32 shows the amino acid sequence of the humanized antibody light chain hL4 (SEQ ID NO: 48) and the nucleotide sequence encoding the humanized antibody light chain hL4 (SEQ ID NO: 49). Each underline in the amino acid sequence indicates a CDR sequence.

[Figure 33] Figure 33 shows the amino acid sequence of the variable region of the humanized antibody light chain hL4 (SEQ ID NO: 50) and the nucleotide sequence encoding the variable region of the humanized antibody light chain hL4 (SEQ ID NO: 51). Each underline in the amino acid sequence indicates a CDR sequence.

[Figure 34] Figure 34 shows the amino acid sequence of the humanized antibody heavy chain hH1 (SEQ ID NO: 52) and the nucleotide sequence encoding the humanized antibody heavy chain hH1 (SEQ ID NO: 53). Each underline in the amino acid sequence indicates a CDR sequence.

[Figure 35] Figure 35 shows the amino acid sequence of the variable region of the humanized antibody heavy chain hH1 (SEQ ID NO: 54) and the nucleotide sequence encoding the variable region of the humanized antibody heavy chain hH1 (SEQ ID NO: 55). Each underline in the amino acid sequence indicates a CDR sequence.

[Figure 36] Figure 36 shows the amino acid sequence of the humanized antibody heavy chain hH2 (SEQ ID NO: 56) and the nucleotide sequence encoding the humanized antibody heavy chain hH2 (SEQ ID NO: 57). Each underline in the amino acid sequence indicates a CDR sequence.

[Figure 37] Figure 37 shows the amino acid sequence of the variable region of the humanized antibody heavy chain hH2 (SEQ ID NO: 58) and the nucleotide sequence encoding the variable region of the humanized antibody heavy chain hH2 (SEQ ID NO: 59).

[Figure 38] Figure 38 shows the amino acid sequence of the humanized antibody heavy chain hH3 (SEQ ID NO: 60) and the nucleotide sequence encoding the humanized antibody heavy chain hH3 (SEQ ID NO: 61). Each underline in the amino acid sequence indicates a CDR sequence.

[Figure 39] Figure 39 shows the amino acid sequence of the variable region of the humanized antibody heavy chain hH3 (SEQ ID NO: 62) and the nucleotide sequence encoding the variable region of the humanized antibody heavy chain hH3 (SEQ ID NO: 63). Each underline in the amino acid sequence indicates a CDR sequence.

[Figure 40] Figure 40 shows the binding abilities of a B 1 antibody and a C7 antibody to human CLDN6 and the family molecules CLDN3, CLDN4, and CLDN9 measured by flow cytometry.

[Figure 41] Figure 41 shows the antibody internalization activities of a B1 antibody and C7 antibody measured by Mab-ZAP.

[Figure 42] Figure 42 shows the binding abilities of the humanized anti-CLDN6 antibodies H1L1, H2L2, H1L3, H2L4, and H3L3 to CLDN6 and the family molecules measured by flow cytometry.

[Figure 43] Figure 43 shows the amino acid sequence of the trastuzumab light chain (SEQ ID NO: 64) and the amino acid sequence of the trastuzumab heavy chain (SEQ ID NO: 65).

[Figure 44] Figure 44 shows the amino acid sequence of a light chain of a trastuzumab variant (SEQ ID NO: 73) and the amino acid sequence of a heavy chain of a trastuzumab variant (SEQ ID NO: 75).

[Figure 45] Figure 45 shows comparison of the amino acid sequences of chB1_H, which is a heavy chain of the chimerized human anti-CLDN6 antibody chB1, and the humanized antibody heavy chains hH1, hH2, and hH3. The symbol "." indicates an amino acid residue identical to the corresponding amino acid residue of chB 1_H, and each position with a symbol of an amino acid residue indicates a substituted amino acid residue.

[Figure 46] Figure 46 shows comparison of the amino acid sequences of chB 1_L, which is a light chain of the chimerized human anti-CLDN6 antibody chB1, and the humanized antibody light chains hL1, hL2, hL3, and hL4. The symbol "." indicates an amino acid residue identical to the corresponding amino acid residue of chB1_L, and each position with symbol of an amino acid residue indicates a substituted amino acid residue.

[Figure 47] Figure 47 shows the effects of the anti-HER2 antibody-drug conjugates ADC7 and ADC14 on subcutaneously transplanted KPL-4 cells, a human breast cancer cell line.

[Figure 48] Figure 48 shows the effect of the anti-HER2 antibody-drug conjugate ADC14 on subcutaneously transplanted JIMT-1 cells, a human breast cancer cell line.

[Figure 49] Figure 49 shows the effects of the anti-HER2 antibody-drug conjugates ADC7 and ADC14, and the anti-LPS antibody-drug conjugate ADC13 on subcutaneously transplanted CFPAC-1 cells, a human pancreatic cancer cell line.

## Description of Embodiments

### [Antibody-drug conjugate]

The antibody-drug conjugate of the present invention is an antitumor drug having an antitumor compound conjugated via a linker structure moiety to an antibody capable of recognizing an antigen expressed on tumor cells or binding to the antigen.

The antibody-drug conjugate of the present invention is represented by the following formula:

[Formula 31]

$$Ab\left[(N297\ glycan)\left[L-D\right]_{m^1}\right]_2$$

wherein

$m^1$ is an integer of 1 or 2 (preferably, 1), D represents a drug, L represents a linker linking the N297 glycan and D, Ab represents an antibody or a functional fragment of the antibody, and the N297 glycan represents a glycan bonding to the side chain of Asn297 of the antibody. The N297 glycan may be a remodeled glycan.

<Drug>

Drug D of the present invention is preferably an antitumor compound. The antitumor compound develops antitumor effect, when a part or the entire of the linker of the antibody-drug conjugate of the present invention is cleaved in a tumor cell and the antitumor compound moiety is released.

The drug in the antibody-drug conjugate of the present invention, namely, the PBD derivative is, for example, any one selected from the following group:

[Formula 32]

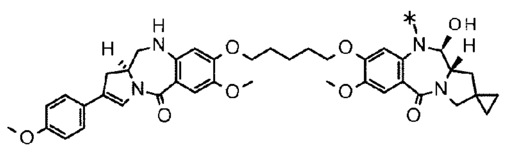

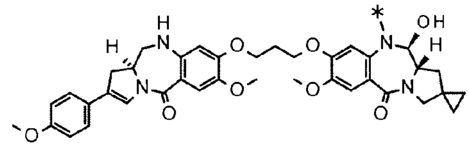

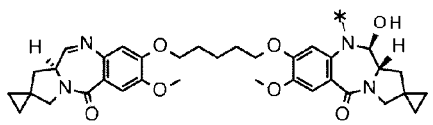

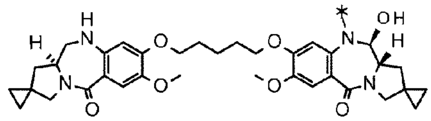

wherein the asterisk * represents bonding to L.

The absolute steric configuration of the hydroxy group at the 11'-position of the PBD derivative of the present invention is S-configuration.

<Linker structure>

Linker L of the present invention is a linker linking the N297 glycan and D.

Linker L is represented by the following formula:

$$-Lb-La-Lp-NH-B-CH_2-O(C=O)-*$$

The asterisk * represents bonding to the nitrogen atom at the N10'-position of drug D, Lb represents a spacer which connects La to a N297 glycan or remodeled N297 glycan.

B represents a phenyl group or a heteroaryl group, and is preferably a 1,4-phenyl group, a 2,5-pyridyl group, a 3,6-pyridyl group, a 2,5-pyrimidyl group, or a 2,5-thienyl group, and more preferably a 1,4-phenyl group.

Lp represents a linker consisting of an amino acid sequence cleavable in vivo or in a target cell. Lp is, for example, cleaved by the action of an enzyme such as esterase and peptidase.

Lp is a peptide residue composed of two to seven (preferably, two to four) amino acids. That is, Lp is composed of an oligopeptide residue in which two to seven amino acids are connected via peptide bonding.

Lp is bound at the N terminal to a carbonyl group of La in Lb-La-, and forms at the C terminal an amide bond with the amino group (-NH-) of the part -NH-B-$CH_2$-O(C=O)- of the linker. The bond between the C terminal of Lp and -NH- is cleaved by the enzyme such as esterase.

The amino acids constituting Lp are not limited to particular amino acids, and, for example, are L- or D-amino acids, and preferably L-amino acids. The amino acids may be not only $\alpha$-amino acids, but may include an amino acid with structure, for example, of $\beta$-alanine, $\varepsilon$-aminocaproic acid, or $\gamma$-aminobutyric acid, and may further include a non-natural amino acid such as an N-methylated amino acid.

The amino acid sequence of Lp is not limited to a particular amino acid sequence, and examples of amino acids that constitute Lp may include, but are not limited to, glycine (Gly; G), valine (Val; V), alanine (Ala; A), phenylalanine (Phe; F), glutamic acid (Glu; E), isoleucine (Ile; I), proline (Pro; P), citrulline (Cit), leucine (Leu; L), serine (Ser; S), lysine (Lys; K), and aspartic acid (Asp; D). Preferred among them are glycine (Gly; G), valine (Val; V), alanine (Ala; A), and citrulline (Cit).

Any of these amino acids may appear multiple times, and Lp has an amino acid sequence including arbitrarily selected amino acids. Drug release pattern may be controlled via amino acid type.

Specific examples of linker Lp may include, but are not limited to, -GGVA-, - GG-(D-)VA-, -VA-, -GGFG-, -GGPI-, -GGVCit-, -GGVK-, -GG(D-)PI-, -GGPL-, - EGGVA, -PI-, -GGF-, DGGF-, (D-)D-GGF-, -EGGF-, -SGGF-, -KGGF-, -DGGFG-, - GGFGG-, -DDGGFG-, -KDGGFG-, and -GGFGGGF-.

Here, "(D-)V" indicates D-valine, "(D)-P" indicates D-proline, and "(D-)D" indicates D-aspartic acid.

Linker Lp is preferably any of the following:
-GGVA-, -GG-(D-)VA-, -VA-, -GGFG-, -GGPI-, -GGVCit-, -GGVK-, -GG(D-)PI-, and -GGPL-.

Linker Lp is more preferably any of the following:
-GGVA-, -GGVCit-, and -VA-.

La represents any one selected from the following group:

- C(=O)-$(CH_2CH_2)n^2$-C(=O)-, -C(=O)-$(CH_2CH_2)n^2$-C(=O)-NH-$(CH_2CH_2)n^3$-C(=O)-, -C(=O)-$(CH_2CH_2)n^2$-C(=O)-NH-$(CH_2CH_2O)n^3$-$CH_2$-C(=O)-,
- C(=O)-$(CH_2CH_2)n^2$-NH-C(=O)-$(CH_2CH_2O)n^3$-$CH_2CH_2$-C(=O)-, and-$(CH_2)n^4$-O-C(=O)-

wherein,
$n^2$ represents an integer of 1 to 3 (preferably, 1 or 2), $n^3$ represents an integer of 1 to 5 (preferably, an integer of 2 to 4, more preferably, 2 or 4), and $n^4$ represents an integer of 0 to 2 (preferably, 0 or 1).

La preferably represents any one selected from the following group:

- C(=O)-$CH_2CH_2$-C(=O)-,-C(=O)-$(CH_2CH_2)_2$-C(=O)-, -C(=O)-$CH_2CH_2$-C(=O)-NH-$(CH_2CH_2)_2$-C(=O)--C(=O)-$CH_2CH_2$-C(=O)-NH-$(CH_2CH_2O)_2$-$CH_2$-C(=O)-, -C(=O)-$CH_2CH_2$-NH-C(=O)-$(CH_2CH_2O)_4$-$CH_2CH_2$-C(=O)-,
- $CH_2$-OC(=O)-, and -OC(=O)-, and

La is more preferably -C(=O)-$CH_2CH_2$-C(=O)- or -C(=O)-$(CH_2CH_2)_2$-C(=O)-.

Spacer Lb is not limited to a particular spacer, and examples thereof may include, but are not limited to, a spacer represented by the following formulas.

[Formula 33]

(Lb-1) 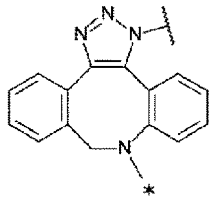 or 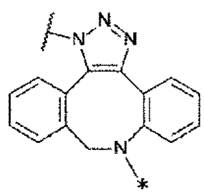

[Formula 34]

(Lb-2) 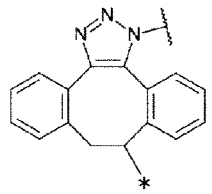 or 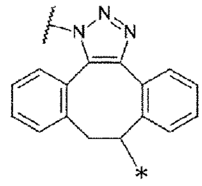

[Formula 35]

(Lb-3) 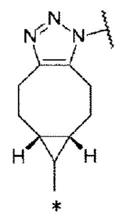 or 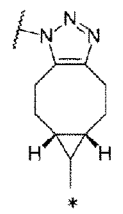

In the structural formulas for Lb shown above, each asterisk * represents bonding to -(C=O) or -$(CH_2)n^4$ at the left end of La, and each wavy line represents bonding to a N297 glycan or remodeled N297 glycan of Ab.

In each structural formula for Lb (Lb-1, Lb-2, or Lb-3) shown above, the triazole ring site formed through click reaction of an azide group and DBCO provides structures of geometric isomers, and one Lb exists as any one of the two structures or as a mixture of both of them. There exist two or four ($m^1$ is 1 or 2) "-L-D" moieties per molecule of the antibody-drug conjugate of the present invention, and either one of the two structures exists or both of them coexist as Lb (Lb-1, Lb-2, or Lb-3) in L of each of the two or four "-L-D" moieties.

L is preferably represented by -Lb-La-Lp-NH-B-$CH_2$-O(C=O)-*, wherein

B is a 1,4-phenyl group,
Lp represents any one selected from the following group:
-GGVA-, -GG-(D-)VA-, -VA-, -GGFG-, -GGPI-, -GGVCit-, -GGVK-, -GG(D-)PI-, and -GGPL-,
La represents any one selected from the following group:

- C(=O)-$CH_2CH_2$-C(=O)-,-C(=O)-$(CH_2CH_2)_2$-C(=O)-, -C(=O)-$CH_2CH_2$-C(=O)-NH-$(CH_2CH_2)_2$-C(=O)-, -C(=O)-$CH_2CH_2$-C(=O)-NH-$(CH_2CH_2O)_2$-$CH_2$-C(=O)-, -C(=O)-$CH_2CH_2$-NH-C(=O)-$(CH_2CH_2O)_4$-$CH_2CH_2$-C(=O)-, -$CH_2$-OC(=O)-, and -OC(=O)-,

and
Lb represents any of the structural formulas above for Lb.

L is more preferably any one selected from the following group:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-GG-(D-)VA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGPI-NH-B-$CH_2$-OC(=O)-,
- $Z^1$-C(=O)-$CH_2CH_2$-C(=O)-GGFG-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-GGVK-NH-B-$CH_2$-OC(=O)-,
- $Z^1$-C(=O)-$CH_2CH_2$-C(=O)-GGPL-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)-,
- $Z^1$-C(=O)-$CH_2CH_2$-NH-C(=O)-$(CH_2CH_2O)_4$-$CH_2CH_2$-C(=O)-VA-NH-B-$CH_2$-OC(=O)-,

- $Z^2$-OC(=O)-GGVA-NH-B-$CH_2$-OC(=O)-, and -$Z^3$-$CH_2$-OC(=O)-GGVA-NH-B-$CH_2$-OC(=O)-

wherein
$Z^1$ represents the following structural formula as described for Lb:

[Formula 36]

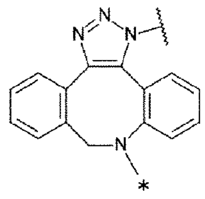

or

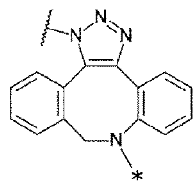

$Z^2$ represents the following structural formula as described for Lb:

[Formula 37]

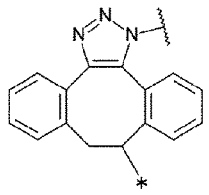

or

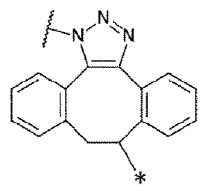

$Z^3$ represents the following structural formula as described for Lb:

[Formula 38]

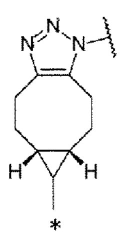

or

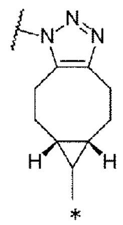

B is a 1,4-phenyl group.
L is most preferably any of the following:

- $Z^1$-C(=O)-$CH_2CH_2$-C(=O)-GGVA-NH-B-$CH_2$-OC(=O)-,
- $Z^1$-C(=O)-$CH_2CH_2$-C(=O)-VA-NH-B-$CH_2$-OC(=O)-,
- $Z^1$-C(=O)-$(CH_2CH_2)_2$-C(=O)-VA-NH-B-$CH_2$-OC(=O)-,
- $Z^1$-C(=O)-$CH_2CH_2$-C(=O)-GGVCit-NH-B-$CH_2$-OC(=O)-,
- $Z^1$-C(=O)-$CH_2CH_2$-C(=O)-NH-$(CH_2CH_2)_2$-C(=O)-VA-NH-B-$CH_2$-OC(=O)-,
- $Z^1$-C(=O)-$CH_2CH_2$-C(=O)-NH-$(CH_2CH_2O)_2$-$CH_2$-C(=O)-VA-NH-B-$CH_2$-OC(=O)-, and -$Z^1$-C(=O)-$CH_2CH_2$-NH-C(=O)-$(CH_2CH_2O)_4$-$CH_2CH_2$-C(=O)-VA-NH-B-$CH_2$-OC(=O)-, wherein

B is a 1,4-phenyl group, and $Z^1$ represents the following structural formula as described for Lb:

[Formula 39]

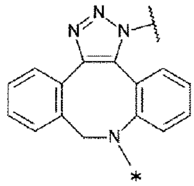

or

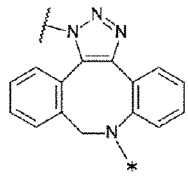

<Free drug>

The free drug of the antibody-drug conjugate of the present invention is one selected from the following group:

[Formula 40]

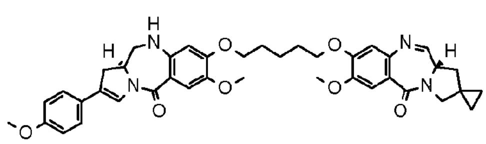

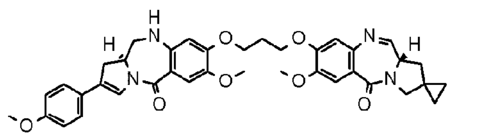

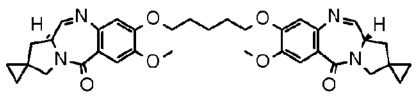

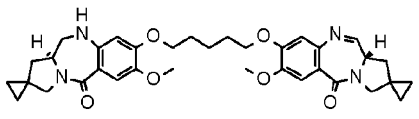

The free drug of the present invention is generated through a process in which the antibody-drug conjugate of the present invention migrates into tumor cells and the portion of linker L is then cleaved. This free drug was found to have anti-tumor cell effect.

<Antibody>

In the present invention, "cancer" and "tumor" are used for the same meaning.

In the present invention, a "gene" refers to nucleotides or a nucleotide sequence including a nucleotide sequence encoding amino acids of protein or a complementary strand thereof. The meaning of a "gene" encompasses, for example, a polynucleotide, an oligonucleotide, DNA, mRNA, cDNA, and RNA as a nucleotide sequence including a nucleotide sequence encoding amino acids of protein or a complementary strand thereof. Examples of the "CLDN6 gene" of the present invention may include, but are not limited to, DNA, mRNA, cDNA, and cRNA including a nucleotide sequence encoding the amino acid sequence of CLDN6 protein.

In the present invention, "nucleotides", "polynucleotide", and "nucleotide sequence" have the same meaning as that of "nucleic acids", and the meaning of "nucleotides" and "nucleotide sequence" encompasses, for example, DNA, RNA, a probe, an oligonucleotide, a polynucleotide, and a primer.

In the present invention, "polypeptide", "peptide", and "protein" are used interchangeably.

In the present invention, "CLDN6" is used for the same meaning as CLDN6 protein.

In the present invention, "cells" include cells in an animal individual and cultured cells.

In the present invention, "cellular cytotoxic activity" refers to causing pathological change to cells in any way, which includes causing, not only direct traumas, but also all types of damage in the structure and function of cells such as cleavage of DNA, formation of a nucleotide dimer, cleavage of a chromosome, damage of the mitotic apparatus, and lowered activity of various enzymes.

In the present invention, a "functional fragment of an antibody" is also referred to as an "antigen-binding fragment of an antibody", and means a partial fragment of an antibody with binding activity to an antigen, and examples thereof may include, but are not limited to, Fab, F(ab')2, Fv, scFv, diabodies, linear antibodies, and multispecific antibodies formed from antibody fragments. In addition, the meaning of an antigen-binding fragment of an antibody encompasses Fab', a monovalent fragment of a variable region of an antibody obtained by treating F(ab')2 under reducing conditions. However, there is no limitation to those molecules as long as the molecules have binding ability to an antigen. Those antigen-binding fragments include not only those obtained by treating a full-length molecule of an antibody protein with an appropriate enzyme, but also protein produced in an appropriate host cell by using a genetically engineered antibody

gene.

The functional fragment of the present invention includes a functional fragment that has well conserved asparagine (Asn297) to be modified with an N-linked glycan in the IgG heavy chain Fc region and amino acids around Asn297, while retains binding activity to an antigen.

In the present invention, an "epitope" refers to a partial peptide or partial three-dimensional structure of an antigen to which a particular antibody (e.g., an anti-CLDN6 antibody) binds (a partial peptide or partial three-dimensional structure of CLDN6). An epitope as such a partial peptide (e.g., a partial peptide of CLDN6) can be determined by using any method well known to those skilled in the art, such as immunoassay.

A "CDR" in the present invention refers to a complementarity determining region. It is known that each of heavy chains and light chains of an antibody molecule have three CDRs. CDRs, which are also called a hypervariable region, are located in variable regions of heavy chains and light chains of an antibody and is a site with particularly high variation of the primary structure. Three CDRs are separately located in the primary structure of the polypeptide chain of each of heavy chains and light chains. Regarding CDRs of antibodies, herein, CDRs of a heavy chain refer to CDRH1, CDRH2, and CDRH3 from the amino terminus of the heavy chain amino acid sequence, and CDRs of a light chain refer to CDRL1, CDRL2, and CDRL3 from the amino terminus of the light chain amino acid sequence. These sites are located in the proximity of each other in the three-dimensional structure, determining specificity to an antibody to bind.

In the present invention, "hybridize under stringent conditions" refers to hybridization in the commercially available hybridization solution ExpressHyb Hybridization Solution (Clontech) at 68°C, or hybridization using a filter with DNA fixed thereto in the presence of 0.7 to 1.0 M NaCl at 68°C and washing at 68°C with 0.1 to 2 × SSC solution (1 × SSC solution contains 150 mM NaCl and 15 mM sodium citrate), or hybridization under conditions equivalent thereto.

In the present invention, "one to several" refers to 1 to 10, one to nine, one to eight, one to seven, one to six, one to five, one to four, one to three, or one or two.

In the present invention, an antibody capable of recognizing or binding to CLDN6 and that capable of recognizing or binding to CLDN6 and CLDN9 are occasionally called as an "anti-CLDN6 antibody" and an "anti-CLDN6/CLDN9 antibody", respectively. Such antibodies include chimeric antibodies, humanized antibodies, and human antibodies. An antibody capable of recognizing or binding to CLDN6 and CLDN9 is occasionally called as an "anti-CLDN6 antibody".

The antibody to be used for the antibody-drug conjugate of the present invention refers to immunoglobulin, and is a molecule including an antigen-binding site which immunospecifically binds to an antigen. The antibody of the present invention may be of any class of IgG, IgE, IgM, IgD, IgA, and IgY, and preferred is IgG. The subclass may be any of IgG1, IgG2, IgG3, IgG4, IgA1, and IgA2, and preferred are IgG1, IgG2, and IgG4. If IgG1 or IgG4 is used, the effector function may be adjusted by substituting some of amino acid residues in the constant region (see WO 88/07089, WO 94/28027, WO 94/29351).

If IgG1 is used as the isotype of the antibody of the present invention, the effector function may be adjusted by substituting some amino acid residues in the constant region. Examples of variants of IgG1 with the effector function lowered or attenuated may include, but are not limited to, IgG1 LALA (IgG1-L234A,L235A) and IgG1 LAGA (IgG1-L235A,G237A), and a preferred variant of IgG1 is IgG1 LALA. The L234A, L235A indicates substitution of leucine with alanine at the 234- and 235-positions specified by EU-index numbering (Proc. Natl. Acad. Sci. U.S.A., Vol. 63, No. 1 (May 15, 1969), pp. 78-85), and the G237A indicates substitution of glycine with alanine at the 237-position specified by EU-index numbering.

The antibody of the present invention may be derived from any species, which preferably include, but are not limited to, a human, a rat, a mouse, and a rabbit. If the antibody is derived from species other than human species, it is preferably chimerized or humanized using a well known technique. The antibody of the present invention may be a polyclonal antibody or a monoclonal antibody, and is preferably a monoclonal antibody. Examples of monoclonal antibodies may include, but are not limited to, monoclonal antibodies derived from non-human animals such as rat antibodies, mouse antibodies, and rabbit antibodies; chimeric antibodies; humanized antibodies; human antibodies; functional fragments of them; and modified variants of them.

The antibody of the present invention is preferably an antibody capable of targeting a tumor cell, specifically, an antibody that binds to an antigen expressed on surfaces of tumor cells.

Since the compound conjugated in the antibody-drug conjugate of the present invention exerts an antitumor effect, it is preferred but not essential that the antibody itself should have an antitumor effect. For the purpose of specifically and selectively exerting the cytotoxicity of the antitumor compound against tumor cells, it is important and preferred that the antibody should have the property of internalizing to migrate into tumor cells. When the antibody internalizes, the antibody-drug conjugate can also migrate into cells. For antitumor effect, it is important and preferred that the antibody or antibody-drug conjugate should have the property of internalizing to migrate into tumor cells, from the viewpoint that the drug specifically and selectively damages tumor cells. The antitumor activity of the antibody refers to the cellular cytotoxic activity or anticellular effect against tumor cells. The antitumor activity may be confirmed by using any known in vitro or in vivo evaluation system. The internalization ability of the antibody can be measured by using a known evaluation system.

Examples of such an antibody may include, but are not limited to, antibodies to tumor-related antigens, including an anti-CLDN6 antibody, an anti-CLDN9 antibody, an anti-CLDN6/CLDN9 antibody, an anti-HER2 antibody, an anti-HER3 antibody, an anti-DLL3 (Delta like protein 3) antibody, an anti-A33 antibody, an anti-CanAg antibody, an anti-CD 19 antibody, an anti-CD20 antibody, an anti-CD22 antibody, an anti-CD25 antibody, an anti-CD30 antibody, an anti-CD33 antibody, an anti-CD37 antibody, an anti-CD56 antibody, an anti-CD70 antibody, an anti-CD98 antibody, an anti-B7-H3 (CD276) antibody, an anti-TROP2 antibody, an anti-CEA antibody, an anti-Cripto antibody, an anti-EphA2 antibody, an anti-FGFR2 antibody (e.g., WO 201315206), an anti-G250 antibody, an anti-MUCI antibody (e.g., WO 2011012309), an anti-GPNMB antibody, an anti-integrin antibody, an anti-PSMA antibody, an anti-tenascin-C antibody, an anti-SLC44A4 antibody, an anti-mesothelin antibody, an anti-EGFR antibody, an anti-5T4 (oncofetal antigen 5T4; also TPBG and trophoblast glycoprotein) antibody, an anti-LRRC15 (Leucine-rich repeat-containing protein 15) antibody, an anti-DR5 antibody, an anti-CDH3 (cadherin 3) antibody, an anti-PDPN (podoplanin) antibody, or an anti-CD123 antibody.

The antibody of the present invention is preferably an anti-CLDN6 antibody, an anti-CLDN6/CLDN9 antibody, an anti-HER2 antibody, an anti-CD98 antibody, or an anti-TROP2 antibody, and more preferably an anti-CLDN6 antibody or an anti-HER2 antibody (e.g., trastuzumab, a trastuzumab variant).

Now, the anti-CLDN6 antibody used in the present invention will be described.

1. CLDN6 and CLDN9

CLDN6, a four-transmembrane protein belonging to the claudin family and consisting of 220 amino acids, has the N terminus and C terminus in a cell.

The amino acid sequence of and DNA sequence for human CLDN6 are published in public databases, and can be referred to, for example, from accession numbers of NP_067018 (SEQ ID NO: 1) and NM_021195 (SEQ ID NO: 2 (both in NCBI).

In the amino acid sequence of human CLDN6 protein (hereinafter, referred to as "CLDN6 amino acid sequence"), the extracellular region is composed of an extracellular domain (EC1) consisting of amino acid residues 29 to 81 of SEQ ID NO: 1 in Sequence Listing and an extracellular domain (EC2) consisting of amino acid residues 138 to 160 of SEQ ID NO: 1 in Sequence Listing.

CLDN9, a four-transmembrane protein belonging to the claudin family and consisting of 217 amino acids, has the N terminus and C terminus in a cell. CLDN9 is highly homologous to CLDN6.

The amino acid sequence of and DNA sequence for human CLDN9 are published in public databases, and can be referred to, for example, from accession numbers of NP_066192 (SEQ ID NO: 3) and NM_020982 (SEQ ID NO: 4 (both in NCBI).

2. Anti-CLDN6 antibody

An example of the anti-CLDN6 antibody of the present invention is an anti-CLDN6 antibody that recognizes a higher order structure including two extracellular regions, specifically, an amino acid sequence of the 29- to 81-positions and amino acid sequence of the 138- to 160-positions from the N terminus of CLDN6 as represented by SEQ ID NO: 1 in Sequence Listing, and has internalization activity.

The anti-CLDN6 antibody of the present invention is an antibody capable of targeting tumor cells, and specifically has a property of recognizing a tumor cell, a property of binding to a tumor cell, a property of being incorporated and internalizing in a tumor cell, and so on. Accordingly, the anti-CLDN6 antibody according to the present invention can be used for an antibody-drug conjugate by conjugating via a linker with a compound having antitumor activity.

The anti-CLDN6 antibody of the present invention may have antitumor activity.

(1) The anti-CLDN6 antibody of the present invention has the following properties (a) and (b).

(a) Recognizing or binding to the CLDN family.

The antibody of the present invention recognizes the CLDN family. In other words, the antibody of the present invention binds to the CLDN family. The antibody of the present invention preferably binds to CLDN6, and more preferably specifically binds to CLDN6. Further, the antibody of the present invention may recognize CLDN9 or bind to CLDN9.

In the present invention, "specific recognition", that is, "specific binding" refers to binding being not nonspecific adsorption. Examples of determination criteria on whether binding is specific or not may include, but are not limited to, dissociation constants (hereinafter, referred to as "KD"). A preferred KD value of the

antibody of the present invention to CLDN6 and/or CLDN9 is $1 \times 10^{-5}$ M or less, $5 \times 10^{-6}$ M or less, $2 \times 10^{-6}$ M or less, or $1 \times 10^{-6}$ M or less, and more preferably $5 \times 10^{-7}$ M or less, $2 \times 10^{-7}$ M or less, or $1 \times 10^{-7}$ M or less.
Binding between an antigen and an antibody in the present invention may be measured or determined by an analysis method such as an ELISA method, an RIA method, and surface plasmon resonance (hereinafter, referred to as "SPR"). Binding between an antigen expressed on a cell surface and an antibody may be measured, for example, by a flow cytometry method.

(b) Having activity to internalize in CLDN6- and/or CLDN9-expressing cells through binding to CLDN6 and/or CLDN9.

(2) The antibody according to (1), wherein CLDN6 and/or CLDN9 are/is human CLDN6 and/or human CLDN9.

For example, the anti-CLDN6 monoclonal antibody of the present invention can be obtained with a method using a hybridoma. Examples of a monoclonal anti-CLDN6 antibody may include, but are not limited to, the mouse anti-CLDN6 antibodies B1 and C7. In the present invention, the "B1" and the "C7" are occasionally called as the "B1 antibody" and the "C7 antibody", respectively.

The nucleotide sequence for and the amino acid sequence of the heavy chain variable region of the B1 antibody are respectively represented by SEQ ID NO: 20 and SEQ ID NO: 21 in Sequence Listing. The nucleotide sequence for and the amino acid sequence of the light chain variable region of the B1 antibody are respectively represented by SEQ ID NO: 18 and SEQ ID NO: 19 in Sequence Listing.

The amino acid sequences of CDRH1, CDRH2, CDRH3, CDRL1, CDRL2, and CDRL3 of the B1 antibody are represented by SEQ ID NO: 9, SEQ ID NO: 10, SEQ ID NO: 11, SEQ ID NO: 5, SEQ ID NO: 6, and SEQ ID NO: 7, respectively.

The nucleotide sequence for and the amino acid sequence of the heavy chain variable region of the C7 antibody are respectively represented by SEQ ID NO: 24 and SEQ ID NO: 25 in Sequence Listing. The nucleotide sequence for and the amino acid sequence of the light chain variable region of the C7 antibody are respectively represented by SEQ ID NO: 22 and SEQ ID NO: 23 in Sequence Listing.

The amino acid sequences of CDRH1, CDRH2, CDRH3, CDRL1, CDRL2, and CDRL3 of the C7 antibody are represented by SEQ ID NO: 15, SEQ ID NO: 16, SEQ ID NO: 17, SEQ ID NO: 12, SEQ ID NO: 13, and SEQ ID NO: 14, respectively.

An example of the anti-CLDN6 antibody of the present invention is an antibody that binds to an epitope for the B1 antibody or C7 antibody. If the antibody binds to a partial peptide or partial three-dimensional structure to which the B1 antibody or C7 antibody binds, it can be determined that the antibody binds to an epitope for the B1 antibody or C7 antibody. By confirming that the antibody competes with the B1 antibody or C7 antibody for binding to CLDN6 (i.e., the antibody interferes with binding between the B1 antibody or C7 antibody and CLDN6), it can be determined, even when the specific sequence or structure of an epitope has not been determined, that the antibody binds to an epitope for the anti-CLDN6 antibody. If epitope identity has been confirmed, the antibody is strongly expected to have antigen-binding ability, biological activity, and/or internalization activity equivalent to that of the B1 antibody or C7 antibody.

The antibody of the present invention includes, in addition to the monoclonal antibody against CLDN6, a gene recombinant antibody obtained by artificial modification for the purpose of decreasing heterologous antigenicity to humans such as a chimeric antibody, a humanized antibody, and a human antibody. These antibodies can be produced using a known method.

(1) Chimeric antibody

Examples of the chimeric antibody may include, but are not limited to, an antibody in which antibody variable and constant regions are derived from different species, for example, a chimeric antibody in which a mouse- or rat-derived antibody variable region is connected to a human-derived antibody constant region.

A chimeric antibody derived from the mouse anti-human CLDN6 antibody B1 antibody, as an example of the chimeric antibody of the present invention, is an antibody comprising a heavy chain comprising a heavy chain variable region consisting of an amino acid sequence represented by SEQ ID NO: 21 and a light chain comprising a light chain variable region represented by SEQ ID NO: 19, which may comprise any human-derived constant region.

Specific examples of the chimeric antibody derived from the mouse anti-human CLDN6 antibody B 1 antibody may include, but are not limited to, the chimeric antibody chB1 antibody (hereinafter, also called as "chB1") derived from the mouse anti-human CLDN6 antibody B1 antibody. Examples of the chB1 antibody, in terms of the amino acid sequence, may include, but are not limited to, an antibody comprising a heavy chain having an amino acid sequence consisting of amino acid residues 20 to 471 of SEQ ID NO: 32 in Sequence Listing and a light chain having an amino acid sequence

consisting of amino acid residues 21 to 234 of SEQ ID NO: 28 in Sequence Listing.

In the heavy chain sequence represented by SEQ ID NO: 32 in Sequence Listing, the amino acid sequence consisting of amino acid residues 1 to 19 is the signal sequence, the amino acid sequence consisting of amino acid residues 20 to 141 is the heavy chain variable region, and the amino acid sequence consisting of amino acid residues 142 to 471 is the heavy chain constant region. In the light chain sequence represented by SEQ ID NO: 28 in Sequence Listing, the amino acid sequence consisting of amino acid residues 1 to 20 is the signal sequence, the amino acid sequence consisting of amino acid residues 21 to 127 is the light chain variable region, and the amino acid sequence consisting of amino acid residues 128 to 234 is the light chain constant region.

The amino acid sequences of the heavy chain and light chain variable regions of the chB1 antibody are respectively represented by SEQ ID NO: 34 and SEQ ID NO: 30 in Sequence Listing.

The heavy chain amino acid sequence of the chB1 antibody is encoded by a nucleotide sequence represented by SEQ ID NO: 33 in Sequence Listing. A nucleotide sequence consisting of nucleotide residues 1 to 57 of a nucleotide sequence represented by SEQ ID NO: 33 in Sequence Listing is encoding the signal sequence of the chB1 antibody heavy chain, a nucleotide sequence consisting of nucleotide residues 58 to 423 of a nucleotide sequence represented by SEQ ID NO: 33 in Sequence Listing is encoding the heavy chain variable region of the chB1 antibody, and a nucleotide sequence consisting of nucleotide residues 424 to 1413 of a nucleotide sequence represented by SEQ ID NO: 33 in Sequence Listing is encoding the heavy chain constant region of the chB1 antibody.

The nucleotide sequence for the heavy chain variable region of the chB1 antibody is represented by SEQ ID NO: 35 in Sequence Listing.

The light chain amino acid sequence of the chB1 antibody is encoded by a nucleotide sequence represented by SEQ ID NO: 29 in Sequence Listing. A nucleotide sequence consisting of nucleotide residues 26 to 85 of a nucleotide sequence represented by SEQ ID NO: 29 in Sequence Listing is encoding the signal sequence of the chB1 antibody light chain, a nucleotide sequence consisting of nucleotide residues 86 to 406 of a nucleotide sequence represented by SEQ ID NO: 29 in Sequence Listing is encoding the light chain variable region of the chB1 antibody, and a nucleotide sequence consisting of nucleotide residues 407 to 727 of a nucleotide sequence represented by SEQ ID NO: 29 in Sequence Listing is encoding the light chain constant region of the chB1 antibody.

The nucleotide sequence for the light chain variable region of the chB1 antibody is represented by SEQ ID NO: 31 in Sequence Listing.

(2) Humanized antibody

Examples of the humanized antibody may include, but are not limited to, an antibody obtained by incorporating only the complementarity determining regions (CDRs) into a human-derived antibody (see Nature (1986) 321, p. 522-525), an antibody obtained by grafting a part of the amino acid residues of a framework as well as the CDR sequences to a human antibody by a CDR-grafting method (WO 90/07861), and an antibody in which a part of the CDR amino acid sequences has been modified with the binding ability to an antigen maintained (WO2012/075581, WO2011/084496, US2018/0501692). The amino acid sequences of CDRs can be determined according to a known method such as the Kabat definition, the Chothia definition, the Abm definition, and IMGT; however, CDRs in the present invention may be those defined according to any method.

If the humanized antibody is derived from the B1 antibody or C1 antibody, however, the humanized antibody may be any humanized antibody, without limited to a particular humanized antibody, that retains all the six CDR sequences of the B1 antibody or C1 antibody and has CLDN6-binding activity, and in addition the humanized antibody may be any humanized antibody, without limited to a particular humanized antibody, such that its humanized antibody variant in which one to several (preferably, one or two, more preferably, one) CDR amino acid sequences have been modified also recognizes CLDN6 protein, or has the CLDN6 protein-binding activity of the original antibody.

Examples of the humanized anti-CLDN6 antibody of the present invention or a functional fragment thereof may include, but are not limited to, an antibody comprising a heavy chain having a variable region comprising:

CDRH1 consisting of an amino acid sequence represented by SEQ ID NO: 9 in Sequence Listing, or an amino acid sequence obtained by substituting one to several (preferably, one or two) amino acids in the aforementioned amino acid sequence;

CDRH2 consisting of an amino acid sequence represented by SEQ ID NO: 10 in Sequence Listing, or an amino acid sequence obtained by substituting one to several (preferably, one or two) amino acids in the aforementioned amino acid sequence; and

CDRH3 consisting of an amino acid sequence represented by SEQ ID NO: 11 in Sequence Listing, or an amino acid sequence obtained by substituting one to several (preferably, one or two) amino acids in the aforementioned amino acid sequence; and a light chain having a variable region comprising:

CDRL1 consisting of an amino acid sequence represented by SEQ ID NO: 5 in Sequence Listing, or an amino acid sequence obtained by substituting one to several (preferably, one or two) amino acids in the aforementioned amino acid sequence;
CDRL2 consisting of an amino acid sequence represented by SEQ ID NO: 6 in Sequence Listing, or an amino acid sequence obtained by substituting one to several (preferably, one or two) amino acids in the aforementioned amino acid sequence; and
CDRL3 consisting of an amino acid sequence represented by SEQ ID NO: 7 in Sequence Listing, or an amino acid sequence obtained by substituting one to several (preferably, one or two) amino acids in the aforementioned amino acid, and
recognizing the CLDN6 protein of the present invention or retaining the CLDN6 protein-binding activity of the antibody,

or a functional fragment of the antibody.

Preferred examples of CDR amino acid substitution in the humanized anti-CLDN6 antibody or functional fragment thereof may include, but are not limited to, substitution of one to several (preferably, one or two) amino acids in CDRL3 as described above, and an example thereof is CDRL3 represented by SEQ ID NO: 8 in Sequence Listing, which is obtained by substituting amino acid residues 4 and 5 of SEQ ID NO: 7 in Sequence Listing.

Examples of the heavy chain variable region of the humanized antibody comprising the above-described CDRHs may include, but are not limited to, an amino acid sequence represented by SEQ ID NO: 54 in Sequence Listing, an amino acid sequence represented by SEQ ID NO: 58 in Sequence Listing, and an amino acid sequence represented by SEQ ID NO: 62 in Sequence Listing, and examples of the light chain variable region of the humanized antibody comprising the above-described CDRLs may include, but not limited to, an amino acid sequence represented by SEQ ID NO: 38 in Sequence Listing, an amino acid sequence represented by SEQ ID NO: 42 in Sequence Listing, an amino acid sequence represented by SEQ ID NO: 46 in Sequence Listing, and an amino acid sequence represented by SEQ ID NO: 50 in Sequence Listing.

Preferred examples of humanized antibodies including a combination of the above heavy chain variable region and light chain variable region may include, but are not limited to:

a humanized antibody comprising a heavy chain variable region consisting of an amino acid sequence represented by SEQ ID NO: 54 in Sequence Listing and a light chain variable region consisting of an amino acid sequence represented by SEQ ID NO: 38 in Sequence Listing;
a humanized antibody comprising a heavy chain variable region consisting of an amino acid sequence represented by SEQ ID NO: 58 in Sequence Listing and a light chain variable region consisting of an amino acid sequence represented by SEQ ID NO: 42 in Sequence Listing;
a humanized antibody comprising a heavy chain variable region consisting of an amino acid sequence represented by SEQ ID NO: 54 in Sequence Listing and a light chain variable region consisting of an amino acid sequence represented by SEQ ID NO: 46 in Sequence Listing;
a humanized antibody comprising a heavy chain variable region consisting of an amino acid sequence represented by SEQ ID NO: 58 in Sequence Listing and a light chain variable region consisting of an amino acid sequence represented by SEQ ID NO: 50 in Sequence Listing; and
a humanized antibody comprising a heavy chain variable region consisting of an amino acid sequence represented by SEQ ID NO: 62 in Sequence Listing and a light chain variable region consisting of an amino acid sequence represented by SEQ ID NO: 46 in Sequence Listing.

Examples of full-length sequences of humanized antibodies including a combination of the above heavy chain variable region and light chain variable region may include, but are not limited to:

a humanized antibody comprising a heavy chain consisting of an amino acid sequence consisting of amino acid residues 20 to 471 of SEQ ID NO: 52 in Sequence Listing and a light chain consisting of an amino acid sequence consisting of amino acid residues 21 to 234 of SEQ ID NO: 36 in Sequence Listing (H1L1);
a humanized antibody comprising a heavy chain consisting of an amino acid sequence consisting of amino acid residues 20 to 471 of SEQ ID NO: 56 in Sequence Listing and a light chain consisting of an amino acid sequence consisting of amino acid residues 21 to 234 of SEQ ID NO: 40 in Sequence Listing (H2L2);
a humanized antibody comprising a heavy chain consisting of an amino acid sequence consisting of amino acid residues 20 to 471 of SEQ ID NO: 52 in Sequence Listing and a light chain consisting of an amino acid sequence consisting of amino acid residues 21 to 234 of SEQ ID NO: 44 in Sequence Listing (H1L3);
a humanized antibody comprising a heavy chain consisting of an amino acid sequence consisting of amino acid residues 20 to 471 of SEQ ID NO: 56 in Sequence Listing and a light chain consisting of an amino acid sequence

consisting of amino acid residues 21 to 234 of SEQ ID NO: 48 in Sequence Listing (H2L4); and
a humanized antibody comprising a heavy chain consisting of an amino acid sequence consisting of amino acid residues 20 to 471 of SEQ ID NO: 60 in Sequence Listing and a light chain consisting of an amino acid sequence consisting of amino acid residues 21 to 234 of SEQ ID NO: 44 in Sequence Listing (H3L3).

In the heavy chain amino acid sequence represented by SEQ ID NO: 52 , 56 , or 60 in Sequence Listing, an amino acid sequence consisting of amino acid residues 1 to 19 is the signal sequence, an amino acid sequence consisting of amino acid residues 20 to 141 is the heavy chain variable region, and an amino acid sequence consisting of amino acid residues 142 to 471 is the heavy chain constant region.
In the light chain amino acid sequence represented by SEQ ID NO: 36, 40, 44, or 48, an amino acid sequence consisting of amino acid residues 1 to 20 is the signal sequence, an amino acid sequence consisting of amino acid residues 21 to 127 is the light chain variable region, and an amino acid sequence consisting of amino acid residues 128 to 234 is the light chain constant region.
As described later, one or two amino acids may be deleted at the carboxyl terminus of each of the humanized antibodies H1L1, H2L2, H1L3, H2L4, and H3L3, and such deletion variants are also included in the present invention.
Examples of the heavy chain of deletion variants may include, but are not limited to, a heavy chain including an amino acid sequence consisting of amino acid residues 20 to 470 of SEQ ID NO: 52, 56, or 60 in Sequence Listing.
Examples of such deletion variants may include:

a humanized antibody comprising a heavy chain consisting of an amino acid sequence consisting of amino acid residues 20 to 470 of SEQ ID NO: 52 in Sequence Listing and a light chain consisting of an amino acid sequence consisting of amino acid residues 21 to 234 of SEQ ID NO: 36 in Sequence Listing (H1L1);
a humanized antibody comprising a heavy chain consisting of an amino acid sequence consisting of amino acid residues 20 to 470 of SEQ ID NO: 56 in Sequence Listing and a light chain consisting of an amino acid sequence consisting of amino acid residues 21 to 234 of SEQ ID NO: 40 in Sequence Listing (H2L2);
a humanized antibody comprising a heavy chain consisting of an amino acid sequence consisting of amino acid residues 20 to 470 of SEQ ID NO: 52 in Sequence Listing and a light chain consisting of an amino acid sequence consisting of amino acid residues 21 to 234 of SEQ ID NO: 44 in Sequence Listing (H1L3);
a humanized antibody comprising a heavy chain consisting of an amino acid sequence consisting of amino acid residues 20 to 470 of SEQ ID NO: 56 in Sequence Listing and a light chain consisting of an amino acid sequence consisting of amino acid residues 21 to 234 of SEQ ID NO: 48 in Sequence Listing (H2L4); and
a humanized antibody comprising a heavy chain consisting of an amino acid sequence consisting of amino acid residues 20 to 470 of SEQ ID NO: 60 in Sequence Listing and a light chain consisting of an amino acid sequence consisting of amino acid residues 21 to 234 of SEQ ID NO: 44 in Sequence Listing (H3L3).

The nucleotide sequence encoding the heavy chain amino acid sequence of the humanized antibody H1L1 and that encoding the light chain amino acid sequence of the humanized antibody H1L1 are a polynucleotide represented by SEQ ID NO: 53 and a polynucleotide represented by SEQ ID NO: 37, respectively;

the nucleotide sequence encoding the heavy chain amino acid sequence of the humanized antibody H2L2 and that encoding the light chain amino acid sequence of the humanized antibody H2L2 are a polynucleotide represented by SEQ ID NO: 57 and a polynucleotide represented by SEQ ID NO: 41, respectively;
the nucleotide sequence encoding the heavy chain amino acid sequence of the humanized antibody H1L3 and that encoding the light chain amino acid sequence of the humanized antibody H1L3 are a polynucleotide represented by SEQ ID NO: 53 and a polynucleotide represented by SEQ ID NO: 45, respectively;
the nucleotide sequences encoding the heavy chain amino acid sequence of the humanized antibody H2L4 and that encoding the light chain amino acid sequence of the humanized antibody H2L4 are a polynucleotide represented by SEQ ID NO: 57 and a polynucleotide represented by SEQ ID NO: 49, respectively; and
the nucleotide sequence encoding the heavy chain amino acid sequence of the humanized antibody H3L3 and that encoding the light chain amino acid sequence of the humanized antibody H3L3 are a polynucleotide represented by SEQ ID NO: 61 and a polynucleotide represented by SEQ ID NO: 45, respectively.

The nucleotide sequence encoding the amino acid sequence of the heavy chain variable region of the humanized antibody H1L1 and that encoding the light chain variable region of the humanized antibody H1L1 are a polynucleotide represented by SEQ ID NO: 55 and a polynucleotide represented by SEQ ID NO: 39, respectively;

the nucleotide sequence encoding the amino acid sequence of the heavy chain variable region of the humanized antibody H2L2 and that encoding the light chain variable region of the humanized antibody H2L2 are a polynucleotide

represented by SEQ ID NO: 59 and a polynucleotide represented by SEQ ID NO: 43, respectively;
the nucleotide sequence encoding the amino acid sequence of the heavy chain variable region of the humanized antibody H1L3 and that encoding the light chain variable region of the humanized antibody H1L3 are a polynucleotide represented by SEQ ID NO: 55 and a polynucleotide represented by SEQ ID NO: 47, respectively;
the nucleotide sequence encoding the amino acid sequence of the heavy chain variable region of the humanized antibody H2L4 and that encoding the light chain variable region of the humanized antibody H2L4 are a polynucleotide represented by SEQ ID NO: 59 and a polynucleotide represented by SEQ ID NO: 51, respectively; and
the nucleotide sequence encoding the amino acid sequence of the heavy chain variable region of the humanized antibody H3L3 and that encoding the light chain variable region of the humanized antibody H3L3 are a polynucleotide represented by SEQ ID NO: 63 and a polynucleotide represented by SEQ ID NO: 47, respectively.

In the nucleotide sequence represented by SEQ ID NO: 53, 57, or 61 in Sequence Listing, a nucleotide sequence consisting of nucleotide residues 1 to 57 is encoding the signal sequence of the humanized antibody heavy chain, a nucleotide sequence consisting of nucleotide residues 58 to 423 is encoding the amino acid sequence of the variable region of the humanized antibody heavy chain, and a nucleotide sequence consisting of nucleotide residues 424 to 1413 is encoding the constant region of the antibody heavy chain.

In the nucleotide sequence represented by SEQ ID NO: 37, 41, 45, or 49 in Sequence Listing, a nucleotide sequence consisting of nucleotide residues 1 to 60 is encoding the signal sequence of the humanized antibody light chain, a nucleotide sequence consisting of nucleotide residues 61 to 381 is encoding the amino acid sequence of the variable region of the humanized antibody light chain, and a nucleotide sequence consisting of nucleotide residues 382 to 702 is encoding the constant region of the antibody light chain.

As long as having binding activity to CLDN6, any antibody that has an identity or homology of 80% or higher, preferably of 90% or higher, more preferably of 95% or higher, even more preferably of 97% or higher, most preferably of 99% or higher, to the amino acid sequence of any of the antibodies including the above combinations of a heavy chain variable region and a light chain variable region and the antibodies including the above combinations of a heavy chain and a light chain is also included in the antibody of the present invention.

As long as having binding activity to CLDN6, any antibody that includes CDRs consisting of the amino acid sequences of the CDRs of any of the antibodies including the above combinations of a heavy chain variable region and a light chain variable region and the antibodies including the above combinations of a heavy chain and a light chain, wherein the amino acid sequence of the antibody excluding the amino acid sequences of the CDRs has an amino acid identity or homology of 80% or higher, preferably of 90% or higher, more preferably of 95% or higher, even more preferably of 97% or higher, most preferably of 99% or higher, is also included in the antibody of the present invention.

Further, an antibody having biological activity equivalent to each of the above antibodies may be selected through combining amino acid sequences obtained by substituting, deleting, or adding one or several amino acid residues in the amino acid sequence of the heavy chain or light chain. The substitution of an amino acid herein is preferably conservative amino acid substitution (WO 2013154206).

The conservative amino acid substitution is substitution that occurs in an amino acid group with related amino acid side chains. Such amino acid substitution is preferably carried out to such a degree that the properties of the substance having the original amino acid sequence are not decreased.

Homology between two amino acid sequences may be determined by using default parameters of Blast algorithm version 2.2.2 (Altschul, Stephen F., Thomas L.Madden, Alejandro A. Schaaffer, Jinghui Zhang, Zheng Zhang, Webb Miller, and David J.Lipman (1997), "Gapped BLAST and PSI-BLAST: a new generation of protein database search programs", Nucleic Acids Res. 25: 3389-3402)
Blast algorithm may be used by accessing www.ncbi.nlm.nih.gov/blast on the Internet.

(3) Human antibody

Further examples of the antibody of the present invention may include, but are not limited to, human antibodies capable of binding to CLDN6 and/or CLDN9. The human anti-CLDN6 and/or CLDN9 antibody refers to a human antibody having only an antibody gene sequence derived from a human chromosome. Human anti-CLDN6 antibodies that can be obtained by using known methods (Nature Genetics (1997) 16, p. 133-143, Nucl. Acids Res. (1998) 26, p.3447-3448, Animal Cell Technology: Basic and Applied Aspects, vol.10, p.69-73, Kluwer Academic Publishers, 1999., Proc. Natl. Acad. Sci. USA (2000) 97, p.722-727, Investigative Ophthalmology & Visual Science. (2002) 43(7), p.2301-2308, Briefings in Functional Genomics and Proteomics (2002), 1(2), p. 189-203, Ophthalmology (2002) 109(3), p.427-431, WO92/01047, WO92/20791, WO93/06213, WO93/11236, WO93/19172, WO95/01438, WO95/15388, Annu. Rev. Immunol (1994) 12, p.433-455, Nature Biotechnology (2005) 23(9), p. 1105-1116) are also known.

The anti-HER2 antibody to be used in the present invention will be described in the following.

The anti-HER2 antibody of the present invention has the following properties.

(1) An anti-HER2 antibody having the following properties:

(a) specifically binding to HER2; and
(b) internalizing into HER2-expressing cells by binding to HER2.

(2) The antibody according to (1), binding to the extracellular domain of HER2.
(3) The antibody according to (1) or (2), being a monoclonal antibody.
(4) The antibody according to any one of (1) to (3), having activities or activity of antibody-dependent cellular cytotoxicity (ADCC) and/or complement-dependent cytotoxicity (CDC).
(5) The antibody according to any one of (1) to (4), being a mouse monoclonal antibody, a chimeric monoclonal antibody, or a humanized monoclonal antibody.
(6) The antibody according to any one of (1) to (3) and (5), wherein the heavy chain constant region is a heavy chain constant region of human IgG1, and comprises a mutation that causes lowering of activities or activity of ADCC and/or CDC.
(7) The antibody according to (6), wherein the heavy chain constant region is a heavy chain constant region of human IgG1, and leucine at the 234- and 235-positions specified by EU Index numbering is substituted with alanine.
(8) The antibody according to any one of (1) to (5), being an antibody comprising a heavy chain consisting of an amino acid sequence represented by SEQ ID NO: 65 and a light chain consisting of an amino acid sequence represented by SEQ ID NO: 64.
(9) The antibody according to any one of (1) to (3) and (5) to (7), being an antibody comprising a heavy chain variable region consisting of an amino acid sequence consisting of amino acid residues 20 to 139 of SEQ ID NO: 75 and a light chain variable region consisting of an amino acid sequence consisting of amino acid residues 21 to 127 of SEQ ID NO: 73.
(10) The antibody according to any one of (1) to (3), (5) to (7), and (9), being an antibody comprising a heavy chain consisting of an amino acid sequence consisting of amino acid residues 20 to 469 of SEQ ID NO: 75 and a light chain consisting of an amino acid sequence consisting of amino acid residues 21 to 234 of SEQ ID NO: 73.
(11) The antibody according to any one of (1) to (10), wherein one or two amino acids are deleted at the carboxyl terminus of the heavy chain.
(12) The antibody according to any one of (1) to (5), (8), and (11), comprising a heavy chain consisting of an amino acid sequence consisting of amino acid residues 1 to 449 of SEQ ID NO: 65 and a light chain consisting of an amino acid sequence consisting of amino acid residues 1 to 214 of SEQ ID NO: 64.
(13) The antibody according to any one of (1) to (3), (5) to (7), and (9) to (11), comprising a heavy chain consisting of an amino acid sequence consisting of amino acid residues 20 to 468 of SEQ ID NO: 75 and a light chain consisting of an amino acid sequence consisting of amino acid residues 21 to 234 of SEQ ID NO: 73.
(14) An antibody obtained by using a method for producing the antibody according to any one of (1) to (13), the method including the steps of: culturing a host cell transformed with an expression vector containing a polynucleotide encoding the antibody; and collecting the targeted antibody from a culture obtained from the step of culturing.

In the present application, such an antibody that leucine at the 234- and 235-positions specified by EU Index numbering in the heavy chain constant region of trastuzumab (SEQ ID NO: 65) is substituted with alanine is referred to as a trastuzumab variant.
Further, another example of the anti-HER2 antibody of the present invention is Trastuzumab variant 2. The heavy chain amino acid sequence and light chain amino acid sequence of Trastuzumab variant 2 are represented by SEQ ID NO: 77 and SEQ ID NO: 76, respectively.
The antibody of the present invention includes modified variants of the antibody. The modified variant refers to a variant obtained by subjecting the antibody of the present invention to chemical or biological modification. Examples of the chemically modified variant may include, but are not limited to, variants including a linkage of a chemical moiety to an amino acid skeleton, and variants with chemical modification of an N-linked or O-linked carbohydrate chain. Examples of the biologically modified variant may include, but are not limited to, variants obtained by post-translational modification (e.g., N-linked or O-linked glycosylation, N- or C-terminal processing, deamidation, isomerization of aspartic acid, oxidation of methionine), and variants in which a methionine residue has been added to the N terminus by being expressed in a prokaryotic host cell. Further, an antibody labeled so as to enable the detection or isolation of the antibody of the present invention or an antigen, for example, an enzymelabeled antibody, a fluorescence-labeled antibody, and an affinity-labeled antibody are also included in the meaning of the modified variant. Such a modified variant of the antibody of the present invention is useful for improving the stability and blood retention of the antibody, reducing the antigenicity thereof, detecting or isolating an antibody or an antigen, and so on.
Further, by regulating the modification of a glycan which is linked to the antibody of the present invention (glycosylation, defucosylation, etc.), the antibody-dependent cellular cytotoxic activity can be enhanced. As the technique

for regulating the modification of a glycan of antibodies, WO 1999/54342, WO 2000/61739, WO 2002/31140, WO 2007/133855, WO 2013/120066 etc., are known. However, the technique is not limited thereto. In the antibody of the present invention, antibodies in which the modification of a glycan is regulated are also included.

Such modification may be applied at any position or a desired position in an antibody or a functional fragment of the antibody, and the same type or two or more different types of modification may be applied at one or two or more positions.

In the present invention, the meaning of a "modified variant of an antibody fragment" also includes a "fragment of a modified variant of an antibody".

If an antibody gene is temporarily isolated and then introduced into an appropriate host to produce an antibody, an appropriate combination of a host and an expression vector can be used. Specific examples of the antibody gene may include, but are not limited to, combination of a gene encoding the heavy chain sequence or the like of an antibody described herein and a gene encoding the light chain sequence or the like of an antibody described herein. To transform host cells, a heavy chain sequence gene or the like and a light chain sequence gene or the like may be inserted into the same expression vector, or inserted into separate expression vectors.

If eukaryotic cells are used as a host, animal cells, plant cells, and eukaryotic microorganisms may be used. Particularly, examples of animal cells may include, but are not limited to, mammalian cells, such as COS cells (Cell (1981) 23, p. 175-182, ATCC CRL-1650), as monkey cells, the mouse fibroblast NIH3T3 (ATCC No. CRL-1658), a dihydrofolate reductase-deficient strain (Proc. Natl. Acad. Sci. U.S.A. (1980) 77, p. 4126-4220) of Chinese hamster ovary cells (CHO cells, ATCC CCL-61), and FreeStyle 293F cells (Invitrogen).

If prokaryotic cells are used, for example, Escherichia coli or Bacillus subtilis may be used.

A targeted antibody gene is introduced into these cells by transformation, and the transformed cells are cultured in vitro to afford an antibody. Sequence difference among antibodies may result in different yields in the culture, and hence antibodies that allow easy production of a medicine may be selected out of antibodies having equivalent binding activity by using yields as an indicator. Accordingly, the antibody of the present invention includes antibodies obtained by using a method for producing the antibody, the method including the steps of: culturing the transformed host cell; and collecting a targeted antibody or a functional fragment of the antibody from a culture obtained in the step of culturing.

The antibody gene is preferably a polynucleotide including a polynucleotide described in any one of (a) to (e):

(a) a combination of a polynucleotide encoding the heavy chain amino acid sequence and a polynucleotide encoding the light chain amino acid sequence of an antibody of any one of the B1 or C7 antibody, the chB1 antibody, the humanized antibodies H1L1, H2L2, H1L3, H2L4, and H3L3, trastuzumab, and a variant thereof;

(b) a combination of a polynucleotide encoding a heavy chain amino acid sequence including the sequences of CDRH1 to CDRH3 and a polynucleotide encoding a light chain amino acid sequence including the sequences of CDRL1 to CDRL3 of an antibody of any one of the B1 or C7 antibody, the chB1 antibody, the humanized antibodies H1L1, H2L2, H1L3, H2L4, and H3L3, trastuzumab, and a variant thereof;

(c) a combination of a polynucleotide encoding a heavy chain amino acid sequence comprising the amino acid sequence of the heavy chain variable region and a polynucleotide encoding a light chain amino acid sequence comprising the amino acid sequence of the light chain variable region of an antibody of any one of the B1 or C7 antibody, the chB1 antibody, the humanized antibodies H1L1, H2L2, H1L3, H2L4, and H3L3, trastuzumab, and a variant thereof;

(d) a polynucleotide that is hybridizable with nucleotides consisting of a polynucleotide complementary to the polynucleotide according to any one of (a) to (c) under stringent conditions and is encoding the amino acid sequence of an antibody capable of binding to CDLN6 or HER2; and

(e) a polynucleotide encoding the amino acid sequence of a polypeptide obtained by substituting, deleting, adding, or inserting 1 to 50, 1 to 45, 1 to 40, 1 to 35, 1 to 30, 1 to 25, 1 to 20, 1 to 15, 1 to 10, one to eight, one to six, one to five, one to four, one to three, one or two, or one amino acid(s) in the polynucleotide according to any one of (a) to (c), and is encoding the amino acid sequence of an antibody capable of binding to CLDN6 or HER2.

The present invention includes a nucleotide encoding the antibody of the present invention or a functional fragment of the antibody, or a modified variant of the antibody or functional fragment; a recombinant vector including the gene inserted therein; and a cell including the gene or the vector introduced therein.

The present invention includes a method for producing an antibody or a functional fragment of the antibody, or a modified variant of the antibody or functional fragment, the method including the steps of: culturing the cell; and collecting from the culture an antibody or a functional fragment of the antibody, or a modified variant of the antibody or functional fragment.

It is known that a lysine residue at the carboxyl terminus of the heavy chain of an antibody produced in a cultured mammalian cell is deleted (Journal of Chromatography A, 705: 129-134 (1995)), and it is also known that two amino acid residues, glycine and lysine, at the carboxyl terminus of the heavy chain of an antibody produced in a cultured

mammalian cell are deleted and that a proline residue newly located at the carboxyl terminus is amidated (Analytical Biochemistry, 360: 75-83 (2007)). However, such deletion and modification of the heavy chain sequence do not affect the antigen-binding ability and the effector function (the activation of complement, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variants having a deletion at the carboxyl terminus of the heavy chain of the antibody according to the present invention is not limited to the above variants as long as the antigen-binding ability and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, an antibody in which one amino acid residue at the carboxyl terminus has been deleted in both of the two heavy chains in the antibody according to the present invention can be preferably exemplified as a main component of molecules of the antibody.

The antibody obtained may be purified to a homogeneous state. For separation/purification of the antibody, separation/purification methods commonly used for protein can be used. For example, the antibody may be separated/purified by appropriately selecting and combining column chromatography, filter filtration, ultrafiltration, salting-out, dialysis, preparative polyacrylamide gel electrophoresis, isoelectric focusing electrophoresis, and so on (Strategies for Protein Purification and Characterization: A Laboratory Course Manual, Daniel R. Marshak et al. eds., Cold Spring Harbor Laboratory Press (1996); Antibodies: A Laboratory Manual. Ed Harlow and David Lane, Cold Spring Harbor Laboratory (1988)), but separation/purification methods are not limited thereto.

<N297 Glycan>

A method for remodeling heterogeneous glycoprotein of an antibody by enzymatic reaction or the like to homogeneously introduce a glycan having a functional group (ACS Chemical Biology 2012, 7, 110, ACS Medicinal Chemistry Letters 2016, 7, 1005, Bioconjugate Chemistry 2015, 26, 2233, Angew. Chem. Int. Ed. 2016, 55, 2361-2367, US 2016361436) has been recently reported.

In the glycan remodeling of the present invention, using hydrolase, heterogeneous glycans added to a protein (e.g., an antibody) are cleaved off to leave only GlcNAc at each terminus, thereby producing a homogenous protein moiety with GlcNAc (hereinafter, referred to as an "acceptor"). Subsequently, an arbitrary glycan separately prepared (hereinafter, referred to as a "donor") is provided, and the acceptor and the donor are linked together by using transglycosidase. Thereby, a homogeneous glycoprotein with arbitrary glycan structure can be synthesized.

In the present invention, a "glycan" refers to a structural unit of two or more monosaccharides bonded together via glycosidic bonds. Specific monosaccharides and glycans are occasionally abbreviated, for example, as "GlcNAc-", "MSG-", and so on. When any of these abbreviations is used in a structural formula, the abbreviation is shown with an intention that an oxygen atom or nitrogen atom involved in a glycosidic bond at the reducing terminal to another structural unit is not included in the abbreviation indicating the glycan, unless specifically defined.

In the present invention, a monosaccharide as a basic unit of a glycan is indicated for convenience so that in the ring structure, the position of a carbon atom bonding to an oxygen atom constituting the ring and directly bonding to a hydroxy group (or an oxygen atom involved in a glycosidic bond) is defined as the 1-position (the 2-position only for sialic acids), unless otherwise specified. The names of compounds in Examples are each provided in view of the chemical structure as a whole, and that rule is not necessarily applied.

When a glycan is indicated as a sign (e.g., GLY, SG, MSG, GlcNAc) in the present invention, the sign is intended, unless otherwise defined, to include carbon atoms ranging to the reducing terminal and not to include N or O involved in an N- or O-glycosidic bond.

In the present invention, unless specifically stated, a partial structure when a glycan is linking to a side chain of an amino acid is indicated in such a manner that the side chain portion is indicated in parentheses, for example, "(SG-)Asn".

The antibody-drug conjugate of the present invention is represented by the following formula:

[Formula 41]

$$Ab-\left[(N297\ glycan)-\left[L-D\right]_{m^1}\right]_2$$

wherein an antibody Ab or a functional fragment of the antibody bonds via a N297 glycan or remodeled N297 glycan to L, and preferably bonds via a remodeled N297 glycan of Ab to L.

Glycans in Ab of the present invention are N-linked glycans or O-linked glycans, and preferably N-linked glycans.

N-linked glycans and O-linked glycans bond to an amino acid side chain of an antibody via an N-glycosidic bond and an O-glycosidic bond, respectively.

IgG has a well conserved N-linked glycan on an asparagine residue at the 297-position of the Fc region of the heavy chain (hereinafter, referred to as "Asn297 or N297"), and the N-linked glycan is known to contribute to the activity and kinetics of the antibody molecule (Biotechnol. Prog., 2012, 28, 608-622, Anal. Chem., 2013, 85, 715-736).

The amino acid sequence in the constant region of IgG is well conserved, and each amino acid is specified by Eu index numbering in Edelman et al. (Proc. Natl. Acad. Sci. U.S.A., Vol. 63, No. 1 (May 15, 1969), p. 78-85). For example, Asn297, to which an N-linked glycan is added in the Fc region, corresponds to the 297-position in Eu index numbering, and each amino acid is uniquely specified by Eu index numbering, even if the actual position of the amino acid has varied through fragmentation of the molecule or deletion of a region.

In the antibody-drug conjugate of the present invention, the antibody or functional fragment of the antibody preferably bonds to L via a glycan bonding to a side chain of Asn297 thereof (hereinafter, referred to as "N297 glycan"), and the antibody or functional fragment of the antibody more preferably bonds via the N297 glycan to L, wherein the N297 glycan is a remodeled glycan.

SGP, an abbreviation for sialyl glycopeptide, is a representative N-linked complex glycan. SGP can be isolated/purified from the yolk of a hen egg, for example, by using a method described in WO 2011/0278681. Purified products of SGP are commercially available (Tokyo Chemical Industry Co., Ltd., FUSHIMI Pharmaceutical Co., Ltd.), and may be purchased. For example, disialooctasaccharide (Tokyo Chemical Industry Co., Ltd.), a glycan formed by deleting one GlcNAc at the reducing terminal in the glycan moiety of SG (hereinafter, referred to as "SG (10)", is commercially available.

In the present invention, a glycan structure formed by deleting a sialic acid at a non-reducing terminal only in either one of the branched chains of β-Man in SG (10) refers to MSG (9), and a structure having a sialic acid only in the 1-3 branched chains is called as MSG1, and a structure having a sialic acid only in the 1-6 branched chains is called as MSG2.

The remodeled N297 glycan of the present invention is N297-(Fuc)MSG1, N297-(Fuc)MSG2, or a mixture of N297-(Fuc)MSG1 and N297-(Fuc)MSG2, or N297-(Fuc)SG, and is preferably N297-(Fuc)MSG1,N297-(Fuc)MSG2, or N297-(Fuc)SG, and is more preferably N297-(Fuc)MSG1 or N297-(Fuc)MSG2.

N297-(Fuc)MSG1 is represented by the following structural formula or sequence formula:

[Formula 42]

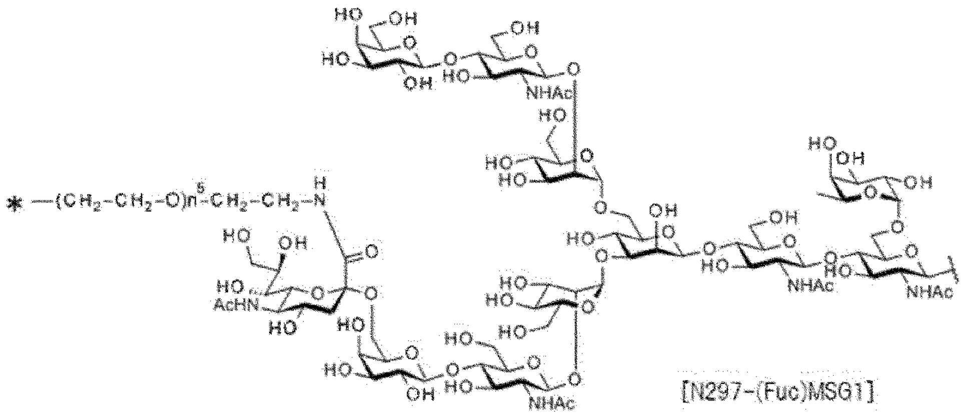

[N297-(Fuc)MSG1]

[Formula 43]

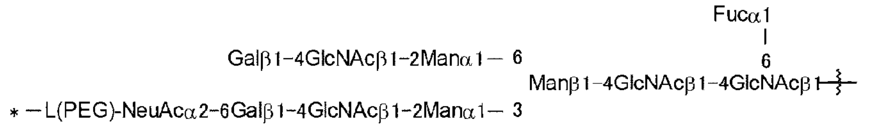

[N297-(Fuc)MSG1]

In the formulas, each wavy line represents bonding to Asn297 of the antibody, L(PEG) represents *-$(CH_2CH_2\text{-}O)n^5\text{-}CH_2CH_2\text{-}NH$-, wherein the amino group at the right end represents bonding via an amide bond to carboxylic acid at the 2-position of a sialic acid at the non-reducing terminal in the 1-3 branched chains of β-Man in the N297 glycan, the asterisk * at the left end represents bonding to a nitrogen atom at the 1- or 3-position of the 1,2,3-triazole ring of Lb in linker L, and $n^5$ is an integer of 2 to 10, and preferably an integer of 2 to 5.

N297-(Fuc)MSG2 is represented by the following structural formula or sequence formula:

[Formula 44]

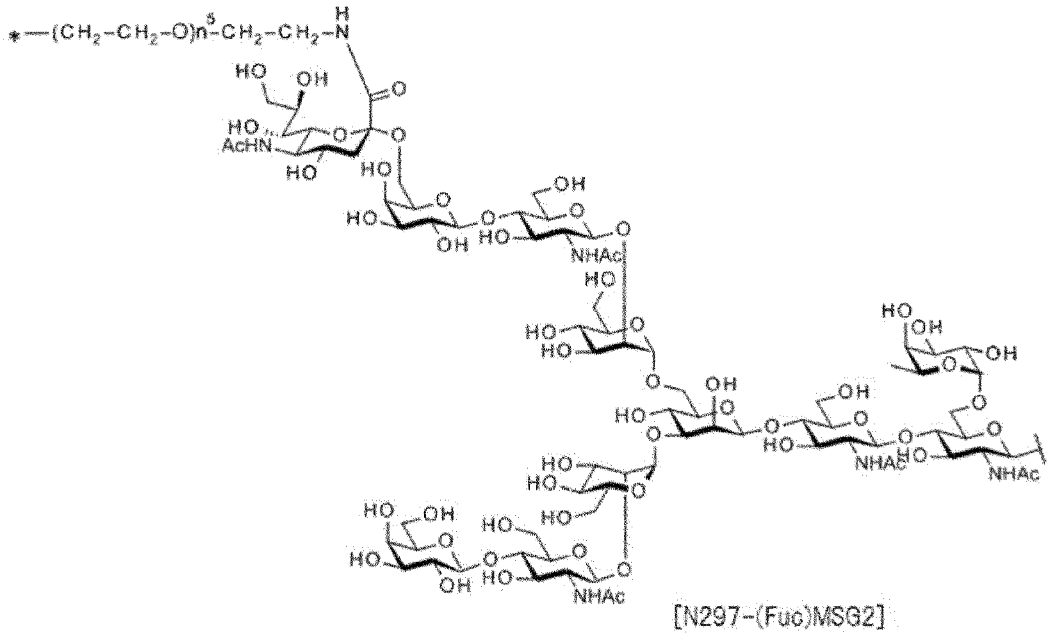

[N297-(Fuc)MSG2]

[Formula 45]

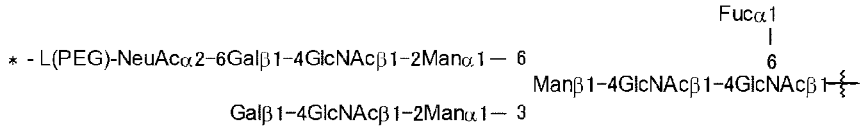

[N297-(Fuc)MSG2]

In the formulas, each wavy line represents bonding to Asn297 of the antibody, L(PEG) represents *-$(CH_2CH_2\text{-}O)n^5$-$CH_2CH_2$-NH-, wherein the amino group at the right end represents bonding via an amide bond to carboxylic acid at the 2-position of a sialic acid at the non-reducing terminal in the 1-6 branched chains of β-Man in the N297 glycan, the asterisk * at the left end represents bonding to a nitrogen atom at the 1- or 3-position of the 1,2,3-triazole ring of Lb in linker L, and $n^5$ is an integer of 2 to 10, and preferably an integer of 2 to 5.

N297-(Fuc)SG is represented by the following structural formula or sequence formula:

[Formula 46]

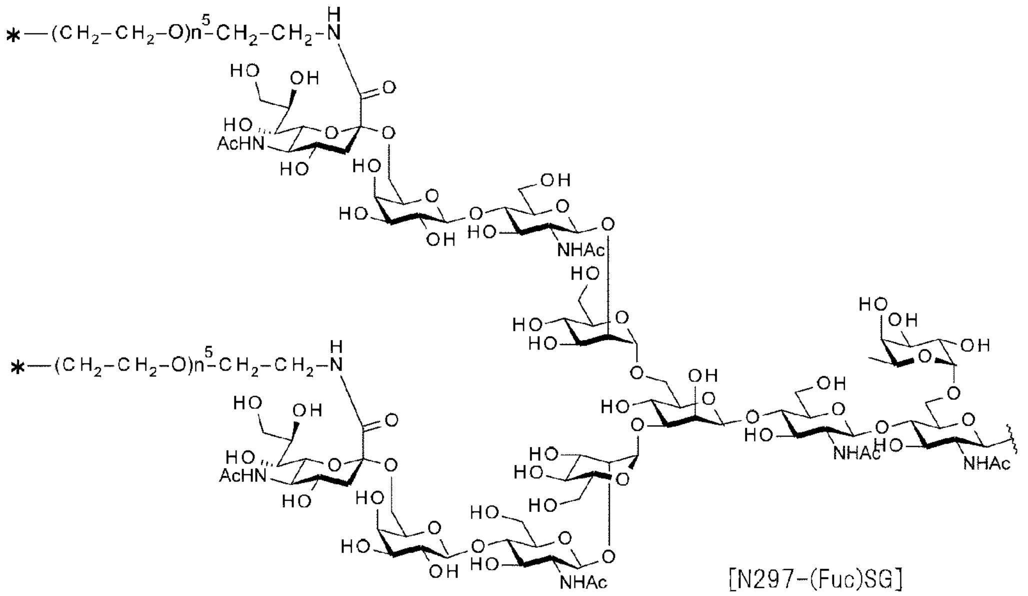

[N297-(Fuc)SG]

[Formula 47]

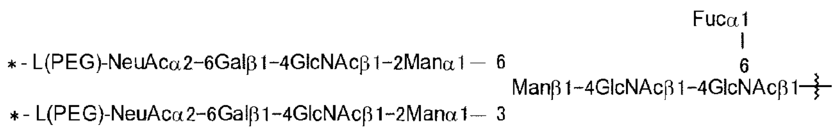

[N297-(Fuc)SG]

In the formulas, each wavy line represents bonding to Asn297 of the antibody, L(PEG) represents *-$(CH_2CH_2\text{-}O)n^5$-$CH_2CH_2$-NH-, wherein the amino group at the right end represents bonding via an amide bond to carboxylic acid at the 2-position of a sialic acid at the non-reducing terminal in each of the 1-3 branched chains and 1-6 branched chains of β-Man in the N297 glycan, the asterisk * at the left end represents bonding to a nitrogen atom at the 1- or 3-position of the 1,2,3-triazole ring of Lb in linker L, and $n^5$ is an integer of 2 to 10, and preferably an integer of 2 to 5.

If N297 glycan of the antibody in the antibody-drug conjugate of the present invention is N297-(Fuc)MSG1,N297-(Fuc)MSG2, or a mixture of them, the antibody-drug conjugate is a molecule to which two molecules of drug-linker (-L-D) have been conjugated ($m^1$ = 1) since the antibody is a dimer (see Figure 1).

For example, Example 27: ADC5 is in the case that N297 glycan is N297-(Fuc)MSG1.

If N297 glycan of the antibody in the antibody-drug conjugate of the present invention is N297-(Fuc)SG, the antibody-drug conjugate is a molecule to which four molecules of drug linker (-L-D) have been conjugated ($m^1 = 2$) since the antibody is a dimer.

N297 glycan is preferably N297-(Fuc)MSG1,N297-(Fuc)MSG2, or N297-(Fuc)SG, more preferably N297-(Fuc)MSG1 or N297-(Fuc)MSG2, and most preferably N297-(Fuc)MSG1.

If N297 glycan of the antibody in the antibody-drug conjugate of the present invention is N297-(Fuc)MSG1,N297-(Fuc)MSG2, or N297-(Fuc)SG, an ADC of homogenous quality can be obtained.

The present invention provides a method for producing a glycan-remodeled antibody or a functional fragment of the antibody, the method including the following steps of:

i) culturing the above-described host cell (e.g., an animal cell (such as a CHO cell)) and collecting a targeted antibody from a culture obtained;
ii) treating the antibody obtained in step i) with hydrolase to produce an antibody with N297 glycan being (Fucα1,6)GlcNAc ((Fucα1,6)GlcNAc-antibody) (Figure 3A);
preferably further purifying the (Fucα1,6)GlcNAc-antibody through a step including purification of the reaction solution with a hydroxyapatite column; and
iii) reacting the (Fucα1,6)GlcNAc-antibody with a glycan donor molecule in the presence of transglycosidase to synthesize a glycan-remodeled antibody with an azide group introduced to a sialic acid, the glycan donor molecule obtained by introducing a PEG linker having an azide group ($N_3$-L(PEG)) to the carbonyl group of carboxylic acid at the 2-position of a sialic acid in MSG (9) or SG (10) and oxazolinating the reducing terminal.

The present invention includes glycan-remodeled antibodies and functional fragments of the antibodies, and modified variants of the antibodies and functional fragments obtained by using the production method.

The production intermediate of the present antibody-drug conjugate has an alkyne structure reactive with an azide group, such as DBCO (dibenzocyclooctyne) (see compound 3-14 in Example 2-1). Therefore, the antibody-drug conjugate of the present invention can be produced by reacting the production intermediate with an MSG1-type, MSG2-type, or SG-type glycan-remodeled antibody or a functional fragment of the antibody, where the antibody, in which a PEG linker having an azide group has been introduced to a sialic acid of a glycan, is obtained through steps i) to iii).

With regard to N297 glycan in the present invention, fucosylated GlcNAc-(Fucα1,6)GlcNAc) at the reducing terminal is preferably derived from an antibody produced in an animal cell, and a portion of the glycan located to the non-reducing terminal side of (Fucα1,6)GlcNAc preferably has been remodeled into the above-described glycan structure as MSG (MSG1, MSG2) or SG. In each case, carboxylic acid bonding to the 2-position of a sialic acid at the non-reducing terminal is used for bonding to L(PEG).

Such a glycan-remodeled antibody having MSG- (MSG1-, MSG2-) or SG-type N297 glycan may be produced by using a method as illustrated in Figure 3, for example, on the basis of a method described in WO 2013/120066. If an antibody is produced as a gene-recombinant protein by using an animal cell as a host on the basis of a known method (step i), the N297 glycan has, as a base structure, a fucosylated N-linked glycan structure, whereas a mixture of antibody molecules having glycans of various structures with various modifications for the structure of the non-reducing terminal or constituent saccharides or fragments of such antibody molecules is provided (IV in Figure 3A). Treatment of such an antibody produced with an animal cell with hydrolase such as EndoS causes hydrolysis of the glycosidic bond at GlcNAcβ1-4GlcNAc in the chitobiose structure at the reducing terminal, providing antibody molecules of single glycan structure having only (Fucα1,6)GlcNAc as N297 glycan (referred to as "(Fucα1,6)GlcNAc-antibody", see A in Figure 2) (Figure 3A) (step ii)).

For the enzyme for the hydrolysis reaction of N297 glycan, for example, Endo S or a variant enzyme retaining the hydrolysis activity may be used.

By reacting the (Fucα1,6)GlcNAc-antibody obtained in the above hydrolysis reaction, as a glycan acceptor molecule, and an MSG- (MSG1-, MSG2-) or SG-type glycan donor molecule with use of transglycosidase (e.g., WO 2017010559) such as EndoS D233Q and EndoS D233Q/Q303L variants, an antibody of the above-described structure including MSG- (MSG1-, MSG2-) or SG type N297 glycan (see B in Figure 2) can be obtained (Figure 3B) (step iii)-1, iii)-2).

If the number of conjugated drug molecules per antibody molecule, $m^1$, in the antibody-drug conjugate is 1, a glycan donor molecule having MSG (MSG1, MSG2) as glycan is employed. For such glycan, commercially available monosialo-Asn free (1S2G/1G2S-10NC-Asn, GlyTech, Inc., hereinafter, referred to as "(MSG-)Asn") as a raw material may be separated on the basis of a method described in Example 3 to obtain (MSG-)Asn1 or (MSG2-)Asn, which may be employed, or a mixture of them may be employed without separation.

If the number of conjugated drug molecules per antibody molecule, $m^1$, in the antibody-drug conjugate is 2, a glycan donor molecule including SG (10) as glycan is used for the transglycosylation reaction. For such SG (10) glycan, for example, that obtained from SGP through hydrolysis or the like may be used, or SG (10) glycan such as commercially

available disialooctasaccharide (Tokyo Chemical Industry Co., Ltd.) may be used.

MSG- (MSG1-, MSG2-) or SG-type glycan included in the donor molecule has a PEG linker having an azide group ($N_3$-L(PEG)) at the 2-position of a sialic acid therein.

It is preferred to use an activated form such as an oxazolinated form formed by treatment with 2-chloro-1,3-dimethyl-1H-benzimidazol-3-ium-chloride for GlcNAc at the reducing terminal of MSG (MSG1, MSG2) or SG-type glycan included in the donor molecule (J.Org.Chem., 2009, 74(5), 2210-2212).

Various enzymes for use in transglycosylation reaction (transglycosidase) may be employed that have activity of transferring complex glycan to N297 glycan; however, EndoS D233Q, a modified product for which hydrolysis reaction is suppressed by substituting Asp at the 233-position of EndoS with Gln, is a preferred transglycosidase. Transglycosylation reaction using EndoS D233Q is described, for example, in WO 2013/120066. Alternatively, a modified enzyme such as EndoS D233Q/Q303L (WO 2017/010559), which is obtained by further adding a mutation to EndoS D233Q, may be used.

The purification operation for the antibody after the glycan remodeling for the antibody (glycohydrolysis and transglycosylation reaction) is intended to separate low-molecular-weight compounds and enzymes used for the reaction, and gel filtration chromatography, ion-exchange chromatography, affinity chromatography, and so on are typically used for such purification, and additional purification with a hydroxyapatite column may be further carried out. That is, the present invention provides a method for producing an antibody-drug conjugate, the method including, in the step of purifying an intermediate from reaction solution after glycohydrolysis of an antibody, the additional step of purifying with a hydroxyapatite column. According to an example of reports on glycan remodeling (JACS. 2012, 134, 12308-12318., Angew. Chem. Int. Ed. 2016, 55, 2361-2367), reaction solution after treatment of an antibody with hydrolase is purified only with a Protein A column (affinity chromatography column); however, this purification method has been proved to be incapable of completely removing hydrolase (e.g., EndoS), and affect the subsequent transglycosylation reaction because of the residual enzyme. In view of such a result, examination was made on purification methods to find that when purification of reaction solution after treatment of an antibody with hydrolase was carried out using a Protein A column and a hydroxyapatite column (CHT column, Bio-Rad Laboratories, Inc.) in the order presented, the reaction efficiency of the subsequent glycosylation reaction was enhanced, without the influence of a residual enzyme.

The antibody-drug conjugate of the present invention is most preferably one antibody-drug conjugate selected from the following group:

[Formula 48]

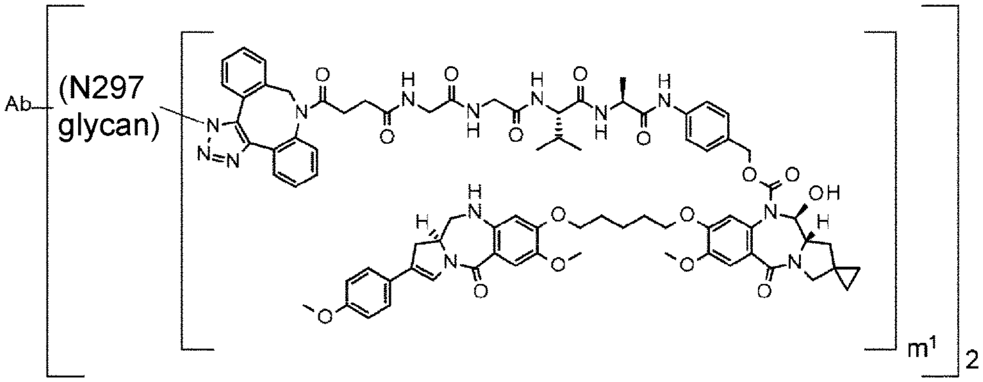

or

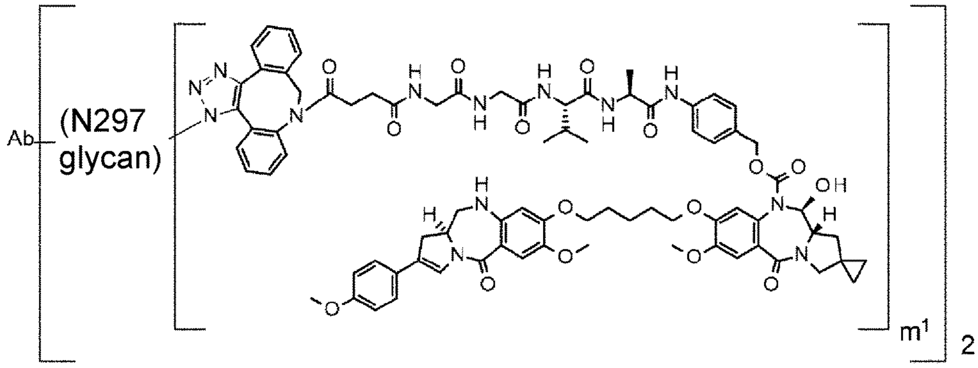

[Formula 49]

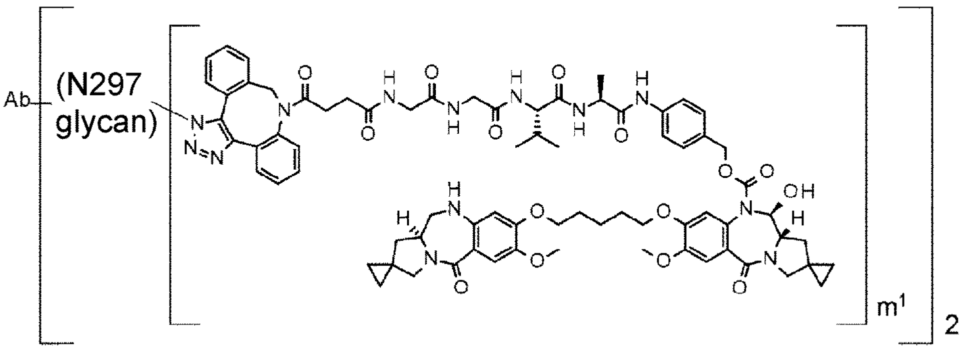

or

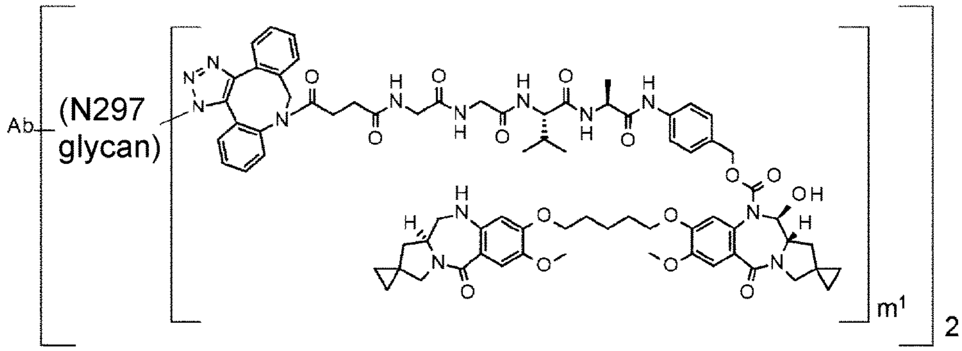

[Formula 50]

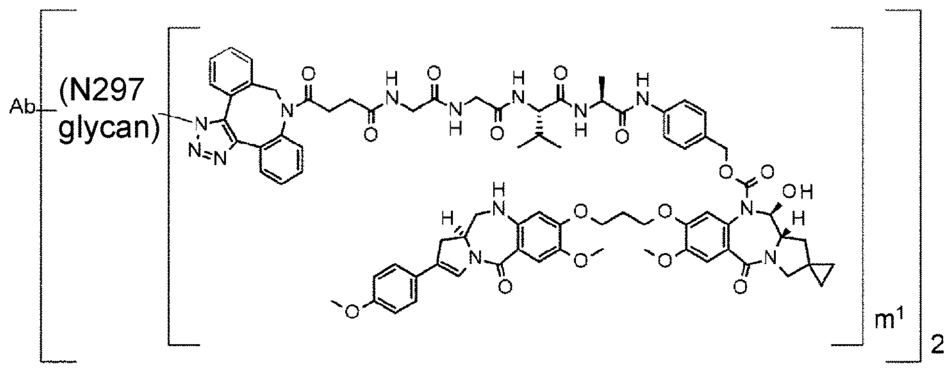

or

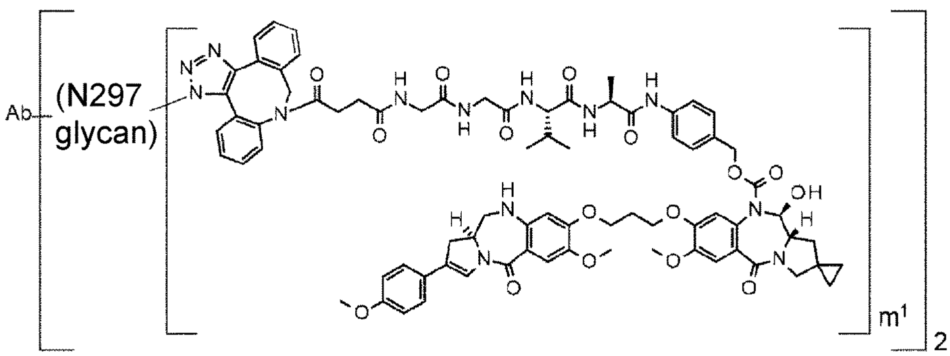

[Formula 51]

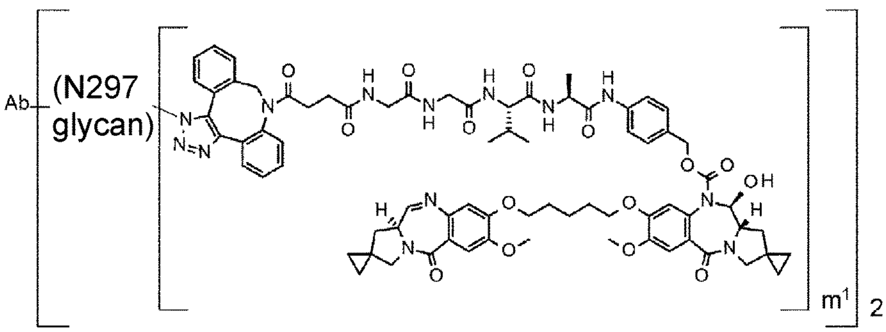

or

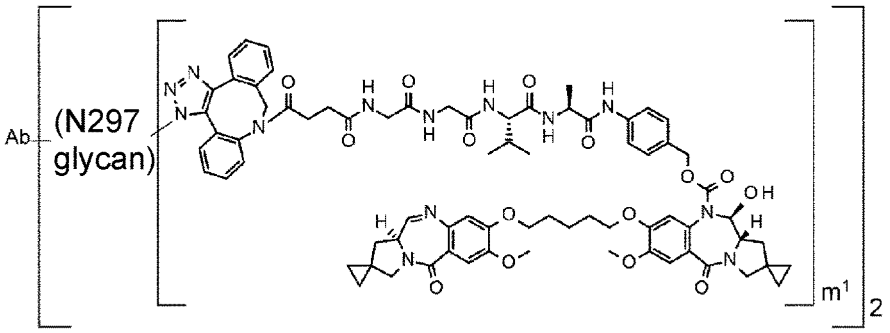

In each of the structural formulas above,

$m^1$ represents an integer of 1 or 2 (preferably, $m^1$ is an integer of 1),
antibody Ab is an anti-CLDN6 antibody, an anti-CLDN9 antibody, an anti-CLDN6/CLDN9 antibody, an anti-HER2 antibody, an anti-HER3 antibody, an anti-DLL3 antibody, an anti-A33 antibody, an anti-CanAg antibody, an anti-CD19 antibody, an anti-CD20 antibody, an anti-CD22 antibody, an anti-CD25 antibody, an anti-CD30 antibody, an anti-CD33 antibody, an anti-CD37 antibody, an anti-CD56 antibody, an anti-CD70 antibody, an anti-CD98 antibody, an anti-B7-H3 antibody, an anti-TROP2 antibody, an anti-CEA antibody, an anti-Cripto antibody, an anti-EphA2 antibody, an anti-FGFR2 antibody (e.g., WO201315206), an anti-G250 antibody, an anti-MUC1 antibody (e.g., WO2011012309), an anti-GPNMB antibody, an anti-Integrin antibody, an anti-PSMA antibody, an anti-Tenascin-C antibody, an anti-SLC44A4 antibody, an anti-Mesothelin antibody, an anti-EGFR antibody, an anti-5T4 antibody, an anti-LRRC15 antibody, an anti-DR5 antibody, an anti-CDH3 antibody, an anti-PDPN antibody, or an anti-CD123 antibody (preferably, the anti-CLDN6 antibody or anti-HER2 antibody,
N297 glycan represents any one of N297-(Fuc)MSG1,N297-(Fuc)MSG2, and a mixture of them, and N297-(Fuc)SG (preferably, N297-(Fuc)MSG1),
L(PEG) represents *-$(CH_2CH_2\text{-}O)_3\text{-}CH_2CH_2$-NH-, wherein the amino group at the right end represents bonding via an amide bond to carboxylic acid at the 2-position of a sialic acid at the non-reducing terminal of each or either one of the 1-3 and 1-6 branched chains (preferably, the 1-3 branched chains) of β-Man in N297 glycan, and the asterisk * at the left end represents bonding to a nitrogen atom at the 1- or 3-position of the triazole ring in the structural formula.

Although structures with two or four units ($m^2$ = 1 or 2) of "-(N297 glycan)-L-D" in each of which N297 glycan bonds to the nitrogen atom at the 1-position of the triazole ring of Lb in L in one conjugate molecule ("(N297 glycan)-(N1Lb)L-D") or structures with two or four units ($m^2$ = 1 or 2) of "-(N297 glycan)-L-D" in each of which N297 glycan bonds to the nitrogen atom at the 3-position of the triazole ring of Lb in L in one conjugate molecule ("(N297 glycan)-(N3Lb)L-D") are illustrated as the most preferred antibody-drug conjugate for convenience, antibody-drug conjugates having both "(N297 glycan)-(N1Lb)L-D" (if $m^2$ = 1, then one unit, if $m^2$ = 2, then one, two, or three units) and "(N297 glycan)-(N3Lb)L-D" (if $m^2$ = 1, then one unit, if $m^2$ = 2, then three, two, or one unit) in one conjugate molecule are also included. In other words, either one of "(N297 glycan)-(N1Lb)L-D" and "(N297 glycan)-(N3Lb)L-D" exists or both of them coexist in one conjugate molecule.

There may exist stereoisomers, optical isomers due to an asymmetric carbon atom, geometric isomers, tautomers, or optical isomers such as d-forms, 1-forms and atropisomers for the antibody-drug conjugate of the present invention, and a free drug or production intermediate of the antibody-drug conjugate, and these isomers, optical isomers, and mixtures of them are all included in the present invention.

The antibody-drug conjugate of the present invention exhibits strong tumor activity (in vivo antitumor activity, in vitro anticellular activity) and satisfactory in vivo kinetics and physical properties, and has high safety, and hence is useful as a pharmaceutical.

The number of conjugated drug molecules per antibody molecule is an important factor having influence on efficacy and safety for the antibody-drug conjugate of the present invention. Antibody-drug conjugates are produced with reaction conditions, such as the amounts of raw materials and reagents to be reacted, specified so as to give a constant number of conjugated drug molecules, but, in contrast to chemical reaction of low-molecular-weight compounds, a mixture with different numbers of conjugated drug molecules is typically obtained. Numbers of conjugated drug molecules per antibody molecule are specified as the average value, namely, the average number of conjugated drug molecules (DAR: Drug to Antibody Ratio). The number of pyrrolobenzodiazepine derivative molecules conjugated to an antibody molecule is controllable, and 1 to 10 pyrrolobenzodiazepine derivative molecules can be conjugated as the average number of conjugated drug molecules per antibody molecule (DAR), but preferably the number is one to eight, and more preferably one to five.

If the antibody bonds via a remodeled glycan of the antibody to L in the antibody-drug conjugate of the present invention, the number of conjugated drug molecules per antibody molecule in the antibody-drug conjugate, $m^2$, is an integer of 1 or 2. If the glycan is N297 glycan and the glycan is N297-(Fuc)MSG1, N297-(Fuc)MSG2, or a mixture of N297-(Fuc)MSG1 and N297-(Fuc)MSG2, $m^2$ is 1, and DAR is in the range of 1 to 3 (preferably, in the range of 1.0 to 2.5, more preferably, in the range of 1.2 to 2.2, or 1.6 to 2.2). If the N297 glycan is N297-(Fuc)SG, $m^2$ is 2, and DAR is in the range of 3 to 5 (preferably, in the range of 3.2 to 4.8, more preferably, in the range of 3.5 to 4.2).

Those skilled in the art could engineer the reaction method to conjugate a required number of drug molecules to each antibody molecule on the basis of the description in Examples herein, and obtain an antibody with a controlled number of conjugated pyrrolobenzodiazepine derivative molecules.

The antibody-drug conjugate, free drug, or production intermediate of the present invention may absorb moisture, allow adhesion of adsorbed water, or become a hydrate when being left to stand in the atmosphere or recrystallized, and such compounds and salts containing water are also included in the present invention.

The antibody-drug conjugate, free drug, or production intermediate of the present invention may be converted into a pharmaceutically acceptable salt, as desired, if it has a basic group such as an amino group. Examples of such salts may include, but are not limited to, hydrohalic acid salts such as hydrochlorides and hydroiodides; inorganic acid salts such as nitrates, perchlorates, sulfates, and phosphates; lower alkanesulfonates such as methanesulfonates, trifluoromethanesulfonates, and ethanesulfonates; arylsufonates such as benzenesulfonates and p-toluenesulfonates; organic acid salts such as formates, acetates, malates, fumarates, succinates, citrates, tartrates, oxalates, and maleates; and amino acid salts such as ornithinates, glutamates, and aspartates.

If the antibody-drug conjugate, free drug, or production intermediate of the present invention has an acidic group such as a carboxy group, a base addition salt can be generally formed. Examples of pharmaceutical acceptable salts may include, but are not limited to, alkali metal salts such as sodium salts, potassium salts, and lithium salts; alkali earth metal salts such as calcium salts and magnesium salts; inorganic salts such as ammonium salts; and organic amine salts such as dibenzylamine salts, morpholine salts, phenylglycine alkyl ester salts, ethylenediamine salts, N-methylglucamates, diethylamine salts, triethylamine salts, cyclohexylamine salts, dicyclohexylamine salts, N,N'-dibenzylethylenediamine salts, diethanolamine salts, N-benzyl-N-(2-phenylethoxy)amine salts, piperazine salts, tetramethylammonium salts, and tris(hydroxymethyl)aminomethane salts.

The antibody-drug conjugate, free drug, or production intermediate of the present invention may exist as a hydrate, for example, by absorbing moisture in the air. The solvate of the present invention is not limited to a particular solvate and may be any pharmaceutically acceptable solvate, and specifically hydrates, ethanol solvates, 2-propanol solvates, and so on are preferred. The antibody-drug conjugate, free drug, or production intermediate of the present invention may be its N-oxide form if a nitrogen atom is present therein. These solvates and N-oxide forms are included in the scope of the present invention.

The present invention includes compounds labeled with various radioactive or nonradioactive isotopes. The antibody-drug conjugate, free drug, or production intermediate of the present invention may contain one or more constituent atoms with non-natural ratios of atomic isotopes. Examples of atomic isotopes may include, but are not limited to, deuterium ($^2H$), tritium ($^3H$), iodine-125 ($^{125}I$), and carbon-14 ($^{14}C$). The compound of the present invention may be radiolabeled with a radioactive isotope such as tritium ($^3H$), iodine-125 ($^{125}I$), and carbon-14 ($^{14}C$). The radiolabeled compound is useful as a therapeutic or prophylactic agent, a reagent for research such as an assay reagent, and a diagnostic agent such as a diagnostic agent for in vivo imaging. Isotopic variants of the antibody-drug conjugate of the present invention are all included in the scope of the present invention, regardless of whether they are radioactive or not.

[Production methods]

Scheme R: Preparation of antibody

A glycan-remodeled antibody may be produced by using a method as illustrated in Figure 3, for example, according to a method described in WO 2013/120066.

[Formula 52]

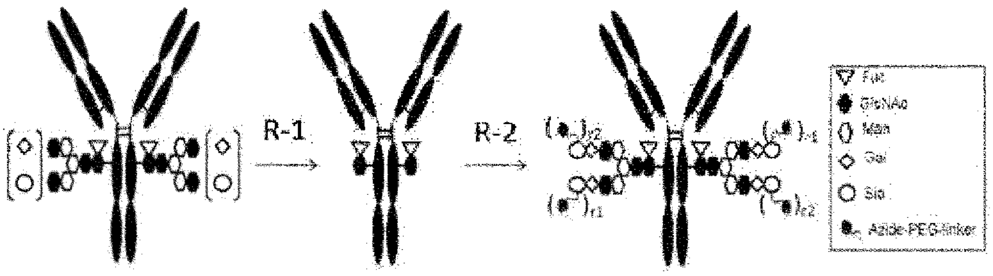


(r1, r2) = any of (1,0), (0,1), and (1,1)

Step R-1: Hydrolysis of glycosidic bond at GlcNAcβ1-4GlcNAc of chitobiose structure at reducing terminal

The step is a step of preparing a glycan-truncated antibody by cleaving N-linked glycan bonding to asparagine at the 297-position of the amino acid sequence of a targeted antibody (N297-linked glycan) with use of a known enzymatic reaction.

A targeted antibody (20 mg/mL) in buffer solution (e.g., 50 mM phosphate buffer solution) is subjected to hydrolysis reaction of the glycosidic bond between GlcNAcβ1 and 4GlcNAc in the chitobiose structure at the reducing terminal with use of hydrolase such as the enzyme EndoS at 0°C to 40°C. The reaction time is 10 minutes to 72 hours, and preferably 1 hour to 6 hours. The amount of the wild-type enzyme EndoS to be used is 0.1 to 10 mg, preferably 0.1 to 3 mg, to 100 mg of the antibody. After the completion of the reaction, purification with affinity chromatography and/or purification with a hydroxyapatite column, each described later, are/is carried out to produce a (Fucα1,6)GlcNAc antibody with the glycan hydrolyzed between GlcNAcβ1 and 4GlcNAc.

Step R-2: Transglycosylation reaction

The step is a step of producing a glycan-remodeled antibody by bonding the (Fucα1,6)GlcNAc antibody to MSG- (MSG1-, MSG2-) or SG-type glycan oxazoline form (hereinafter, referred to as "azide glycan oxazoline form") having a PEG linker including an azide group with use of enzymatic reaction.

The glycan-truncated antibody in buffer solution (e.g., phosphate buffer solution) is subjected to transglycosylation reaction by reacting with an azide glycan oxazoline form in the presence of a catalytic amount of transglycosidase such as EndoS (D233Q/Q303L) at 0°C to 40°C. The reaction time is 10 minutes to 72 hours, and preferably 1 hour to 6 hours. The amount of the enzyme EndoS (D233Q/Q303L) to be used is 1 to 10 mg, preferably 1 to 3 mg, to 100 mg of the antibody, and the amount of the azide glycan oxazoline form to be used is 2 equivalents to an excessive equivalent, preferably 2 equivalents to 20 equivalents.

After the completion of the reaction, purification with affinity chromatography and purification with a hydroxyapatite column are carried out to afford a purified glycan-remodeled antibody.

The azide glycan oxazoline form may be prepared according to methods described in Examples 3 to 5. By using a reaction known in the field of synthetic organic chemistry (e.g., condensation reaction), $N_3$-$(CH_2CH_2$-$O)n_5$-$CH_2CH_2$-$NH_2$, a PEG linker including an azide group ($N_3$-L(PEG)), may be introduced to MSG (MSG1, MSG2) or disialooctasaccharide (Tokyo Chemical Industry Co., Ltd.). Specifically, carboxylic acid at the 2-position of a sialic acid and the amino group at the right end of $N_3$-$(CH_2CH_2$-$O)n_5$-$CH_2CH_2$-$NH_2$ undergo a known condensation reaction to form an amide bond.

MSG, MSG1, or MSG2 may be obtained by hydrolysis of the (MSG-)Asn or separated/purified (MSG1-)Asn or (MSG2-)Asn (Example 56) with hydrolase such as EndoM.

In preparing the glycan-remodeled antibody, concentration of an aqueous solution of an antibody, measurement of concentration, and buffer exchange may be carried out according to common operations A to C in the following.

(Common operation A: Concentration of aqueous solution of antibody)

A solution of an antibody or antibody-drug conjugate was placed in a container of an Amicon Ultra (30,000 to 50,000 MWCO, Millipore Corporation), and the solution of an antibody or antibody-drug conjugate, which is described later, was concentrated through a centrifugation operation (centrifugation at 2000 G to 4000 G for 5 to 20 minutes) using a centrifuge (Allegra X-15R, Beckman Coulter, Inc.).

(Common operation B: Measurement of antibody concentration)

Measurement of antibody concentration was carried out by using a UV measurement apparatus (Nanodrop 1000, Thermo Fisher Scientific Inc.) according to a method specified by the manufacturer. Then, 280 nm absorption coefficients, being different among antibodies (1.3 mL $mg^{-1}$ $cm^{-1}$ to 1.8 mL $mg^{-1}$ $cm^{-1}$), were used.

(Common operation C: Buffer exchange for antibody)

A buffer solution (e.g., phosphate buffered saline (pH 6.0), phosphate buffer (pH 6.0)) was added to an aqueous solution of an antibody, which was concentrated according to common operation A. This operation was carried out several times, and the antibody concentration was then measured by using common operation B, and adjusted to 10 mg/mL with a buffer solution (e.g., phosphate buffered saline (pH 6.0), phosphate buffer (pH 6.0)).

Scheme S: Conjugation

The production method is a method for producing an antibody-drug conjugate by conjugating the above-described glycan-remodeled antibody to production intermediate (2) through SPAAC reaction (strain-promoted alkyne azide cycloaddition: JACS. 2004, 126, 15046-15047).

[Formula 53]

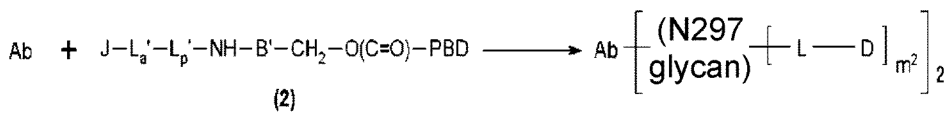

(2)

In the formula, Ab represents the glycan-remodeled antibody,

La', Lp', and B' are synonymous with La, Lp, and B, respectively,
J represents any one of the following structures,
wherein each asterisk * represents bonding to La'.

[Formula 54]

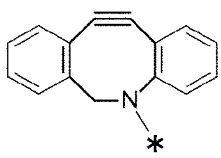

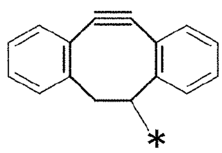

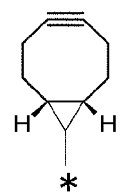

J-La'-Lp'-NH-B'-$CH_2$-O(C=O)-PBD can be synthesized, for example, by using any of methods described in Examples 2-1 to 2-6.

SPAAC reaction proceeds by mixing a buffer solution (sodium acetate solution, sodium phosphate, sodium borate solution, or the like, or a mixture thereof) of antibody Ab and a solution obtained by dissolving compound (2) in an appropriate solvent (dimethyl sulfoxide (DMSO), dimethylformamide (DMF), dimethylacetamide (DMA), N-methyl-2-pyridone (NMP), propylene glycol (PG), or the like, or a mixture thereof).

The amount of moles of compound (2) to be used is 2 mol to an excessive amount of moles, preferably 1 mol to 30 mol, per mole of the antibody, and the ratio of the organic solvent is preferably 1 to 200% v/v to the buffer of the antibody. The reaction temperature is 0°C to 37°C, and preferably 10°C to 25°C, and the reaction time is 1 to 150 hours, and preferably 6 hours to 100 hours. The pH in the reaction is preferably 5 to 9.

Antibody-drug conjugate compounds (ADCs) can be identified from each other through buffer exchange, purification, and measurement of antibody concentration and average number of conjugated drug molecules per antibody molecule according to common operations A to C described above and common operations D to F described later.

Common operation D: Purification of antibody-drug conjugate

An NAP -25 column was equilibrated with acetic acid buffer solution (10 mM, pH 5.5; herein, referred to as ABS) containing commercially available sorbitol (5%). To this NAP-25 column, an aqueous reaction solution of an antibody-drug conjugate (about 1.5 to 2.5 mL) was applied, and eluted with a buffer in an amount specified by the manufacturer to separate and collect an antibody fraction. The fraction separated and collected was again applied to the NAP-25 column, and a gel filtration purification operation to elute with a buffer was repeated twice or three times in total to afford the antibody-drug conjugate with an unbound drug-linker, dimethyl sulfoxide, and propylene glycol removed. As necessary, the concentration of the solution of the antibody-drug conjugate was adjusted through common operations A to C.

Common operation E: Measurement of antibody concentration of antibody-drug conjugate

The concentration of the conjugated drug in an antibody-drug conjugate can be calculated by using the Lambert-Beer's law shown below.
Expression (I) using the Lambert-Beer's law is as follows.
[Expression 1]

$$\underline{A_{280}} = \underline{\varepsilon_{280}(L\cdot mol^{-1}\cdot cm^{-1})} \cdot \underline{C(mol\cdot L^{-1})} \cdot \underline{l(cm)} \qquad \text{Expression(I)}$$

$$\text{Absorbance} = \text{Molar absorption coefficient} \times \text{Molarity} \times \text{Optical path length}$$

Here, A280 denotes absorbance of an aqueous solution of an antibody-drug conjugate at 280 nm, ε280 denotes the molar absorption coefficient of an antibody-drug conjugate at 280 nm, and C ($mol\cdot L^{-1}$) denotes the molarity of an antibody-drug conjugate. From expression (I), the molarity of an antibody-drug conjugate, C ($mol\cdot L^{-1}$), can be determined by using expression (II) below.
[Expression 2]

$$C(mol\cdot L^{-1}) = \frac{A_{280}}{\varepsilon_{280}(L\cdot mol^{-1}\cdot cm^{-1})\cdot l(cm)} \qquad \text{Expression (II)}$$

Further, the both sides are multiplied by the molar mass of the antibody-drug conjugate, MW ($g\cdot mol^{-1}$), to determine the weight concentration of the antibody-drug conjugate, C' ($mg\cdot mL^{-1}$) (expression (III)).
[Expression 3]

$$C'(mg\cdot mL^{-1}) = MW(g\cdot mol^{-1})\cdot C(mol\cdot L^{-1}) = \frac{A_{280}\cdot MW(g\cdot mol^{-1})}{\varepsilon_{280}(L\cdot mol^{-1}\cdot cm^{-1})\cdot l(cm)} \qquad \text{Expression(III)}$$

Values used for the expression and applied to Examples will be described.
The absorbance A280 used was a measured value of UV absorbance of an aqueous solution of an antibody-drug conjugate at 280 nm. For molar mass, MW ($g\cdot mol^{-1}$), an estimated value of the molecular weight of an antibody was calculated from the amino acid sequence of the antibody, and used as an approximate value of the molar mass of an antibody-drug conjugate. The optical path length, 1 (cm), used in measurement was 1 cm.
The molar absorption coefficient, ε280, of the antibody-drug conjugate can be determined by using expression (IV) below.
[Expression 4]

$$\varepsilon_{280} = \underset{\text{Coefficient of antibody}}{\text{Molar absorption}}\ \varepsilon_{Ab,\,280} + \underset{\text{Coefficient of drug}}{\text{Molar absorption}}\ \varepsilon_{DL,\,280} \times \underset{\text{drug molecules}}{\text{Number of conjugated}} \qquad \text{Expression (IV)}$$

Here, $\varepsilon_{Ab,\,280}$ denotes the molar absorption coefficient of an antibody at 280 nm, and $\varepsilon_{DL,\,280}$ denotes the molar

absorption coefficient of a drug at 280 nm.

By using a known calculation method (Protein Science, 1995, vol. 4, 2411-2423), $\varepsilon_{Ab, 280}$ can be estimated from the amino acid sequence of an antibody. In Examples, the molar absorption coefficient of trastuzumab used was $\varepsilon_{Ab, 280}$ = 215400 (calculated estimated value). The molar absorption coefficient of the CLDN6 antibody used was $\varepsilon_{Ab, 280}$ = 221340 (calculated estimated value), the molar absorption coefficient of the TROP2 antibody used was $\varepsilon_{Ab, 280}$ = 226400 (calculated estimated value), the molar absorption coefficient of the CD98 antibody used was $\varepsilon_{Ab, 280}$ = 240400 (calculated estimated value), the molar absorption coefficient of the LPS antibody used was $\varepsilon_{Ab, 280}$ = 230300 (calculated estimated value), and the molar absorption coefficient of the trastuzumab variant used was $\varepsilon_{Ab, 280}$ = 215057 (calculated estimated value).

$\varepsilon_{DL, 280}$ was calculated for use from a measured value obtained in each UV measurement. Specifically, the absorbance of a solution dissolving a conjugate precursor (drug) with a certain molarity was measured, and expression (I), the Lambert-Beer's law, was applied thereto, and the resulting value was used.

Common operation F: Measurement of average number of conjugated drug molecules per antibody molecule in antibody-drug conjugate

The average number of conjugated drug molecules per antibody molecule in an antibody-drug conjugate can be determined through high-performance liquid chromatography (HPLC) with the following method.

[F-1. Preparation of sample for HPLC analysis (reduction of antibody-drug conjugate)]

A solution of an antibody-drug conjugate (about 1 mg/mL, 60 μL) is mixed with an aqueous solution of dithiothreitol (DTT) (100 mM, 15 μL). The mixture is incubated at 37°C for 30 minutes to prepare a sample in which the disulfide bond between the L chain and H chain of the antibody-drug conjugate cleaved, and this sample is used for HPLC analysis.

[F-2. HLPC analysis]

HPLC analysis is carried out under the following conditions.

HPLC system: Agilent 1290 HPLC system (Agilent Technologies)
Detector: Ultraviolet absorption spectrometer (measurement wavelength: 280 nm, 329 nm)
Column: BEH Phenyl (2.1 × 50 mm, 1.7 μm, Waters Acquity)
Column temperature: 75°C
Mobile phase A: 0.1% trifluoroacetic acid (TFA)-15% isopropyl alcohol aqueous solution
Mobile phase B: 0.075% TFA-15% isopropyl alcohol acetonitrile solution
Gradient program: 14%-36% (0 min to 15 min), 36%-80% (15 min to 17 min), 80%-14% (17 min to 17.1 min), 14%-14% (17.1 min to 23 min)
Sample injection volume: 5μL

[F-3. Data analysis]

[F-3-1] An H chain with a conjugated drug molecule(s) (H chain with one conjugated drug molecule: $H_1$, H chain with two conjugated drug molecules: $H_2$) have hydrophobicity increased in proportion to the number of conjugated drug molecules and have longer retention time as compared to H chain (Ho) of an antibody without any conjugated drug molecule, and hence Lo, $H_0$, $H_1$, and $H_2$, are eluted in the presented order. Through comparison of retention time, each peak detected can be assigned to Lo, $H_0$, $H_1$, or $H_2$. In addition, conjugation of the drug can be confirmed via absorption at a wavelength of 329 nm, which is characteristic to the drug.

[F-3-2] Since each drug-linker absorbs UV, peak area values are corrected by using the following expression with the molar absorption coefficients of an L chain, H chain, and drug-linker according to the number of conjugated drug-linker molecules.

[Expression 5]

$$\text{Corrected H chain peak area (Hi)} = \text{Peak area} \times \frac{\text{Molar absorption coefficient of H chain}}{\text{Molar absorption coefficient of H chain} + \text{Number of conjugated drug molecules} \times \text{Molar absorption coefficient of drug-linker}}$$

Here, for the molar absorption coefficients (280 nm) of the L chain and H chain of each antibody, values estimated from the amino acid sequences of the L chain and H chain of the antibody by using a known calculation method (Protein Science, 1995, vol. 4, 2411-2423) may be used. In the case of trastuzumab, 81290 was used as the molar absorption coefficient of the H chain estimated from the amino acid sequence. In the case of the CLDN6 antibody, similarly, 77280 was used as the molar absorption coefficient of the H chain; in the case of the TROP2 antibody, 68990 was used as the molar absorption coefficient of the H chain; in the case of the CD98 antibody, 78500 was used as the molar absorption coefficient of the H chain; in the case of the LPS antibody, 77470 was used as the molar absorption coefficient of the H chain; in the case of the trastuzumab variant, 81488 was used as the molar absorption coefficient of the H chain; and the molar absorption coefficient (280 nm) measured for compound (1), as a conjugate precursor, was used as the molar absorption coefficient (280 nm) of each drug-linker.

[F-3-3] The peak area ratio (%) of each chain to the total of corrected peak areas is calculated by using the following expression.

[Expression 6]

$$\text{H chain peak area ratio} = \frac{A_{Hi}}{A_{H0} + A_{H1} + A_{H2}} \times 100$$

$A_{Hi}$ : *Hi Respective corrected peak areas*

[F-3-4] The average number of conjugated drug molecules per antibody molecule in an antibody-drug conjugate is calculated by using the following expression.

[Expression 7]

$$\text{Average number of conjugated drug molecules} = (\text{H}_0 \text{ peak area ratio} \times 0 + \text{H}_1 \text{ peak area ratio} \times 1 + \text{H}_2 \text{ peak area ratio} \times 2) / 100 \times 2$$

<Medicine>

The antibody-drug conjugate of the present invention exhibits cellular cytotoxic activity to cancer cells, and hence may be used as a medicine, in particular, a therapeutic agent and/or prophylactic agent for cancer.

Examples of the type of cancer to which the anti-CLDN6 antibody-drug conjugate of the present invention is applied may include lung cancer (e.g., non-small cell lung cancer, small cell lung cancer), kidney cancer, urothelial cancer, colorectal cancer, prostate cancer, glioblastoma multiforme, ovarian cancer (e.g., surface epithelial tumor, stromal tumor, germ cell tumor), pancreatic cancer, breast cancer, melanoma, liver cancer, bladder cancer, gastric cancer, esophageal cancer, endometrial cancer, testicular cancer (seminoma, non-seminoma), uterine cervix cancer, placental choriocarcinoma, brain tumor, and head-and-neck cancer, and metastatic forms of them; examples of the type of cancer to which the anti-HER2 antibody-drug conjugate is applied may include lung cancer, urothelial cancer, colorectal cancer, prostate cancer, ovarian cancer, pancreatic cancer, breast cancer, bladder cancer, gastric cancer, gastric and intestinal stromal tumor, uterine cervix cancer, esophageal cancer, squamous cell carcinoma, peritoneal cancer, liver cancer, hepatocellular cancer, colon cancer, rectal cancer, colon and rectal cancer, endometrial cancer, uterine cancer, salivary gland cancer, kidney cancer, vulvar cancer, thyroid cancer, and penis cancer, and metastatic forms of them; however, there is no limitation thereto as long as cancer cells to be treated are expressing a protein recognizable to the antibody in the antibody-drug conjugate.

The antibody-drug conjugate of the present invention can be preferably administered to mammals, and are

more preferably administered to humans.

Substances used in a pharmaceutical composition containing the antibody-drug conjugate of the present invention may be suitably selected and applied from formulation additives or the like that are generally used in the field in view of the dose or concentration for administration.

The antibody-drug conjugate of the present invention may be administered as a pharmaceutical composition containing one or more pharmaceutically applicable components. For example, the pharmaceutical composition typically contains one or more pharmaceutical carriers (e.g., sterilized liquid (including water and oil (petroleum oil and oil of animal origin, plant origin, or synthetic origin (such as peanut oil, soybean oil, mineral oil, and sesame oil)))). Water is a more typical carrier when the pharmaceutical composition above is intravenously administered. Saline solution, an aqueous dextrose solution, and an aqueous glycerol solution can be also used as a liquid carrier, in particular, for an injection solution. Suitable pharmaceutical vehicles are known in the art. If desired, the composition above may also contain a trace amount of a moisturizing agent, an emulsifying agent, or a pH buffering agent. Examples of suitable pharmaceutical carriers are disclosed in "Remington's Pharmaceutical Sciences" by E. W. Martin. The formulations correspond to the administration mode.

Various delivery systems are known and they may be used for administering the antibody-drug conjugate of the present invention. Examples of the administration route may include, but not limited to, intradermal, intramuscular, intraperitoneal, intravenous, and subcutaneous routes. The administration may be made by injection or bolus injection, for example. According to a specific preferred embodiment, the administration of the above ligand-drug conjugate form is done by injection.

Parenteral administration is a preferred administration route.

According to a representative embodiment, the pharmaceutical composition is prescribed, as a pharmaceutical composition suitable for intravenous administration to humans, according to conventional procedures. The composition for intravenous administration is typically a solution in a sterile and isotonic aqueous buffer. If necessary, the medicine may contain a solubilizing agent and a local anesthetic to alleviate pain at an injection site (e.g., lignocaine). Generally, the ingredients above are provided either individually as a dried lyophilized powder or an anhydrous concentrate contained in each container which is obtained by sealing in an ampoule or a sachet with indication of the amount of the active agent, or as a mixture in a unit dosage form. When the pharmaceutical composition is to be administered by injection, it may be administered from an injection bottle containing water or saline of sterile pharmaceutical grade. When the medicine is administered by injection, an ampoule of sterile water or saline for injection may be provided so that the aforementioned ingredients are admixed with each other before administration.

The pharmaceutical composition of the present invention may be a pharmaceutical composition containing only the antibody-drug conjugate of the present invention, or a pharmaceutical composition containing the antibody-drug conjugate and at least one cancer treating agent other than the antibody-drug conjugate. The antibody-drug conjugate of the present invention may be administered in combination with other cancer treating agents, and thereby the anti-cancer effect may be enhanced. Other anti-cancer agents used for such purpose may be administered to an individual simultaneously with, separately from, or subsequently to the antibody-drug conjugate, and may be administered while varying the administration interval for each. Examples of such cancer treating agents may include abraxane, carboplatin, cisplatin, gemcitabine, irinotecan (CPT-11), paclitaxel, pemetrexed, sorafenib, vinblastin, agents described in International Publication No. WO 2003/038043, LH-RH analogues (e.g., leuprorelin, goserelin), estramustine phosphate, estrogen antagonists (e.g., tamoxifen, raloxifene), and aromatase inhibitors (e.g., anastrozole, letrozole, exemestane), but are not limited thereto as long as they are agents having an antitumor activity.

The pharmaceutical composition can be formulated into a lyophilization formulation or a liquid formulation as a formulation having the selected composition and required purity. When formulated as a lyophilization formulation, it may be a formulation containing suitable formulation additives that are used in the art. Also for a liquid formulation, it may be formulated as a liquid formulation containing various formulation additives that are used in the art.

The composition and concentration of the pharmaceutical composition may vary depending on the administration method. However, the antibody-drug conjugate contained in the pharmaceutical composition of the present invention can exhibit a pharmaceutical effect even at a small dosage when the antibody-drug conjugate has a higher affinity for an antigen, that is, a higher affinity (lower Kd value) in terms of the dissociation constant (Kd value) for the antigen. Thus, for determining the dosage of the antibody-drug conjugate, the dosage may be set in view of the situation relating to the affinity of the antibody-drug conjugate with the antigen. When the antibody-drug conjugate of the present invention is administered to a human, for example, about 0.001 to 100 mg/kg can be administered once or administered in several portions with intervals of 1 to 180 days.

The antibody of the present invention or a functional fragment of the antibody may be used as a medicine. In this case, the above description of "antibody-drug conjugate" in the above chapter <Medicine> may be appropriately read as a description of the "antibody or functional fragment of the antibody."

Further, the free drug of the present invention (novel PBD derivative compound), a salt of the free drug, and hydrates of them may be used as a medicine. In this case, the above description of "antibody-drug conjugate" in the

above chapter <Medicine> may be appropriately read as a description of the "free drug (novel PBD derivative compound), a salt of the free drug, and hydrates of them."

Examples

The present invention will be specifically described with reference to Examples shown below; however, the present invention is not limited to Examples. Examples should not be interpreted as limitation in any sense. Reagents, solvents, and starting materials without any description herein can be readily obtained from commercially available sources of supply.

Reference Example 1: Trastuzumab tesirine

Step 1: Conjugation of antibody and drug-linker

To a 5 mM solution of trastuzumab (Reference Example 3) in ethylenediamine tetraacetate-phosphate buffered saline (pH 6.5) (9.91 mg/mL, 0.70 mL), an aqueous solution of dipotassium phosphate (1.0 M, 0.0112 mL) and an aqueous solution of tris(2-carboxyethyl)phosphine hydrochloride (10 mM, 0.0086 mL) were added at 20°C, and reacted at 20°C for 60 minutes and then at room temperature for 30 minutes. A solution of tesirine (0.36 mg) synthesized with reference to a literature (Med. Chem. Lett. 2016, 7, 983-987) in dimethylacetamide (0.0415 mL) was added to the reaction solution, and reacted at room temperature for 1 hour. An aqueous solution of N-acetylcysteine (100 mM, 0.0024 mL) was added to the reaction solution, and reacted for 30 minutes to terminate the reaction.

Purification operation: The solution was purified by using common operation D to afford 3.5 mL of a solution of the targeted compound.
Characterization: The following characteristic values were obtained by using common operations E and F.
Antibody concentration: 1.40 mg/mL, antibody yield: 4.90 mg (71%), average number of conjugated drug molecules per antibody molecule (n): 2.0

Reference Example 2: Anti-CLDN6 (H1L1) antibody tesirine

Step 1: Conjugation of antibody and drug-linker

To a 5 mM solution of an anti-CLDN6 (H1L1) antibody in ethylenediamine tetraacetate-phosphate buffered saline (pH 6.5) (9.87 mg/mL, 0.45 mL), an aqueous solution of dipotassium phosphate (1.0 M, 0.0072 mL) and an aqueous solution of tris(2-carboxyethyl)phosphine hydrochloride (10 mM, 0.0041 mL) were added at 20°C, and reacted at 20°C for 90 minutes. A solution of tesirine (0.15 mg) synthesized with reference to a literature (Med. Chem. Lett. 2016, 7, 983-987) in N,N-dimethylacetamide (0.0277 mL) was added to the reaction solution, and reacted at 20°C for 1 hour. An aqueous solution of N-acetylcysteine (100 mM, 0.001 mL) was added to the reaction solution, and reacted for 30 minutes to terminate the reaction.

Purification operation: The solution was purified by using common operation D to afford 3.5 mL of a solution of the targeted compound.
Characterization: The following characteristic values were obtained by using common operations E and F.
Antibody concentration: 1.56 mg/mL, antibody yield: 3.90 mg (88%), average number of conjugated drug molecules per antibody molecule (n): 2.1

Reference Example 3: Anti-HER2 antibody trastuzumab

The anti-HER2 antibody was produced with reference to US 5821337. The amino acid sequences of the light chain and heavy chain of trastuzumab are represented by SEQ ID NO: 64 and SEQ ID NO: 65, respectively.

Reference Example 4: Anti-LPS antibody h#1G5-H1L1

The anti-LPS antibody was produced with reference to WO 2015/046505. The amino acid sequences of the light chain and heavy chain of h#1G5-H1L1 were represented by SEQ ID NO: 66 and SEQ ID NO: 67, respectively.

Reference Example 5: Anti-TROP2 antibody hRS7

The anti-TROP2 antibody was produced with reference to WO 2003/074566 and WO 2015/098099 (Reference Example 1). The amino acid sequences of the light chain and heavy chain of hRS7 are represented by SEQ ID NO: 68 and SEQ ID NO: 69, respectively.

Reference Example 6: Anti-CD98 antibody hM23-H1L1

The anti-CD98 antibody was produced with reference to WO 2015/146132. The amino acid sequences of the light chain and heavy chain of hM23-H1L1 were represented by SEQ ID NO: 70 and SEQ ID NO: 71, respectively.

[Synthesis of production intermediate (Drug-linker)]

Example 1

[Example 1-1: Intermediate 1]

[Formula 55]

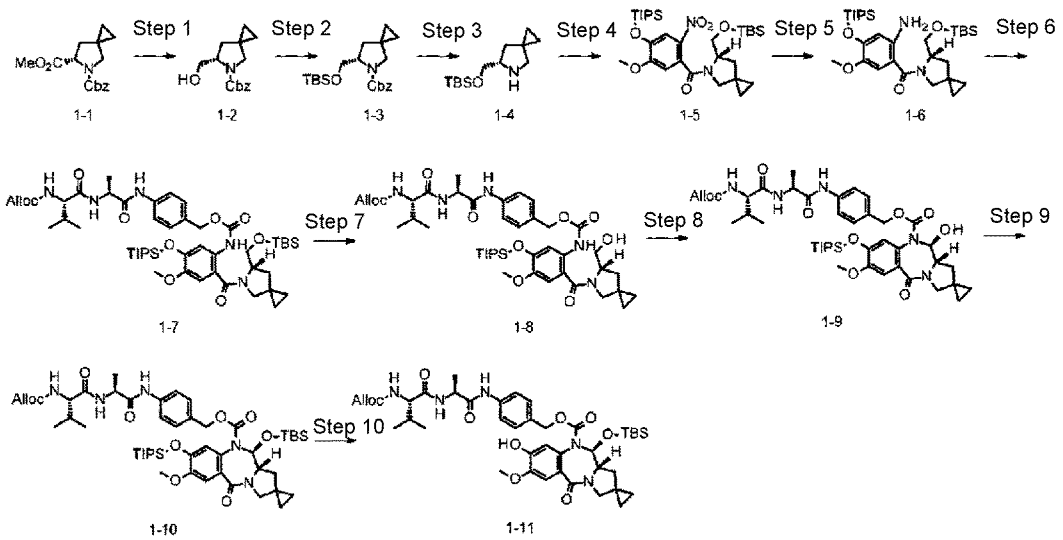


Step 1: Benzyl (6S)-6-(hydroxymethyl)-5-azaspiro[2.4]heptane-5-carboxylate (1-2)

To a solution of 5-benzyl 6-methyl (6S)-5-azaspiro[2.4]heptane-5,6-dicarboxylate (1-1) (104 mmol, WO 2012087596) in tetrahydrofuran (500 mL), lithium borohydride (4.30 g, 178 mmol) was added in small portions at 0°C. The resultant was stirred at 0°C for 30 minutes, and then stirred at room temperature for 2 hours. Water (180 mL) and 2 N hydrochloric acid (186 mL) were added at 0°C, and the resultant was distillated under reduced pressure. The resulting residue was extracted with ethyl acetate four times, and the organic layer was washed with brine and then dried over anhydrous sodium sulfate. The resultant was distillated under reduced pressure, and the resulting residue (1-2) (27.9 g, 90%) was directly used for the subsequent reaction.

Step 2: Benzyl (6S)-6-({[tert-butyl(dimethyl)silyl]oxy}methyl)-5-azaspiro[2.4]heptane-5-carboxylate (1-3)

To a solution of compound (1-2) obtained in Step 1 (27.9 g, 107 mmol) and imidazole (14.5 g, 214 mmol) in dichloromethane (300 mL), tert-butyldimethylsilyl chloride (24.2 g, 160 mmol) was added at room temperature, and the resultant was stirred at room temperature for 18 hours. The reaction solution was washed with a saturated aqueous citric acid, a saturated aqueous sodium hydrogen carbonate, and brine, dried over anhydrous sodium sulfate, and then distillated under reduced pressure. The resulting residue was purified by silica gel column chromatography [hexane:ethyl acetate = 100:0 (v/v) to 50:50 (v/v)] to afford the desired compound (1-3) (32.5 g, 81%).
$^{1}H$-

NMR($CDCl_3$)δ:7.39-7.34(5H,m),5.23-5.11(2H,m),4.10-3.48(4H,m),3.16-3.14(1H,m),2.15-2.04(1H,m),1.81-1.77(1H,m), 0.91-0.88(9H,m),0.65-0.55(4H,m),0.08-0.01(6H,m).

MS(APCI)m/z:376$(M+H)^+$

Step 3: (6S)-6-({[tert-Butyl(dimethyl)silyl]oxy}methyl)-5-azaspiro[2.4]heptane (1-4)

To a solution of compound (1-3) obtained in Step 2 (32.5 g, 86.5 mmol) in ethanol (400 mL), 7.5% palladium carbon catalyst (moisture content: 54%, 5.00 g) was added at room temperature, and the resultant was stirred under the hydrogen atmosphere at room temperature for 6 hours. The reaction solution was filtered through a Celite, and the filtrate was distilled under reduced pressure to afford the desired compound (1-4) (21.3 g, quantitative).
$^1H$-NMR($CDCl_3$)δ:3.79-3.77(1H,m),3.71-3.69(1H,m),3.65-3.60(1H,m),3.01-2.98(2H,m),1.81-1.71(2H,m),0.90(9H,s),0.65-0.57(4H,m),0.08(3H,s),0.07(3H,s). MS(APCI, ESI)m/z:242$(M+H)^+$

Step 4: [(6S)-6-({[tert-Butyl(dimethyl)silyl]oxy}methyl)-5-azaspiro[2.4]hept-5-yl] (5-methoxy-2-nitro-4-{[tri(propan-2-yl)silyl]oxy}phenyl)methanone (1-5)

To a solution of 5-methoxy-2-nitro-4-{tri(propan-2-yl)silyl]oxy}benzoic acid (52.2 g, 141 mmol, US 20150283262) and 1-hydroxybenzotriazole monohydrate (23.8 g, 155 mmol) in dichloromethane (500 mL), N,N'-dicyclohexylcarbodiimide (35.0 g, 170 mmol) was added under ice-cooling. The reaction mixture was stirred at room temperature. After the carboxylic acid disappeared, a solution of compound (1-4) obtained in Step 3 (34.1 g, 141 mmol) and triethylamine (29.4 mL, 212 mmol) in dichloromethane (100 mL) was slowly added dropwise thereto. After the reaction solution was stirred at room temperature overnight, saturated aqueous sodium hydrogen carbonate was added to the reaction mixture, and the reaction mixture was extracted with chloroform. The organic layer was washed with water and brine, and dried over anhydrous magnesium sulfate. The resultant was distilled under reduced pressure, and to the resulting residue ethyl acetate and diethyl ether were added, and the solid contents were removed through filtration, and the filtrate was distillated under reduced pressure, and the resulting residue was purified by silica gel column chromatography [hexane:ethyl acetate = 100:0 (v/v) to 25:75 (v/v)] to afford the desired compound (1-5) (55.0 g, 66%).
$^1H$-NMR($CDCl_3$)δ:7.72-7.66(1H,m),6.80-6.73(1H,m),4.53-4.49(1H,m),4.04-3.95(1H,m),3.91-3.88(3H,m),3.59-3.54(1H,m), 3.36-3.25(0.5H,m),3.01-2.96(1,5H,m),2.24-2.20(0.3H,m),2.09-2.05(0.7H,m),2.00-1,97(0.7H,m),1.69-1.67(0.3H,m),1.32-1.24(3H,m),1.12-1.05(18H,m),0.93-0.91(6H,m),0.79-0.77(3H,m),0.71-0.62(2H,m),0.57-0.40(2H,m),0.12-0.10(4H,m),0.11-0.15(2H,m). MS(APCI, ESI)m/z:593$(M+H)^+$

Step 5: (2-Amino-5-methoxy-4-{[tri(propan-2-yl)silyl]oxy}phenyl)[(6S)-6-({[tert-butyl(dimethyl)silyl]oxy}methyl)-5-azaspiro[2.4]hept-5-yl]methanone (1-6)

To a solution of compound (1-5) obtained in Step 4 (55.0 g, 92.8 mmol) in ethanol (300 mL), 7.5% palladium carbon (10.0 g) was added under the nitrogen atmosphere. The nitrogen balloon was immediately replaced with a hydrogen balloon, and the reaction mixture was vigorously stirred under the hydrogen atmosphere at room temperature. After the raw materials disappeared, the reaction mixture was filtered, and the filtrate was distillated under reduced pressure to afford the desired compound (1-6) (52.2 g, 100%), which was directly used for the subsequent reaction.
$^1H$-NMR($CDCl_3$)5:6.71(1H,s),6.25(1H,s),4.55-4.28(2H,m),3.97(1H,m),3.75-3.62(3H,m),3.70(3H,s),3.09-3.07(1H,m),2.24-2.19(1H,m),1.81-1.68(1H,m),1.27-1.22(3H,m),1.09-1.05(18H,m),0.90(9H,s),0.65-0.46(4H,m),0.07-0.03(6H,m).
MS(APCI, ESI)m/z:563$(M+H)^+$

Step 6: N-[(Prop-2-en-1-yloxy)carbonyl]-L-valyl-N-[4-({[(2-{[(6S)-6-({[tert-butyl(dimethyl)silyl]oxy}methyl)-5-azaspiro[2.4]hept-5-yl]carbonyl}-4-methoxy-5-{[tri(propan-2-yl)silyl]oxy}phenyl)carbamoyl]oxy}methyl)phenyl]-L-alaninamide (1-7)

To a solution of compound (1-6) obtained in Step 5 (18.6 g, 33.0 mmol) and triethylamine (6.26 mL, 45.2 mmol) in THF (300 mL), triphosgene (4.22 g, 14.2 mmol) was slowly added on an ethanol-ice bath. After the addition, a mixed solution of N-[(prop-2-en-1-yloxy)carbonyl]-L-valyl-N-[4-(hydroxymethyl)phenyl]-L-alaninamide (11.4 g, 30.2 mmol, WO 2011130598) and triethylamine (6.26 mL, 45.2 mmol) in tetrahydrofuran (100 mL) and N,N-dimethylformamide (30 mL) was slowly added dropwise to the ice-cooled reaction mixture. After the dropwise addition, the ice bath was removed, and the reaction mixture was stirred under the nitrogen atmosphere at 40°C. After the raw materials disappeared, water was added to the reaction mixture, and the reaction mixture was extracted with ethyl acetate. The organic layer was

washed with brine, and dried over anhydrous sodium sulfate. After filtration followed by distillation under reduced pressure, the resulting residue was purified by silica gel column chromatography [hexane:ethyl acetate = 100:0 (v/v) to 40:60 (v/v)] to afford the desired compound (1-7) (23.5 g, 74%).

$^1$H-NMR($CDCl_3$)δ:8.99(1H,m),8.58(1H,s),7.80(1H,s),7.55-7.53(2H,m),7.34-7.32(2H,m),6.77-6.75(2H,m),5.94-5.87(1H,m),5.40-5.38(1H,m),5.33-5.29(1H,m),5.23-5.21(1H,m),5.13(1H,m),5.10(2H,m),4.69-4.64(1H,m),4.62-4.52(2H,m),4.06-4.03(1H,m),3.98(1H,m),3.76-3.65(6H,m),3.04(1H,m),2.28-2.26(1H,m),2.18-2.13(1H,m),1.46(3H,m),1.32-1.25(3H,m),1.11-1.09(18H,m),0.99-0.84(15H,m),0.65-0.40(4H,m),0.08-0.00(6H,m).

MS(APCI, ESI)m/z:966(M+H)$^+$

Step 7: N-[(Prop-2-en-1-yloxy)carbonyl]-L-valyl-N-[4-({[(2-{[(6S)-6-(hydroxymethyl)-5-azaspiro[2.4]hept-5-yl]carbonyl}-4-methoxy-5-{[tri(propan-2-yl)silyl]oxy}phenyl)carbamoyl]oxy}methyl)phenyl]-L-alaninamide (1-8)

To a solution of compound (1-7) obtained in Step 6 (23.5 g, 24.3 mmol) in tetrahydrofuran (50 mL), methanol (50 mL) and water (44 mL), acetic acid (200 mL) was added at room temperature. The reaction mixture was stirred at room temperature. After the raw materials disappeared, the reaction mixture was extracted with ethyl acetate. The organic layer was washed with water and brine, and dried over anhydrous sodium sulfate. After filtration followed by distillation under reduced pressure, the resulting residue was purified by silica gel column chromatography [hexane:ethyl acetate = 100:0 (v/v) to 0:100 (v/v)] to afford the desired compound (1-8) (18.0 g, 87%).

$^1$H-NMR($CDCl_3$)δ:8.64-8.62(1H,m),8.50(1H,m),7.69(1H,m),7.55-7.53(2H,m),7.34-7.32(2H,m),6.79-6.75(3H,m),5.91-5.89(1H,m),5.39(1H,m),5.32-5.29(1H,m),5.23-5.21(1H,m),4.68-4.54(4H,m),4.31(1H,m),4.06-4.04(1H,m),3.81-3.79(3H,m),3.76(3H,s),3.63-3.61(1H,m),3.13-3.11(1H,m),2.16-2.13(1H,m),1.87-1.81(2H,m),1.46-1.43(3H,m),1.30-1.24(3H,m),1.12-1.08(18H,m),0.98-0.91(6H,m),0.63-0.45(4H,m).

MS(APCI, ESI)m/z:852(M+H)$^+$

Step 8: N-[(Prop-2-en-1-yloxy)carbonyl]-L-valyl-N-{4-[({[(11'S,11a'S)-11'-hydroxy-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (1-9)

To a solution of dimethyl sulfoxide (3.75 mL, 52.8 mmol) in dichloromethane (300 mL), oxalyl chloride (2.17 mL, 25.3 mmol) was slowly added dropwise under the nitrogen atmosphere at -78°C. After the dropwise addition, the reaction mixture was stirred at -78°C. A solution of compound (1-8) obtained in Step 7 (18.0 g, 21.1 mmol) in dichloromethane (50.0 mL) was slowly added to the reaction mixture. Triethylamine (14.6 mL, 105 mmol) was added to the reaction solution at -78°C. After the addition, the refrigerant bath was removed, and the temperature was slowly raised to room temperature. After the raw materials disappeared, water was added to the reaction mixture, and the reaction mixture was extracted with chloroform (200 mL). The organic layer was washed with water and brine, and dried over anhydrous magnesium sulfate. After filtration followed by distillation under reduced pressure, the resulting residue was purified by silica gel column chromatography [hexane:ethyl acetate = 100:0 (v/v) to 0:60 (v/v)] to afford the desired compound (1-9) (16.5 g, 92%). $^1$H-NMR($CDCl_3$)δ:8.51-8.36(1H,m),7.54-7.38(2H,m),7.22-7.07(3H,m),6.73-6.64(1H,m),5.94-5.87(2H,m),5.33-5.22(3H,m),5.09(1H,m),4.97(1H,m),4.64-4.58(4H,m),4.02-4.00(1H,m),3.86-3.83(3H,m),3.75-3.70(1H,m),3.61-3.54(2H,m),3.38-3.29(1H,m),2.40(1H,m),2.16-2.14(1H,m),1.74-1.71(1H,m),1.44(3H,m),1.18-1.16(3H,m),1.05-1.00(18H,m),0.97-0.92(6H,m),0.72-0.60(4H,m).

MS(APCI, ESI)m/z:850(M+H)$^+$

Step 9: N-[(Prop-2-en-1-yloxy)carbonyl]-L-valyl-N-{4-[({[(11'S,11a'S)-11'-{[tert-butyl(dimethyl)silyl]oxy}-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (1-10)

To a solution of compound (1-9) obtained in Step 8 (12.0 g, 14.1 mmol) and 2,6-lutidine (6.58 mL, 56.5 mmol) in dichloromethane (200 mL), tert-butyldimethylsilyl trifluoromethylsulfonate (9.73 mL, 42.3 mmol) was slowly added dropwise under the nitrogen atmosphere at 0°C. After stirring under ice-cooling for 10 minutes, the ice bath was removed, and stirring was performed at room temperature. After the raw materials disappeared, water was added to the reaction mixture, and the reaction mixture was extracted with chloroform, washed with water and brine, and dried over anhydrous sodium sulfate. After filtration followed by distillation under reduced pressure, the resulting residue was purified by silica gel column chromatography [hexane:ethyl acetate = 100:0(v/v) to 25:75(v/v)] to afford the desired compound (1-10) (8.12 g, 60%).

$^1$H-NMR($CDCl_3$)δ:8.67-8.45(1H,m),7.50-7.44(2H,m),7.19(1H,s),7.13(2H,m),6.95(2H,m),6.62-6.57(2H,m),6.01(1H,m),5.95-5.86(1H,m),5.33-5.13(3H,m),4.82(1H,m),4.65-4.54(3H,m),4.03-4.01(1H,m),3.84-3.82(3H,m),3.73-3.66(1H,m),3.50-3.48(1H,m),3.27(1H,m),2.37-2.33(1H,m),2.19-2.13(1H,m),1.54-1.43(3H,m),1.22-1.13(3H,m),1.10-1.00(18H,m),0.97-0.91(6H,m),0.81(9H,s),0.76-0.59(4H,m),0.19--0.09(6H,m).

MS(APCI, ESI)m/z:964(M+H)$^+$

Step 10: N-[(Prop-2-en-1-yloxy)carbonyl]-L-valyl-N-{4-[({[(11'S,11a'S)-11'-{[tert-butyl(dimethyl)silyl]oxy}-8'-hydroxy-7'-methoxy-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (1-11)

To a solution of compound (1-10) obtained in Step 9 (8.12 g, 8.42 mmol) in N,N-dimethylformamide (90 mL) and water (2 mL), lithium acetate (0.611 g, 9.26 mmol) was added, and the resultant was stirred at room temperature. After the raw materials disappeared, water was added to the reaction mixture, and the reaction mixture was extracted with ethyl acetate. The organic layer was washed with water and brine, and dried over anhydrous sodium sulfate. After filtration followed by distillation under reduced pressure, the resulting residue was purified by silica gel column chromatography [hexane:ethyl acetate = 100:0 (v/v) to 0:100 (v/v)] to afford the desired compound (1-11) (5.48 g, 81%). $^1$H-NMR(400MHz, $CDCl_3$, 20.9°C)δ:8.76-8.60(1H,m),7.45-7.44(2H,m),7.21(1H,s),7.10-7.09(2H,m),6.81-6.74(1H,m),6.65(1H,s),6.23(1H,s),6.01-5.99(1H,m),5.95-5.84(1H,m),5.41-5.20(2H,m),5.16(1H,m),4.84(1H,m),4.67-4.54(4H,m),4.05-4.03(1H,m),3.87(3H,s),3.71(1H,m),3.55-3.51(1H,m),3.26(1H,m),2.35(1H,m),2.18-2.12(1H,m),1.55-1.42(4H,m),0.97-0.92(6H,m),0.81(9H,s),0.76-0.61(4H,m),0.20--0.06(6H,m).

MS(APCI, ESI)m/z:808(M+H)$^+$

$^1$H-NMR (500M Hz, $CDCl_3$, 27°C) δ: 8.76 (1H, s), 7.43 (2H, brd), 7.20 (1H, s), 7.08 (2H, d, J=8.3 Hz), 7.00 (1H, br), 6.66 (1H, s), 6.44 (1H, s), 6.00 (1H, H-11', d, J11', 11'a=9.2 Hz), 5.89 (1H, m), 5.53 (1H, brd), 5.30 (1H, d, J=17.2 Hz), 5.20 (1H, d, J=10.3 Hz), 5.15 (1H, d, JABq=12.5 Hz), 4.85 (1H, d, JABq=12.5 Hz), 4.66 (1H, m), 4.60-4.52 (2H, m), 4.07 (1H, m), 3.84 (3H, s), 3.71 (1H, H-3'β, d, Jgem=11.7 Hz), 3.53 (1H, H-11'a, m), 3.26 (1H, H-3'α, d, Jgem=11.7 Hz), 2.35 (1H, H-1'β, dd, J1' β, 11'a=8.30 Hz, Jgem=13.1 Hz), 2.14 (1H, m), 1.54 (1H, H-1' α, d, Jgem=13.1 Hz), 1.41 (3H, d, J=6.90 Hz), 0.95 (3H, d, J=6.80 Hz), 0.92 (3H, d, J=6.80 Hz), 0.81 (9H, s), 0.80-0.70 (1H, m), 0.70-0.59 (3H, m), 0.2-0.06 (6H, m)

The absolute steric configuration at the 11'-position of compound (1-11) was analyzed by correlation obtained from its selective ID ROESY spectrum (a figure below). Correlation was found between 1'α-H and 11'-H, between 3'α-H and 11'-H, and between 1'β-H and 3'β-H, and thus the absolute steric configuration at the 11'-position was revealed to be S-configuration.

[Formula 56]

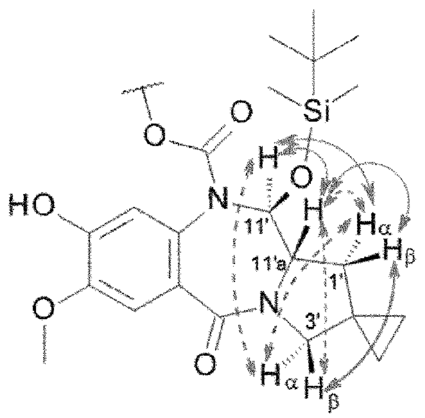

Key ROESY Correlation
(dashed : weak correlation)

Significant correlation obtained from Selective 1D ROESY spectrum

Accordingly, the absolute steric configuration at the 11'-position of each of compound (1-11), compound (1-9) and compound (1-10), each of which had the same absolute steric configuration as compound (1-11), compound (3-11), which was synthesized with compound (1-11), compound (3-12), compound (3-13), and drug-linker 1 (compound (3-14)), compound (4-9), compound (4-10), compound (4-11), and drug-linker 2 (compound (4-12)), and compound (6-10), compound (6-11), compound (6-12), and drug-linker 4 (compound (6-13)) was revealed to be S-configuration. Further, the absolute steric configuration at the 11'-position of each of compound (5-9), compound (5-10), and drug-linker 3 (compound (5-11)), which were obtained by the same synthesis procedure, was determined to be S-configuration.

[Example 1-2: Intermediate 2]

[Formula 57]

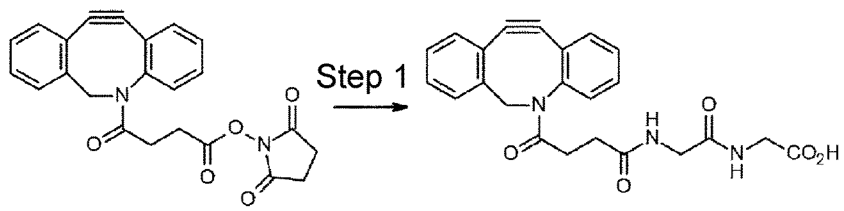


2-1

2-2

Step 1: N-[4-(11,12-Didehydrodibenzo[b,f]azocin-5(6H)-yl))-4-oxobutanoyl]glycylglycine (2-2)

To a solution of glycylglycine (0.328 g, 2.49 mmol) and N,N-diisopropylethylamine (0.433 mL, 2.49 mmol) in N,N-dimethylformamide (20 mL), 1-{[4-(11,12-didehydrodibenzo[b,f]azocin-5(6H)-yl))-4-oxobutanoyl]oxy}pyrrolidine-2,5-dione (2-1) (1.00 g, 2.49 mmol, Click Chemistry Tools) and water (10 mL) were added at room temperature, and the resultant was stirred at the same temperature overnight. The resultant was distillated under reduced pressure, and the resulting residue was purified by silica gel column chromatography [an organic layer for distribution with chloroform to chloroform:methanol:water = 7:3:1(v/v/v)] to afford the desired compound (0.930 g, 89%).
$^1$H-NMR(DMSO-$D_6$)$\delta$:12.58(1H,s),8.14-8.12(1H,m),8.08-8.07(1H,m),7.69-7.68(1H,m),7.62-7.61(1H,m),7.53-7.45(3H,m),7.40-7.29(3H,m),5.05-5.01(1H,m),3.73-3.72(2H,m),3.66-3.60(3H,m),2.66-2.60(1H,m),2.33-2.24(1H,m),2.08-2.04(1H,m),1.81-1.77(1H,m).
MS(APCI, ESI)m/z:420[(M+H)$^+$].

Example 2

[Example 2-1: Drug-linker 1]

[Formula 58]

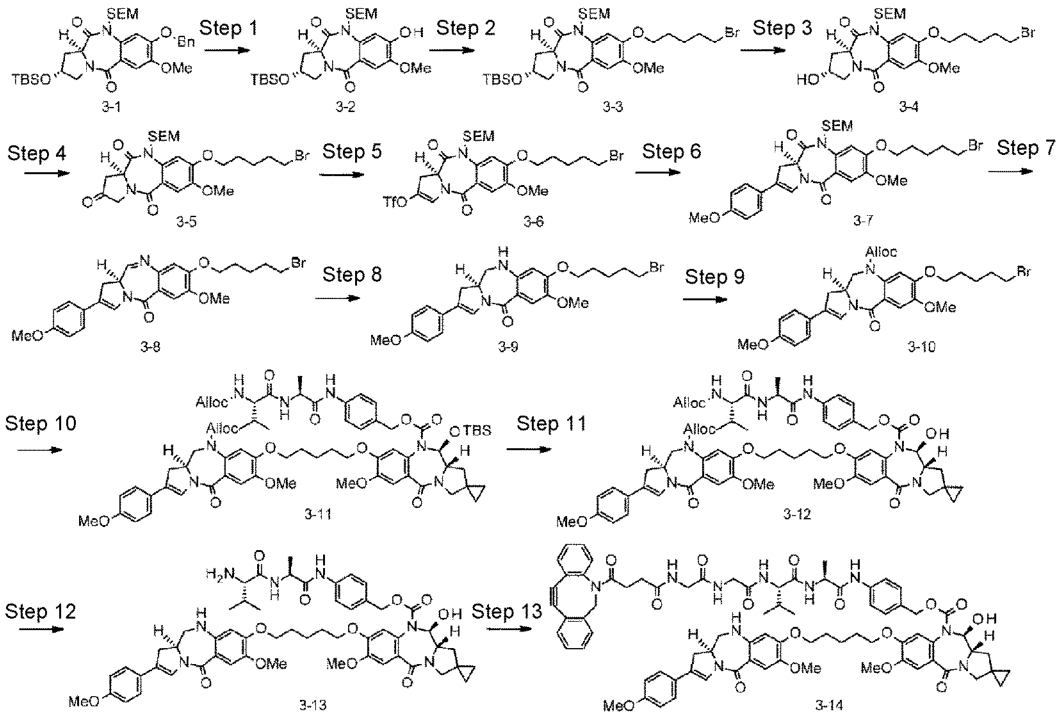


Step 1: (2R,11aS)-2-{[tert-Butyl(dimethyl)silyl]oxy}-8-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione (3-2)

To a solution of (2R,11aS)-8-(benzyloxy)-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (3-1) (25.5 g, 41.6 mmol, WO 2016149546) in tetrahydrofuran (150 mL) and ethanol (150 mL), 5% palladium carbon (moisture content: 54%, 10.0 g) was added under the nitrogen atmosphere, and the reaction solution was then stirred under the hydrogen atmosphere at room temperature for 3 days. Chloroform was added to the reaction solution, which was filtered through a Celite, and the filtrate was then distilled under reduced pressure. The resulting residue was purified by silica gel column chromatography [hexane:ethyl acetate = 100:0 (v/v) to 50:50 (v/v)] to afford the desired compound (3-2) (19.4 g, 89%). $^1$H-NMR($CDCl_3$)δ:7.36(1H,s),7.25(1H,s),6.01(1H,s),5.45-5.43(1H,m),4.69-4.67(1H,m),4.60-4.55(1H,m),4.23-4.21(1H,m),3.96(3H,s),3.76-3.68(2H,m),3.63-3.61(1H,m),3.56-3.53(1H,m),2.88-2.83(1H,m),2.03-2.00(1H,m),1.00-0.98(2H,m),0.87(9H,s),0.10(6H,s),0.02(9H,s).

MS(APCI, ESI)m/z:523$(M+H)^+$

Step 2: (2R,11aS)-8-[(5-Bromopentyl)oxy]-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (3-3)

To a solution of compound (3-2) obtained in Step 1 (10.8 g, 20.7 mmol) in N,N-dimethylformamide (30 mL), 1,5-dibromopentane (23.8 g, 103 mmol) and potassium carbonate (3.43 g, 24.8 mmol) were added at room temperature. After stirring at room temperature for 3 hours, water was added to the reaction solution, which was extracted with ethyl acetate. The organic layer obtained was washed with brine and dried over sodium sulfate, and distilled under reduced pressure. The resulting residue was purified by silica gel column chromatography [hexane:ethyl acetate = 90:10 (v/v) to 50:50 (v/v)] to afford the desired compound (3-3) (14.5 g, quantitative). $^1$H-NMR($CDCl_3$)δ:7.34(1H,s),7.21(1H,s),5.52-5.49(1H,m),4.63-4.62(1H,m),4.58-4.55(1H,m),4.24-4.22(1H,m),4.07-4.04(2H,m),3.92(3H,s),3.82-3.64(3H,m),3.56-3.53(1H,m),3.45-3.43(2H,m),2.86-2.84(1H,m),2.04-2.00(1H,m),1.97-1.87(4H,m),1.66-1.62(2H,m),1.01-0.98(2H,m),0.87(9H,s),0.10(6H,s),0.04(9H,s).
MS(APCI, ESI)m/z:673[81Br,$(M+H)^+$],671[79Br,$(M+H)^+$],

Step 3: (2R,11aS)-8-[(5-Bromopentyl)oxy]-2-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione (3-4)

To a solution of compound (3-3) obtained in Step 2 (21.5 mmol) in tetrahydrofuran (40 mL), a 1 mol/L tetrahydrofuran solution of tetrabutylammonium fluoride (28.0 mL, 28.0 mmol) was added at 0°C. After stirring at room temperature for 30 minutes, water was added to the reaction solution, which was extracted with ethyl acetate, and the organic layer obtained was washed with brine. The resultant was dried over sodium sulfate, and then distillated under reduced pressure. The resulting residue was purified by silica gel column chromatography [chloroform:methanol = 97.5:2.5 (v/v) to 92.5:7.5 (v/v)] to afford the desired compound (3-4) (11.3 g, 94%). $^1$H-NMR($CDCl_3$)5:7.34(1H,s),7.21(1H,s),5.53-5.50(1H,m),4.69-4.64(2H,m),4.32-4.30(1H,m),4.10-4.00(2H,m),3.91(3H,s), 3.88-3.75(2H,m),3.73-3.64(2H,m),3.45-3.44(2H,m),2.99-2.96(1H,m),2.15-2.09(1H,m),1.99-1.85(5H,m),1.68-1.62(2H, m),1.01-0. 95(2H,m),0. 04(9H,s).

MS(APCI, ESI)m/z:559[81Br,$(M+H)^+$],557[79Br,$(M+H)^+$],

Step 4: (11aS)-8-[(5-Bromopentyl)oxy]-7-methoxy-10-{[2-(trimethylsilyl)ethoxy]methyl}-1H-pyrrolo[2,1-c][1,4]benzodiazepin-2,5,11 (3H,10H,11aH)-trione (3-5)

Compound (3-4) obtained in Step 3 (11.3 g, 20.2 mmol), tetrabutylammonium bromide (0.325 g, 1.01 mmol), and potassium bromide (0.240 g, 2.02 mmol) were dissolved in a saturated aqueous sodium hydrogen carbonate (60 mL)/dichloromethane (60 mL), to which nor-AZADO (0.0279 g, 0.202 mmol) and sodium hypochlorite pentahydrate (2.03 g, 27.2 mmol) were added at 0°C, and the resultant was stirred at 0°C for 30 minutes. Because the raw materials remained, sodium hypochlorite pentahydrate (1.00 g, 13.4 mmol) was added thereto at 0°C, and the resultant was stirred at 0°C for 15 minutes. Sodium hypochlorite pentahydrate (0.300 g, 4.03 mmol) was further added thereto at 0°C, and the resultant was stirred at 0°C for 15 minutes, and the disappearance of the raw materials was confirmed by TLC. An aqueous solution of sodium thiosulfate was added to the reaction solution, which was extracted with chloroform, and the organic layer obtained was dried over sodium sulfate. The resultant was distillated under reduced pressure, and the resulting residue was purified by silica gel column chromatography [hexane:ethyl acetate = 75:25(v/v) to 40:60(v/v)] to afford the desired compound (3-5) (9.74 g, 87%).

$^1$H-NMR($CDCl_3$)δ:7.33(1H,s),7.24(1H,s),5.56-5.53(1H,m),4.71-4.69(1H,m),4.66-4.63(1H,m),4.27-4.22(1H,m),4.12-4. 02(2H,m),3.93-3.88(4H,m),3.82-3.75(1H,m),3.69-3.67(1H,m),3.61-3.56(1H,m),3.46-3.44(2H,m),2.82-2.77(1H,m), 1.97-1.89(4H,m),1.68-1,64(2H,m),1.05-0.93(2H,m),0.04(9H,s).
MS(APCI, ESI)m/z:557[81Br,$(M+H)^+$],555[79Br,$(M+H)^+$],

Step 5: (11aS)-8-[(5-Bromopentyl)oxy]-7-methoxy-5,11-dioxo-10-{[2-(trimethylsilyl)ethoxy]methyl}-5,10,11,11a-tetrahydro-1H-pyrrolo[2,1-c][1,4]benzodiazepin-2-yl trifluoromethanesulfonate (3-6)

To a solution of compound (3-5) obtained in Step 4 (9.74 g, 17.5 mmol) in dichloromethane (160 mL), 2,6-lutidine (8.17 mL, 70.1 mmol) was added at -40°C, and the resultant was stirred at -40°C for 10 minutes. Anhydrous trifluoromethanesulfonic acid (8.85 mL, 52.6 mmol) was added to the reaction solution at -40°C, and the resultant was stirred at -40°C for 30 minutes. To the reaction solution, a 10% aqueous solution of citric acid was added, which was extracted with chloroform, and the organic layer obtained was dried over sodium sulfate. The resultant was distillated under reduced pressure, and the resulting residue was purified by silica gel column chromatography [hexane:ethyl acetate = 95:5 (v/v) to 70:35 (v/v)] and then purified by NH2 silica gel chromatography [hexane:ethyl acetate = 95:5 (v/v) to 65:35 (v/v)] to afford the desired compound (3-6) (7.10 g, 59%).

$^1$H-NMR($CDCl_3$)δ:7.32(1H,s),7.24(1H,s),7.15-7.14(1H,m),5.56-5.53(1H,m),4.70-4.68(1H,m),4.66-4.63(1H,m),4.11-4. 01(2H,m),3.94-3.90(4H,m),3.84-3.75(1H,m),3.73-3.68(1H,m),3.46-3.44(2H,m),3.18-3.14(1H,m),1.96-1.88(4H,m), 1.69-1.61(2H,m),1.02-0. 92(2H,m),0. 04(9H,s).
MS(APCI, ESI)m/z:689[81Br,$(M+H)^+$],687[79Br,$(M+H)^+$],

Step 6: (11aS)-8-[(5-Bromopentyl)oxy]-7-methoxy-2-(4-methoxyphenyl)-10-{[2-(trimethylsilyl)ethoxy]methyl}-1H-pyrrolo[2,1-c][1,4]benzodiazepin-5,11(10H,11aH)-dione (3-7)

To a mixture of compound (3-6) obtained in Step 5 (2.00 g, 2.91 mmol), 4-methoxyphenylboronic acid (0.884 g, 5.82 mmol), tetrakis(triphenylphosphine)palladium (0) (0.336 g, 0.291 mmol) and sodium carbonate (1.23 g, 11.6

mmol), toluene (20 mL), ethanol (10 mL) and water (10 mL) were added at room temperature. The reaction solution was stirred at room temperature for 30 minutes, and the reaction solution was then extracted with ethyl acetate, and the extract was washed with water and brine. The organic layer was dried over sodium sulfate, and then distillated under reduced pressure. The resulting residue was purified by silica gel column chromatography [hexane:ethyl acetate = 90:10 (v/v) to 50:50 (v/v)] to afford the desired compound (3-7) (1.71 g, 91%).

$^1$H-NMR($CDCl_3$)δ:7.38-7.37(3H,m),7.33(1H,s),7.25(1H,s),6.89-6.88(2H,m),5.56-5.54(1H,m),4.71-4.68(1H,m),4.65-4.62(1H,m),4.09-4.04(2H,m),3.96-3.91(4H,m),3.85-3.66(5H,m),3.46-3.45(2H,m),3.16-3.12(1H,m),1.99-1.94(4H,m),1.69-1.64(2H,m),1.00-0. 98(2H,m),0. 04(9H,s).
MS(APCI, ESI)m/z:647[81Br,$(M+H)^+$],645[79Br,$(M+H)^+$],

Step 7: (11aS)-8-[(5-Bromopentyl)oxy]-7-methoxy-2-(4-methoxyphenyl)-1,11a-dihydro-5H-pyrrolo[2,1-c][1,4]benzodiazepin-5-one (3-8)

Compound (3-7) obtained in Step 6 (0.789 g, 1.22 mmol) was dissolved in ethanol (10 mL) and tetrahydrofuran (10 mL), and 2.0 M tetrahydrofuran solution of lithium borohydride (6.11 mL, 12.2 mmol) was added thereto at 0°C, and the resultant was stirred at 0°C for 3 hours. Water was added to the reaction solution, which was extracted with chloroform, and the organic layer obtained was dried over sodium sulfate. The resultant was distillated under reduced pressure, and the resulting residue was dissolved in dichloromethane (10 mL), ethanol (20 mL) and water (10 mL), to which silica gel (4 g) was added at room temperature, and the resultant was stirred at room temperature for 4 days. The silica gel was removed through filtration, and water was added thereto, and the resultant was extracted with chloroform. The organic layer obtained was dried over sodium sulfate. The resultant was distillated under reduced pressure, and the resulting residue was purified by silica gel column chromatography [hexane:ethyl acetate = 60:40 (v/v) to 25:75 (v/v)] to afford the desired compound (3-8) (0.496 g, 81%).

$^1$H-NMR($CDCl_3$)δ:7.90-7.89(1H,m),7.53(1H,s),7.40-7.40(1H,m),7.35-7.34(2H,m),6.92-6.90(2H,m),6.83-6.81(1H,m),4.43-4.40(1H,m),4.13-4.06(2H,m),3.96(3H,s),3.84(3H,s),3.61-3.57(1H,m),3.47-3.36(3H,m),2.00-1.92(4H,m),1.67-1.63(2H,m).
MS(APCI, ESI)m/z:501[81Br,$(M+H)^+$],499[79Br,$(M+H)^+$],

Step 8: (11aS)-8-[(5-Bromopentyl)oxy]-7-methoxy-2-(4-methoxyphenyl)-1,10,11,11a-tetrahydro-5H-pyrrolo[2,1-c][1,4]benzodiazepin-5-one (3-9)

To a solution of compound (3-8) obtained in Step 7 (0.496 g, 0.992 mmol) in dichloromethane (20 mL), sodium triacetoxyborohydride (0.421 g, 1.99 mmol) was added at 0°C. After stirring at room temperature for 2 hours, a saturated aqueous sodium hydrogen carbonate was added thereto, and the resultant was extracted with chloroform. The organic layer was dried over sodium sulfate, and distillated under reduced pressure, and the resulting residue was then purified by silica gel column chromatography [hexane:ethyl acetate = 60:40 (v/v) to 25:75 (v/v)] to afford the desired compound (3-9) (0.426 g, 86%).

$^1$H-NMR($CDCl_3$)δ:7.53-7.53(2H,m),7.32-7.30(2H,m),6.89-6.87(2H,m),6.05(1H,s),4.33-4.27(2H,m),4.00-3.98(2H,m),3.86(3H,s),3.82(3H,s),3.57-3.55(2H,m),3.42-3.38(3H,m),2.76-2.72(1H,m),1.96-1.88(4H,m),1.65-1.62(2H,m).
MS(APCI, ESI)m/z:503[81Br,$(M+H)^+$],501[79Br,$(M+H)^+$].

Step 9: Prop-2-en-1-yl(11aS)-8-[(5-bromopentyl)oxy]-7-methoxy-2-(4-methoxyphenyl)-5-oxo-11,11a-dihydro-1H-pyrrol0[2,1-c][1,4]benzodiazepin-10(5H)-carboxylate (3-10)

To a solution of compound (3-9) obtained in Step 8 (0.426 g, 0.849 mmol) in dichloromethane (30 mL), pyridine (0.102 mL 1.27 mmol) and allyl chloroformate (0.374 mL, 3.54 mmol) were added at 0°C, and the resultant was stirred at 0°C for 15 minutes. To the reaction solution, a 10% aqueous solution of citric acid was added, which was extracted with chloroform, and the organic layer obtained was washed with a saturated aqueous sodium hydrogen carbonate, and then dried over sodium sulfate.
The resultant was distillated under reduced pressure, and the resulting residue was purified by silica gel column chromatography [hexane:ethyl acetate = 90:10 (v/v) to 50:50 (v/v)] to afford the desired compound (3-10) (0.465 g, 94%).
$^1$H-

NMR($CDCl_3$)δ:7.38(1H,s),7.31-7.29(2H,m),7.26-7.25(1H,m),6.89-6.87(2H,m),6.71(1H,s),5.80-5.78(1H,m),5.14-5.11(2H,m),4.65-4.62(1H,m),4.39-4.26(3H,m),4.03-4.01(2H,m),3.92(3H,s),3.82(3H,s),3.66-3.64(1H,m),3.46-3.44(2H,m),3.30-3.27(1H,m),2.72-2.68(1H,m),1.96-1.88(4H,m),1.68-1.60(2H,m). MS(APCI, ESI)m/z:587[81Br,$(M+H)^+$],585[79Br,$(M+H)^+$],

Step 10: N-[(Prop-2-en-1-yloxy)carbonyl]-L-valyl-N-{4-[({[(11'S,11a'S)-11'-{[tert-butyl(dimethyl)silyl]oxy}-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-11)

To a solution of compound (1-11) obtained in Step 10 of Example 1-1 (0.130 g, 0.161 mmol) and compound (3-10) obtained in Step 9 (0.104 g, 0.177 mmol) in N,N-dimethylformamide (3 mL), potassium carbonate (0.0266 g, 0.193 mmol) was added at room temperature, and the resultant was stirred at room temperature overnight. The reaction solution was diluted with ethyl acetate, and washed with water and brine, and then dried over sodium sulfate. The resultant was distillated under reduced pressure, and the resulting residue was then purified by NH2-silica gel column chromatography [hexane:ethyl acetate = 70:30 (v/v) to 0:100 (v/v)] to afford the desired compound (3-11) (0.184 g, 87%).
$^1$H-NMR($CDCl_3$)5:8.76(1H,s),7.58-7.56(2H,m),7.39(1H,s),7.32-7.30(2H,m),7.26-7.24(2H,m),7.19-7.17(3H,m),6.90-6.88(2H,m),6.78(1H,s),6.68-6.66(1H,m),6.37(1H,s),5.99-5.93(3H,m),5.34-5.20(6H,m),4.66-4.01(11H,m),3.90(3H,s),3.89(3H,s),3.78-3.54(9H,m),3.31-3.28(2H,m),2.73-2.69(1H,m),2.38-2.35(1H,m),2.19-2.13(1H,m),1.82-1.80(2H,m),1.46-1.29(6H,m),0.98-0.90(6H,m),0.83(9H,s),0.69-0.63(4H,m),0.19-0.16(6H,m).

MS(APCI, ESI)m/z:1312$(M+H)^+$

Step 11: N-[(Prop-2-en-1-yloxy)carbonyl]-L-valyl-N-{4-[({[(11'S,11a'S)-11'-hydroxy-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-12)

To a solution of compound (3-11) obtained in Step 10 (0.1837 g, 0.140 mmol) and acetic acid (0.048 mL, 0.840 mmol) in tetrahydrofuran (5.00 mL), a 1 mol/L tetrahydrofuran solution of tetrabutylammonium fluoride (0.700 mL, 0.700 mmol) was added at room temperature, and the resultant was stirred at room temperature for 3 hours. The reaction solution was diluted with ethyl acetate, and the organic layer was washed with a saturated aqueous sodium hydrogen carbonate and brine, and then dried over sodium sulfate. The resultant was distillated under reduced pressure, and the resulting residue was purified by silica gel chromatography [chloroform:methanol = 99.5:0.5(v/v) to 95:5(v/v)] to afford the desired compound (3-12) (0.178 g, quantitative).
$^1$H-NMR($CDCl_3$)δ:8.86(1H,s),7.60-7.59(2H,m),7.39(1H,s),7.32-7.20(7H,m),6.90-6.88(2H,m),6.78(1H,s),6.68(1H,s),6.38(1H,s),5.90-5.87(3H,m),5.39-5.22(6H,m),4.72-4.02(11H,m),3.90(3H,s),3.88(3H,s),3.83(3H,s),3.70-3.63(6H,m),3.32-3.29(3H,m),2.73-2.69(1H,m),2.43-2.40(1H,m),2.12-2.06(1H,m),1.77-1.74(2H,m),1.39-1.25(6H,m),0.96-0.89(6H,m),0.73-0.66(4H,m).

MS(APCI, ESI)m/z: 1198$(M+H)^+$

Step 12: L-Valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-13)

To a solution of compound (3-12) obtained in Step 11 (0.140 mmol) in dichloromethane (2 mL), pyrrolidine (0.0579 mL, 0.700 mmol) and tetrakis(triphenylphosphine)palladium (0) (0.0162 g, 0.0140 mmol) were added at room temperature, and the resultant was stirred at room temperature for 15 minutes. After distillation under reduced pressure, the resulting residue was purified by silica gel chromatography [chloroform:methanol = 99.5:0.5(v/v) to 92.5:7.5(v/v)] to afford the desired compound (3-13) (0.143 g, 99%).
$^1$H-NMR($CDCl_3$)δ:9.12(1H,s),7.94-7.92(1H,m),7.57-7.53(4H,m),7.33-7.31(2H,m),7.20-7.18(3H,m),6.90-6.88(2H,m),6.36(1H,s),6.07(1H,s),5.91-5.88(1H,m),5.47-5.44(1H,m),5.21-5.13(1H,m),4.66-4.58(3H,m),4.32(1H,s),4.03-3.49(17H,m),3.38-3.29(4H,m),3.15-3.14(1H,m),2.77-2.73(1H,m),2.57(2H,s),2.43-2.40(1H,m),2.32-2.27(1H,m),1.81-1.39(8H,m),0.98-0.96(3H,m),0.85-0.83(3H,m),0.75-0.62(4H,m).

MS(APCI, ESI)m/z: 1030$(M+H)^+$

Step 13: N-[4-(11,12-Didehydrodibenzo[b,f]azocin-5 (6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (3-14)

To a mixture of compound (2-2) obtained in Step 1 of Example 1-2 (0.0640 g, 0.153 mmol) and N-ethoxycarbonyl-2-ethoxy-1,2-dihydroquinoline (0.0446 g, 0.180 mmol), dichloromethane (2 mL) was added at room temperature, and the resultant was stirred at room temperature for 15 minutes. To the reaction solution, a solution of compound (3-13) obtained in Step 12 (0.143 g, 0.139 mmol) in dichloromethane (2 mL) was added, and the resultant was stirred at room temperature for 5 hours, and then distillated under reduced pressure. The resulting residue was purified by silica gel column chromatography [chloroform:methanol = 99.5:0.5 (v/v) to 92.5:7.5 (v/v)] to afford the desired compound (3-14) (0.103 g, 52%).

1H-NMR (DMSO-D6) δ: 9.93 (1H, s), 8.21-8.16 (2H, m), 8.07-8.04 (1H, m), 7.83-7.64 (2H, m), 7.60-7.55 (3H, m), 7.51-7.28 (10H, m), 7.19-7.16 (2H, m), 7.10-7.04 (1H, m), 6.92-6.90 (2H, m), 6.76-6.70 (1H, m), 6.39 (1H, s), 5.77-5.75 (1H, m), 5.21-5.18 (1H, m), 5.03-4.99 (1H, m), 4.82-4.79 (1H, m), 4.37-4.35 (1H, m), 4.21-4.20 (2H, m), 4.02-3.24 (26H, m), 3.16-3.13 (1H, m), 2.79-2.59 (2H, m), 2.39-2.28 (2H, m), 2.05-1.97 (2H, m), 1.91-1.77 (4H, m), 1.57-1.54 (3H, m), 1.28-1.23 (3H, m), 0.85-0.80 (6H, m), 0.67-0.61 (4H, m).
MS(APCI, ESI)m/z: 1431$(M+H)^+$

[Example 2-2: Drug-linker 2]

[Formula 59]

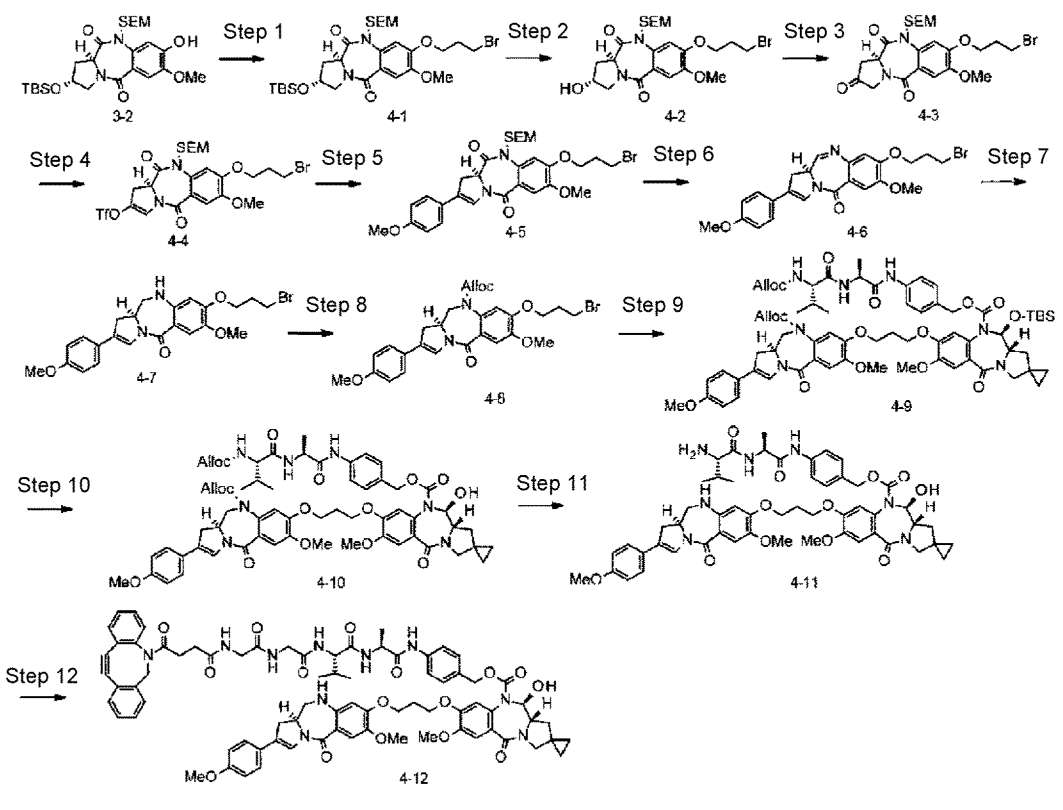


Step 1: (2R,11aS)-8-(3-Bromopropoxy)-2-{[tert-butyl(dimethyl)silyl]oxy}-7-methoxy-10-{[2-(trimethylsilyl)ethoxy]methyl}-2,3-dihydro-1H-pyrrolo[2,1-c][1,4]benzodiazepin-5,11(10H,11aH)-dione (4-1)

Compound (3-2) obtained in Step 1 of Example 2-1 (5.06 g, 9.67 mmol) and 1,3-dibromopropane (4.93 mL, 48.4 mmol) were reacted in the same manner as in Step 2 of Example 2-1 to afford the desired compound (4-1) (4.85 g, 78%).
MS(APCI, ESI)m/z:645[81Br,$(M+H)^+$],643[79Br,$(M+H)^+$],

Step 2: (2R,11aS)-8-(3-Bromopropoxy)-2-hydroxy-7-methoxy-10-{[2-(trimethylsilyl)ethoxy]methyl}-2,3-dihydro-1H-pyrrolo[2,1-c][1,4]benzodiazepin-5,11(10H,11aH)-dione (4-2)

Compound (4-1) obtained in Step 1 (4.85 g, 7.54 mmol) was reacted in the same manner as in Step 3 of Example 2-1 to afford the desired compound (4-2) (4.05 g, quantitative).
MS(APCI, ESI)m/z:531[81Br,$(M+H)^+$],529[79Br,$(M+H)^+$].

Step 3: (11aS)-8-(3-Bromopropoxy)-7-methoxy-10-{[2-(trimethylsilyl)ethoxy]methyl}-1H-pyrrolo[2,1-c][1,4]benzodiazepin-2,5,11 (3H,10H,11aH)-trione (4-3)

Compound (4-2) obtained in Step 2 (7.54 mmol) was reacted in the same manner as in Step 4 of Example 2-1 to afford the desired compound (4-3) (3.73 g, 93%). $^1$H-NMR($CDCl_3$)δ:7.34(1H,s),7.29(1H,s),5.56-5.53(1H,m),4.72-4.69(1H,m),4.67-4.61(1H,m),4.23-4.17(3H,m),3.97-3.88(4H,m),3.82-3.75(1H,m),3.74-3.56(4H,m),2.82-2.77(1H,m),2.43-2.38(2H,m),1.06-0.94(2H,m),0.08-0.00(9H,m).

Step 4: (11aS)-8-(3-Bromopropoxy)-7-methoxy-5,11-dioxo-10-{[2-(trimethylsilyl)ethoxy]methyl}-5,10,11,11a-tetrahydro-1H-pyrrolo[2,1-c][1,4]benzodiazepin-2-yl trifluoromethanesulfonate (4-4)

Compound (4-3) obtained in Step 3 (3.73 g, 7.08 mmol) was reacted in the same manner as in Step 5 of Example 2-1 to afford the desired compound (4-4) (3.27 g, 70%).

MS(APCI, ESI)m/z:661[81Br,$(M+H)^+$],659[79Br,$(M+H)^+$].

Step 5: (11aS)-8-(3-Bromopropoxy)-7-methoxy-2-(4-methoxyphenyl-10-{[2-(trimethylsilyl)ethoxy]methyl}-1H-pyrrolo[2,1-c][1,4]benzodiazepin-5,11(10H,11aH)-dione (4-5)

Compound (4-4) obtained in Step 4 (3.27 g, 4.96 mmol) was reacted in the same manner as in Step 6 of Example 2-1 to afford the desired compound (4-5) (2.49 g, 81%). MS(APCI, ESI)m/z:619[81Br,$(M+H)^+$],617[79Br,$(M+H)^+$].

Step 6: (11aS)-8-(3-Bromopropoxy)-7-methoxy-2-(4-methoxyphenyl)-1,11a-dihydro-5H-pyrrolo[2,1-c][1,4]benzodiazepin-5-one (4-6)

Compound (4-5) obtained in Step 5 (2.49 g, 4.04 mmol) was reacted in the same manner as in Step 7 of Example 2-1 to afford the desired compound (4-6) (1.59 g, 84%).

MS(APCI, ESI)m/z:473[81Br,$(M+H)^+$],471[79Br,$(M+H)^+$].

Step 7: (11aS)-8-(3-Bromopropoxy)-7-methoxy-2-(4-methoxyphenyl)-1,10,11,11a-tetrahydro-5H-pyrrolo[2,1-c][1,4]benzodiazepin-5-one (4-7)

Compound (4-6) obtained in Step 6 (1.59 g, 3.38 mmol) was reacted in the same manner as in Step 8 of Example 2-1 to afford the desired compound (4-7) (1.39 g, 87%). MS(APCI, ESI)m/z:475[81Br,$(M+H)^+$],473[79Br,$(M+H)^+$].

Step 8: Prop-2-en-1-yl (11aS)-8-(3-bromopropoxy)-7-methoxy-2-(4-methoxyphenyl)-5-oxo-11,11a-dihydro-1H-pyrrolo[2,1-c][1,4]benzodiazepin-10(5H)-carboxylate (4-8)

Compound (4-7) obtained in Step 7 (1.40 g, 2.95 mmol) was reacted in the same manner as in Step 9 of Example 2-1 to afford the desired compound (4-8) (0.885 g, 54%).
MS(APCI, ESI)m/z:559[81Br,$(M+H)^+$],557[79Br,$(M+H)^+$].

Step 9: N-{[(Prop-2-en-1-yl)oxy]carbonyl}-L-valyl-N-[4-({[(11'S,11'aS)-11'-{[tert-butyl(dimethyl)silyl]oxy}-7'-methoxy-8'-(3-{[(11aS)-7-methoxy-2-(4-methoxyphenyl)-5-oxo-10-{[(prop-2-en-1-yl)oxy]carbonyl}-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-9)

Compound (4-8) obtained in Step 8 (0.0381 g, 0.0683 mmol) and compound (1-11) obtained in Step 10 of Example 1-1 (0.0552 g, 0.0683 mmol) were reacted in the same manner as in Step 10 of Example 2-1 to afford the desired compound (4-9) (0.0712 g, 81%).

MS(APCI, ESI)m/z: $1284(M+H)^+$.

Step 10: N-{[(Prop-2-en-1-yl)oxy]carbonyl}-L-valyl-N-[4-({[(11'S,11'aS)-11'-hydroxy-7'-methoxy-8'-(3-{[(11aS)-7-methoxy-2-(4-methoxyphenyl)-5-oxo-10-{[(prop-2-en-1-yl)oxy]carbonyl}-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-10)

Compound (4-9) obtained in Step 9 (0.0712 g, 0.0554 mmol) was reacted in the same manner as in Step 11 of Example 2-1 to afford the desired compound (4-10) (0.0671 g, quantitative).

MS(APCI, ESI)m/z: 1170$(M+H)^+$.

Step 11: L-Valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-11)

Compound (4-10) obtained in Step 10 (0.0571 mmol) was reacted in the same manner as in Step 12 of Example 2-1 to afford the desired compound (4-11) (0.0574 g, 99%).

$^1$H-NMR($CDCl_3$)δ:9.16(1H,s),7.93-7.91(1H,m),7.55-7.52(1H,m),7.50-7.47(3H,m),7.35-7.32(2H,m),7.21(1H,s),7.13-7.11(2H,m),6.90-6.87(2H,m),6.40(1H,s),6.08(1H,s),5.90-5.87(1H,m),5.37-5.34(1H,m),4.73-4.53(3H,m),4.23-4.08(5H,m),3.89(3H,s),3.82(3H,s),3.78-3.72(5H,m),3.57-3.51(3H,m),3.38-3.30(3H,m),2.76-2.71(1H,m),2.36-2.24(4H,m),1.78-1.42(6H,m),1.00-0.98(3H,m),0.87-0.84(3H,m),0.74-0.62(4H,m).

MS(APCI, ESI)m/z: 1002$(M+H)^+$.

Step 12: N-[4-(11,12-Didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide (4-12)

Compound (4-11) obtained in Step 11 (0.189 g, 0.189 mmol) and compound (2-2) obtained in Step 1 of Example 1-2 (0.087 g, 0.207 mmol) were reacted in the same manner as in Step 13 of Example 2-1 to afford the desired compound (4-12) (0.169 g, 64%).

MS(APCI, ESI)m/z: 1402(M+H)+.

[Example 2-3: Drug-linker 3]

[Formula 60]

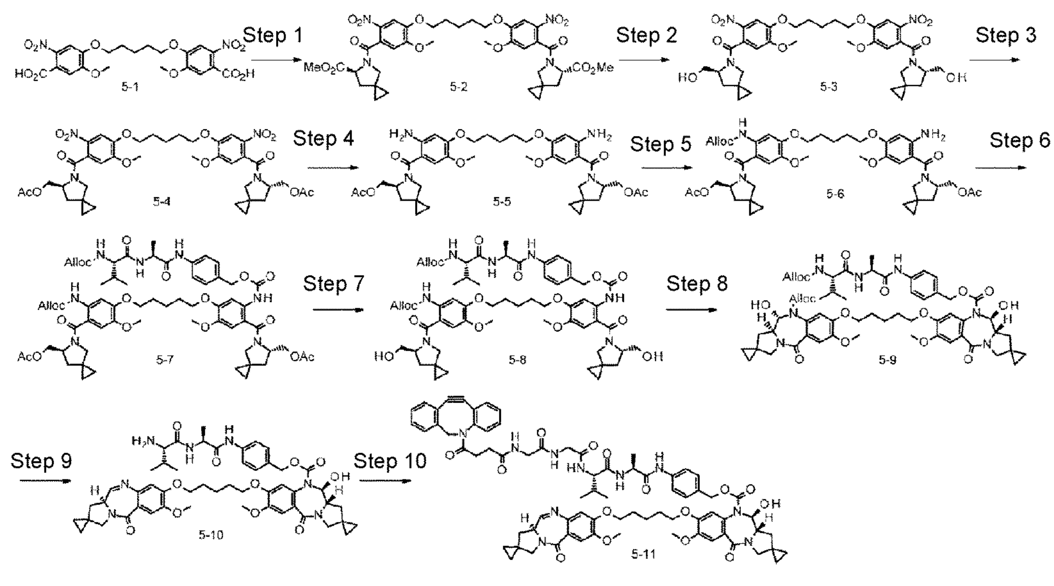


Step 1: Dimethyl(6S,6'S)-5,5'-{1,5-pentanediylbis[oxy(5-methoxy-2-nitrobenzene-4,1-diyl)carbonyl] }bis(5-azaspiro[2.4]heptane-6-carboxylate) (5-2)

To a solution of 4,4'-[1,5-pentanediylbis(oxy)]bis(5-methoxy-2-nitrobenzoic acid) (5-1) (5.41 g, 10.9 mmol, Journal of Medicinal Chemistry 2004, 47, 1161) in dichloromethane (50 mL), oxalyl chloride (5.63 mL, 65.7 mmol) was added

at 0°C, and N,N-dimethylformamide (0.0844 mL, 1.09 mmol) was added dropwise. The temperature of the reaction solution was raised to room temperature, and the reaction solution was stirred for 2 hours. The resultant was distillated under reduced pressure, and the resulting residue was dissolved in dichloromethane (100 mL), which was added dropwise to dichloromethane solution (100 mL) of methyl (6S)-5-azaspiro[2.4]heptane-6-carboxylate hydrochloride (4.28 g, 24.1 mmol, Tetrahedron Letters 2012. 53. 3847) and triethylamine (6.07 mL, 43.8 mmol) under the nitrogen atmosphere at -40°C. The temperature of the reaction solution was raised to 0°C, and the reaction solution was stirred for 2 hours. To the reaction mixture, 1 N hydrochloric acid (100 mL) was added, and the organic layer was washed with water and brine, and dried over anhydrous sodium sulfate. The resultant was distillated under reduced pressure to afford the desired compound (5-2) (8.40 g, quantitative).
MS(APCI, ESI)m/z:769(M+H)+.

Step 2: {1,5-Pentanediylbis[oxy (5-methoxy-2-nitrobenzen-4,1-diyl)]}bis{[(6S)-6-(hydroxymethyl)-5-azaspiro[2.4]hept-5-yl]methanone} (5-3)

To a solution of compound (5-2) obtained in Step 1 (8.40 g, 10.9 mmol) in tetrahydrofuran (100 mL), lithium borohydride (714 mg, 32.8 mmol) was added, and the resultant was stirred at 0°C for 30 minutes, and the temperature was raised to room temperature, and stirring was performed for 1 hour. After 1 N hydrochloric acid was added at 0°C, the resultant was extracted with ethyl acetate, and washed with brine, and then dried over anhydrous sodium sulfate. The solvent was distilled off under reduced pressure to afford the desired compound (5-3) (7.70 g, 99%).
MS(APCI, ESI)m/z:713(M+H)+.

Step 3: Pentan-1,5-diylbis[oxy (5-methoxy-2-nitrobenzen-4,1-diyl)carbonyl (6*S*)-5-azaspiro[2.4]heptan-5,6-diylmethanediyl] diazetate (5-4)

Compound (5-3) obtained in Step 2 (7.70 g, 10.8 mmol) was dissolved in pyridine (20 mL) and acetic anhydride (10 mL, 105.9 mmol), which was stirred at room temperature. The resultant was distillated under reduced pressure to afford the desired compound (5-4) (8.38 g, 97%).
MS(APCI, ESI)m/z:797(M+H)+.

Step 4: 1,5-Pentanediylbis[oxy (2-amino-5-methoxybenzen-4,1-diyl)carbonyl (6S)-5-azaspiro[2.4]heptan-5,6-diylmethanediyl] diacetate (5-5)

To a solution of compound (5-4) obtained in Step 3 (8.28 g, 10.4 mmol) in N,N-dimethylformamide (100 mL), 5% palladium carbon (moisture content: 54%, 1.00 g) was added, and the reaction solution was then vigorously stirred under the hydrogen atmosphere at room temperature for 6 hours. The resultant was filtered through a Celite, and the filtrate was then distillated under reduced pressure, and the resulting residue was purified by silica gel column chromatography [chloroform:methanol = 100:0(v/v) to 90:10(v/v)] to afford the desired compound (5-5) (5.05 g, 66%). MS(APCI, ESI)m/z:737(M+H)+.

Step 5: {(6*S*)-5-[4-({5-[4-({(6*S)*-6-[(Acetyloxy)methyl]-5-azaspiro[2.4]hept-5-yl}carbonyl)-5-amino-2-methoxyphenoxy]pentyl}oxy)-5-methoxy-2-{[(prop-2-en-1-yloxy)carbonyl]amino}benzoyl]-5-azaspiro[2.4]hept-6-yl}methylacetate (monoallyloxycarbonyl form) (5-6)

To a solution of compound (5-5) obtained in Step 4 (5.05 g, 6.85 mmol) in dichloromethane (100 mL), pyridine (1.10 mL, 13.7 mmol) was added, and allyl chloroformate (0.725 mL, 6.85 mmol) was added thereto under the nitrogen atmosphere at -78°C, and the resultant was stirred for 2 hours. The resultant was distillated under reduced pressure, and the resulting residue was purified by silica gel column chromatography [hexane:ethyl acetate = 70:30 (v/v) to 100:0 (v/v), chloroform:methanol = 100:0 (v/v) to 90:10 (v/v)] to afford the monoallyloxycarbonyl form (5-6) (2.63 g, 47%) as the desired compound.
MS(APCI, ESI)m/z: 821(M+H)+.

Step 6: N-[(2-Propen-1-yloxy)carbonyl]-L-valyl-N-{4-[({[2-({(6S)-6-[(acetyloxy)methyl]-5-azaspiro[2.4]hept-5-yl}carbonyl)-5-({5-[4-({(6S)-6-[(acetyloxy)methyl]-5-azaspiro[2.4]hept-5-yl}carbonyl)-2-methoxy-5-{[(2-propen-1-yloxy)carbonyl]amino}phenoxy]pentyl}oxy)-4-methoxyphenyl]carbamoyl}oxy)methyl]phenyl}-L-alaninamide (5-7)

Monoallyloxycarbonyl form (5-6) obtained in Step 5 (2.00 g, 2.44 mmol) and N-[(prop-2-en-1-yloxy)carbonyl]-L-valyl-N-[4-(hydroxymethyl)phenyl]-L-alaninamide (1.10 g, 2.92 mmol, WO2011130598) were reacted in the same manner as in Step 6 of Example 1-1 to afford the desired compound (5-7) (2.64 g, 89%).

MS(APCI, ESI)m/z: 1224(M+H)+.

Step 7: N-[(2-Propen-1-yloxy)carbonyl]-L-valyl-N-[4-({[(2-{[(6S)-6-(hydroxymethyl)-5-azaspiro[2.4]hept-5-yl]carbonyl}-5-{[5-(4-{[(6S)-6-(hydroxymethyl)-5-azaspiro[2.4]hept-5-yl]carbonyl}-2-methoxy-5-{[(2-propen-1-yloxy)carbonyl]amino}phenoxy)pentyl]oxy}-4-methoxyphenyl)carbamoyl]oxy}methyl)phenyl]-L-alaninamide (5-8)

To a solution of compound (5-7) obtained in Step 6 (2.64 g, 2.16 mmol) in methanol (10 mL), potassium carbonate (1.49 g, 10.8 mmol) was added, and the resultant was stirred at room temperature for 3 hours. A saturated aqueous ammonium chloride (100 mL) was added to the reaction mixture, which was extracted with ethyl acetate. The organic layer was dried over anhydrous sodium sulfate. The resultant was distillated under reduced pressure to afford the desired compound (5-8) (2.21 g, 90%).
MS(APCI, ESI)m/z: 1140$(M+H)^+$.

Step 8: N-[(2-Propen-1-yloxy)carbonyl]-L-valyl-N-{4-[({[(11'S,11a'S)-11'-hydroxy-8'-{[5-({(11'S,11a'S)-11'-hydroxy-7'-methoxy-5'-oxo-10'-[(2-propen-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-7'-methoxy-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-9)

To a solution of compound (5-8) obtained in Step 7 (2.03 g, 1.78 mmol) in dichloromethane (50 mL), Dess-Martin periodinane (1.59 g, 3.74 mmol) was added, and the resultant was stirred at room temperature overnight. A saturated aqueous sodium hydrogen carbonate (100 mL) was added to the reaction mixture, which was extracted with chloroform. The organic layer was dried over anhydrous sodium sulfate. The resultant was distillated under reduced pressure, and the resulting residue was purified by silica gel column chromatography [chloroform:methanol = 100:0(v/v) to 90:10(v/v)] to afford the desired compound (5-9) (2.05 g, quantitative).
MS(APCI, ESI)m/z: 1136$(M+H)^+$.

Step 9: L-Valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepine]-8'-yl]oxy}pentyl)oxy]-5'-oxo-11',1 1a'-dihydro-1'H-spiro[cyclopropane-1,2'-pyrrolo[2,1-c][1,4]benzodiazepine]-10'(5'H)-yl]carbonyl}oxy)methyl]phenyl} -L-alaninamide (5-10)

Compound (5-9) obtained in Step 8 (2.05 g, 1.80 mmol) was reacted in the same manner as in Step 12 of Example 2-1 to afford the desired compound (5-10) (1.02 g, 60%).
MS(APCI, ESI)m/z:950$(M+H)^+$.

Step 10: N-[4-(11,12-Didehydrodibenzo[b,f]azocin-5 (6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (5-11)

Compound (5-10) obtained in Step 9 (0.710 g, 0.747 mmol) and compound (2-2) obtained in Step 1 of Example 1-2 (0.313 g, 0.747 mmol) were dissolved in mixed solvent of dichloromethane (1.5 mL) and methanol (0.1 mL). Thereto, 4-(4,6-dimethoxy-1,3,5-triazin-2-yl)-4-methylmorpholinium chloride (0.264 g, 0.897 mmol) was added, and the resultant was stirred at room temperature for 1 hour. The resultant was distillated under reduced pressure, and the resulting residue was purified by silica gel column chromatography [chloroform:methanol = 100:0 (v/v) to 80:20 (v/v)] to afford the desired compound (5-11) (0.671 g, 66%).

$^1$H-NMR(DMSO-$D_6$)$\delta$:9.91(1H,s),8.32(1H,s),8.23-7.91(3H,m),7.81-7.19(14H,m),7.04(1H,m),6.80-6.62(3H,m),5.77-5.75(1H,m),5.20(1H,m),5.01(1H,m),4.79(1H,m),4.46-4.35(1H,m),4.04(4H,m),3.86-3.38(18H,m),3.22-3.15(2H,m),2.67-2.63(1H,m),2.46-2.23(3H,m),2.09-1.91(2H,m),1.80-1.78(5H,m),1.57(3H,m),1.27(3H,s),1.11-1.04(1H,m),0.87-0.79(6H,m),0.63-0.55(6H,m).
MS(APCI, ESI)m/z: 1351$(M+H)^+$.

[Example 2-4: Drug-linker 4]

[Formula 61]

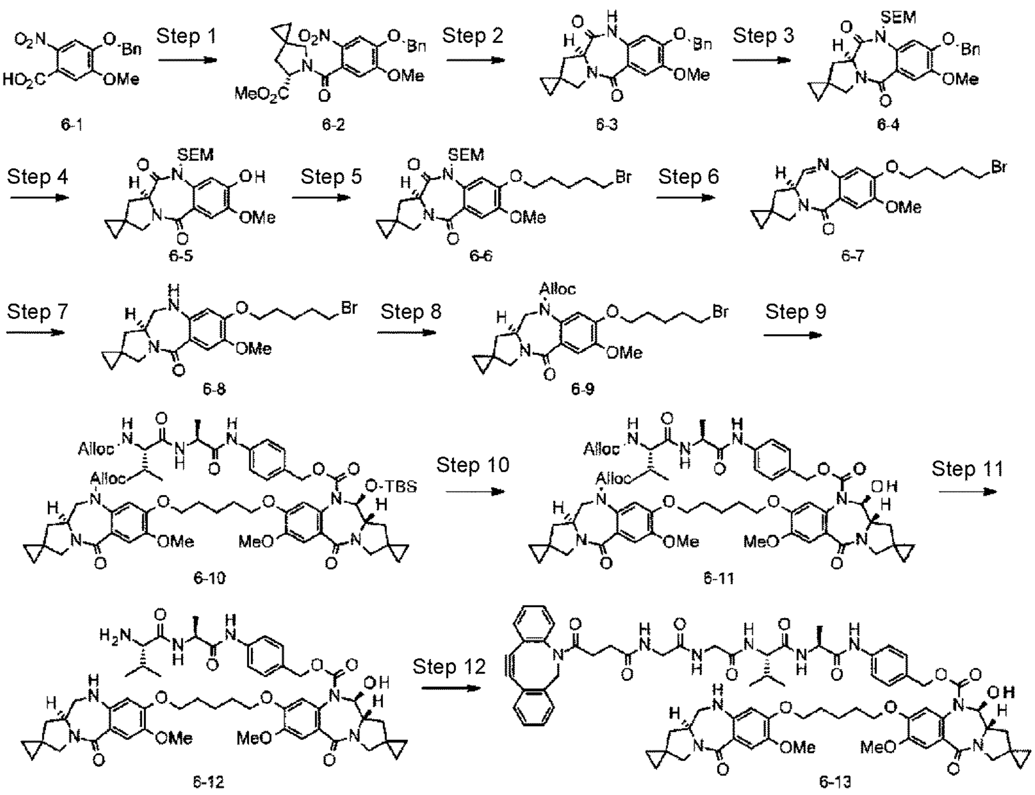


Step 1: Methyl (6S)-5-[4-(benzyloxy)-5-methoxy-2-nitrobenzoyl]-5-azaspiro[2.4]heptane-6-carboxylate (6-2)

To a solution of 4-(benzyloxy)-5-methoxy-2-nitrobenzoic acid (6-1) (6.07 g, 20.0 mmol, Tetrahedron 1995, 51, 5617) and N,N-dimethylformamide (1.08 mL, 13.9 mmol) in dichloromethane (100 mL), oxalyl chloride (3.43 mL, 40.0 mmol) was added dropwise under ice-cooling over 5 minutes. The reaction solution was stirred at room temperature for 5 hours, and then distillated under reduced pressure, and the resulting residue was dissolved in dichloromethane (20 mL), which was distillated under reduced pressure. After this operation was repeated three times, the residue was suspended in dichloromethane (5 mL), to which excessive amounts of diethyl ether and hexane were added, and the following filtration and drying under reduced pressure afforded the crude acyl chloride. The acyl chloride obtained was dissolved in dichloromethane and cooled to -40°C (dry ice-acetonitrile bath), to which methyl (6S)-5-azaspiro[2.4]heptane-6-carboxylate hydrochloride (4.22 g, 22.0 mmol, Tetrahedron Letters 2012. 53. 3847) and triethylamine (3.36 mL, 24.2 mmol) were gradually added. The temperature of the reaction mixture was raised to room temperature overnight. To the reaction mixture, 1 N hydrochloric acid was added, and the reaction mixture was extracted with dichloromethane. The organic layer was washed with water, a saturated aqueous sodium hydrogen carbonate, and brine, and dried over anhydrous sodium sulfate. The resultant was distillated under reduced pressure, and the resulting residue was purified by silica gel column chromatography [hexane:ethyl acetate = 100:0 to 50:50] to afford the desired compound (6-2) (6.55 g, 80%).
MS (APCI, ESI)m/z:441 $(M+H)^+$

Step 2: (11a'S)-8'-(Benzyloxy)-7'-methoxy-1'H-spiro[cyclopropane-1,2'-pyrrolo[2,1-c][1,4]benzodiazepine]-5',11'(10'H,11a'H)-dione (6-3)

To a solution of compound (6-2) obtained in Step 1 (6.55 g, 16.0 mmol) in ethanol (150 mL) and tetrahydrofuran (150 mL), Raney-nickel (7.00 g) was added under the nitrogen atmosphere. Hydrazine monohydrate (7 mL) was added to the reaction mixture, and the temperature was gradually raised to 50°C. After stirring at 50°C for 2 hours, Raney-nickel (3.00 g) and hydrazine monohydrate (3 mL) were added thereto, and the resultant was stirred for 1 hour. THF (100 mL) was added to the reaction mixture, which was filtered through a Celite. The resultant was distillated under reduced pressure, and the resulting residue was purified by silica gel column chromatography [hexane:ethyl acetate = 100:0 to 25:75] to afford the desired compound (6-3) (4.42 g, 73%).

MS(APCI, ESI)m/z:379(M+H)+

Step 3: (11a'S)-8'-(Benzyloxy)-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-4)

To a solution of compound (6-3) obtained in Step 2 (10.0 g, 26.4 mmol) in tetrahydrofuran (150 mL), a 2.6 mol/L normal-hexane solution of normal-butyllithium (12.0 mL, 31.8 mmol) was added slowly dropwise at -40°C. The reaction solution was stirred at -40°C for 15 minutes, and 2-(chloromethoxy)ethyltrimethylsilane (5.57 mL, 31.7 mmol) was then added slowly dropwise thereto. After the reaction solution was stirred at room temperature for 3 hours, water was added thereto, and the resultant was extracted with ethyl acetate. The organic layer was washed with water and brine, and dried over anhydrous sodium sulfate. After distillation under reduced pressure, the resulting residue was purified by silica gel column chromatography [hexane:ethyl acetate = 100:0 to 30:70] to afford the desired compound (6-4) (11.8 g, 88%). MS(APCI, ESI)m/z:509(M+H)+

Step 4: (11a'S)-8'-Hydroxy-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-5)

To a solution of compound (6-4) obtained in Step 3 (18.7 g, 36.8 mmol) in tetrahydrofuran (50 mL) and ethanol (100 mL), a 5% palladium carbon catalyst (5.00 g) was added under the nitrogen atmosphere. The nitrogen balloon was immediately replaced with a hydrogen balloon, and the reaction mixture was stirred under the hydrogen atmosphere for 6 hours. The reaction mixture was diluted by addition of chloroform and filtered through a Celite, and the filtrate was then distillated under reduced pressure, and the resulting residue was purified by silica gel column chromatography [hexane:ethyl acetate = 100:0 to 25:75] to afford the desired compound (6-5) (15.1 g, 98%).
MS(APCI, ESI)m/z:419(M+H)+

Step 5: (11a'S)-8'-[(5-Bromopentyl)oxy]-7'-methoxy-10'-{[2-(trimethylsilyl)ethoxy]methyl}-1'H-spiro[cyclopropane-1,2'-pyrrolo[2,1-c][1,4]benzodiazepine]-5',11'(10'H,11a'H)-dione (6-6)

Compound (6-5) obtained in Step 4 (2.77 g, 6.62 mmol) was reacted in the same manner as in Step 2 of Example 2-1 to afford the desired compound (6-6) (3.31 g, 88%). $^1$H-NMR($CDCl_3$)δ:7.36(1H,s),7.25(1H,s),5.55(1H,m),4.65(1H,m),4.24-4.23(1H,m),4.11-4.03(2H,m),3.93(3H,s),3.85-3.78(1H,m),3.72-3.69(2H,m),3.46-3.39(3H,m),2.47-2.44(1H,m),2.25-2.22(1H,m),1.95-1.91(4H,m),1.67-1,59(1H,m),1.03-0.95(2H,m),0.90-0.85(1H,m),0.70-0.66(4H,m),0.05(9H,s).

Step 6: (11a'S)-8'-[(5-Bromopentyl)oxy]-7'-methoxy-1',11a'-dihydro-5'H-spiro[cyclopropane-1,2'-pyrrolo[2,1-c][1,4]benzodiazepine]-5'-one (6-7)

Compound (6-6) obtained in Step 5 (3.31 g, 5.83 mmol) was reacted in the same manner as in Step 7 of Example 2-1 to afford the desired compound (6-7) (1.11 g, 45%). $^1$H-NMR($CDCl_3$)δ:7.81(1H,m),7.53(1H,s),6.82(1H,s),4.13-4.06(2H,m),3.97(3H,s),3.88-3.83(1H,m),3.69(1H,m),3.52-3.39(3H,m),2.55-2.52(1H,m),2.06-1.89(5H,m),1.67-1.63(2H,m),0.76-0.72(4H,m).

Step 7: (11a'S)-8'-[(5-Bromopentyl)oxy]-7'-methoxy-1',10',11,11a'-tetrahydro-5'H-spiro[cyclopropane-1,2'-pyrrolo[2,1-c][1,4]benzodiazepine]-5'-one (6-8)

Compound (6-7) obtained in Step 6 (2.56 g, 6.08 mmol) was reacted in the same manner as in Step 8 of Example 2-1 to afford the desired compound (6-8) (1.15 g, 45%). $^1$H-NMR($CDCl_3$)δ:7.60(1H,s),6.07(1H,s),4.11-4.04(1H,m),3.99(2H,m),3.87-3.84(1H,m),3.85(3H,s),3.73(1H,m),3.58-3.53(2H,m),3.47-3.42(3H,m),2.03-1.78(6H,m),1.65-1.63(2H,m),0.77-0.56(4H,m).

Step 8: Prop-2-en-1-yl (11a'S)-8'-[(5-bromopentyl)oxy]-7'-methoxy-5'-oxo-11',11a'-dihydro-1'H-spiro[cyclopropane-1,2'-pyrrolo[2,1-c][1,4]benzodiazepine]-10'(5'H)-carboxylate (6-9)

Compound (6-8) obtained in Step 7 (1.15 g, 2.72 mmol) was reacted in the same manner as in Step 9 of Example 2-1 to afford the desired compound (6-9) (1.14 g, 82%). $^1$H-NMR($CDCl_3$)δ:7.23(1H,s),6.69(1H,s),5.79(1H,s),5.13-5.10(2H,m),4.68-4.66(1H,m),4.48-4.45(2H,m),4.01(2H,m),3.92(3H,s),3.76(1H,m),3.54-3.37(3H,m),2.39(1H,m),1.95-1.90(4H,m),1.68-1.61(3H,m),1.44(1H,m),0.75-0.66(4H,m).

Step 9: N-[(Prop-2-en-1-yloxy)carbonyl]-L-valyl-N-{4-[({[(11'S,11a'S)-11'-{[tert-butyl(dimethyl)silyl]oxy}-7'-methoxy-8'-{[5-({(11a'S)-7'-methoxy-5'-oxo-10'-[(prop-2-en-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-10)

Compound (6-9) obtained in Step 8 (0.374 g, 0.737 mmol) and compound (1-11) obtained in Step 10 of Example 1-1 (0.452 g, 0.56 mmol) were reacted in the same manner as in Step 10 of Example 2-1 to afford the desired compound (6-10) (0.589 g, 65%).

MS (APCI, ESI)m/z:1234 $(M+H)^+$

Step 10: N-[(Prop-2-en-1-yloxy)carbonyl]-L-valyl-N-{4-[({[(11'S,11a'S)-11'-hydroxy-7'-methoxy-8'-{[5-({(11a'S)-7'-methoxy-5'-oxo-10'-[(prop-2-en-1-yloxy)carbonyl]-5',10',11',11a'-tetrahydro-1'H-spiro[cyclopropane-1,2'-pyrrolo[2,1-c][1,4]benzodiazepine]-8'-yl}oxy)pentyl]oxy}-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-11)

Compound (6-10) obtained in Step 9 (0.589 g, 0.477 mmol) was reacted in the same manner as in Step 11 of Example 2-1 to afford the desired compound (6-11) (0.382 g, 71%).
$^1$H-NMR($CDCl_3$)$\delta$:8.90(1H,s),7.55(2H,m),7.25-7.21(2H,m),6.74(2H,m),6.38(1H,s),5.90-5.87(5H,m),5.33-5.09(8H,m),4.66-4.60(8H,m),3.98-3.91(10H,m),3.77-3.30(12H,m),2.42-2.36(2H,m),1.77-1.39(6H,m),0.91-0.70(14H,m).

Step 11: L-Valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-12)

Compound (6-11) obtained in Step 10 (0.382 g, 0.341 mmol) was reacted in the same manner as in Step 12 of Example 2-1 to afford the desired compound (6-12) (0.200 g, 62%).

MS (APCI, ESI)m/z:952 $(M+H)^+$

Step 12: N-[4-(11,12-Didehydrodibenzo[b,f]azocin-5 (6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepine]-8'-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alaninamide (6-13)

Compound (6-12) obtained in Step 11 (0.0560 g, 0.0588 mmol) and compound (2-2) obtained in Step 1 of Example 1-2 (0.022 g, 0.053 mmol) were reacted in the same manner as in Step 13 of Example 2-1 to afford the desired compound (6-13) (0.0500 g, 63%).

MS (APCI, ESI)m/z:1354 $(M+H)^+$

[Synthesis of glycan donor]

Example 3: [$N_3$-PEG(3)]-MSG1-Ox

[Formula 62]

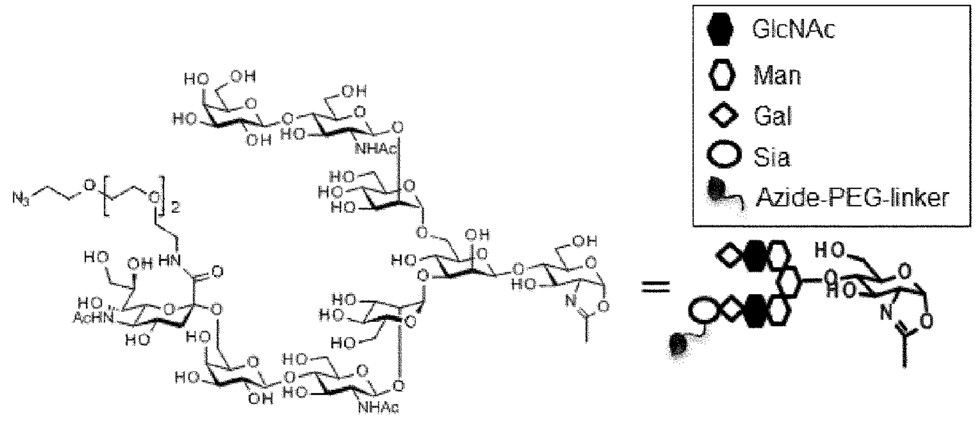


Step 1: (MSG1-)Asn

The commercially available product monosialo-Asn free (1S2G/1G2S-10NC-Asn, produced by GlyTech, Inc.) (referred to as "(MSG-)Asn") (500 mg) was subjected to separation/purification by reversed-phase HPLC under conditions below to separate into (MSG1-)Asn eluted as the 1st main peak (retention time: around 15 to 19 min) and (MSG2-)Asn eluted as the 2nd main peak (retention time: around 21 to 26 min). The eluent used was a 0.1% formic acid aqueous solution, the apparatus used was an ELS-PDA trigger preparative system (produced by JASCO Corporation), the column used was an Inertsil ODS-3 (10 um, 30ϕ × 250 mm, produced by GL Sciences, Inc.), and the flow rate was 30 mL/min. Fractions of the first peak UV-detected (210 nm) during the elution were separated, and freeze-dried to afford the desired compound (238 mg).

Step 2: MSG1

The compound obtained in Step 1 (229 mg) was dissolved in 200 mM phosphate buffer solution (pH 6.25) (1145 μL), to which an aqueous solution (100 μL) of EndoM (produced by Tokyo Chemical Industry Co., Ltd., 1 U/mL)) was added, and the resultant was incubated at 35°C for 6 days. After the completion of the reaction, the reaction solution was subjected to ultrafiltration with a VIVASPIN 15R (Hydrosart membrane, 30K, 6,000 ×G), and the filtered solution obtained was subjected to separation/purification by reversed-phase HPLC. The eluent used was a 0.1% trifluoroacetic acid aqueous solution, the apparatus used was an ELS-PDA trigger preparative system (produced by JASCO Corporation), and the column used was an Inertsil ODS-3 (produced by GL Sciences, Inc.). Fractions corresponding to the peak of the desired compound UV-detected (210 nm) during the elution were separated, and freeze-dried to afford the desired compound (117 mg).

Step 3: [$N_3$-PEG(3)]-MSG1

Into a 5 mL sampling tube (Ina-Optica Co., Ltd.), 11-azide-3,6,9-trioxaundecane-1-amine (0.108 mL, 0.541 mmol) and an aqueous solution (1.2 mL) of MSG1 obtained in Step 2 (117 mg, 0.068 mmol) were added, and the resultant was stirred for 1 hour and then freeze-dried. Into the 5 mL sampling tube after freeze-drying, an N,N-dimethylformamide solution (1.2 mL) of O-(7-azabenzotriazol-1-yl)-N,N,N',N'-tetramethyluronium hexafluorophosphate (103 mg, 0.27 mmol) and diisopropylethylamine (0.046 mL, 0.27 mmol) were added, followed by stirring at 37°C for 3 hours. After the completion of the reaction, the reaction solution was transferred into a centrifuge tube (50 mL) into which diethyl ether (20 mL) had been added in advance. The solid matter was precipitated by using a small centrifuge (Hitachi Koki Co., Ltd., CF16RX) and the supernatant was removed. Diethyl ether (10 mL) was further added, and the resultant was centrifuged and then decanted. Subsequently, acetonitrile (10 mL) was added and the resultant was subjected twice to an operation of centrifugation followed by decantation, and dried under reduced pressure to afford a crude product. The resulting solid matter was subjected to separation/purification by reversed-phase HPLC under the same conditions as in Step 2 to afford the desired compound (94.2 mg).

Step 4: [$N_3$-PEG(3)]-MSG1-Ox

Into a 5 mL sampling tube (produced by Ina-Optica Co., Ltd.), the compound synthesized in Step 3 (100 mg)

and an aqueous solution (520 μL) of 2-chloro-1,3-dimethyl-1H-benzimidazol-3-ium-chloride (produced by FUSHIMI Pharmaceutical Co., Ltd., 56 mg, 0.257 mmol) was added. To the reaction solution after being ice-cooled, an aqueous solution (520 μL) of tripotassium phosphate (165 mg, 0.78 mmol) was added, followed by stirring under ice-cooling for 3 hours. The resulting reaction solution was subjected to ultrafiltration with an Amicon Ultra (Ultracel 30K, produced by Merck Millipore) to remove the solid matter. The filtered solution was purified by gel filtration chromatography. The apparatus used was a Purif-Rp2 (produced by Shoko Scientific Co., Ltd.), the column used was a HiPrep 26/10 Desalting (produced by GE Healthcare), the mobile phase used was 0.03%-$NH_3$ aqueous solution, and the flow rate was 10 mL/min and the fraction volume was 10 mL. Fractions containing the desired compound UV-detected (220 nm) during the elution were collected together, to which a 1 N aqueous solution of sodium hydroxide (104 μL, 0.104 mmol) was added, and the resultant was freeze-dried to afford the desired compound (84 mg).

Example 4: [$N_3$-PEG(3)]-MSG-Ox

[Formula 63]

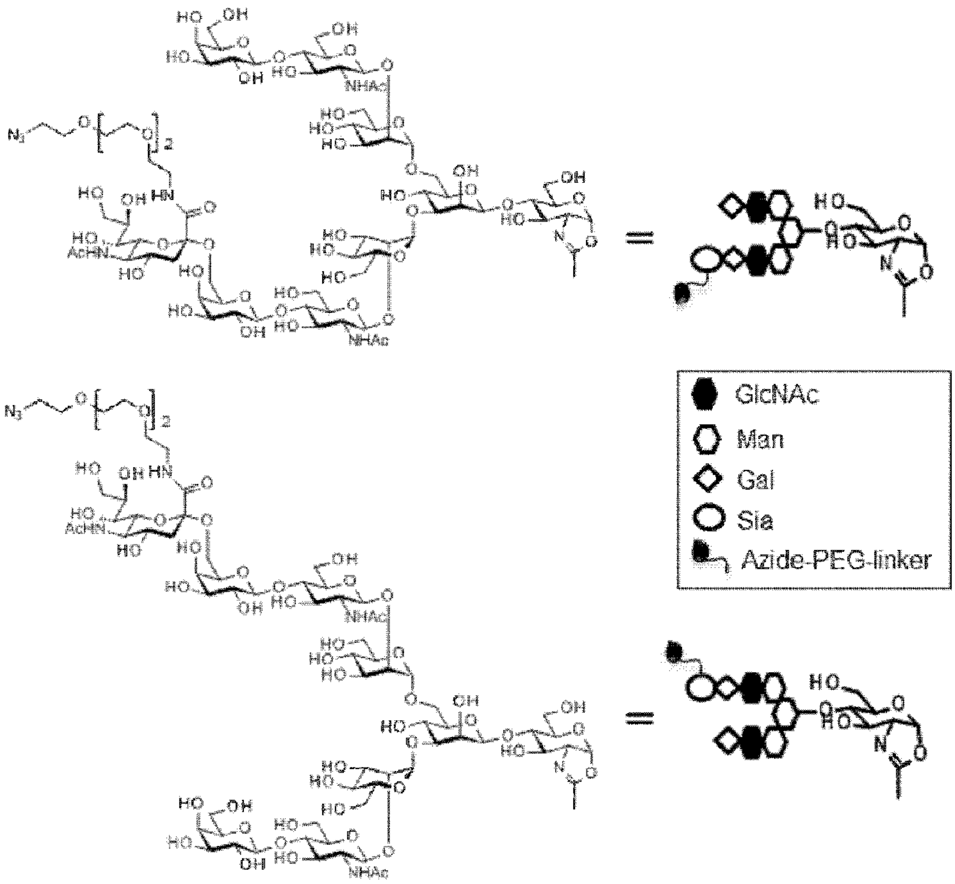


Step 1: Preparation of (MSG-)Asn

The commercially available product 1S2G/1G2S-10NC-Asn-Fmoc (produced by GlyTech, Inc.) (referred to as "Fmoc-(MSG-)Asn") (1000 mg) was dissolved in ethanol/water (1/1) (10 mL), to which a 1 N aqueous solution of sodium hydroxide (1.75 mL, 4 equivalents) was added, followed by stirring at room temperature for 3 hours. After the completion of the reaction, the reaction solution was subjected to ultrafiltration with an Amicon Ultra (30K, produced by Millipore Corporation) to remove the solid matter, and 1 N hydrochloric acid (832 μL, 1.9 equivalents) was added to the filtered solution obtained. The solvent was removed with the high-speed evaporator V-10 (produced by Biotage). Acetonitrile was added thereto, and the solvent was removed with the high-speed evaporator V-10 (produced by Biotage), and the resultant was then subjected to separation/purification by reversed-phase HPLC. The eluent was a 0.1% trifluoroacetic acid aqueous solution and a 0.1% trifluoroacetic acid acetonitrile solution, the apparatus used was a Purif-Rp2 (produced by Shoko Scientific Co., Ltd.), and the column used was an Inertsil ODS-3 (produced by GL Sciences, Inc.). Fractions

containing the desired compound UV-detected (220 nm) during the elution were collected together, and freeze-dried. This was dissolved again in pure water, and a pH test paper strip indicated that the solution was acidic. Hence, 18% aqueous ammonia (150 μL) was added thereto and it was confirmed with a pH test paper strip that the solution had become basic, and the solution was freeze-dried again. The desired compound obtained (840 mg) was directly used for the subsequent reaction.

Step 2: Synthesis of MSG

The compound obtained in Step 1 (840 mg) was dissolved in 200 mM phosphate buffer solution (pH 6.25) (6000 μL), to which an aqueous solution (200 μL) of EndoM (produced by Tokyo Chemical Industry Co., Ltd., 1 U/mL)) was added, and the resultant was incubated at 28°C for 26 hours. Because the reaction had not completed, an aqueous solution (50 μL) of EndoM (produced by Tokyo Chemical Industry Co., Ltd., 1 U/mL)) was added, and the resultant was incubated at 28°C for 2 hours, and then left to stand at room temperature until the completion of the reaction. After the completion of the reaction, the reaction solution was subjected to ultrafiltration with an Amicon Ultra (30K, produced by Millipore Corporation). Trifluoroacetic acid (80 μL) was added to the filtered solution obtained, which was subjected to separation/purification by reversed-phase HPLC. The eluent was a 0.1% trifluoroacetic acid aqueous solution and a 0.1% trifluoroacetic acid acetonitrile solution, the apparatus used was a Purif-Rp2 (produced by Shoko Scientific Co., Ltd.), and the column used was an Inertsil ODS-3 (produced by GL Sciences, Inc.). Fractions containing the desired compound UV-detected (220 nm) during the elution were collected together, and freeze-dried. This was dissolved again in pure water in order to remove the residual trifluoroacetic acid, and thus the desired compound (618 mg) was obtained as a colorless solid.
ESI-MS: Calcd for $C_{66}H_{110}N_{4}0_{49}$ : $[M+H]^+$ 1743.62, Found 1743.63

Step 3: Synthesis of $[N_3$-PEG (3)]-MSG

In accordance with the procedure of Step 3 of Example 3 using the compound obtained in Step 2 (120 mg), the desired compound (88.6 mg) was obtained.
ESI-MS: Calcd for $C_{73}H_{124}N_{8}O_{51}$ :$[M+2H]^{2+}$ 965.37, Found 965.37

Step 4 Synthesis of $[N_3$-PEG (3)]-MSG-Ox

In accordance with the procedure of Step 4 of Example 3 using the compound obtained in Step 3 (100 mg), the desired compound (88 mg) was obtained.

Example 5: $[N_3$-PEG $(3)]_2$-SG (10)-Ox

[Formula 64]

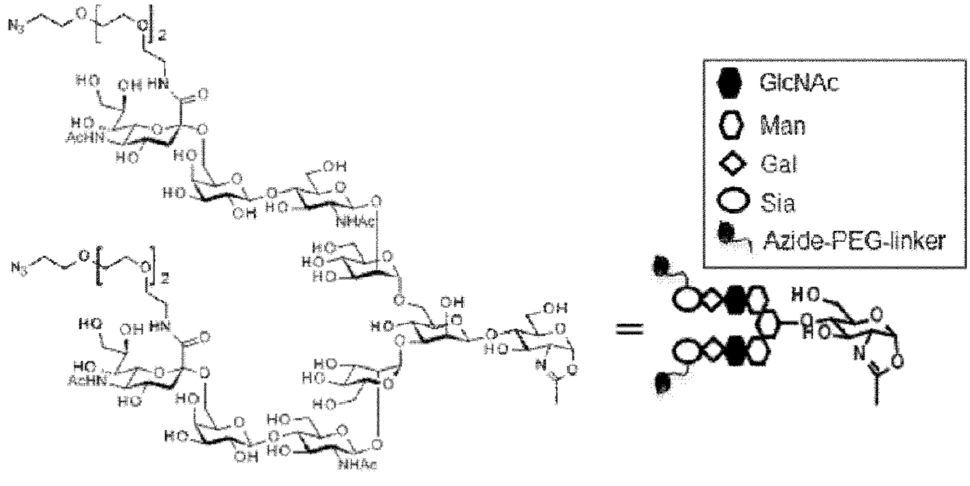

Step 1: $[N_3$-PEG $(3)]_2$-SG (10)

Into a 5 mL sampling tube (Ina-Optica Co., Ltd), an aqueous solution (0.5 mL) of 11-azide-3,6,9-trioxaundecane-1-amine (0.096 mL, 0.485 mmol) and disialooctasaccharide (50 mg, 0.24 mmol) were added, and the resultant was stirred for 1 hour and then freeze-dried. Into the 5 mL sampling tube after freeze-drying, an N,N-dimethylformamide solution (0.6 mL) of O-(7-azabenzotriazol-1-yl)-N,N,N',N'-tetramethyluronium hexafluorophosphate (92 mg, 0.24 mmol) and diisopropylethylamine (0.042 mL, 0.24 mmol) were added, followed by stirring at 37°C for 4 hours. After the completion of the reaction, the reaction solution was transferred into a centrifuge tube (50 mL) into which diethyl ether (20 mL) had been added in advance. The solid matter was precipitated by using a small centrifuge (Hitachi Koki Co., Ltd., CF16RX) and the supernatant was removed. Diethyl ether (20 mL) was added and the resultant was decanted. Subsequently, acetonitrile (20 mL) was added and the resultant was decanted, and then dried under reduced pressure to afford a crude product. The resulting solid matter was dissolved in an appropriate amount of a 0.2% trifluoroacetic acid aqueous solution, and subjected to separation/purification by reversed-phase HPLC. The eluent was a 0.1% trifluoroacetic acid aqueous solution and a 0.1% trifluoroacetic acid acetonitrile solution, the apparatus used was a Purif-Rp2 (produced by Shoko Scientific Co., Ltd.), and the column used was an Inertsil ODS-3 (produced by GL Sciences, Inc.). Fractions containing the desired compound UV-detected (220 nm) during the elution were collected together, and freeze-dried to afford the desired compound (42 mg).

Step 2: $[N_3$-PEG $(3)]_2$-SG (10)-Ox

Into a 5 mL sampling tube (produced by Ina-Optica Co., Ltd), the compound synthesized in Step 1 (40 mg) and an aqueous solution (200 $\mu$L) of 2-chloro-1,3-dimethyl-1H-benzimidazol-3-ium-chloride (produced by FUSHIMI Pharmaceutical Co., Ltd. 17.9 mg, 0.083 mmol) was added. To the reaction solution after being ice-cooled, an aqueous solution (200 $\mu$L) of tripotassium phosphate (52.6 mg, 0.25 mmol) was added, followed by stirring under ice-cooling for 2 hours. The resulting reaction solution was subjected to ultrafiltration with an Amicon Ultra (Ultracel 30K, produced by Merck Millipore) to remove the solid matter. The filtered solution was purified by gel filtration chromatography. The apparatus used was a Purif-Rp2 (produced by Shoko Scientific Co., Ltd.), the column used was a HiPrep 26/10 Desalting (produced by GE Healthcare), the mobile phase used was 0.03% $NH_3$ aqueous solution, and the flow rate was 10 mL/min and the fraction volume was 10 mL. Fractions containing the desired compound UV-detected (220 nm) during the elution were collected together, to which a 1 N aqueous solution of sodium hydroxide (33 $\mu$L, 0.033 mmol) was added, and the resultant was freeze-dried to afford the desired compound (34 mg).

Example 6: Mouse anti-CLDN6 antibody B1-producing hybridoma (218B1) and mouse anti-CLDN6 antibody C7-producing hybridoma (218C7)

6-1. Immunization of mice and acquisition of hybridomas

1-1) Preparation of cells for immunization of mice

In RPMI-1640 (Roswell Park Memorial Institute-1640) 10% FBS (fetal bovine serum) (+) medium (10 mL or 20 mL), $2 \times 10^6$ or $5 \times 10^6$ NOR-P1 cells (human pancreatic cancer cell line, RIKEN RCB-2139) were cultured for 5 days and then collected, and washed twice with PBS (phosphate buffered saline) and resuspended in PBS (300 $\mu$L).

1-2) Immunization of mice

Each BALB/c mouse (12-week-old) was intraperitoneally immunized with NOR-P1 cells ($2 \times 10^6$ cells) at intervals of about 1 week for the first to fifth immunization. About 2 weeks after the fifth immunization, each BALB/c mouse was intraperitoneally immunized with NOR-P1 cells ($5 \times 10^6$ cells). About 3 weeks after the sixth immunization, each BALB/c mouse was intraperitoneally immunized with NOR-P1 cells ($2 \times 10^6$ cells). Each BALB/c mouse was intraperitoneally immunized with $2 \times 10^6$ NOR-P1 cells at intervals of about 2 weeks for the 8th to 10th immunization. About 3 weeks after the 10th immunization (11th immunization) and 3 days thereafter (12th immunization, final immunization), each BALB/c mouse was intraperitoneally immunized with $5 \times 10^6$ NOR-P1 cells. Splenocytes were isolated 3 days after the final immunization.

1-3) Preparation of splenocytes from immunized mice

The spleen was isolated from each immunized mouse, triturated, and suspended in RPMI1640 10% FBS (+) medium. The cell suspension was passed through a Cell Strainer (70 $\mu$m, BD Falcon), and then centrifuged at 1500

rpm at room temperature for 5 minutes to discard the supernatant. Tris-$NH_4Cl$ solution (20 mM Tris-HCl pH 7.2, 77.6 mM $NH_4Cl$; 20 mL) was added thereto, and the resultant was treated at room temperature for 5 minutes. PBS (20 mL) was added thereto, and the resultant was centrifuged at 1500 rpm at room temperature for 5 minutes. After the supernatant was discard, RPMI1640 FBS (+) medium (10 mL) was added to the residue.

1-4) Preparation of myeloma cells

P3U1 cells (mouse myeloma cell line) was cultured in RPMI1640 FBS (+) medium for 5 days, and then collected and resuspended in RPMI1640 FBS (+) medium (20 mL).

1-5) Cell fusion

Splenocytes and myeloma cells were mixed together at 5:1, and centrifuged at 1500 rpm at room temperature for 5 minutes. The cells were washed twice with RPMI1640 FBS (-) medium (10 mL), and then centrifuged (1500 rpm, 5 minutes). The group of cells in the precipitated fraction obtained was sufficiently loosened, and polyethylene glycol-1500 (PEG-1500; 1 mL) was then gradually added thereto with stirring over about 1 minute. After stirring for 3 minutes 30 seconds, the resultant was left to stand at room temperature for 30 seconds. Thereafter, RPMI medium 10% Low IgG FBS (+) (10 mL) was added to the cell solution over 1 minute. The cell suspension was centrifuged (1500 rpm, 5 minutes), and the cells in the precipitated fraction obtained were gently loosened, and then gently suspended in HAT medium (RPMI1640 medium containing 10% Low IgG FBS, HAT Media Supplement, and 5% BriClone; 200 mL). The suspension was aliquoted into a 96-well culture plate at 200 μL/well, and cultured in an incubator at 37°C and 5% $CO_2$ for 6 days.

1-6) Screening of hybridomas/preparation of probe

DT3C, a recombinant complex protein, was produced for the purpose of assaying internalization of antibodies and immunotoxin activity. This DT3C is a protein formed by fusing the catalytic domain of diphtheria toxin (DT) and the antibody-binding domain of streptococcal protein G through genetic engineering. DT3C specifically binds to the Fc region of antibodies, and induce cell death through protein synthesis inhibition when being incorporated in a cell. Use of this system allows simultaneous observation of the internalization of an antibody and the cytocidal effect of immunotoxin (Yamaguchi, M. et al., Biochemical and Biophysical Research Communications 454 (2014) 600-603).

1-7) Screening of hybridomas with DT3C

To a 96-well plate, 4 μg/mL DT3C (25 μL) was added, and the culture supernatant of the hybridoma obtained in Step 1-5 (25 μL) was further added, and the resultant was incubated at room temperature for 30 minutes. NOR-P1 cells (50 μL) were seeded at $2 \times 10^5$ cells/mL (RPMI medium 10% Low IgG FBS (+)), and cultured in a $CO_2$ incubator at 37°C for 3 days. Through microscopic observation after culturing, wells with the number of adhering cells being about 25% or less of that in using a negative control antibody were determined to be positive. Selected clones were subjected to one or two subcloning steps to establish eight monoclonal hybridoma cell lines.

6-2: Identification of antigen to which antibody produced by hybridoma binds

Antigens were identified for two clones, 218B1 and 218C7, of antibodies produced by the hybridomas prepared in Example 6-1.

2-1) Immunoprecipitation of biotin-labeled cell surface protein with 218B1 antibody and 218C7 antibody

Culture supernatant of $2 \times 10^6$ NTERA-2 cells (human testicular cancer cell line, ATCC CRL-1973) was removed, and the residue was washed twice with PBS. EZ-Link Sulfo-NHS-Biotin (Thermo Fisher Scientific) was suspended in PBS to a concentration of 0.1 mg/mL. After PBS was removed, Biotin/PBS solution was added, and the resultant was incubated on a shaker for 30 minutes, and then washed twice with 100 mM glycine/PBS solution (25 mL) and then washed once with PBS (10 mL). The washed cells were resuspended in 200 μL of lysis buffer (150 mM NaCl, 50 mM Tris-HCl pH 7.4, 1% DDM, Protease inhibitor, Complete EDTA free (F. Hoffmann-La Roche, Ltd.) 1 particle/50 mL), and treated at 4°C for 30 minutes. The resultant was centrifuged (13000 rpm, 20 minutes, 4°C) to prepare a cell lysate. To the cell lysate, Protein G Sepharose/lysis buffer (50% slurry; 30 μL) obtained by substituting the buffer of Protein G Sepharose (Protein G Sepharose 4 Fast Flow (GE Healthcare)) with the lysis buffer was added, and the resultant was rotated at 4°C for 30 minutes and then centrifuged at 4°C for 1 minute, and the supernatant was collected. To this

supernatant the 218B1 antibody or 218C7 antibody (about 3 μg) was added, and the resultant was rotated at 4°C for 30 minutes, to which Protein G Sepharose/lysis buffer (50% slurry; 60 μL) was then added, and the resultant was rotated at 4°C for 1 hour. The Protein G Sepharose was washed six times with the lysis buffer (1 mL), and then resuspended in 1 × SDS sample buffer (Bio-Rad Laboratories, Inc.). After the suspension was treated at 100°C for 5 minutes, the solution was collected as a sample for SDS-PAGE (polyacrylamide gel electrophoresis).

2-2) SDS-PAGE and Western blotting

The SDS-PAGE sample prepared in 2-1) was stacked with SuperSep Ace 5-20% (Wako Pure Chemical Industries, Ltd.) at 50 mV for 30 minutes, and then subjected to electrophoresis at 200 mV for 1 hour, and blotted from the gel onto a membrane at 12 mV for 47 minutes. The membrane was washed with PBS-T (PBS (-)-0.02% Tween 20), and then blocked for 1 hour. The membrane was washed three times with PBS-T for 5 minutes, and then reacted with a Streptavidin-horseradish peroxidase conjugate (GE Healthcare; 2000-fold diluted with PBS-T in use) for 1 hour. The membrane was washed twice with PBS-T for 5 minutes, and a targeted band was then detected by using an enhanced chemiluminescence (ECL) method. A band indicating a molecular weight of 18 kDa was detected for any of the case with the 218B1 antibody and the case with the 218C7 antibody, regardless of the presence or absence of DTT added.

2-3) Mass spectrometry of immunoprecipitated product of cell protein with 218B1 antibody and 218C7 antibody

2 × $10^7$ NTERA-2 cells were collected and washed twice with PBS. The cells were collected by using a cell scraper, and centrifuged at 1500 rpm for 5 minutes.
After the supernatant was removed, the cells were resuspended in 2 mL of the lysis buffer, and treated at 4°C for 30 minutes. The resultant was centrifuged (13000 rpm, 20 minutes, 4°C) to prepare a cell lysate. Protein G Sepharose/lysis buffer (50% slurry; 180 μL) was added to the cell lysate, and the resultant was rotated at 4°C for 30 minutes and then centrifuged at 4°C for 1 hour, and the supernatant was collected. The 218B1 antibody (about 9 μg) was added to the supernatant, and the resultant was rotated at 4°C for 30 minutes, to which Protein G Sepharose/lysis buffer (50% slurry; 180 μL) was then added, and the resultant was rotated at 4°C for 1 hour. The Protein G Sepharose was washed six times with the lysis buffer (1 mL), and then resuspended in 1 × SDS sample buffer. After the suspension was treated at 100°C for 5 minutes, the solution was collected as a sample for SDS-PAGE. SDS-PAGE was carried out in the same manner as in 2-2), and the electrophoresis gel was stained with CBB. The part corresponding to 18 kDa was cut out of the electrophoresis gel, and subjected to mass spectrometry. The mass spectrometry found that the gel piece contained claudin-6.

2-4) FACS analysis

Since the antigen for the 218B1 antibody and 218C7 antibody was estimated to be claudin-6 from the mass spectrometry, forced expression analysis by cDNA transfection was carried out. FACS analysis results showed that the 218B1 antibody and 218C7 antibody exhibited strong positive reaction for human claudin-6-expressing CHO-K1 cells, demonstrating that the antigen for the 218B1 antibody and 218C7 antibody is claudin-6.

2-5) Purification of antibody from hybridoma culture supernatant

The mouse anti-CLDN6 antibody B 1-producing hybridoma (218B1) and mouse anti-CLDN6 antibody C7-producing hybridoma (218C7) were cultured in Hybridoma-SFM (Thermo Fisher Scientific) containing 10% Fetal Bovine Serum, Ultra-Low IgG (Thermo Fisher Scientific). The culture supernatant was collected by centrifugation, and filtered through a filter of 0.45 μm (produced by Corning Incorporated). The antibody was purified from the culture supernatant through rProtein A affinity chromatography (at 4 to 6°C) in one step. The step of buffer displacement after rProtein A affinity chromatography was carried out at 4 to 6°C. First, the culture supernatant was applied to a column packed with MabSelectSuRe (produced by GE Healthcare Bioscience) equilibrated with PBS. After the culture solution completely entered the column, the column was washed with PBS in an amount twice or more the column volume. Subsequently, elution was carried out with a 2 M solution of arginine hydrochloride (pH 4.0), and a fraction containing the antibody was collected. The fraction was subjected to liquid displacement to PBS (-) by dialysis (Thermo Scientific, Slide-A-Lyzer Dialysis Cassette). Finally, the fraction was concentrated with a Centrifugal UF Filter Device VIVASPIN20 (molecular weight cutoff: UF10K, Sartorius AG, at 4°C) to adjust the IgG concentration to 1 mg/mL or more. The fraction was filtered through a Minisart-Plus filter (Sartorius AG), and the resultant was used as a purified sample.

Example 7: In vitro evaluation of mouse anti-CLDN6 antibodies B1 and C7

7-1: Evaluation of binding ability of mouse anti-CLDN6 antibodies by flow cytometry

Binding activity of the mouse anti-CLDN6 antibodies produced in Example 6 to human CLDN6 and its family molecules, CLDN3, CLDN4, and CLDN9, was evaluated by using a flow cytometry method. Human CLDN3/pCMV6-Entry, human CLDN4/pCMV6-Entry, human CLDN6/pCMV-Entry, human CLDN9/pCMV6-Entry, or pCMV6-Entry purchased from OriGene Technologies, Inc. was transiently transferred into 293T cells (Thermo Fisher Scientific, HCL4517) by using Lipofectamine 2000 (Thermo Fisher Scientific), and the cells were cultured under conditions of 37°C and 5% $CO_2$ overnight, and then a cell suspension was prepared. The transfected 293T cell suspension was centrifuged to remove the supernatant, and a mouse anti-CLDN6 antibody (clone number: B1 or C7) or a mouse IgGI control antibody (R&D Systems, Inc.) was then added and suspended to a final concentration of 30 μg/mL, 10 μg/mL, 3.3 μg/mL, or 1.1 μg/mL, and the resultant was left to stand at 4°C for 1 hour. The cells were washed twice with Dulbecco's phosphate buffered saline (Sigma-Aldrich Co. LLC) containing 5% fetal bovine serum (Hyclone) (hereinafter, referred to as 5% FBS-containing PBS), and FLUORESCEIN-CONJUGATED GOAT IGG FRACTION TO MOUSE IGG (WHOLE MOLECULE) (MP Biomedicals, Inc.) 500-fold diluted with 5% FBS-containing PBS was then added thereto, and the cells were suspended and left to stand at 4°C for 1 hour. After washing twice with 5% FBS-containing PBS, detection was carried out by using a flow cytometer (FC500; Beckman Coulter, Inc.). Data analysis was carried out by using FlowJo (Tree Star, Inc.). To confirm each transfection, the cells were permeabilized with 0.25% Tween 20-containing PBS, and then a mouse anti-FLAG antibody (Sigma-Aldrich Co. LLC) was used. Figure 40 shows the results. In each graph in Figure 40, the ordinate represents FITC fluorescence intensity indicating the amount of the binding antibody and the abscissa represents antibody concentrations. The mouse anti-CLDN6 antibodies produced bound to human CLDN6 and human CLDN9 to a similar degree, and did not bind to human CLDN3 or human CLDN4. The mouse control IgG1 did not bind to any of the cells.

7-2: Internalization activity of antibodies

Internalization activity of the mouse anti-CLDN6 antibodies B1 and C7 was evaluated by using the anti-mouse IgG reagent, to which a toxin that inhibits protein synthesis (saporin) had been conjugated, Mab-ZAP (Advanced Targeting Systems). In this evaluation, Mab-ZAP is incorporated into cells in a manner depending on the internalization activity of a mouse anti-CLDN6 antibody, and saporin, which inhibits protein synthesis, is released in the cells to suppress cell growth.

JEG-3(ATCC HTB-36), a human choriocarcinoma cell line of human CLDN6-positive cells, NIH:OVCAR-3 (ATCC HTB-161), a human ovarian cancer cell line of human CLDN6-positive cells, or BxPC-3 (ATCC CRL-1687), a human pancreatic cancer cell line of human CLDN6-negative cells, was seeded in a 96-well cell culture microplate at $2 \times 10^3$ cells/well, and cultured under conditions of 37°C and 5% $CO_2$ overnight. On the next day, a mixed solution obtained by mixing each mouse anti-CLDN6 antibody or mouse IgG1 antibody (R&D Systems, Inc.) to a final concentration of 1 nM, with Mab-ZAP (final concentration: 0.5 nM) or AffiniPure Goat Anti-Mouse IgG, Fcγ Fragment Specific (Jackson ImmunoResearch Laboratories Inc.) (final concentration: 0.5 nM), without conjugated toxin, was added, and the cells were cultured under conditions of 37°C and 5% $CO_2$ for 5 days. The number of surviving cells was determined through quantification of ATP activity by using CellTiter-Glo Luminescent Cell Viability Assay (Promega Corporation). The cell growth-suppressing effect by addition of each anti-CLDN6 antibody was determined as a relative survival rate to the value for the well without the mixed solution as 100%. Figure 41 shows the results. The mouse anti-CLDN6 antibodies (B1, C7) were found to have cell growth-suppressing effect on the human CLDN6-positive cell lines JEG-3 and NIH:OVCAR-3. On the other hand, they were found to have no cell growth-suppressing effect on the human CLDN6-negative cell line BxPC-3. The mouse IgG1 antibody was found to have no cell growth-suppressing effect on any of the cell lines. These results suggest that the anti-CLDN6 antibodies (B1, C7) produced have internalization activity and are each suitable as an antibody for antibody-drug conjugates.

Example 8: Nucleotide sequencing of cDNA encoding variable region of each of mouse anti-CLDN6 antibodies B1 and C7

8-1: Nucleotide sequencing of cDNA encoding variable region of B1 antibody

8-1-1: Preparation of total RNA of B1 antibody-producing hybridoma

To amplify cDNA encoding the variable region of the B1 antibody, total RNA was prepared from the B1 antibody-producing hybridoma by using TRIzol Reagent (Ambion).

8-1-2: Amplification and sequencing of cDNA encoding light chain variable region of B1 antibody through 5'-RACE PCR

Amplification of cDNA encoding the light chain variable region was carried out by using about 1 μg of the total RNA prepared in Example 8-1-1 and a SMARTer RACE 5'/3' Kit (Clontech). As a primer to amplify cDNA encoding the variable region of the light chain gene of the B1 antibody through PCR, UPM (Universal Primer A Mix: attached to the SMARTer RACE 5'/3' Kit) and a primer designed on the basis of the sequence of a known mouse light chain constant region were used.

The cDNA encoding the variable region of the light chain amplified through 5'-RACE PCR was cloned into a plasmid, and subsequently sequence analysis was carried out for the nucleotide sequence of the cDNA encoding the variable region of the light chain.

The determined nucleotide sequence of the cDNA encoding the variable region of the light chain of the B1 antibody is represented by SEQ ID NO: 18, and the corresponding amino acid sequence is represented by SEQ ID NO: 19.

8-1-3: Amplification and sequencing of cDNA encoding heavy chain variable region of B1 antibody through 5'-RACE PCR

Amplification of cDNA encoding the heavy chain variable region was carried out by using about 1 μg of the total RNA prepared in Example 8-1-1 and a SMARTer RACE 5'/3' Kit (Clontech). As a primer to amplify cDNA encoding the variable region of the heavy chain gene of the LB1 antibody through PCR, UPM (Universal Primer A Mix: attached to the SMARTer RACE 5'/3' Kit) and a primer designed on the basis of the sequence of a known mouse heavy chain constant region were used.

The cDNA encoding the variable region of the heavy chain amplified through 5'-RACE PCR was cloned into a plasmid, and subsequently sequence analysis was carried out for the nucleotide sequence of the cDNA encoding the variable region of the heavy chain.

The determined nucleotide sequence of the cDNA encoding the variable region of the heavy chain of the B1 antibody is represented by SEQ ID NO: 20, and the corresponding amino acid sequence is represented by SEQ ID NO: 21.

8-2: Nucleotide sequencing of cDNA encoding variable region of C7 antibody Nucleotide sequencing was carried out in the same manner in Example 8-1.

The determined nucleotide sequence of the cDNA encoding the variable region of the light chain of the C7 antibody is represented by SEQ ID NO: 22, and the corresponding amino acid sequence is represented by SEQ ID NO: 23. The nucleotide sequence of the cDNA encoding the variable region of the heavy chain of the C7 antibody is represented by SEQ ID NO: 24, and the corresponding amino acid sequence is represented by SEQ ID NO: 25.

Example 9: Production of chimeric anti-CLDN6 antibody chB1

9-1: Construction of expression vector for chimeric anti-CLDN6 antibody chB 1

9-1-1: Construction of expression vector pCMA-LK for chimeric and humanized light chains

About 5.4 kb of a fragment obtained by digesting the plasmid pcDNA3.3-TOPO/LacZ (Invitrogen) with the restriction enzymes XbaI and PmeI was linked to a DNA fragment including a DNA sequence encoding the human light chain signal sequence and human κ chain constant region, as represented by SEQ ID NO: 26, by using an In-Fusion HD PCR Cloning Kit (Clontech) to prepare pcDNA3.3/LK. A neomycin expression unit was removed from the pcDNA3.3/LK to construct pCMA-LK.

9-1-2: Construction of expression vector pCMA-G1LALA for chimeric and humanized IgG1LALA-type heavy chains

A DNA fragment obtained by digesting the pCMA-LK with XbaI and PmeI to remove the light chain signal sequence and human κ chain constant region was linked to a DNA fragment including a DNA sequence encoding the human heavy chain signal sequence and human IgG1LALA constant region, as represented by SEQ ID NO: 27, by using an In-Fusion HD PCR Cloning Kit (Clontech) to construct pCMA-G1LALA.

9-1-3: Construction of chimeric chB1 heavy chain expression vector

The DNA fragment consisting of nucleotide residues 36 to 440 of the nucleotide sequence for the chB1 heavy chain, as represented by SEQ ID NO: 33, was synthesized (GeneArt). The pCMA-G1LALA was cleaved with the restriction enzyme BlpI, and the synthesized DNA fragment was inserted into the cleaved portion by using an In-Fusion HD PCR

Cloning Kit (Clontech) to construct a chB 1 heavy chain expression vector. The amino acid sequence of the chB1 heavy chain is represented by SEQ ID NO: 32.

9-1-4: Construction of chimeric chB1 light chain expression vector

A DNA fragment including a DNA sequence encoding the chB 1 light chain, as represented by SEQ ID NO: 29, was synthesized (GeneArt). By using an In-Fusion HD PCR Cloning Kit (Clontech), the synthesized DNA fragment was linked to a DNA fragment obtained by digesting the pCMA-LK with XbaI and PmeI for removal of the light chain signal sequence and human κ chain constant region to construct a chB 1 light chain expression vector. The amino acid sequence of the chB1 light chain is represented by SEQ ID NO: 28.

9-2: Production and purification of chimeric anti-CLDN6 antibody chB 1

9-2-1: Production of chimeric antibody chB1

FreeStyle 293F cells (Invitrogen) were passaged and cultured in accordance with the instruction manual. Into a 3 L Fernbach Erlenmeyer Flask (Corning Incorporated), $1.2 \times 10^9$ FreeStyle 293F cells (Invitrogen) in the logarithmic growth phase were seeded, and diluted with FreeStyle293 expression medium (Invitrogen) to adjust to $2.0 \times 10^6$ cells/mL. To 40 mL of Opti-Pro SFM medium (Invitrogen), 0.24 mg of the heavy chain expression vector, 0.36 mg of the light chain expression vector, and 1.8 mg of polyethyleneimine (Polyscience, Inc., #24765) were added and gently stirred, and further left to stand for 5 minutes, and then added to the FreeStyle 293F cells. After shaking culture at 90 rpm in an incubator at 37°C and 8% $CO_2$ for 4 hours, 600 mL of EX-CELL VPRO medium (SAFC Biosciences, Inc.), 18 mL of GlutaMAX I (Gibco), and 30 mL of Yeastolate Ultrafiltrate (Gibco) were added, and the resultant was subjected to shaking culture at 90 rpm in an incubator at 37°C and 8%$CO_2$ for 7 days, and the resulting culture supernatant was filtered through a Disposable Capsule Filter (ADVANTEC, #CCS-045-E1H). The chimeric anti-CLDN6 antibody obtained was designated as "chB1".

9-2-2: Purification of chimeric antibody chB1

The antibody was purified from the culture supernatant obtained in Example 9-2-1 through rProtein A affinity chromatography in one step. The culture supernatant was applied to a column packed with MabSelectSuRe (produced by GE Healthcare Bioscience) equilibrated with PBS, and the column was then washed with PBS in an amount twice or more the column volume. Subsequently, elution was carried out with a 2 M solution of arginine hydrochloride (pH 4.0), and a fraction containing the antibody was collected. The antibody was subjected to buffer displacement to PBS (-) by dialysis (Thermo Scientific, Slide-A-Lyzer Dialysis Cassette). The fraction was concentrated with a Centrifugal UF Filter Device VIVASPIN20 (molecular weight cutoff: UF10K, Sartorius AG) to adjust the IgG concentration to 1 mg/mL or more. Finally, the fraction was filtered through a Minisart-Plus filter (Sartorius AG), and the resultant was used as a purified sample.

Example 10: Production of humanized anti-CLDN6 antibody

10-1: Design of humanized form of anti-CLDN6 antibody

10-1-1: Molecular modeling of variable region of chimeric antibody chB1

A method known as homology modeling (Methods in Enzymology, 203, 121-153 (1991)) was used for molecular modeling of the variable region of chB1. Molecular modeling was carried out by using the commercially available protein conformational analysis program BioLuminate (Schrodinger, Inc.) with a structure (PDB ID: 1XIW), as a template, registered in Protein Data Bank (Nuc. Acid Res. 35, D301-D303 (2007)) with high sequence identity to the variable regions of the heavy chain and light chain of chB 1.

10-1-2: Design of humanized amino acid sequence

chB1 was humanized by CDR grafting (Proc. Natl. Acad. Sci. USA 86, 10029-10033 (1989)). The consensus sequence of human gamma chain subgroup 1 and that of human kappa chain subgroup 1 specified in Kabat et al. (Sequences of Proteins of Immunological Interest, 5th Ed. Public Health Service National Institutes of Health, Bethesda, MD. (1991)) had high identity to the framework regions of the chB1, and hence were respectively selected as acceptors for the heavy chain and the light chain. Donor residues to be transferred on the acceptors were selected through analysis

of the three-dimensional model, for example, with reference to criteria provided by Queen et al. (Proc. Natl. Acad. Sci. USA 86, 10029-10033 (1989)). Because the CDRL3 was rich in hydrophobic amino acids, a humanized light chain with mutation in the CDRL3 was additionally designed.

10-2: Humanization of chB1 heavy chain

The three heavy chains designed were designated as hH1, hH2, and hH3. The heavy chain full-length amino acid sequence of hH1 is represented by SEQ ID NO: 52. The nucleotide sequence encoding the amino acid sequence of SEQ ID NO: 52 is represented by SEQ ID NO: 53. The heavy chain full-length amino acid sequence of hH2 is represented by SEQ ID NO: 56. The nucleotide sequence encoding the amino acid sequence of SEQ ID NO: 56 is represented by SEQ ID NO: 57. The heavy chain full-length amino acid sequence of hH3 is represented by SEQ ID NO: 60. The nucleotide sequence encoding the amino acid sequence of SEQ ID NO: 60 is represented by SEQ ID NO: 61.

10-3: Humanization of chB1 light chain

The four light chains designed were designated as hL1, hL2, hL3, and hL4. The light chain full-length amino acid sequence of hL1 is represented by SEQ ID NO: 36. The nucleotide sequence encoding the amino acid sequence of SEQ ID NO: 36 is represented by SEQ ID NO: 37. The light chain full-length amino acid sequence of hL2 is represented by SEQ ID NO: 40. The nucleotide sequence encoding the amino acid sequence of SEQ ID NO: 40 is represented by SEQ ID NO: 41. The light chain full-length amino acid sequence of hL3 is represented by SEQ ID NO: 44. The nucleotide sequence encoding the amino acid sequence of SEQ ID NO: 44 is represented by SEQ ID NO: 45. The light chain full-length amino acid sequence of hL4 is represented by SEQ ID NO: 48. The nucleotide sequence encoding the amino acid sequence of SEQ ID NO: 48 is represented by SEQ ID NO: 49.

10-4: Design of humanized antibody with combination of heavy chain and light chain

An antibody consisting of hH1 and hL1 is referred to as "H1L1 antibody" or

"H1L1". An antibody consisting of hH2 and hL2 is referred to as "H2L2 antibody" or "H2L2". An antibody consisting of hH1 and hL3 is referred to as "H1L3 antibody" or "H1L3". An antibody consisting of hH2 and hL4 is referred to as "H2L4 antibody" or "H2L4". An antibody consisting of hH3 and hL3 is referred to as "H3L3 antibody" or "H3L3".

10-5: Production of humanized anti-CLDN6 antibody

10-5-1: Construction of humanized heavy chain expression vector

10-5-1-1: Construction of hH1 expression vector

The DNA fragment consisting of nucleotide residues 36 to 440 of the nucleotide sequence of SEQ ID NO: 53 for hH1 was synthesized (GeneArt). An hH1 expression vector was constructed in the same manner as in Example 9-1-3.

10-5-1-2: Construction of hH2 expression vector

The DNA fragment consisting of nucleotide residues 36 to 440 of the nucleotide sequence of SEQ ID NO: 57 for hH2 was synthesized (GeneArt). An hH2 expression vector was constructed in the same manner as in Example 9-1-3.

10-5-1-3: Construction of hH3 expression vector

The DNA fragment consisting of nucleotide residues 36 to 440 of the nucleotide sequence of SEQ ID NO: 61 for hH2 was synthesized (GeneArt). An hH3 expression vector was constructed in the same manner as in Example 9-1-3.

10-5-2: Construction of humanized light chain expression vector

10-5-2-1: Construction of hL1 expression vector

The DNA fragment consisting of nucleotide residues 37 to 402 of the nucleotide sequence of SEQ ID NO: 37 for hL1 was synthesized (GeneArt). The pCMA-LK was cleaved with the restriction enzyme BsiWI, and the synthesized

DNA fragment was inserted into the cleaved portion by using an In-Fusion HD PCR Cloning Kit (Clontech) to construct an hL1 expression vector.

10-5-2-2: Construction of hL2 expression vector

The DNA fragment consisting of nucleotide residues 37 to 402 of the nucleotide sequence of SEQ ID NO: 41 for hL2 was synthesized (GeneArt). An hL2 expression vector was constructed in the same manner as in Example 10-5-2-1.

10-5-2-3: Construction of hL3 expression vector

The DNA fragment consisting of nucleotide residues 37 to 402 of the nucleotide sequence of SEQ ID NO: 45 for hL3 was synthesized (GeneArt). An hL3 expression vector was constructed in the same manner as in Example 10-5-2-1.

10-5-2-4: Construction of hL4 expression vector

The DNA fragment consisting of nucleotide residues 37 to 402 of the nucleotide sequence of SEQ ID NO: 49 for hL4 was synthesized (GeneArt). An hL4 expression vector was constructed in the same manner as in Example 10-5-2-1.

10-5-3: Preparation of humanized antibodies

10-5-3-1: Production of humanized antibodies H1L1, H2L2, H1L3, H2L4, and H3L3

They were produced in the same manner as in Example 9-2-1. H1L1, H2L2, H1L3, H2L4, and H3L3 were produced by using the combinations of a heavy chain expression vector and a light chain expression vector corresponding to the combinations of a heavy chain and a light chain shown in Example 10-4.

10-5-3-2: Two-step purification of humanized antibodies H1L1, H2L2, H1L3, H2L4, and H3L3

The culture supernatant obtained in Example 10-5-3-1 was purified in two steps through rProtein A affinity chromatography and ceramic hydroxyapatite. The culture supernatant was applied to a column packed with MabSelect-SuRe (produced by GE Healthcare Bioscience) equilibrated with PBS, and the column was then washed with PBS in an amount twice or more the column volume. Subsequently, the antibody was eluted with a 2 M solution of arginine hydrochloride (pH 4.0). A fraction containing the antibody was subjected to buffer displacement to PBS by dialysis (Thermo Scientific, Slide-A-Lyzer Dialysis Cassette), 5-fold diluted with a buffer of 5 mM sodium phosphate/50 mM MES/pH 7.0, and then applied to a ceramic hydroxyapatite column (Bio-Rad Laboratories Japan, Inc., Bio-Scale CHT Type-1 Hydroxyapatite Column) equilibrated with a buffer of 5 mM NaPi/50 mM MES/30 mM NaCl/pH 7.0. Linear concentration gradient elution was carried out with sodium chloride, and a fraction containing the antibody was collected. The fraction was subjected to buffer displacement to HBSor (25 mM histidine/5% sorbitol, pH 6.0) by dialysis (Thermo Scientific, Slide-A-Lyzer Dialysis Cassette). The antibody was concentrated with a Centrifugal UF Filter Device VIVASPIN20 (molecular weight cutoff: UF10K, Sartorius AG) to adjust the IgG concentration to 50 mg/mL. Finally, the fraction was filtered through a Minisart-Plus filter (Sartorius AG), and the resultant was used as a purified sample.

Example 11: Evaluation of binding ability of humanized anti-CLDN6 antibody by flow cytometry

The binding activity of the humanized anti-CLDN6 antibody produced in Example 10 to human CLDN6 and its family molecules, CLDN3, CLDN4, and CLDN9, was evaluated by using a flow cytometry method. Used were 293T cells transiently transfected in the same manner as in Example 7-1. To cells into which a human CLDN6 or human CLDN9 gene had been transferred, the humanized anti-CLDN6 antibody H1L1, H2L2, H1L3, H2L4, or H3L3, or a human IgGI control antibody (Calbiochem) was added and suspended to a final concentration of 100 nM, 20 nM, 4 nM, or 0.8 nM, and the resultant was left to stand at 4°C for 30 minutes. To cells into which a human CLDN3 or human CLDN4 gene, or an empty vector had been transferred, the humanized anti-CLDN6 antibody H1L1, H2L2, H1L3, H2L4, or H3L3 was added and suspended to a final concentration of 100 nM, and the resultant was left to stand at 4°C for 30 minutes. The cells were washed with Dulbecco's phosphate buffered saline (Sigma-Aldrich Co. LLC) containing 5% fetal bovine serum (Hyclone) (hereinafter, referred to as 5% FBS-containing PBS), and FITC AffiniPureF (ab')2 Fragment Goat Anti-Human IgG (H+L) (Jackson ImmunoResearch Laboratories Inc.) 150-fold diluted with 5% FBS-containing PBS was then added

thereto, and the cells were suspended and left to stand at 4°C for 30 minutes. After washing with 5% FBS-containing PBS, detection was carried out by using a flow cytometer (FC500; Beckman Coulter, Inc.). Data analysis was carried out by using FlowJo (Tree Star, Inc.), and mean fluorescence intensity (MFI) of FITC, which indicates the amount of the binding antibody, was calculated. Figure 42 shows the results. In each graph in Figure 42, the abscissa represents antibody concentrations and the ordinate represents MFI. The humanized anti-CLDN6 antibody produced bound to human CLDN6 and human CLDN9 to a similar degree, and did not bind to human CLDN3 or human CLDN4. The human control IgG1 did not bind to any of the cells.

Example 12: Production of trastuzumab variant

12-1: Construction of heavy chain expression vector for trastuzumab-LALA

The DNA fragment consisting of nucleotide residues 36 to 434 of the nucleotide sequence of SEQ ID NO: 74 for the heavy chain of trastuzumab-LALA was synthesized (GeneArt). An expression vector was constructed in the same manner as in Example 9-1-3. The amino acid sequence of the heavy chain of trastuzumab-LALA is represented by SEQ ID NO: 75.

12-2: Construction of light chain expression vector for trastuzumab-LALA

The DNA fragment consisting of nucleotide residues 37 to 402 of the nucleotide sequence of SEQ ID NO: 72 for the light chain of trastuzumab-LALA was synthesized (GeneArt). An expression vector was constructed in the same manner as in Example 10-5-2-1. The amino acid sequence of the light chain of trastuzumab-LALA is represented by SEQ ID NO: 73.

12-3: Production of trastuzumab variant

A trastuzumab variant was produced in the same manner as in Example 9-2-1.

12-4: Purification of trastuzumab variant

Trastuzumab-LALA was purified from the culture supernatant obtained in Example 12-3 in the same manner as in Example 9-2-2, except that buffer displacement was carried out not to PBS (-) but to 50 mM phosphate buffer solution (pH 6.0).

[Preparation of glycan remodelling antibodies]

Example 13: Sugar chain remodeling 1 (T-SG)

[Formula 65]

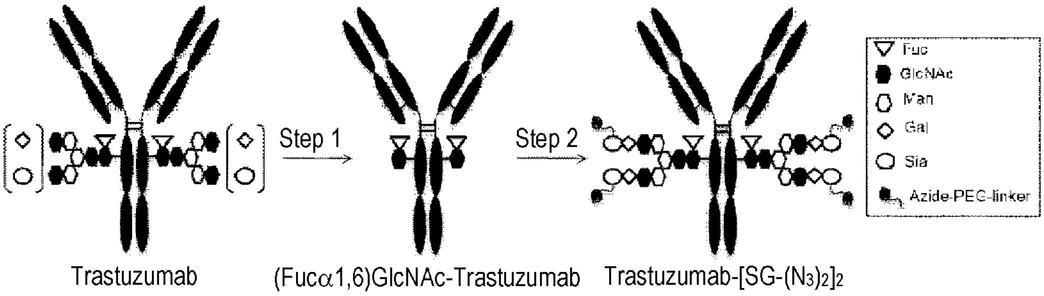


Trastuzumab (Fucα1,6)GlcNAc-Trastuzumab Trastuzumab-[SG-$(N_3)_2$]$_2$

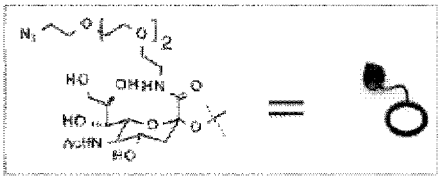

Step 1: Preparation of (Fucα1,6)GlcNAc-Trastuzumab

The 22 mg/mL trastuzumab solution (25 mM histidine solution (pH 6.0), 5% sorbitol solution) (45.5 mL) prepared in Reference Example 3 was halved and according to common operation C, buffer exchange to 50 mM phosphate buffer (pH 6.0) was carried out twice separately. To the resulting 28.1 mg/mL (18 mL) and 28.0 mg/mL (18 mL) trastuzumab solutions (50 mM phosphate buffer (pH 6.0)), 1.26 mL and 1.27 mL of wild-type EndoS solution (2.0 mg/mL, PBS) were respectively added, and the solutions were incubated at 37°C for 4 hours. The progress of the reaction was confirmed by Experion electrophoresis station (produced by Bio-Rad Laboratories, Inc.). After the completion of the reaction, purification by affinity chromatography and purification with a hydroxyapatite column were carried out in accordance with the following methods.

(1) Purification by affinity chromatography

Purification apparatus: AKTA pure150 (produced by GE Healthcare)
Column: HiTrap rProtein A FF (5 mL) (produced by GE Healthcare)
Flow rate: 5 mL/min (1.25 mL/min in charging)

Each reaction solution obtained above was purified in multiple separate operations. Two columns were linked together into one column, and in connecting to the column, the reaction solution was added to the upper part of the column, and 2 CV of binding buffer (20 mM phosphate buffer (pH 6.0)) was flowed at 1.25 mL/min and 5 CV thereof was further flowed at 5 mL/min. In intermediate washing, 15 CV of washing solution (20 mM phosphate buffer (pH 7.0), 0.5 M sodium chloride solution) was flowed. In elution, 6 CV of elution buffer (ImmunoPure IgG Eution buffer, produced by Pierce) was flowed. The eluate was immediately neutralized with 1 M Tris buffer (pH 9.0). Fractions UV-detected (280 nm) during the elution were confirmed by using the micro-volume spectrophotometer Xpose (produced by Trinean NV) and an Experion electrophoresis station (produced by Bio-Rad Laboratories, Inc.). Fractions containing the desired compound were subjected to buffer exchange to 5 mM phosphate buffer/50 mM 2-morpholinoethanesulfonic acid (MES) solution (pH 6.8) by using common operation C.

(2) Purification by hydroxyapatite chromatography

Purification apparatus: AKTA avant25 (produced by GE Healthcare)
Column: Bio-Scale Mini CHT Type I cartridge (5 mL) (produced by Bio-Rad Laboratories, Inc.)
Flow rate: 5 mL/min (1.25 mL/min in charging)

Two columns were linked together into one column, and the solution obtained in (1) was purified in multiple separate operations. The solution was added to the upper part of the column, and 2 CV of solution A (5 mM phosphate buffer/50 mM morpholinoethanesulfonic acid (MES) solution (pH 6.8)) was flowed at 1.25 mL/min and 3 CV thereof was

further flowed at 5 mL/min. Thereafter, elution was carried out with solution A and solution B (5 mM phosphate buffer/50 mM morpholinoethanesulfonic acid (MES) solution (pH 6.8), 2 M sodium chloride solution). The elution conditions were solution A:solution B = 100:0 to 0:100 (15 CV). Further, 5 CV of washing solution (500 mM phosphate buffer (pH 6.5)) was flowed.

Fractions containing the desired compound were subjected to buffer exchange by using common operation C to afford a 25.5 mg/mL (Fucα1,6)GlcNAc-Trastuzumab solution (50 mM phosphate buffer (pH 6.0)) (35 mL).

Step 2: Preparation of Trastuzumab [SG-$(N_3)_2$]$_2$

To the 23.9 mg/mL (Fucα1,6)GlcNAc-Trastuzumab solution (50 mM phosphate buffer (pH 6.0)) obtained in Step 1 (3,37 mL), a solution (0.258 mL) of the compound synthesized in Step 2 of Example 5 (12.9 mg) in 50 mM phosphate buffer (pH 6.0) and 4.90 mg/mL EndoS D233Q/Q303L solution (PBS) (0.328 mL) were added, and the resultant was incubated at 30°C for 4.5 hours. These operations were carried out in two lots. The progress of the reaction was confirmed by using an Experion electrophoresis station (produced by Bio-Rad Laboratories, Inc.). After the completion of the reaction, purification by affinity chromatography and purification by hydroxyapatite chromatography were carried out as in Step 1, and fractions containing the desired compound were then subjected to buffer exchange to phosphate buffered saline (pH 6.0) by using common operation C to afford a 10.0 mg/mL Trastuzumab [SG-$(N_3)_2$]$_2$ solution (phosphate buffered saline (pH 6.0)) (15.5 mL).

Example 14: Sugar chain remodeling 2 (T-MSG)

[Formula 66]

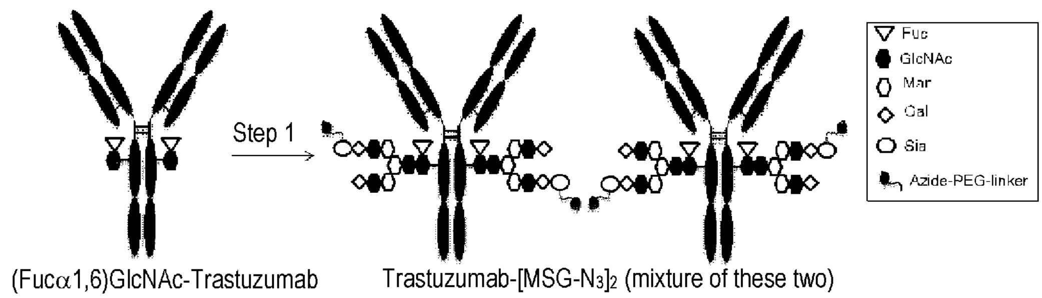


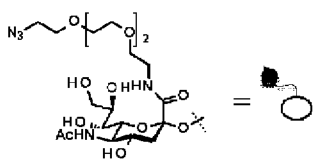

Step 1: Trastuzumab[MSG-$N_3$]$_2$

The following operations were carried out in five lots. The compound obtained in Step 1 of Example 13 (20 mg/mL, 15.0 mL) was used together with the compound obtained in Step 4 of Example 4 (25.5 mg) as a glycan donor, and incubated at 30°C for 3 hours, and the operations same as in Step 2 of Example 13 were carried out. With the five lots combined, a 14.4 mg/mL Trastuzumab[MSG-$N_3$]$_2$ solution (phosphate buffered saline (pH 6.0)) (93.5 mL) was obtained.

Example 15: Sugar chain remodeling 3 (T-MSG1)

[Formula 67]

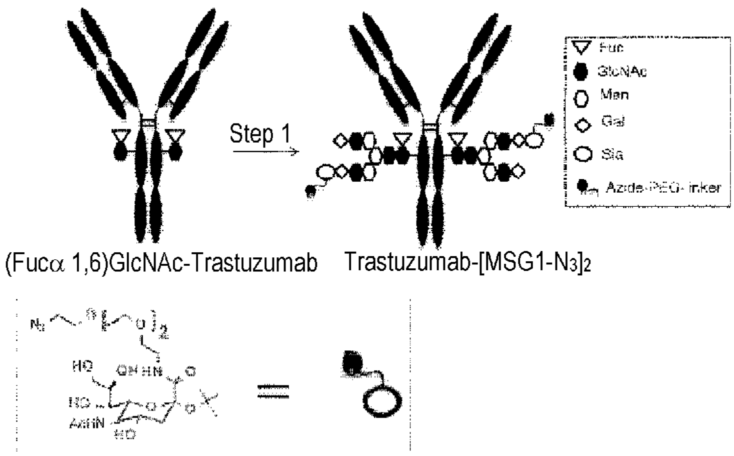


Step 1: Trastuzumab[MSG1-$N_3$]$_2$

The following operations were carried out in two lots. The compound obtained in Step 1 of Example 13 (25.5 mL, 7.8 mL) was used together with the compound obtained in Step 4 of Example 3 (25.5 mg) as a glycan donor, and incubated at 30°C for 3 hours, and the operations same as in Step 2 of Example 13 were carried out. With the two lots combined, a 10.6 mg/mL Trastuzumab[MSG1-$N_3$]$_2$ solution (phosphate buffered saline (pH 6.0)) (31 mL) was obtained.

Example 16: Sugar chain remodeling 4 (CLDN6-MSG1 (H1L1))

[Formula 68]

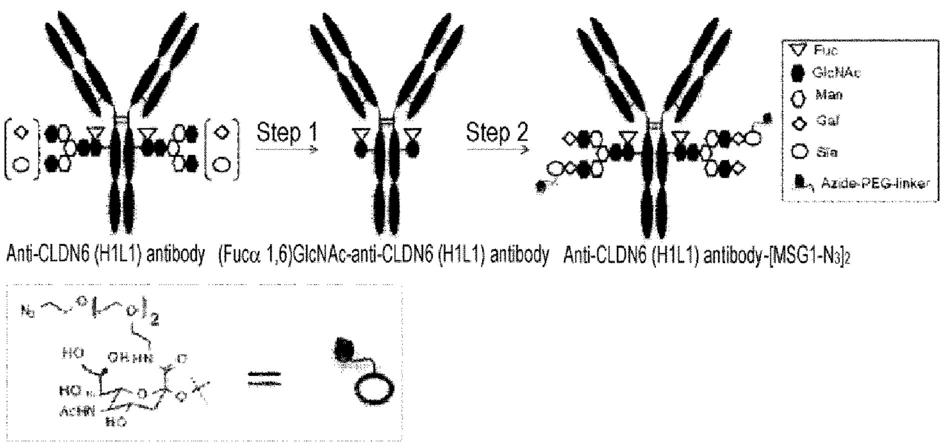


Step 1: (Fucα1,6)GlcNAc-anti-CLDN6 antibody (H1L1)

The operations same as in Step 1 of Example 13 were carried out using a ca. 37.7 mg/mL anti-CLDN6 antibody (H1L1) solution (25 mM histidine solution (pH 6.0), 5% sorbitol solution) prepared in Example 10 (2.5 mL) to afford a 19.2 mg/mL (Fucα1,6)GlcNAc-anti-CLDN6 antibody (H1L1) solution (50 mM phosphate buffer (pH 6.0)) (4.8 mL).

Step 2: Anti-CLDN6 antibody (H1L1)-[MSG1-$N_3$]$_2$

The operations same as in Step 1 of Example 15 were carried out using the 19.2 mg/mL (Fucα1,6)GlcNAc-anti-CLDN6 (H1L1) antibody solution (50 mM phosphate buffer (pH 6.0)) obtained in Step 1 (4.8 mL) to afford a 10.2 mg/mL anti-CLDN6 antibody (H1L1)-[MSG1-$N_3$]$_2$ solution (phosphate buffered saline (pH 6.0)) (7.2 mL).

Example 17: Sugar chain remodeling 5 (CLDN6-MSG1 (H2L2))

Step 1: (Fucα1,6)GlcNAc-anti-CLDN6 antibody (H2L2)

The operations same as in Step 1 of Example 13 were carried out using a ca. 20 mg/mL anti-CLDN6 antibody (H2L2) solution (25 mM histidine solution (pH 6.0), 5% sorbitol solution) prepared in Example 10 (6 mL) to afford a 21.84 mg/mL (Fucα1,6)GlcNAc-anti-CLDN6 antibody (H2L2) solution (50 mM phosphate buffer (pH 6.0)) (5.7 mL).

Step 2: Anti-CLDN6 antibody (H2L2)-[MSG1-$N_3$]$_2$

The operations same as in Step 1 of Example 15 were carried out using the 21.8 mg/mL (Fucα1,6)GlcNAc-anti-CLDN6 (H2L2) antibody solution (50 mM phosphate buffer (pH 6.0)) obtained in Step 1 (5.7 mL) to afford a 10.2 mg/mL anti-CLDN6 antibody (H2L2)-[MSG1-$N_3$]$_2$ solution (phosphate buffered saline (pH 6.0)) (11.1 mL).

Example 18: Sugar chain remodeling 6 (CLDN6-MSG1 (H1L3))

Step 1: (Fucα1,6)GlcNAc-anti-CLDN6 antibody (H1L3)

The operations same as in Step 1 of Example 13 were carried out using a ca. 39.4 mg/mL anti-CLDN6 antibody (H1L3) solution (25 mM histidine solution (pH 6.0), 5% sorbitol solution) prepared in Example 10 (3 mL) to afford a 39.2 mg/mL (Fucα1,6)GlcNAc-anti-CLDN6 antibody (H1L3) solution (50 mM phosphate buffer (pH 6.0)) (4.5 mL).

Step 2: Anti-CLDN6 antibody (H1L3)-[MSG1-$N_3$]$_2$

The operations same as in Step 1 of Example 15 were carried out using the 39.2 mg/mL (Fucα1,6)GlcNAc-anti-CLDN6 (H1L3) antibody solution (50 mM phosphate buffer (pH 6.0)) obtained in Step 1 (4.5 mL) to afford a 9.83 mg/mL anti-CLDN6 antibody (H1L3)-[MSG1-$N_3$]$_2$ solution (phosphate buffered saline (pH 6.0)) (7.2 mL).

Example 19: Sugar chain remodeling 7 (CD98-MSG1)

Step 1: (Fucα1,6)GlcNAc-anti-CD98 antibody

The operations same as in Step 1 of Example 13 were carried out using a ca. 20 mg/mL anti-CD98 antibody solution (25 mM histidine solution (pH 6.0), 5% sorbitol solution) prepared in Reference Example 6 (6 mL) to afford a 21.7 mg/mL (Fucα1,6)GlcNAc-anti-CD98 antibody solution (50 mM phosphate buffer (pH 6.0)) (4.7 mL).

Step 2: Anti-CD98 antibody-[MSG1-$N_3$]$_2$

The operations same as in Step 1 of Example 15 were carried out using the 21.7 mg/mL (Fucα1,6)GlcNAc-anti-CD98 antibody solution (50 mM phosphate buffer (pH 6.0)) obtained in Step 1 (4.7 mL) to afford a 10.1 mg/mL anti-CD98 antibody-[MSGl-$N_3$]$_2$ solution (phosphate buffered saline (pH 6.0)) (7.6 mL).

Example 20: Sugar chain remodeling 8 (TROP2-MSG1)

Step 1: (Fucα1,6)GlcNAc-anti-Trop2 antibody

The operations same as in Step 1 of Example 13 were carried out using a ca. 20 mg/mL anti-Trop2 antibody solution (25 mM histidine solution (pH 6.0), 5% sorbitol solution) prepared in Reference Example 5 (6 mL) to afford a 21.69 mg/mL (Fucα1,6)GlcNAc-anti-Trop2 antibody solution (50 mM phosphate buffer (pH 6.0)) (3.3 mL).

Step 2: Anti-Trop2 antibody-[MSG1-$N_3$]$_2$

The operations same as in Step 1 of Example 15 were carried out using the 21.69 mg/mL (Fucα1,6)GlcNAc-anti-Trop2 antibody solution (50 mM phosphate buffer (pH 6.0)) obtained in Step 1 (3.35 mL) to afford a 10.3 mg/mL anti-Trop2 antibody-[MSGl-$N_3$]$_2$ solution (phosphate buffered saline (pH 6.0)) (6.4 mL).

Example 21: Sugar chain remodeling 9 (LPS-MSG1)

Step 1: (Fucα1,6)GlcNAc-anti-LPS antibody

The operations same as in Step 1 of Example 13 were carried out using a ca. 17 mg/mL anti-LPS antibody solution (25 mM histidine solution (pH 6.0), 5% sorbitol solution) prepared in Reference Example 4 (6.6 mL) to afford a 21.03 mg/mL (Fucα1,6)GlcNAc-anti-LPS antibody solution (50 mM phosphate buffer (pH 6.0)) (5.4 mL).

Step 2: Anti-LPS antibody-[MSG1-$N_3$]$_2$

The operations same as in Step 1 of Example 15 were carried out using the 21.03 mg/mL (Fucα1,6)GlcNAc-anti-LPS antibody solution (50 mM phosphate buffer (pH 6.0)) obtained in Step 1 (5.4 mL) to afford a 9.89 mg/mL anti-LPS antibody-[MSGl-$N_3$]$_2$ solution (phosphate buffered saline (pH 6.0)) (7.9 mL).

Example 22: Sugar chain remodeling (trastuzumab variant-MSGI)

Step 1: (Fucα1,6)GlcNAc-trastuzumab variant

The operations same as in Step 1 of Example 13 were carried out using a ca. 22.3 mg/mL trastuzumab variant solution (50 mM phosphate buffer (pH 6.0)) prepared in Example 12 (2.7 mL) to afford a 6.1 mg/mL (Fucα1,6)GlcNAc-trastuzumab variant solution (50 mM phosphate buffer (pH 6.0)) (6.1 mL).

Step 2: Trastuzumab variant-[MSG1-$N_3$]$_2$

The operations same as in Step 1 of Example 15 were carried out using the 6.1 mg/mL (Fucα1,6)GlcNAc-trastuzumab variant solution (50 mM phosphate buffer (pH 6.0)) obtained in Step 1 (6.1 mL) to afford a 10.2 mg/mL trastuzumab variant-[MSGl-$N_3$]$_2$ solution (phosphate buffered saline (pH 6.0)) (3.7 mL).

[Synthesis of ADC]

ADC1, 2, and 5 described in Examples 23, 24, and 52 were synthesized, as illustrated in the following reaction formula, by conjugating the antibody obtained in Step 1 of Example 14 with a drug-linker. In the formula, R differs among drug-linkers used in those Examples.

[Formula 69]

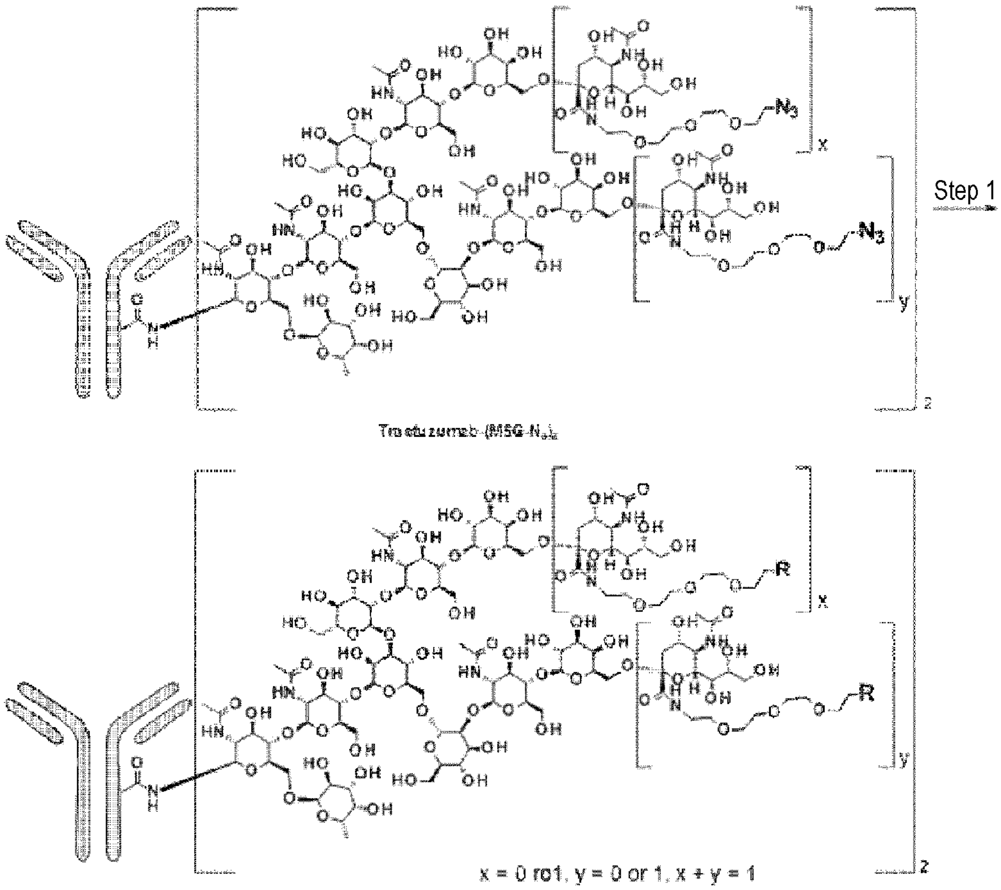


Example 23: ADC1

[Formula 70]

R =

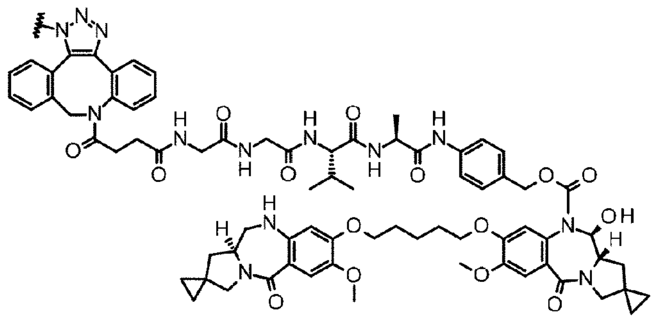

or

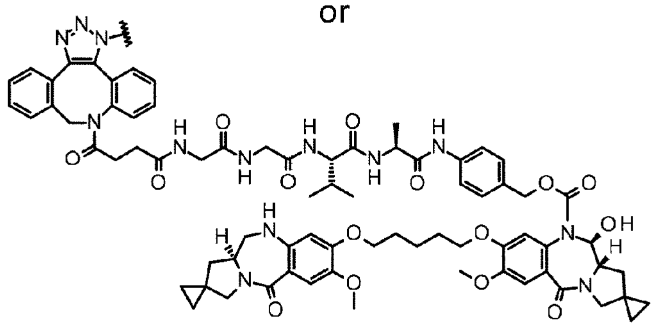

(In the compound obtained in Step 1 of Example 23, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.0 mg/mL, 1.00 mL) obtained in Step 1 of Example 14, 1,2-propanediol (0.917 mL) and a 10 mM dimethyl sulfoxide solution of compound (6-13) obtained in Step 12 of Example 2-4 (0.0825 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 6.0 mL of a solution of the desired compound.
Characterization: The following characteristic values were obtained by using common operations E and F.
Antibody concentration: 1.33 mg/mL, antibody yield: 7.97 mg (80%), average number of conjugated drug molecules per antibody molecule (n): 1.9

Example 24: ADC2

[Formula 71]

R =

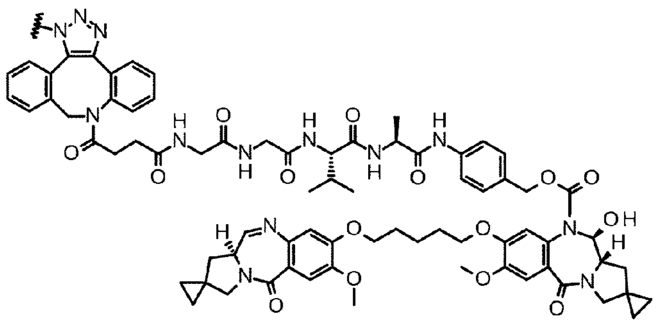

or

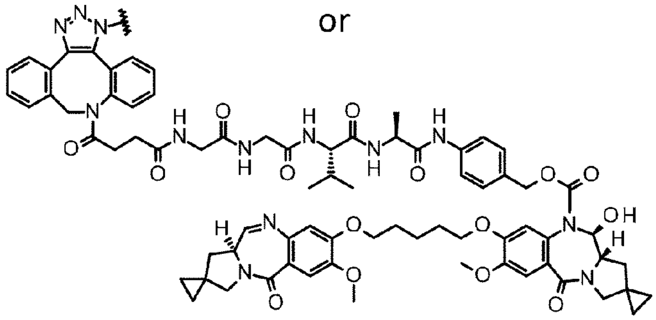

(In the compound obtained in Step 1 of Example 24, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (9.88 mg/mL, 0.500 mL) obtained in Step 1 of Example 14, 1,2-propanediol (0.459 mL) and a 10 mM dimethyl sulfoxide solution of compound (5-11) obtained in Step 10 of Example 2-3 (0.0408 mL; 12 equivalents per antibody molecule) were added at room temperature, and the reaction was carried out using a tube rotator (MTR-103, AS ONE Corporation) at room temperature for 1 day.

Purification operation: The solution was purified by using common operation D to afford 6.00 mL of a solution of the desired compound. This solution was concentrated by using common operation A to afford 0.420 mL of a solution of the desired compound.
Characterization: The following characteristic values were obtained by using common operations E and F.
Antibody concentration: 7.27 mg/mL, antibody yield: 3.54 mg (72%), average number of conjugated drug molecules per antibody molecule (n): 1.8

ADC3 and 4 described in Examples 25 and 26 were synthesized, as illustrated in the following reaction formula, by conjugating the antibody obtained in Step 2 of Example 13 with a drug-linker. In the formula, R differs among drug-linkers used in those Examples.

[Formula 72]

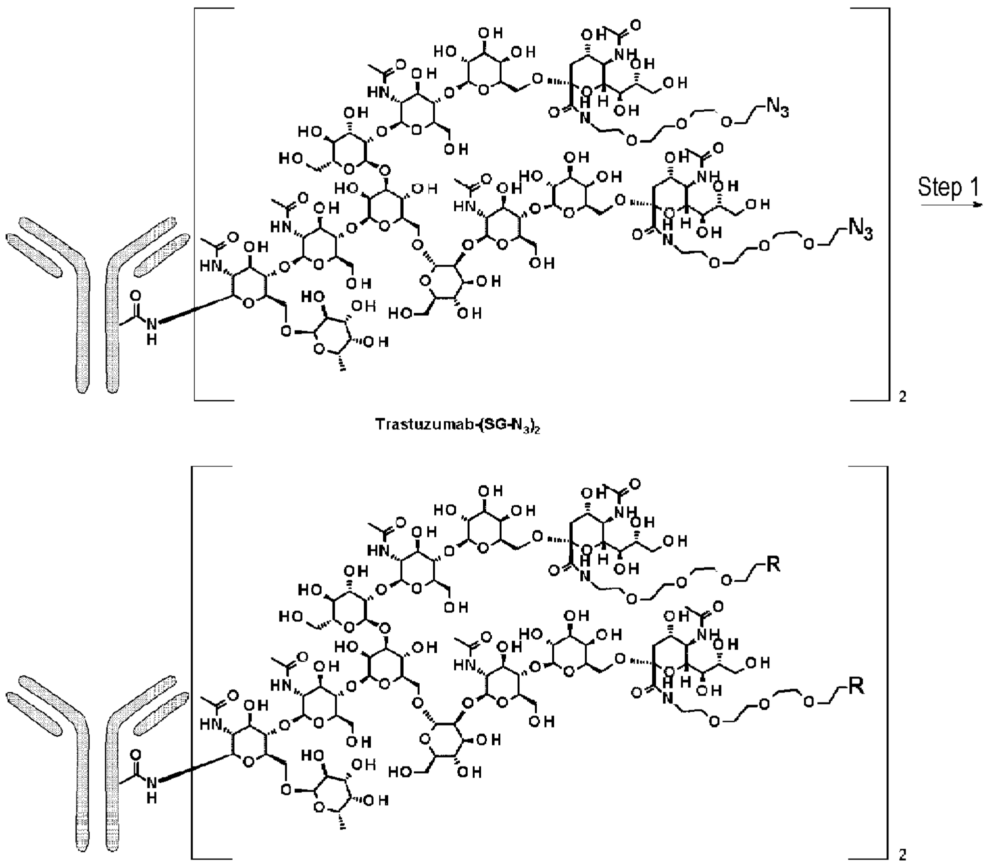


Trastuzumab-(SG-$N_3$)$_2$

Example 25: ADC3

[Formula 73]

R =

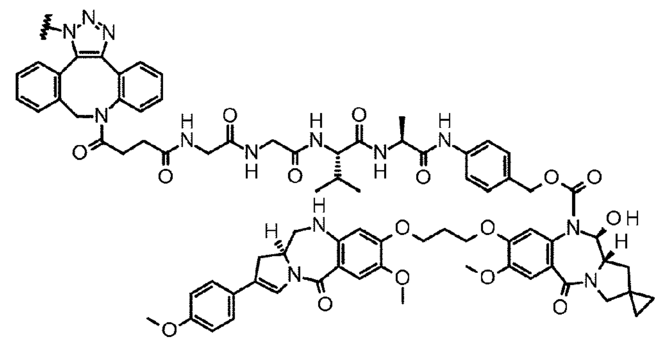

or

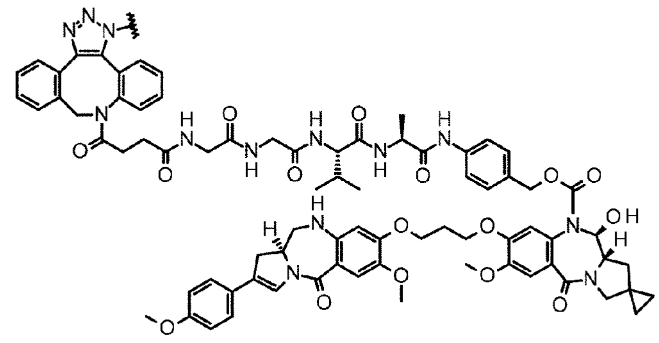

(In the compound obtained in Step 1 of Example 25, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.0 mg/mL, 1.00 mL) obtained in Step 2 of Example 13, 1,2-propanediol (0.835 mL) and a 10 mM dimethyl sulfoxide solution of compound (4-12) obtained in Step 12 of Example 2-2 (0.165 mL; 24 equivalents per antibody molecule) were added at room temperature, and the reaction was carried out using a tube rotator (MTR-103, AS ONE Corporation) at room temperature for 48 hours.

Purification operation: The solution was purified by using common operation D to afford 4.50 mL of a solution of the desired compound.
Characterization: The following characteristic values were obtained by using common operations E and F.
Antibody concentration: 1.66 mg/mL, antibody yield: 7.48 mg (75%), average number of conjugated drug molecules per antibody molecule (n): 3.8

Example 26: ADC4

[Formula 74]

R =

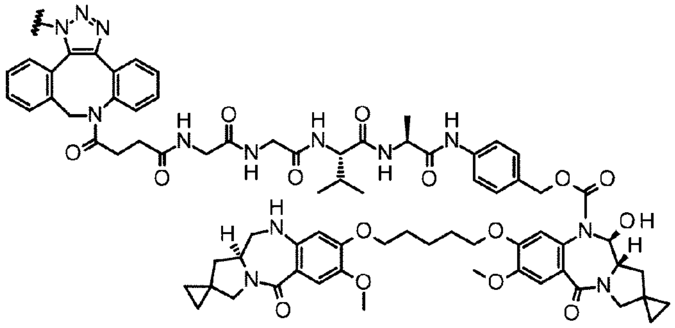

or

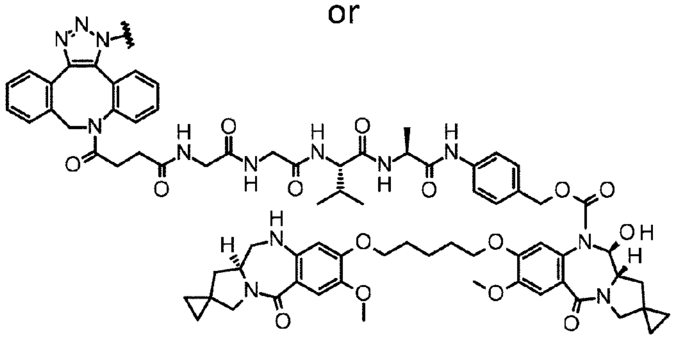

(In the compound obtained in Step 1 of Example 26, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.0 mg/mL, 1.00 mL) obtained in Step 2 of Example 13, 1,2-propanediol (0.835 mL) and a 10 mM dimethyl sulfoxide solution of compound (6-13) obtained in Step 12 of Example 2-4 (0.165 mL; 24 equivalents per antibody molecule) were added at room temperature, and the reaction was carried out using a tube rotator (MTR-103, AS ONE Corporation) at room temperature for 2 days.

Purification operation: The solution was purified by using common operation D to afford 10.5 mL of a solution of the desired compound.
Characterization: The following characteristic values were obtained by using common operations E and F.
Antibody concentration: 0.73 mg/mL, antibody yield: 7.70 mg (77%), average number of conjugated drug molecules per antibody molecule (n): 3.8

ADC5 to 7 and 16 to 19 described in Examples 27, 28, 29, and 53 to 56 were synthesized, as illustrated in the following reaction formula, by conjugation of the antibody obtained in Step 1 of Example 15 with a drug-linker. In the formula, R group differs among drug-linkers used in those Examples.

[Formula 75]

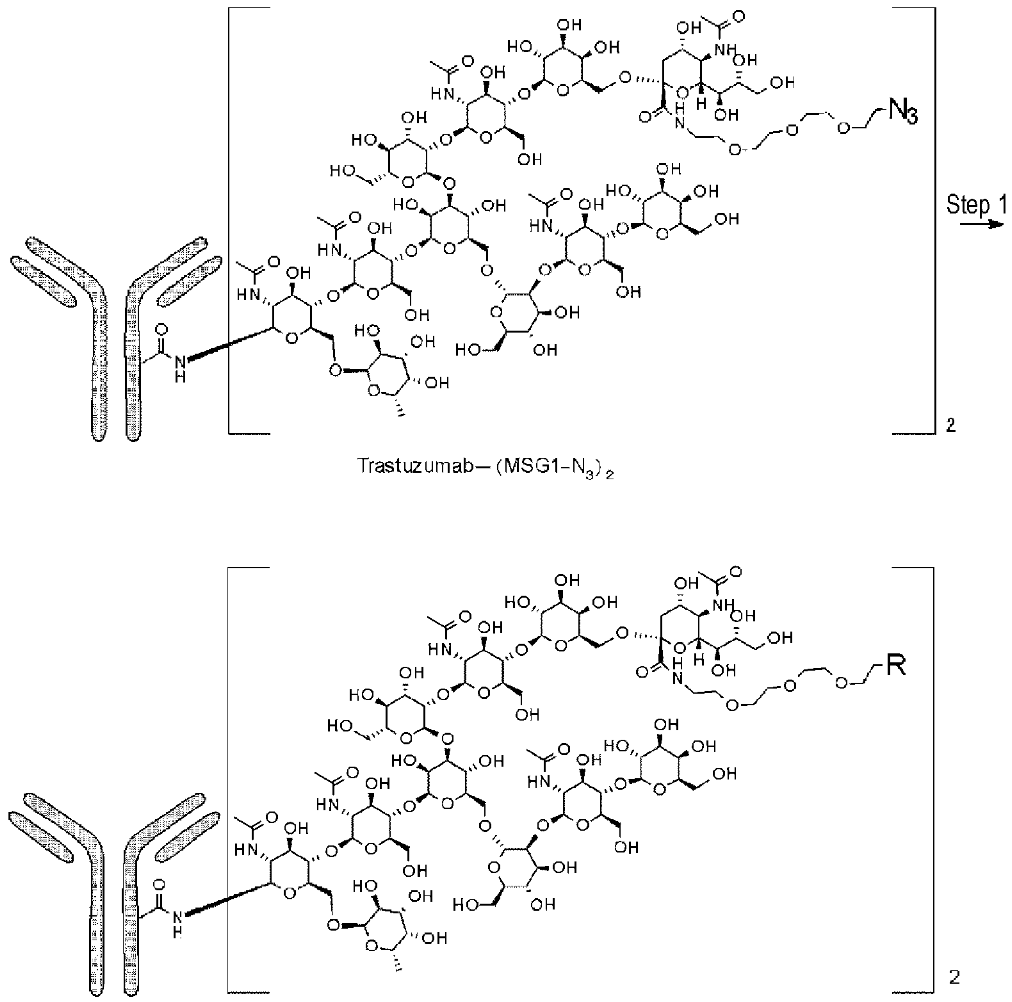


Trastuzumab—$(MSG1\text{-}N_3)_2$

Example 27: ADC5

[Formula 76]

R =

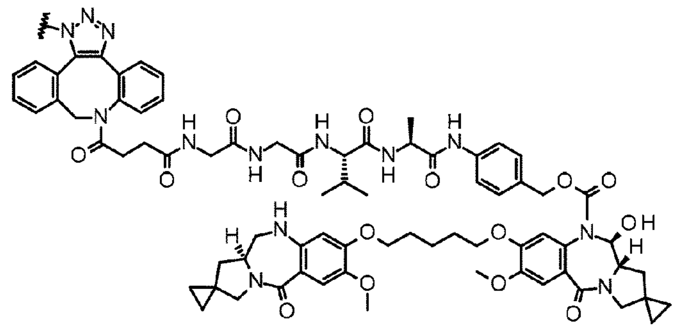

or

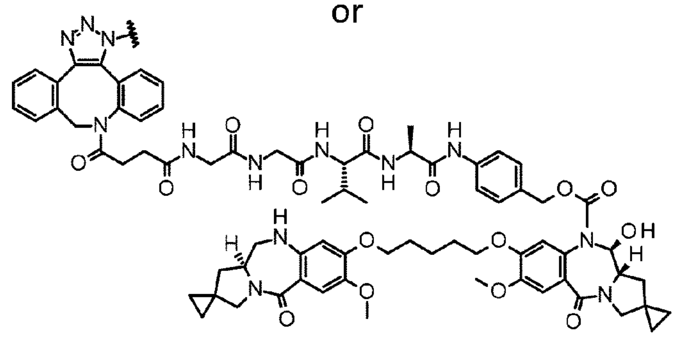

(In the compound obtained in Step 1 of Example 27, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.0 mg/mL, 1.00 mL) obtained in Step 1 of Example 15, 1,2-propanediol (0.917 mL) and a 10 mM dimethyl sulfoxide solution of compound (6-13) obtained in Step 12 of Example 2-4 (0.0825 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 6.0 mL of a solution of the desired compound.
Characterization: The following characteristic values were obtained by using common operations E and F.
Antibody concentration: 1.46 mg/mL, antibody yield: 8.76 mg (88%), average number of conjugated drug molecules per antibody molecule (n): 1.9

Example 28: ADC6

[Formula 77]

R = 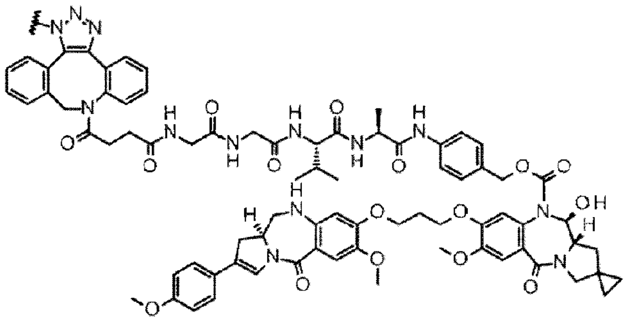

or

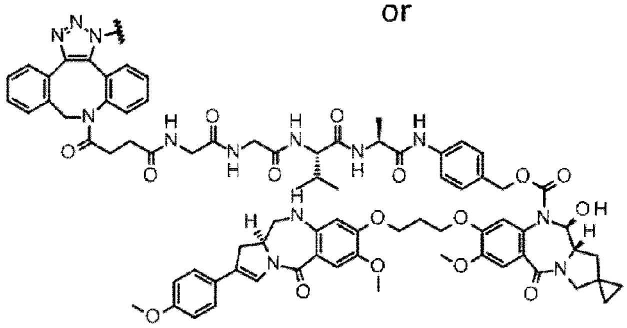

(In the compound obtained in Step 1 of Example 28, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.0 mg/mL, 5.00 mL) obtained in Step 1 of Example 15, 1,2-propanediol (4.59 mL) and a 10 mM dimethyl sulfoxide solution of compound (4-12) obtained in Step 12 of Example 2-2 (0.413 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 30.0 mL of a solution of the desired compound.
Characterization: The following characteristic values were obtained by using common operations E and F.
Antibody concentration: 1.30 mg/mL, antibody yield: 38.9 mg (78%), average number of conjugated drug molecules per antibody molecule (n): 1.9

Example 29: ADC7

[Formula 78]

R =

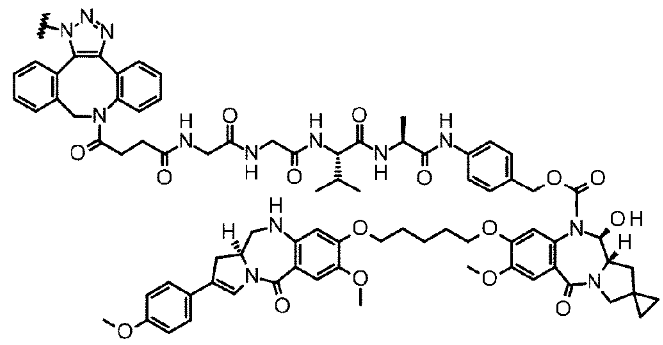

or

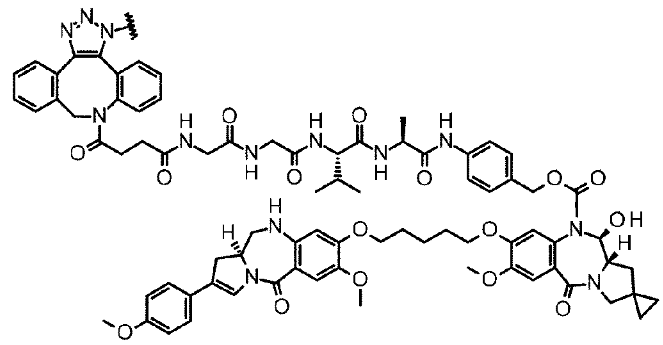

(In the compound obtained in Step 1 of Example 29, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.2 mg/mL, 1.00 mL) obtained in Step 1 of Example 15, 1,2-propanediol (0.917 mL) and a 10 mM dimethyl sulfoxide solution of compound (3-14) obtained in Step 13 of Example 2-1 (0.0825 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 6.00 mL of a solution of the desired compound.
Characterization: The following characteristic values were obtained by using common operations E and F.
Antibody concentration: 1.41 mg/mL, antibody yield: 8.45 mg (85%), average number of conjugated drug molecules per antibody molecule (n): 1.9

Example 30: ADC8

[Formula 79]

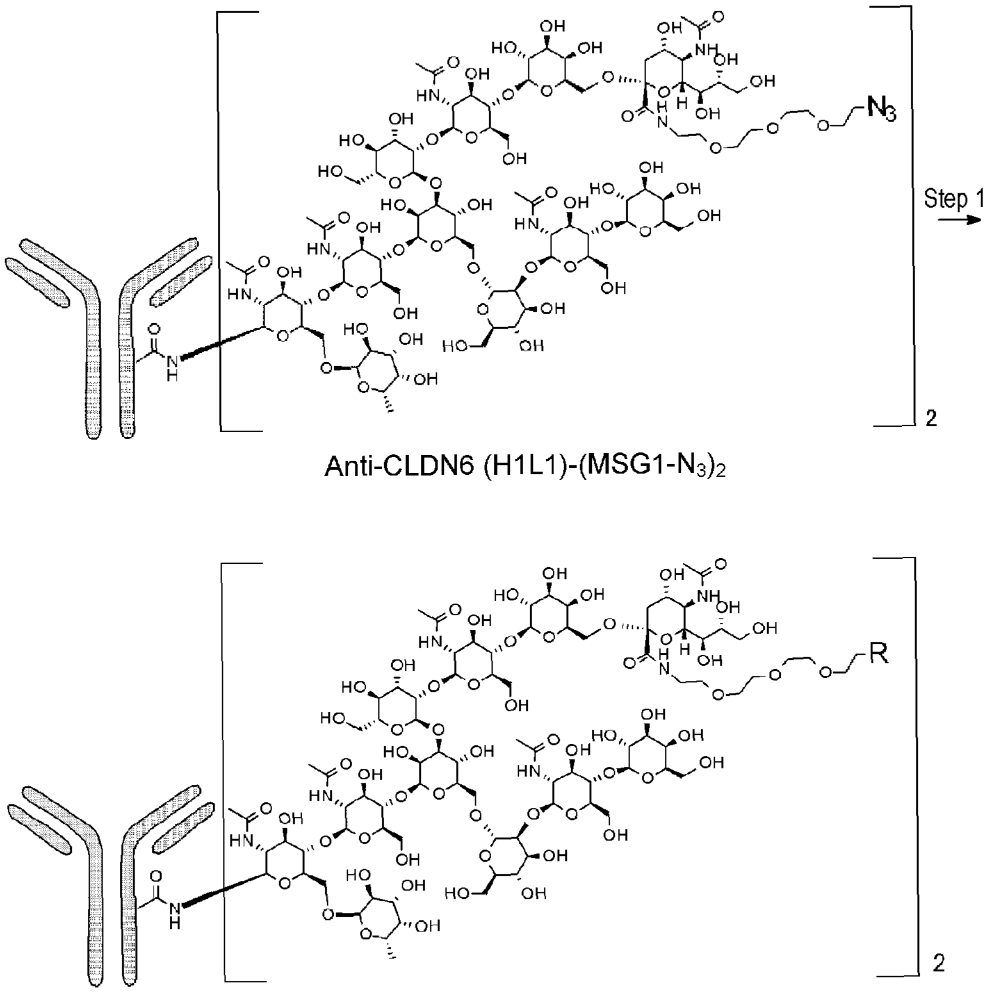


Anti-CLDN6 (H1L1)-(MSG1-$N_3$)$_2$

[Formula 80]

R =

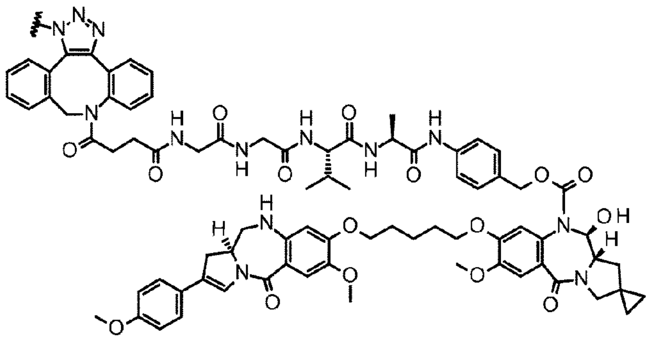

or

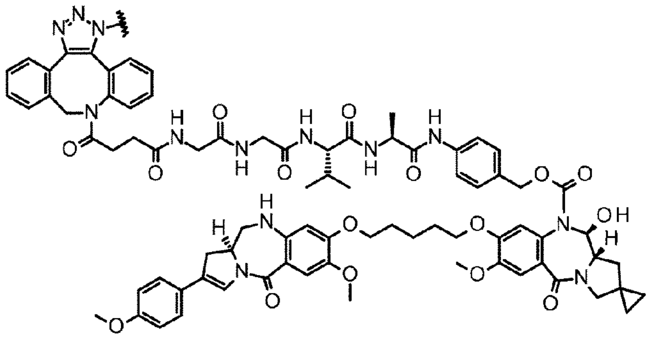

(In the compound obtained in Step 1 of Example 30, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.2 mg/mL, 2.50 mL) obtained in Step 2 of Example 16, 1,2-propanediol (2.29 mL) and a 10 mM dimethyl sulfoxide solution of compound (3-14) obtained in Step 13 of Example 2-1 (0.206 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 14.5 mL of a solution of the desired compound.
Characterization: The following characteristic values were obtained by using common operations E and F.
Antibody concentration: 1.54 mg/mL, antibody yield: 22.3 mg (89%), average number of conjugated drug molecules per antibody molecule (n): 1.9

Example 31: ADC9

[Formula 81]

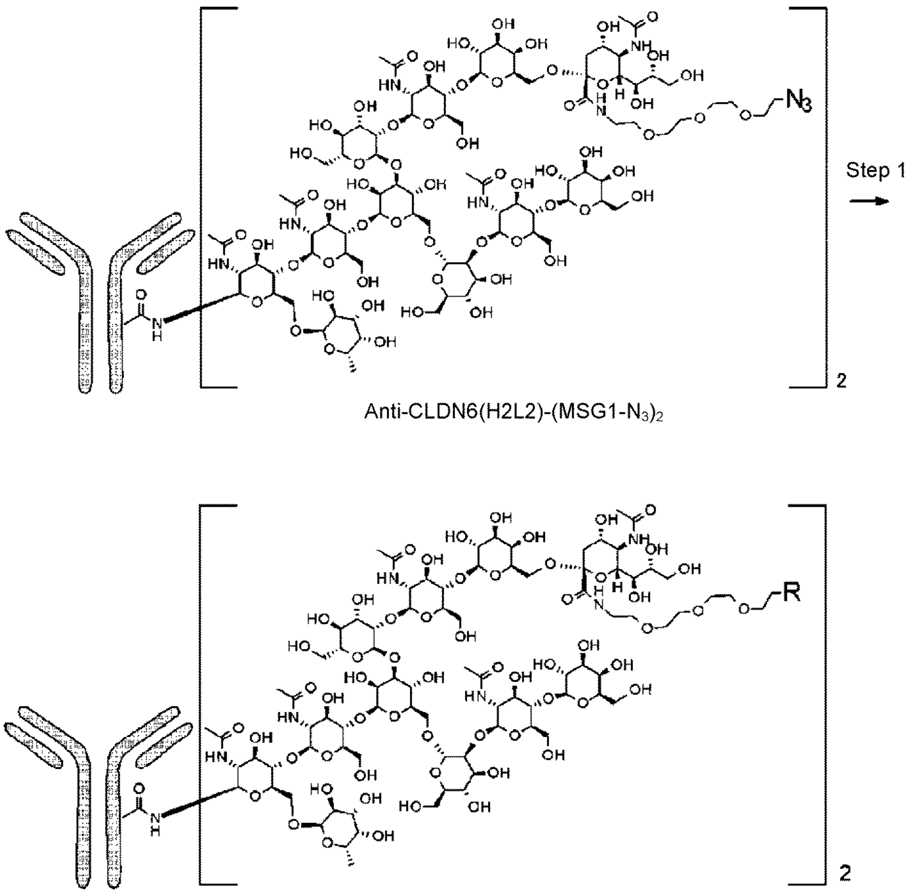


Anti-CLDN6(H2L2)-(MSG1-$N_3$)$_2$

[Formula 82]

R =

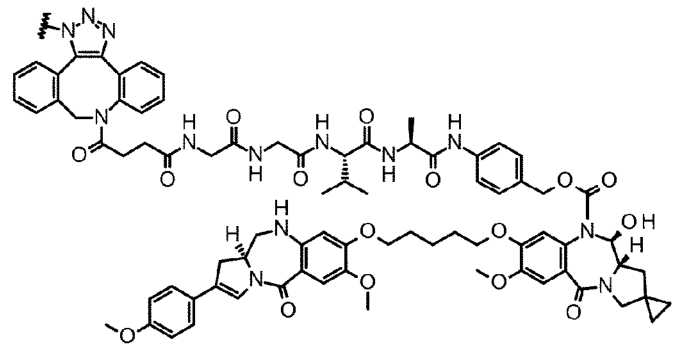

or

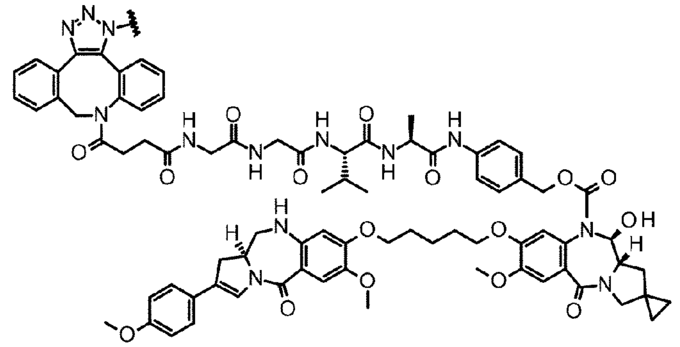

(In the compound obtained in Step 1 of Example 31, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (9.96 mg/mL, 2.50 mL) obtained in Step 2 of Example 17, 1,2-propanediol (2.29 mL) and a 10 mM dimethyl sulfoxide solution of compound (3-14) obtained in Step 13 of Example 2-1 (0.206 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 14.5 mL of a solution of the desired compound.
Characterization: The following characteristic values were obtained by using common operations E and F.
Antibody concentration: 1.52 mg/mL, antibody yield: 22.0 mg (88%), average number of conjugated drug molecules per antibody molecule (n): 1.9

Example 32: ADC10

[Formula 83]

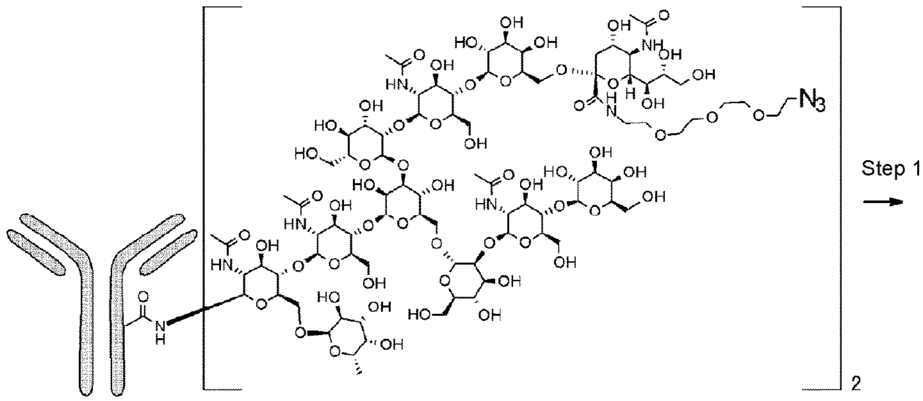


Anti-CLDN6(H1L3)-(MSG1-$N_3$)$_2$

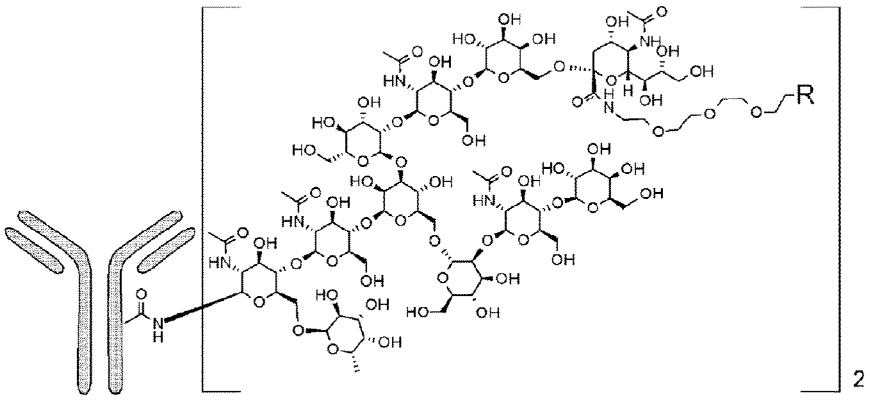

[Formula 84]

R =

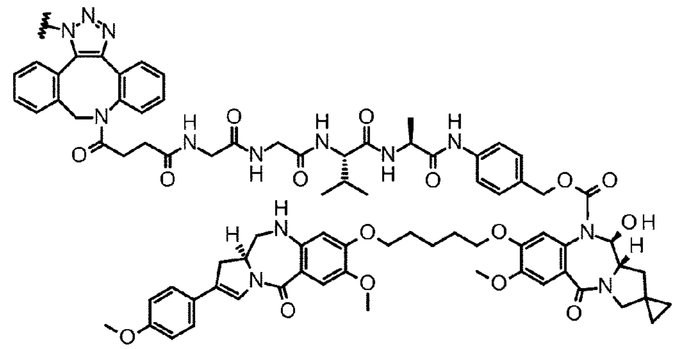

or

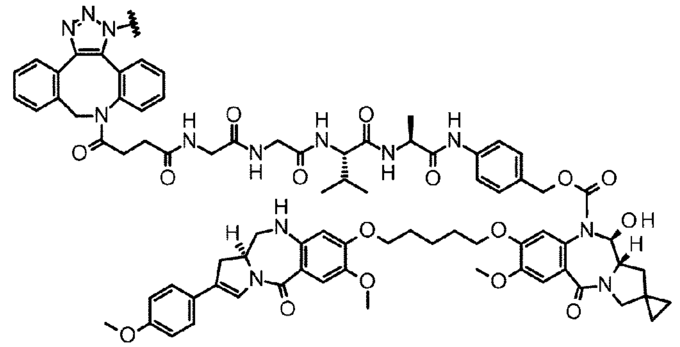

(In the compound obtained in Step 1 of Example 32, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (9.83 mg/mL, 2.50 mL) obtained in Step 2 of Example 18, 1,2-propanediol (2.29 mL) and a 10 mM dimethyl sulfoxide solution of compound (3-14) obtained in Step 13 of Example 2-1 (0.206 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 14.5 mL of a solution of the desired compound.
Characterization: The following characteristic values were obtained by using common operations E and F.
Antibody concentration: 1.45 mg/mL, antibody yield: 21.0 mg (84%), average number of conjugated drug molecules per antibody molecule (n): 1.9

Example 33: ADC11

[Formula 85]

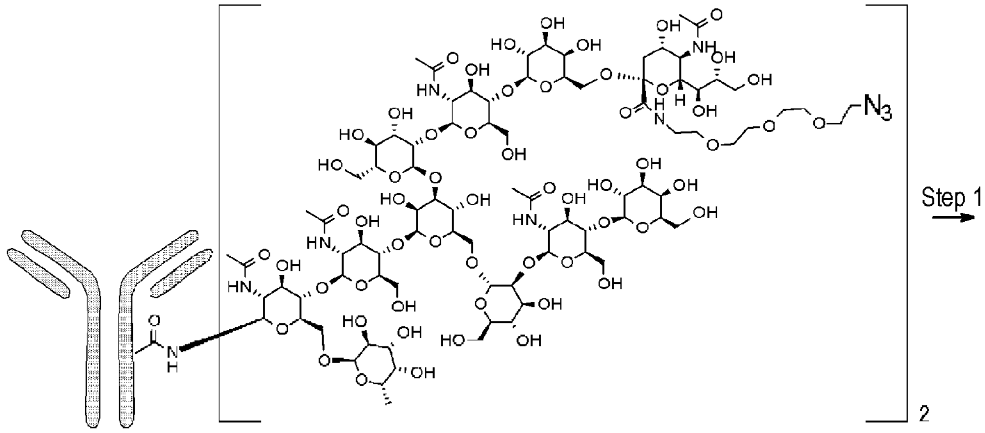


Anti-Trop2-(MSG1-$N_3$)$_2$

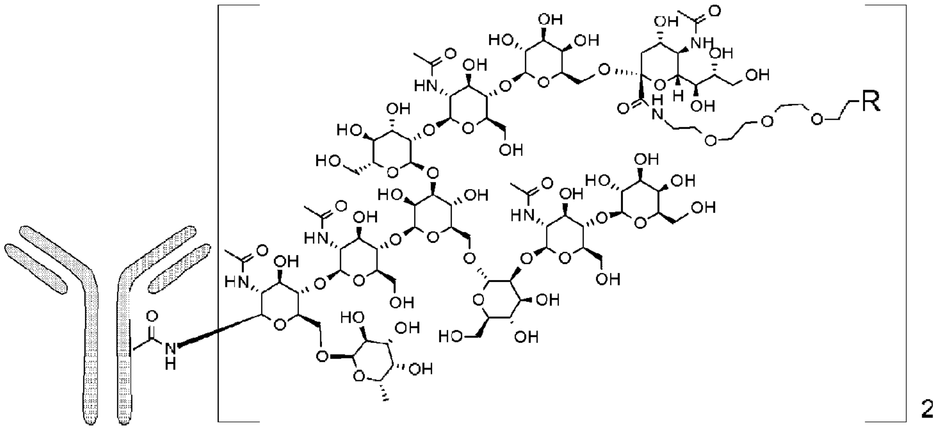

[Formula 86]

R =

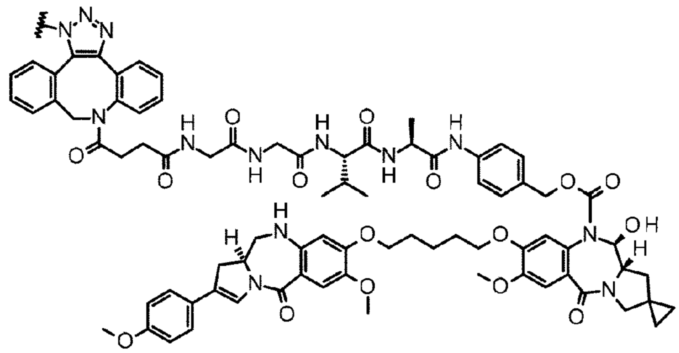

or

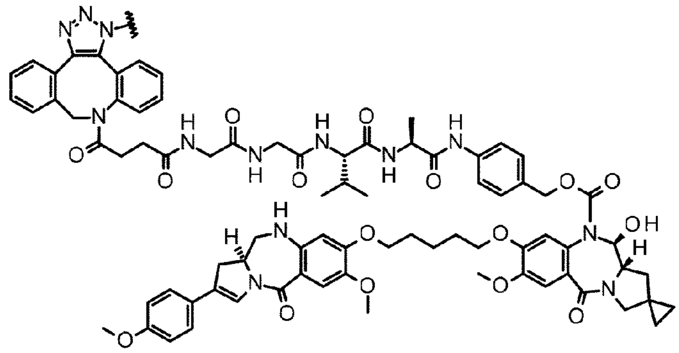

(In the compound obtained in Step 1 of Example 33, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10 mg/mL, 1.00 mL) obtained in Step 2 of Example 20, 1,2-propanediol (0.917 mL) and a 10 mM dimethyl sulfoxide solution of compound (3-14) obtained in Step 13 of Example 2-1 (0.0825 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 6.00 mL of a solution of the desired compound.
Characterization: The following characteristic values were obtained by using common operations E and F.
Antibody concentration: 1.47 mg/mL, antibody yield: 8.8 mg (88%), average number of conjugated drug molecules per antibody molecule (n): 1.9

Example 34: ADC12

[Formula 87]

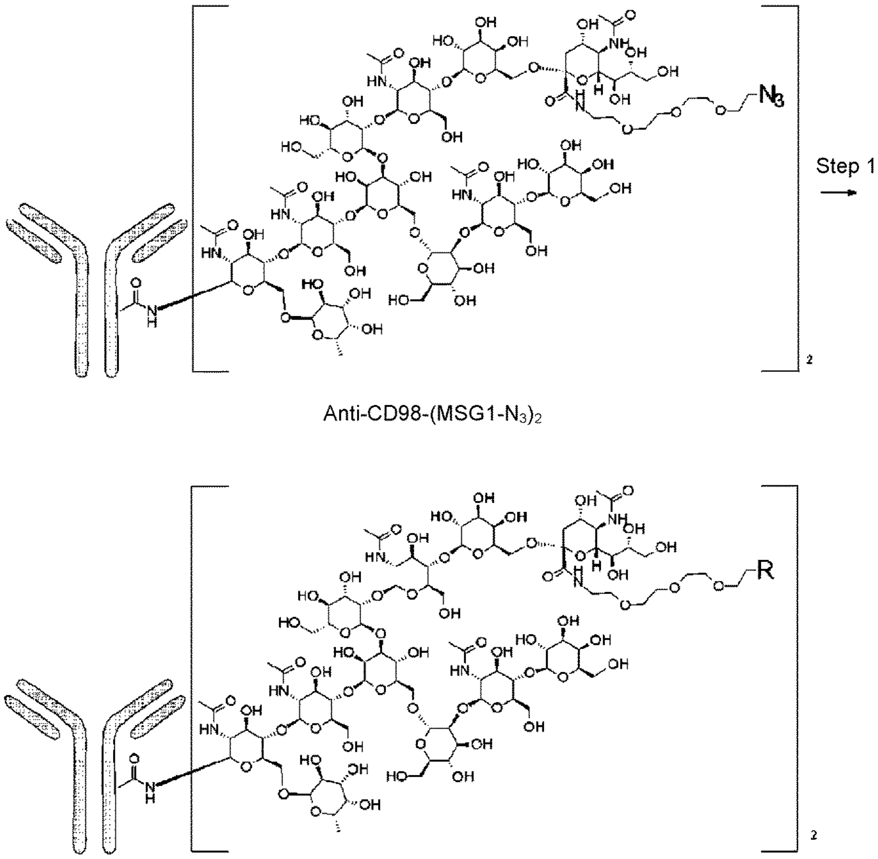


Anti-CD98-(MSG1-$N_3$)$_2$

[Formula 88]

R =

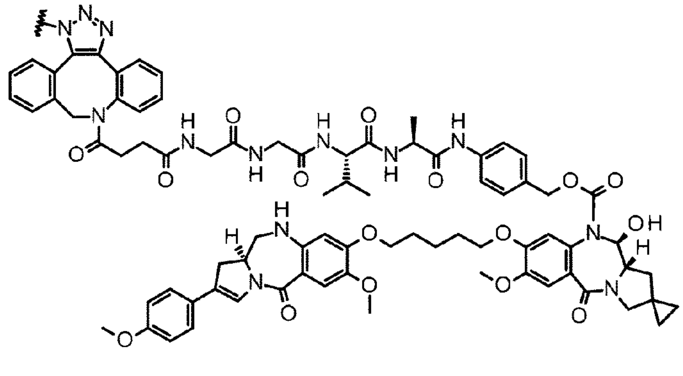

or

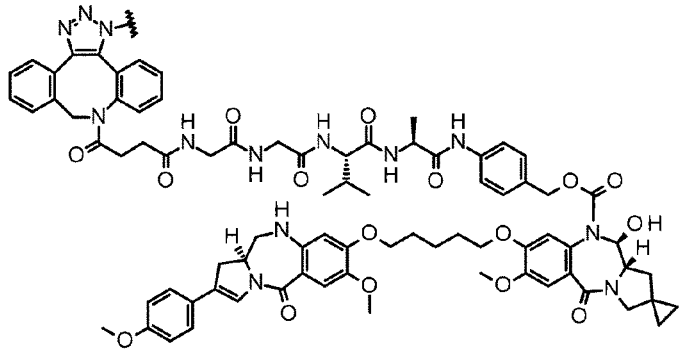

(In the compound obtained in Step 1 of Example 34, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.2 mg/mL, 1.00 mL) obtained in Step 2 of Example 19, 1,2-propanediol (0.917 mL) and a 10 mM dimethyl sulfoxide solution of compound (3-14) obtained in Step 13 of Example 2-1 (0.0825 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 6.00 mL of a solution of the desired compound.
Characterization: The following characteristic values were obtained by using common operations E and F.
Antibody concentration: 1.36 mg/mL, antibody yield: 8.19 mg (82%), average number of conjugated drug molecules per antibody molecule (n): 1.8

Example 35: ADC13

[Formula 89]

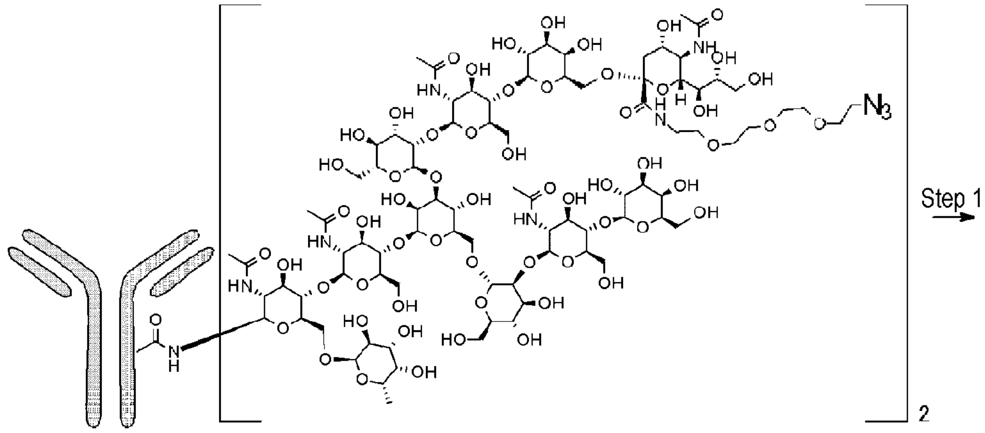


Anti-LPS-(MSG1-$N_3$)$_2$

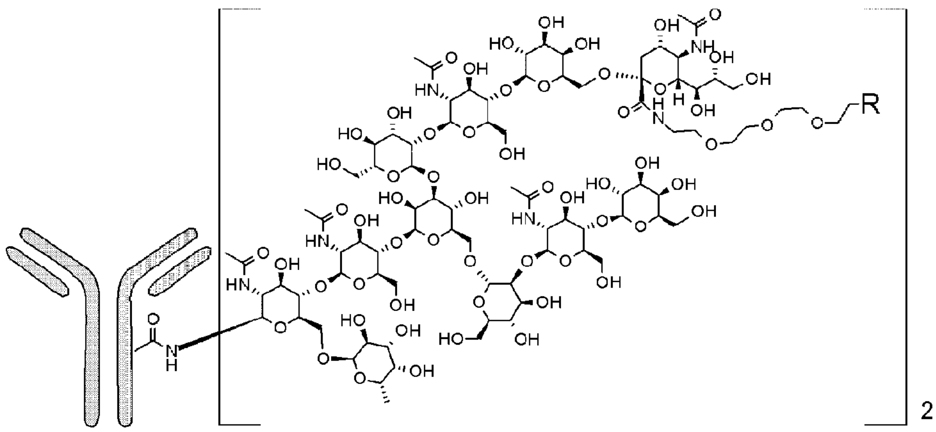

[Formula 90]

R =

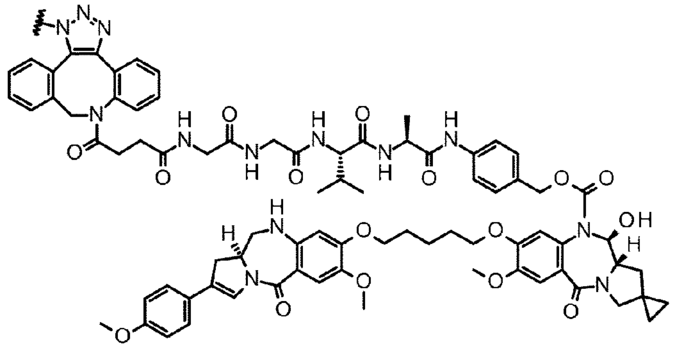

or

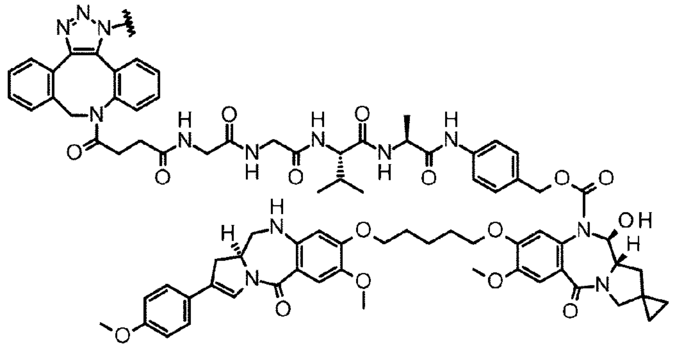

(In the compound obtained in Step 1 of Example 35, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (9.89 mg/mL, 0.40 mL) obtained in Step 2 of Example 21, 1,2-propanediol (0.367 mL) and a 10 mM dimethyl sulfoxide solution of compound (3-14) obtained in Step 13 of Example 2-1 (0.0328 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 2.50 mL of a solution of the desired compound.
Characterization: The following characteristic values were obtained by using common operations E and F.
Antibody concentration: 1.16 mg/mL, antibody yield: 2.89 mg (72%), average number of conjugated drug molecules per antibody molecule (n): 1.8

Example 36: ADC14

[Formula 91]

R = 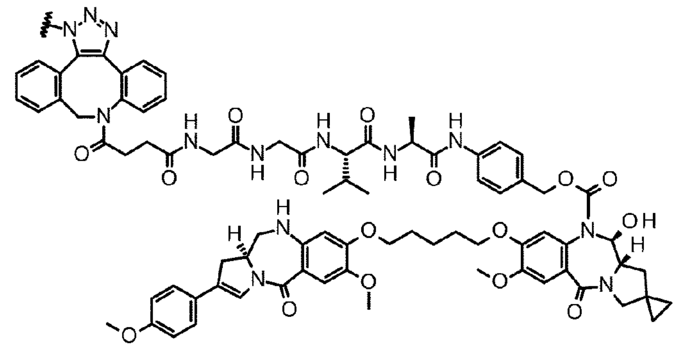

or

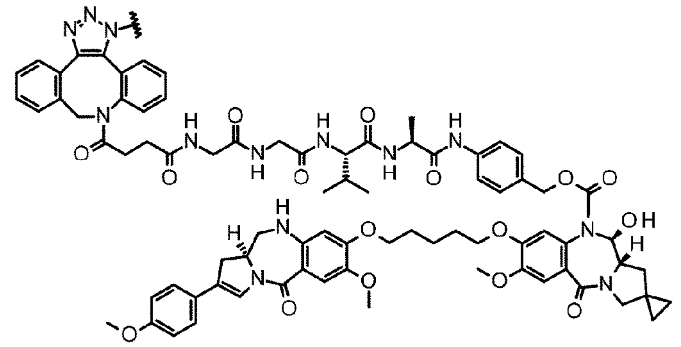

(In the compound obtained in Step 1 of Example 36, the triazole ring has geometric isomers as illustrated in the formula, and the compound has a drug-linker as a mixture of the two structures shown above as R.)

Step 1: Conjugation of antibody and drug-linker

To a phosphate buffered saline (pH 6.0) solution of the antibody (10.2 mg/mL, 0.40 mL) obtained in Step 2 of Example 22, phosphate buffered saline (pH 6.0) solution (0.40 mL), 1,2-propanediol (0.767 mL), dimethylformamide (0.20 mL), and a 10 mM dimethylformamide solution of compound (3-14) obtained in Step 13 of Example 2-1 (0.033 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 7.00 mL of a solution of the desired compound.
Characterization: The following characteristic values were obtained by using common operations E and F.
Antibody concentration: 0.39 mg/mL, antibody yield: 1.38 mg (35%), average number of conjugated drug molecules per antibody molecule (n): 1.8

Example 37: Evaluation of cell growth inhibition of antibody-drug conjugate (1)

NCI-N87 (American Type Culture Collection; ATCC CRL-5822), a human gastric cancer cell line of HER2 antigen-positive cells, was cultured in RPMI1640 Medium (Thermo Fisher Scientific; hereinafter, referred to as RPMI medium) containing 10% fetal bovine serum (Hyclone). MDA-MB-468 (ATCC HTB-132), HER2 antigen-negative cells, was cultured in Leibovitz's L-15 Medium (Thermo Fisher Scientific; hereinafter, referred to as Leibovitz's medium) containing 10% fetal bovine serum (Hyclone). NCI-N87 and MDA-MB-468 cells were prepared with RPMI medium and Leibovitz's medium, respectively, to reach $2.5 \times 10^4$ cells/mL, and 80 μL portions of them were added to a 96-well cell culture microplate. After addition of the cells, NCI-N87 was cultured at 37°C and 5% $CO_2$ overnight, and MDA-MB-468 was cultured at 37°C overnight, without setting of $CO_2$ concentration.

On the next day, 20 μL portions of an anti-HER2 antibody-drug conjugate diluted with RPMI medium or Leibovitz's medium to 100 nM, 10 nM, 1 nM, 0.1 nM, 0.01 nM, and 0.001 nM were added to the microplate. To each well without any antibody-drug conjugate, 20 μL of RPMI medium or Leibovitz's medium was added. NCI-N87 was cultured

at 37°C and 5% $CO_2$ for 6 days, and MDA-MB-468 was cultured at 37°C for 6 days, without setting of $CO_2$ concentration. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer).

Cell survival rates were calculated by using the following formula.

$$\text{Cell survival rate (\%)} = a \div b \times 100$$

a: Mean value of amounts of emission from wells with test substance
b: Mean value of amounts of emission from wells with medium

$IC_{50}$ values were calculated by using the following formula.

$$IC_{50}\ (nM) = \text{antilog}((50\text{-}d) \times (LOG_{10}(b)\text{-}LOG_{10}(a)) \div (d\text{-}c) + LOG_{10}(b))$$

a: Concentration of test substance, a
b: Concentration of test substance, b
c: Cell survival rate when test substance of concentration a was added
d: Cell survival rate when test substance of concentration b was added a and b satisfy a > b at points sandwiching a cell survival rate of 50%.

The antibody-drug conjugates ADC5, ADC4, and ADC16 each exhibited an anticellular effect of $IC_{50}$ < 0.001 (nM) on the NCI-N87 cells. The antibody-drug conjugates ADC2, ADC15, ADC3, ADC6, ADC17, and ADC18 each exhibited an anticellular effect of 0.001 ≤ $IC_{50}$ < 0.1 (nM). None of the antibody-drug conjugates exhibited anticellular effect on the MDA-MB-468 cells ($IC_{50}$ > 0.1 (nM)).

Example 38: Evaluation of cell growth inhibition of antibody-drug conjugate (2)

NCI-N87 (ATCC CRL-5822), a human gastric cancer cell line of HER2 antigen-positive cells, was cultured in RPMI1640 Medium (Thermo Fisher Scientific; hereinafter, referred to as RPMI medium) containing 10% fetal bovine serum (Hyclone). JIMT-1 (Deutsche Sammlung von Mikroorganismen und Zellkulturen GmbH; DSMZ ACC 589), a human breast cancer cell line of HER2 antigen-positive cells, was cultured in Dulbecco's Modified Eagle Medium (Thermo Fisher Scientific; hereinafter, referred to as DMEM medium) containing 10% fetal bovine serum (Hyclone). NCI-N87 were prepared with RPMI medium to reach $5.0 \times 10^4$ cells/mL and JIMT-1 cells were prepared with DMEM medium to reach $1.3 \times 10^4$ cells/mL, and 80 μL portions of them were added to a 96-well cell culture microplate, and the cells were cultured at 37°C and 5% $CO_2$ overnight.

On the next day, 20 μL portions of the anti-HER2 antibody-drug conjugate ADC7 or anti-LPS antibody-drug conjugate ADC13 diluted with RPMI medium or DMEM medium to 400 nM, 80 nM, 16 nM, 3.2 nM, 0.64 nM, 0.13 nM, 0.026 nM, 0.0051 nM, and 0.0010 nM were added to the microplate. To each well without any antibody-drug conjugate, 20 μL of RPMI medium or DMEM medium was added. The cells were cultured at 37°C and 5% $CO_2$ for 6 days. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer). Cell survival rates were calculated by using the same formula as in Example 37.

The anti-HER2 antibody-drug conjugate ADC7 exhibited an anticellular effect of 0.001 < IC50 < 0.1 (nM) on both of the NCI-N87 and JIMT-1 cells. The anti-LPS antibody-drug conjugate ADC13 exhibited, by contrast, no anticellular effect on both cells ($IC_{50}$ > 0.1 (nM)).

Example 39: Evaluation of cell growth inhibition of antibody-drug conjugate (3)

OV-90 (ATCC CRL-11732), a human ovarian cancer cell line of CLDN6 antigen-positive cells, was cultured in 1:1 mixed medium (hereinafter, referred to as the medium) of Medium 199 (Thermo Fisher Scientific) and MCDB 105 Medium (Sigma-Aldrich Co. LLC) containing 15% fetal bovine serum (Hyclone). OV-90 cells were prepared with the medium to reach $1.9 \times 10^4$ cells/mL, and 80 μL portions of them were added to a 96-well cell culture microplate, and

the cells were cultured at 37°C and 5% $CO_2$ overnight.

On the next day, 20 μL portions of the anti-CLDN6 antibody-drug conjugate ADC8, ADC9, or ADC10 diluted with the medium to 50 nM, 10 nM, 2.0 nM, 400 pM, 80 pM, 16 pM, 3.2 pM, 0.64 pM, and 0.13 pM were added to the microplate. To each well without any antibody-drug conjugate, 20 μL of the medium was added. The cells were cultured at 37°C and 5% $CO_2$ for 6 days. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer). Cell survival rates were calculated by using the same formula as in Example 37.

The anti-CLDN6 antibody-drug conjugates ADC8, ADC9, and ADC10 each exhibited an anticellular effect of $0.001 < IC_{50} < 0.1$ (nM) on the OV-90 cells.

Example 40: Antitumor test for antibody-drug conjugate (1)

Mouse: Four- to five-week-old female BALB/c nude mice (Charles River Laboratories Japan, Inc.) were habituated under SPF conditions for 4 to 7 days before being used for experiment. To the mice, sterilized pellets (FR-2, Funabashi Farms Co., Ltd.) were fed and sterilized tap water (prepared by adding 5 to 15 ppm sodium hypochlorite solution) was provided.

Assay and calculation formula: In all of the studies, the major axis and minor axis of a tumor were measured twice or three times a week by using an electronic digital caliper (CD-15CX, Mitutoyo Corp.), and the tumor volume ($mm^3$) was calculated.
The calculation formula is as shown below.

$$\text{Tumor volume (mm}^3\text{)} = \text{Major axis (mm)} \times [\text{Minor axis (mm)}]^2 \times 1/2$$

Each of the antibody-drug conjugates and antibodies was diluted with 10 mM acetate buffer, 5% sorbitol, pH 5.5 (NACALAI TESQUE, INC.; ABS buffer), and a liquid volume of 10 mL/kg was intravenously administered into the tail vein. As a control group (Vehicle group), ABS buffer was administered in the same manner.

NCI-N87 cells (ATCC CRL-5822) were suspended in physiological saline (Otsuka Pharmaceutical Factory, Inc.), and $1 \times 10^7$ cells were subcutaneously transplanted to the right flank of each female nude mouse (Day 0), and the mice were randomly grouped on Day 7. The anti-HER2 antibody-drug conjugate ADC2 of ADC1 was intravenously administered into the tail vein on Day 7 at a dose of 0.3 mg/kg for ADC2 and on Day 7 at a dose of 1 mg/kg for ADC1. As a control group (Vehicle group), ABS buffer was administered in the same manner.

Figure 4 shows the results. The anti-HER2 antibody-drug conjugates ADC2 and ADC1 were found to have strong antitumor effect causing regression of tumor. No weight loss was found for any of the mice with administration of any of the anti-HER2 antibody-drug conjugates.

In the following evaluation examples relating to antitumor test, the method used in the present evaluation example was conducted, unless otherwise stated.

Example 41: Antitumor test for antibody-drug conjugate (2)

NCI-N87 cells (ATCC CRL-5822) were suspended in Dulbecco's phosphate buffered saline (Sigma-Aldrich Co. LLC), and $1 \times 10^7$ cells were subcutaneously transplanted to the right flank of each female nude mouse (Day 0), and the mice were randomly grouped on Day 4. The anti-HER2 antibody-drug conjugate ADC7, the anti-HER2 antibody trastuzumab (Reference Example 3), or the anti-LPS antibody-drug conjugate ADC13 was intravenously administered into the tail vein on Day 4 at a dose of 0.33 mg/kg for all cases. As a control group (Vehicle group), ABS buffer was administered in the same manner.

Figure 5 shows the results. The anti-HER2 antibody-drug conjugate ADC7 was found to have strong antitumor effect causing regression of tumor. By contrast, the anti-HER2 antibody trastuzumab and the anti-LPS antibody-drug conjugate ADC13 did not suppress tumor growth. No weight loss caused by administration of the antibody-drug conjugate ADC7 or ADC13, or the anti-HER2 antibody was found for the mice.

Example 42: Antitumor test for antibody-drug conjugate (3)

KPL-4 cells (Dr. Junichi Kurebayashi, Kawasaki Medical School, British Journal of Cancer, (1999) 79(5/6). 707-717) were suspended in Dulbecco's phosphate buffered saline (Sigma-Aldrich Co. LLC), and $1.5 \times 10^7$ cells were subcutaneously transplanted to the right flank of each female nude mouse (Day 0), and the mice were randomly grouped

on Day 14. The anti-HER2 antibody-drug conjugate ADC7, the anti-LPS antibody-drug conjugate ADC13, or trastuzumab tesirine (Reference Example 1) was intravenously administered into the tail vein on Day 14 at a dose of 0.4 mg/kg. As a control group (Vehicle group), ABS buffer was administered in the same manner.

Figure 6 shows the results. The anti-HER2 antibody-drug conjugate ADC7 was found to have strong antitumor effect causing regression of tumor. By contrast, regression of tumor was not found for ADC13 and trastuzumab tesirine. No weight loss caused by administration of ADC7 or trastuzumab tesirine was found for the mice.

Example 43: Antitumor test for antibody-drug conjugate (4)

JIMT-1 cells (DSMZ ACC 589) were suspended in physiological saline (Otsuka Pharmaceutical Factory, Inc.), and $5 \times 10^6$ cells were subcutaneously transplanted to the right flank of each female nude mouse (Day 0), and the mice were randomly grouped on Day 10. The anti-HER2 antibody-drug conjugate ADC7 or trastuzumab tesirine (Reference Example 1) was intravenously administered into the tail vein on Day 10 at a dose of 0.4 mg/kg. As a control group (Vehicle group), ABS buffer was administered in the same manner.

Figure 7 shows the results. The anti-HER2 antibody-drug conjugate ADC7 was found to have strong antitumor effect causing regression of tumor. For trastuzumab tesirine, by contrast, antitumor effect was found but regression of tumor was not found. No weight loss caused by administration of ADC7 or trastuzumab tesirine was found for the mice.

Example 44: Antitumor test for antibody-drug conjugate (5)

OV-90 cells (ATCC CRL-11732) were suspended in Matrigel (Corning Incorporated), and $2.5 \times 10^6$ cells were subcutaneously transplanted to the right flank of each female nude mouse (Day 0), and the mice were randomly grouped on Day 15. The anti-CLDN6 antibody-drug conjugate ADC8 or anti-CLDN6 antibody (H1L1) tesirine was intravenously administered into the tail vein on Day 15 at a dose of 0.33 mg/kg. As a control group (Vehicle group), ABS buffer was administered in the same manner.

Figure 8 shows the results. The anti-CLDN6 antibody-drug conjugate ADC8 was found to have strong antitumor effect causing regression of tumor. By contrast, regression of tumor was not found for the anti-CLDN6 antibody (H1L1) tesirine. No weight loss caused by administration of ADC8 or H1L1 tesirine was found for the mice.

Example 45: Antitumor test for antibody-drug conjugate (6)

NIH:OVCAR-3 cells (ATCC HTB-161) were suspended in Matrigel (Corning Incorporated), and $1 \times 10^7$ cells were subcutaneously transplanted to the right flank of each female nude mouse (Day 0), and the mice were randomly grouped on Day 25. The anti-CLDN6 antibody-drug conjugate ADC8 or anti-CLDN6 antibody (H1L1)-tesirine was intravenously administered into the tail vein on Day 25 at a dose of 0.33 mg/kg. As a control group (Vehicle group), ABS buffer was administered in the same manner.

Figure 9 shows the results. The anti-CLDN6 antibody-drug conjugate ADC8 was found to have strong antitumor effect causing regression of tumor. For H1L1-tesirine, by contrast, antitumor effect was found but regression of tumor was not found. No weight loss caused by administration of ADC8 or HILI-tesirine was found for the mice.

Example 46: Antitumor test for antibody-drug conjugate (7)

FaDu cells (ATCC HTB-43) were suspended in physiological saline (Otsuka Pharmaceutical Factory, Inc.), and $3 \times 10^6$ cells were subcutaneously transplanted to the right flank of each female nude mouse (Day 0), and the mice were randomly grouped on Day 10. The anti-TROP2 antibody-drug conjugate ADC11 or the anti-LPS antibody-drug conjugate ADC13 was intravenously administered into the tail vein on Day 10 at a dose of 0.4 mg/kg. As a control group (Vehicle group), ABS buffer was administered in the same manner.

Figure 10 shows the results. The anti-TROP2 antibody-drug conjugate ADC11 was found to have strong antitumor effect causing regression of tumor. By contrast, the anti-LPS antibody-drug conjugate ADC13 did not suppress tumor growth. No weight loss caused by administration of ADC11 or ADC13 was found for the mice.

Example 47: Antitumor test for anti-HER2 antibody-drug conjugate (8)

Antitumor effect of the anti-HER2 antibody-drug conjugates was measured by using the same experiment animals and method as in Example 40.

KPL-4 cells (Dr. Junichi Kurebayashi, Kawasaki Medical School) were suspended in Dulbecco's phosphate buffered saline (Sigma-Aldrich Co. LLC), and $1.5 \times 10^7$ cells were subcutaneously transplanted to the right flank of each female nude mouse (Day 0), and the mice were randomly grouped on Day 14. The anti-HER2 antibody-drug conjugate

ADC7 or the anti-HER2 antibody-drug conjugate ADC14 was intravenously administered into the tail vein on Day 14 at a dose of 0.33 mg/kg. As a control group (Vehicle group), ABS buffer was administered in the same manner.

Figure 47 shows the results. ADC7 and ADC14 exhibited comparable antitumor activity by administration of 0.33 mg/kg. No weight loss caused by administration of ADC7 or ADC14 was found for the mice.

Example 48: Antitumor test for anti-HER2 antibody-drug conjugate (9)

JIMT-1 cells (DSMZ ACC 589) were suspended in physiological saline (Otsuka Pharmaceutical Factory, Inc.), and 5 × $10^6$ cells were subcutaneously transplanted to the right flank of each female nude mouse (Day 0), and the mice were randomly grouped on Day 11. ADC14 was intravenously administered into the tail vein on Day 11 at a dose of 0.4 mg/kg or 0.2 mg/kg. As a control group (Vehicle group), ABS buffer was administered in the same manner.

Figure 48 shows the results. ADC14 was found to have strong antitumor effect causing regression of tumor in administration of 0.4 mg/kg. No weight loss caused by administration of ADC14 was found for the mice with administration at any dose.

Example 49: Antitumor test for anti-HER2 antibody-drug conjugate (3)

CFPAC-1 cells (ATCC CRL-1918) were suspended in physiological saline (Otsuka Pharmaceutical Factory, Inc.), and 5 × $10^6$ cells were subcutaneously transplanted to the right flank of each female nude mouse (Day 0), and the mice were randomly grouped on Day 10. The anti-HER2 antibody-drug conjugate ADC7 or ADC14, or the anti-LPS antibody-drug conjugate ADC13 was intravenously administered into the tail vein on Day 10 at a dose of 0.4 mg/kg. As a control group (Vehicle group), ABS buffer was administered in the same manner.

Figure 49 shows the results. Strong antitumor effect causing regression of tumor was found for any mice with administration of any of ADC7 and ADC14. No weight loss caused by administration of ADC7, ADC14, or ADC13 was found for the mice.

[Synthesis of drug D]

Example 50: Drug 1

A free drug (drug 1) from ADC7 to ADC1 was synthesized in accordance with the following scheme.

[Formula 92]

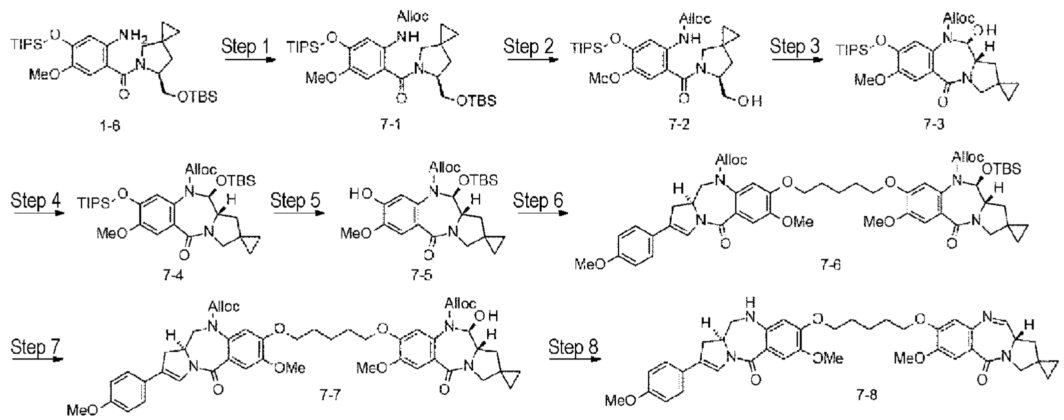


Step 1: Prop-2-en-1-yl (2-{[(6S)-6-({[tert-butyl(dimethyl)silyl]oxy}methyl)-5-azaspiro[2.4]hept-5-yl]carbonyl}-4-methoxy-5-{[tri(propan-2-yl)silyl]oxy}phenyl)carbamate (7-1)

Compound (1-6) obtained in Step 5 of Example 1-1 (4.59 g, 8.15 mmol) was reacted in the same manner as in Step 9 of Example 2-1 to afford the desired compound (7-1) (4.86 g, 92%).
MS(APCI, ESI)m/z:647(M+H)$^+$

Step 2: Prop-2-en-1-yl (2-{[(6S)-6-(hydroxymethyl)-5-azaspiro[2.4]hept-5-yl]carbonyl}-4-methoxy-5-{[tri(propan-2-yl)silyl]oxy}phenyl)carbamate (7-2)

Compound (7-1) obtained in Step 1 (4.86 g, 7.51 mmol) was reacted in the same manner as in Step 7 of Example 1-1 to afford the desired compound (7-2) (3.42 g, 86%). MS(APCI, ESI)m/z:533(M+H)+

Step 3: Prop-2-en-1-yl (1 1a'S)-11'-hydroxy-7'-methoxy-5'-oxo-8'-{[tri(propan-2-yl)silyl]oxy }-11',11a'-dihydro-1'H-spiro[cyclopropane-1,2'-pyrrolo[2,1-c][1,4]benzodiazepine]-10'(5'H)-carboxylate (7-3)

Compound (7-2) obtained in Step 2 (6.68 g, 12.5 mmol) was reacted in the same manner as in Step 8 of Example 1-1 to afford the desired compound (7-3) (6.44 g, 97%). MS(APCI, ESI)m/z:531(M+H)+

Step 4: Prop-2-en-1-yl (11a'S)-11'-{[tert-butyl(dimethyl)silyl]oxy}-7'-methoxy-5'-oxo-8'-{[tri(propan-2-yl)silyl]oxy}-11',11a'-dihydro-1'H-spiro[cyclopropane-1,2'-pyrrolo[2,1-c][1,4]benzodiazepine]-10'(5'H)-carboxylate (7-4)

Compound (7-3) obtained in Step 3 (3.24 g, 6.10 mmol) was reacted in the same manner as in Step 9 of Example 1-1 to afford the desired compound (7-4) (3.86 g, 98%). MS(APCI, ESI)m/z:645(M+H)+

Step 5: Prop-2-en-1-yl (11a'S)-11'-{[tert-butyl(dimethyl)silyl]oxy}-8'-hydroxy-7'-methoxy-5'-oxo-11',11a'-dihydro-1'H-spiro[cyclopropane-1,2'-pyrrolo[2,1 - c][1,4]benzodiazepine]-10'(5'H)-carboxylate (7-5)

Compound (7-4) obtained in Step 4 (4.49 g, 6.96 mmol) was reacted in the same manner as in Step 10 of Example 1-1 to afford the desired compound (7-5) (3.24 g, 95%).
MS(APCI, ESI)m/z:489(M+H)+

Step 6: Prop-2-en-1-yl (11a'S)-11'-{ [tert-butyl(dimethyl)silyl]oxy}-7'-methoxy-8'-{ [5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c][1,4]benzodiazepin-8-yl}oxy)pentyl]oxy}-5'-oxo-11',11a'-dihydro-1'H-spiro[cyclopropane-1,2'-pyrrolo[2,1-c] [1,4]benzodiazepine]-10'(5'H)-carboxylate (7-6)

Compound (7-5) obtained in Step 5 (0.080 g, 0.164 mmol) was reacted in the same manner as in Step 10 of Example 2-1 to afford the desired compound (7-6) (0.160 g, 98%).
MS(APCI, ESI)m/z:993(M+H)+

Step 7: Prop-2-en-1-yl (1 1a'S)-11'-hydroxy-7'-methoxy-8'-{[5-({(11aS)-7-methoxy-2-(4-methoxyphenyl)-5-oxo-10-[(prop-2-en-1-yloxy)carbonyl]-5,10,11,11a-tetrahydro-1H-pyrrolo[2,1-c] [1,4]benzodiazepin-8-yl }oxy)pentyl]oxy}-5'-oxo-11',11a'-dihydro-1'H-spiro[cyclopropane-1,2'-pyrrolo[2,1-c][1,4]benzodiazepine]-10'(5'H)-carboxylate (7-7)

Compound (7-6) obtained in Step 6 (160 mg, 0.161 mmol) was reacted in the same manner as in Step 11 of Example 2-1 to afford the desired compound (7-7) (141 mg, quantitative).
MS(APCI, ESI)m/z:879(M+H)+

Step 8: (11a'S)-7'-Methoxy-8'-[(5-{[(11aS)-7-methoxy-2-(4-methoxyphenyl)-5-oxo-5,10,11,11a-tetrahydro- 1H-pyrrolo[2,1-c][1,4]benzodiazepin-8-yl]oxy}pentyl)oxy]-1',11a'-dihydro-5'H-spiro[cyclopropane-1,2'-pyrrolo[2,1-c][1,4]benzodiazepine]-5'-one (7-8)

Compound (7-7) obtained in Step 7 (141 mg, 0.161 mmol) was reacted in the same manner as in Step 12 of Example 2-1 to afford the desired compound (7-8: drug 1) (109.8 mg, 99%).
$^1$H-NMR (DMSO-$D_6$) δ: 7.92-7.91 (1H, m), 7.45 (1H, s), 7.39-7.37 (2H, m), 7.33 (1H, s), 7.29 (1H, s), 6.92-6.89 (2H, m), 6.85 (1H, s), 6.56-6.54 (1H, m), 6.31 (1H, s), 4.19-4.12 (2H, m), 4.05-3.99 (1H, m), 3.95-3.93 (2H, m), 3.82-3.79 (4H, m), 3.76 (3H, s), 3.66 (3H, s), 3.52-3.46 (3H, m), 3.30-3.21 (2H, m), 2.78-2.74 (1H, m), 2.45-2.42 (1H, m), 2.06-2.05 (1H, m), 1.89-1.82 (4H, m), 1.60-1.58 (2H, m), 0.80-0.63 (4H, m). MS(APCI, ESI)m/z:693(M+H)+

Example 51: Evaluation of cell growth inhibition of drug 1 (4)

KPL-4 cells (Dr. Junichi Kurebayashi, Kawasaki Medical School, British Journal of Cancer, (1999) 79(5/6). 707-717), a human breast cancer cell line, were prepared with RPMI1640 Medium (Thermo Fisher Scientific; hereinafter, referred to as RPMI medium) containing 10% fetal bovine serum (Hyclone) to reach 6.25 × $10^3$ cells/mL, and 80 μL

portions of them were added to a 96-well cell culture microplate. After addition of the cells, the cells were cultured at 37°C and 5% $CO_2$ overnight.

On the next day, 20 μL portions of drug 1 diluted with RPMI medium to 100 nM, 20 nM, 4 nM, 0.8 nM, 0.16 nM, 0.032 nM, 6.4 pM, 1.3 pM, and 0.26 pM were added to the microplate. To each well without any antibody-drug conjugate, 20 μL of RPMI medium was added. KPL-4 was cultured at 37°C and 5% $CO_2$ for 6 days. After culturing, the microplate was taken out of the incubator, and left to stand at room temperature for 30 minutes. CellTiter-Glo Luminescent Cell Viability Assay (Promega Corporation) in an amount equivalent to that of the culture solution was added, and stirred using a plate mixer. The microplate was left to stand at room temperature for 10 minutes, and thereafter the amount of emission was measured by using a plate reader (PerkinElmer). Cell survival rates were calculated by using the same formula as in Example 37.

Drug 1 exhibited an anticellular effect of $0.01 < IC_{50} < 0.02$ (nM) on the KPL-4 cells.

Example 52-1: Drug-linker 5

[Formula 93]

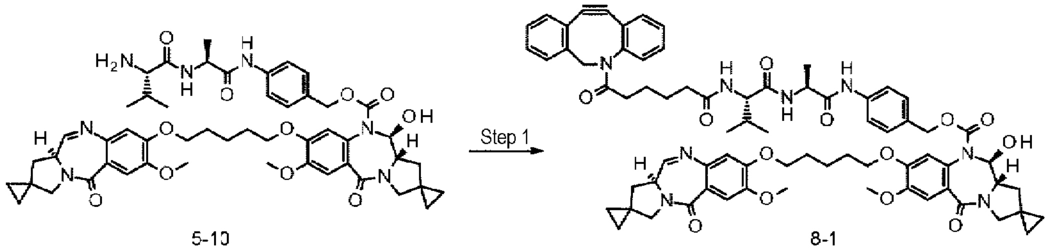


5-10

8-1

Step 1: Compound (8-1)

Compound (5-10) obtained in Step 9 of Example 2-3 (0.100 g, 0.105 mmol) and azadibenzocyclooctynoic acid (0.0351 g, 0.105 mmol) were reacted in the same manner as in Step 10 of Example 2-3 to afford the desired compound (8-1) (0.0702 g, 53%). MS(APCI, ESI)m/z: $1265(M+H)^+$.

Example 52-2: ADC15

[Formula 94]

R = 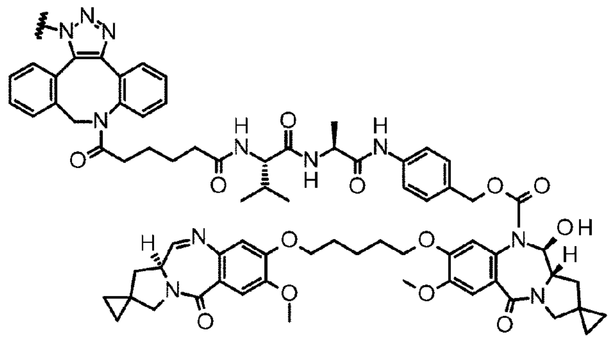

or

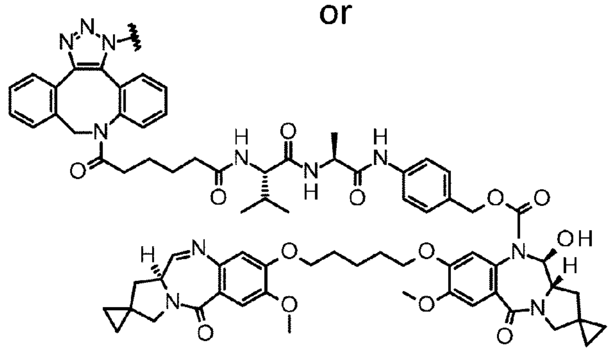

In the compound obtained in Step 1 of Example 52-2, the triazole ring has geometric isomers as illustrated in the formula, and the compound has a drug-linker as a mixture of the two structures shown above as R.

Step 1: Conjugation of antibody and drug-linker

To a phosphate buffered saline (pH 6.0) solution of the antibody (9.88 mg/mL, 1.00 mL) obtained in Step 1 of Example 14, 1,2-propanediol (0.917 mL) and a 10 mM dimethyl sulfoxide solution of compound (8-1) obtained in Step 1 of Example 52-1 (0.0825 mL; 12 equivalents per antibody molecule) were added at room temperature, and the reaction was carried out using a tube rotator (MTR-103, AS ONE Corporation) at room temperature for 1 day.

Purification operation: The solution was purified by using common operation D to afford 10.5 mL of a solution of the desired compound. This solution was concentrated by using common operation A to afford 0.850 mL of a solution of the desired compound.
Characterization: The following characteristic values were obtained by using common operations E and F.
Antibody concentration: 8.90 mg/mL, antibody yield: 7.56 mg (77%), average number of conjugated drug molecules per antibody molecule (n): 1.8

Example 53-1: Drug-linker 6

[Formula 95]

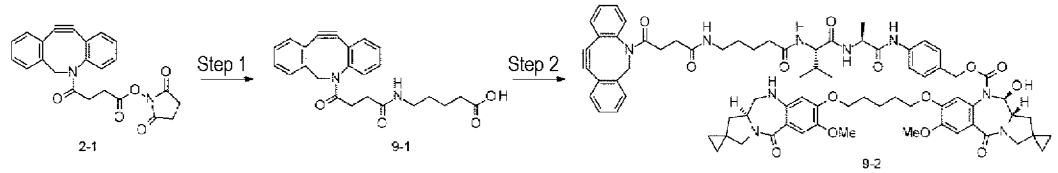

Step1: Compound (9-1)

In N,N-dimethylformamide (10 mL), 5-aminovaleric acid (0.436 g, 3.72 mmol) was dissolved, to which 1-{[4-(11,12-didehydrodibenzo[b,f]azocin-5 (6H)-yl)-4-oxobutanoyl]oxy}pyrrolodin-2,5-dione (1.36 g, 3.38 mmol) and triethylamine (0.937 mL, 6.76 mmol) were added at room temperature, and the resultant was stirred at room temperature for 1.5 hours. To the reaction solution, 1 N hydrochloric acid was added, which was extracted with chloroform, and the organic layer obtained was washed with water and brine and then dried over magnesium sulfate. After distillation under reduced pressure, the resulting residue was purified by silica gel column chromatography [organic layer for distribution with chloroform to chloroform:methanol:water = 7:3:1 (v/v/v)] to afford an N,N-dimethylformamide adduct of the desired compound (9-1) (0.730 g, 45%) as a solid.
MS(APCI, ESI)m/z:405$(M+H)^+$

Step 2: Compound (9-2)

Compound (9-1) obtained in Step 1 (0.0176 g, 0.0368 mmol) was reacted in the same manner as in Step 13 of Example 2-1 to afford the desired compound (9-2) (0.00880 g, 18%).
MS(APCI, ESI)m/z: 1339(M+H)

Example 53-2: ADC16

[Formula 96]

R =

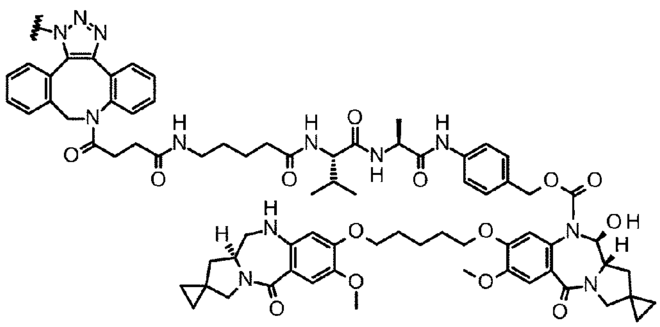

or

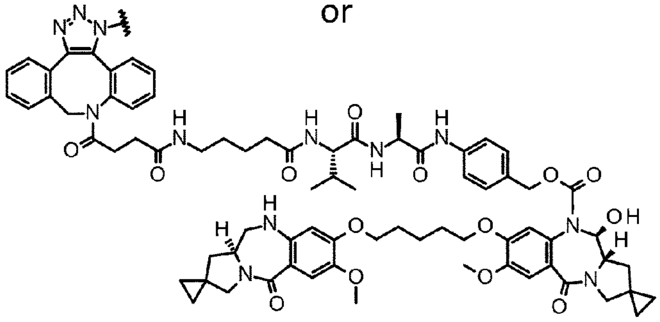

In the compound obtained in Step 1 of Example 53-2, the triazole ring has geometric isomers as illustrated in the formula, and the compound has a drug-linker as a mixture of the two structures shown above as R.

Step 1: Conjugation of antibody and drug-linker

To a phosphate buffered saline (pH 6.0) solution of the antibody (10.0 mg/mL, 1.00 mL) obtained in Step 1 of Example 15, 1,2-propanediol (0.917 mL) and a 10 mM dimethyl sulfoxide solution of compound (9-2) obtained in Step 2 of Example 53-1 (0.0825 mL; 12 equivalents per antibody molecule) were added at room temperature, and the reaction was carried out using a tube rotator (MTR-103, AS ONE Corporation) at room temperature for 2 days.

Purification operation: The solution was purified by using common operation D to afford 6.0 mL of a solution of the

desired compound.

Characterization: The following characteristic values were obtained by using common operations E and F.

Antibody concentration: 1.53 mg/mL, antibody yield: 9.17 mg (92%), average number of conjugated drug molecules per antibody molecule (n): 1.9

Example 54-1: Drug-linker 7

[Formula 97]

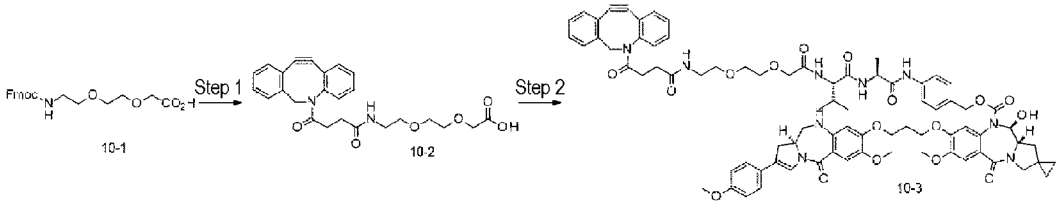


Step 1: Compound (10-2)

To a solution of starting material (10-1) (3.00 g, 7.78 mmol, Tokyo Chemical Industry Co., Ltd.) in N,N-dimethylformamide (10 mL), 1,8-diazabicyclo[5.4.0]-7-undecene (1.16 mL, 7.78 mL) was added, and the resultant was stirred at room temperature for 2 hours, and 1-{[4-(11,12-didehydrodibenzo[b,flazocin-5 (6H)-yl)-4-oxobutanoyl]oxy}pyrrolidin-2,5-dione (1.10 g, 2.72 mmol) and triethylamine (1.94 mL, 14.0 mmol)were then added thereto at room temperature. The reaction solution was distillated under reduced pressure, and the resulting residue was then purified by silica gel column chromatography [chloroform:methanol = 100:0 (v/v) to chloroform:methanol = 90:10 (v/v)] to afford the desired compound (10-2) (0.410 g, 12%).

MS(APCI, ESI)m/z:451(M+H)$^+$

Step 2: Compound (10-3)

Compound (10-2) obtained in Step 1 (0.050 g, 0.0499 mmol) was reacted in the same manner as in Step 13 of Example 2-1 to afford the desired compound (10-3) (0.0590 g, 82%).

MS(APCI, ESI)m/z: 1434(M+H)$^+$

Example 54-2: ADC17

[Formula 98]

R = 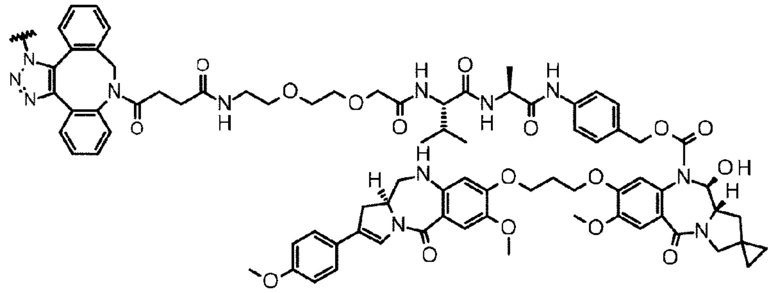

or

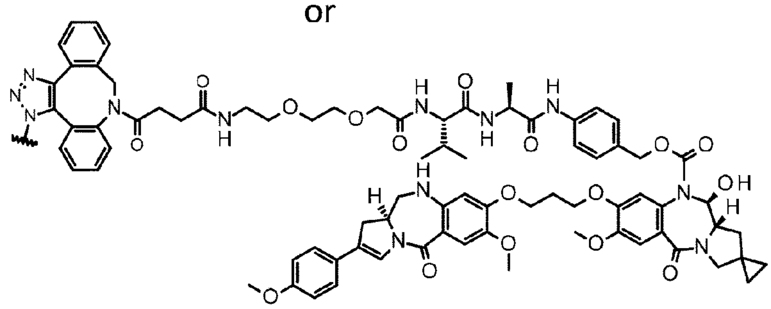

In the compound obtained in Step 1 of Example 54-2, the triazole ring has geometric isomers as illustrated in the formula, and the compound has a drug-linker as a mixture of the two structures shown above as R.

Step 1: Conjugation of antibody and drug-linker

To a phosphate buffered saline (pH 6.0) solution of the antibody (10.0 mg/mL, 1.00 mL) obtained in Step 1 of Example 15, 1,2-propanediol (0.917 mL) and a 10 mM dimethyl sulfoxide solution of compound (10-3) obtained in Step 2 of Example 54-1 (0.0825 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 6.0 mL of a solution of the desired compound.
Characterization: The following characteristic values were obtained by using common operations E and F.
Antibody concentration: 1.63 mg/mL, antibody yield: 9.80 mg (98%), average number of conjugated drug molecules per antibody molecule (n): 1.8

Example 55-1: Drug-linker 8

[Formula 99]

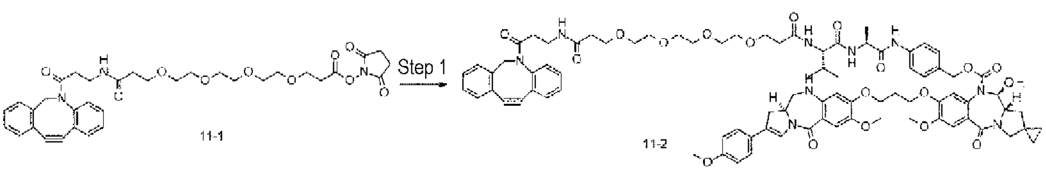


11-1 11-2

Step 1: Compound (11-2)

A solution of compound (4-11) obtained in Step 11 of Example 2-2 (0.0500 g, 0.0499 mmol), starting material (11-1) (0.050 g, 0.0499 mmol, commercially available from Alfa Aesar), and triethylamine (0.00830 mL, 0.0599 mmol) in dichloromethane (3 mL) was stirred at room temperature overnight. The resultant was distillated under reduced

pressure, and the resulting residue was then purified by silica gel column chromatography [chloroform:methanol = 100:0 (v/v) to chloroform:methanol = 90:10 (v/v)] to afford the desired compound (11-2) (0.0490 g, 64%).
MS(APCI, ESI)m/z: 1536(M+H)$^+$

Example 55-2: ADC18

[Formula 100]

R =

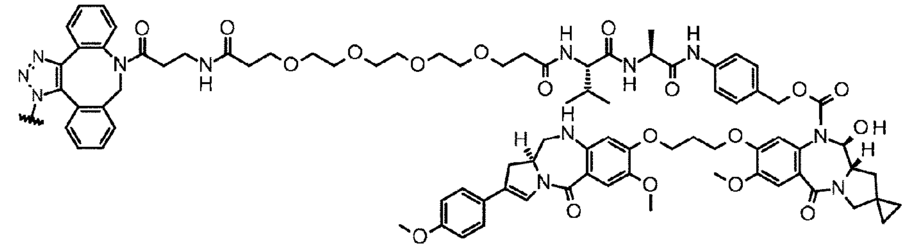

or

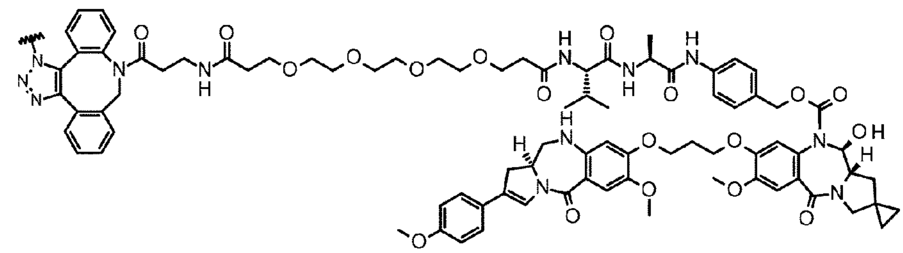

In the compound obtained in Step 1 of Example 55-2, the triazole ring has geometric isomers as illustrated in the formula, and ADC obtained has a drug-linker as a mixture of the two structures shown above as R.

Step 1: Conjugation of antibody and drug-linker

To a phosphate buffered saline (pH 6.0) solution of the antibody (10.0 mg/mL, 1.00 mL) obtained in Step 1 of Example 15, 1,2-propanediol (0.917 mL) and a 10 mM dimethyl sulfoxide solution of compound (11-2) obtained in Step 1 of Example 55-1 (0.0825 mL; 12 equivalents per antibody molecule) were added at room temperature, and the reaction was carried out using a tube rotator (MTR-103, AS ONE Corporation) at room temperature for 48 hours.

Purification operation: The solution was purified by using common operation D to afford 6.0 mL of a solution of the desired compound.
Characterization: The following characteristic values were obtained by using common operations E and F.
Antibody concentration: 1.72 mg/mL, antibody yield: 10.3 mg (quantitative), average number of conjugated drug molecules per antibody molecule (n): 1.9

Example 56-1: Drug-linker 9

[Formula 101]

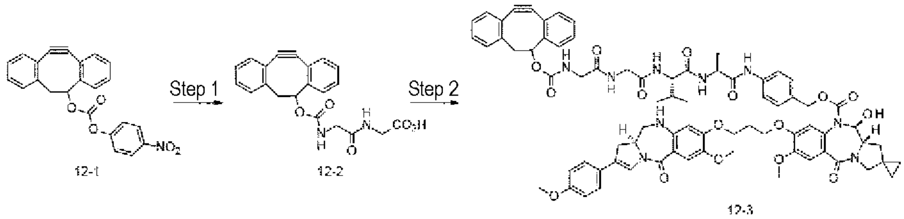


Step 1: Compound (12-2)

To a solution of 11,12-didehydro-5,6-dihydrodibenzo[a,e][8]annulen-5-yl 4-nitrophenylcarbamate (12-1) (0.437 g, 1.14 mmol) and N,N-diisopropylethylamine (0.198 mL, 1.14 mmol) in N,N-dimethylformamide (6 mL), glycylglycine (0.150 g, 1.14 mmol) and water (3 mL) were added at room temperature, and the resultant was stirred at room temperature overnight. The resultant was distilled under reduced pressure, and the resulting residue was purified by silica gel column chromatography [chloroform:CMW = 100:0(v/v) to 0:100(v/v)] to afford the desired compound (12-2) (0.324 g, 75%).
MS(APCI, ESI)m/z:378$(M+H)^+$

Step 2: Compound (8-3)

Compound (12-2) obtained in Step 1 (0.0306 g, 0.0305 mmol) was reacted with compound (4-11) obtained in Step 11 of Example 2-2 in the same manner as in Step 13 of Example 2-1 to afford the desired compound (12-3) (0.0361 g, 87%).
MS(APCI, ESI)m/z: 1362$(M+H)^+$

Example 56-2: ADC19

[Formula 102]

R = 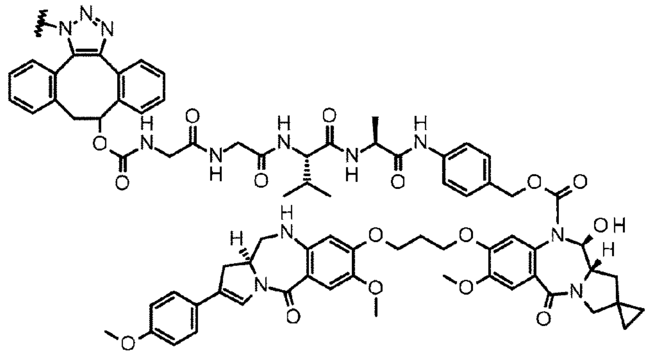

or

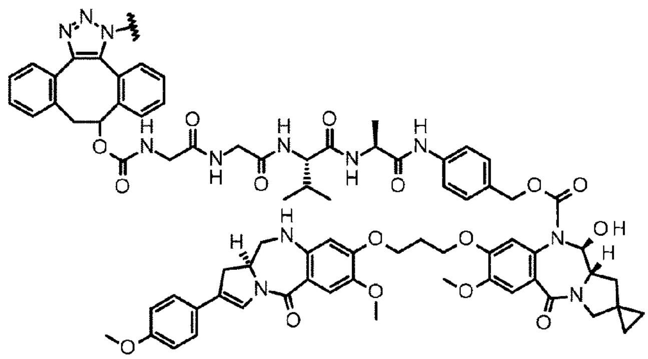

In the compound obtained in Step 1 of Example 56-2, the triazole ring has geometric isomers as illustrated in the formula, and the compound has a drug-linker as a mixture of the two structures shown above as R.

Step 1: Conjugation of antibody and drug-linker

To a phosphate buffered saline (pH 6.0) solution of the antibody (10.0 mg/mL, 1.00 mL) obtained in Step 1 of Example 15, 1,2-propanediol (0.917 mL) and a 10 mM dimethyl sulfoxide solution of compound (12-3) obtained in Step 2 of Example 56-1 (0.0825 mL; 12 equivalents per antibody molecule) were added at room temperature, and the reaction was carried out using a tube rotator (MTR-103, AS ONE Corporation) at room temperature for 3 days.

Purification operation: The solution was purified by using common operation D to afford 6.00 mL of a solution of the desired compound.
Characterization: The following characteristic values were obtained by using common operations E and F.
Antibody concentration: 1.06 mg/mL, antibody yield: 6.35 mg (63%), average number of conjugated drug molecules per antibody molecule (n): 1.2

Industrial Applicability

Use of the antibody-drug conjugate, antibody and/or PBD derivative, and so on of the present invention enables treatment or prevention of various cancers.

Free Text of Sequence Listing

SEQ ID NO: 1 - Amino acid sequence of human CLDN6
SEQ ID NO: 2 - Nucleotide sequence of cDNA encoding amino acid sequence of human CLDN6
SEQ ID NO: 3 - Amino acid sequence of human CLDN9
SEQ ID NO: 4 - Nucleotide sequence of cDNA encoding amino acid sequence of human CLDN9
SEQ ID NO: 5 - Amino acid sequence of CDRL1 ofB1 antibody light chain

SEQ ID NO: 6 - Amino acid sequence of CDRL2 ofB1 antibody light chain
SEQ ID NO: 7 - Amino acid sequence of CDRL3 ofB1 antibody light chain
SEQ ID NO: 8 - Amino acid sequence of CDRL3 of humanized B 1 antibody light chain L4
SEQ ID NO: 9 - Amino acid sequence of CDRH1 ofB1 antibody heavy chain
SEQ ID NO: 10 - Amino acid sequence of CDRH2 of B1 antibody heavy chain
SEQ ID NO: 11 - Amino acid sequence of CDRH3 of B1 antibody heavy chain
SEQ ID NO: 12 - Amino acid sequence of CDRL1 of C7 antibody light chain
SEQ ID NO: 13 - Amino acid sequence of CDRL2 of C7 antibody light chain
SEQ ID NO: 14 - Amino acid sequence of CDRL3 of C7 antibody light chain
SEQ ID NO: 15 - Amino acid sequence of CDRH1 of C7 antibody heavy chain
SEQ ID NO: 16 - Amino acid sequence of CDRH2 of C7 antibody heavy chain
SEQ ID NO: 17 - Amino acid sequence of CDRH3 of C7 antibody heavy chain
SEQ ID NO: 18 - Nucleotide sequence of cDNA encoding variable region of B1 antibody light chain
SEQ ID NO: 19 - Amino acid sequence of variable region ofB1 antibody light chain
SEQ ID NO: 20 - Nucleotide sequence of cDNA encoding variable region of B1 antibody heavy chain
SEQ ID NO: 21 - Amino acid sequence of variable region ofB1 antibody heavy chain
SEQ ID NO: 22 - Nucleotide sequence of cDNA encoding variable region of C7 antibody light chain
SEQ ID NO: 23 - Amino acid sequence of variable region of C7 antibody light chain
SEQ ID NO: 24 - Nucleotide sequence of cDNA encoding variable region of C7 antibody heavy chain
SEQ ID NO: 25 - Amino acid sequence of variable region of C7 antibody heavy chain
SEQ ID NO: 26 - DNA fragment including DNA sequence encoding human light chain signal sequence and human κ chain constant region
SEQ ID NO: 27 - DNA fragment including DNA sequence encoding human heavy chain signal sequence and human IgG1 LALA constant region
SEQ ID NO: 28 - Amino acid sequence of chB1 light chain
SEQ ID NO: 29 - DNA fragment including DNA sequence encoding amino acid sequence of chB 1 light chain
SEQ ID NO: 30 - Amino acid sequence of variable region of chB1 light chain
SEQ ID NO: 31 - Nucleotide sequence encoding chB1 light chain variable region
SEQ ID NO: 32 - Amino acid sequence of chB1 heavy chain
SEQ ID NO: 33 - Nucleotide sequence encoding chB1 heavy chain
SEQ ID NO: 34 - Amino acid sequence of variable region of chB1 heavy chain
SEQ ID NO: 35 - Nucleotide sequence encoding variable region of chB1 heavy chain
SEQ ID NO: 36 - Amino acid sequence of humanized antibody light chain hL1
SEQ ID NO: 37 - Nucleotide sequence encoding humanized antibody light chain hL1
SEQ ID NO: 38 - Amino acid sequence of variable region of humanized antibody light chain hL1
SEQ ID NO: 39 - Nucleotide sequence encoding variable region of humanized antibody light chain hL1
SEQ ID NO: 40 - Amino acid sequence of humanized antibody light chain hL2
SEQ ID NO: 41 - Nucleotide sequence encoding humanized antibody light chain hL2
SEQ ID NO: 42 - Amino acid sequence of variable region of humanized antibody light chain hL2
SEQ ID NO: 43 - Nucleotide sequence encoding variable region of humanized antibody light chain hL2
SEQ ID NO: 44 - Amino acid sequence of humanized antibody light chain hL3
SEQ ID NO: 45 - Nucleotide sequence encoding humanized antibody light chain hL3
SEQ ID NO: 46 - Amino acid sequence of variable region of humanized antibody light chain hL3
SEQ ID NO: 47 - Nucleotide sequence encoding variable region of humanized antibody light chain hL3
SEQ ID NO: 48 - Amino acid sequence of humanized antibody light chain hL4
SEQ ID NO: 49 - Nucleotide sequence encoding humanized antibody light chain hL4
SEQ ID NO: 50 - Amino acid sequence of variable region of humanized antibody light chain hL4
SEQ ID NO: 51 - Nucleotide sequence encoding variable region of humanized antibody light chain hL4
SEQ ID NO: 52 - Amino acid sequence of humanized antibody heavy chain hH1
SEQ ID NO: 53 - Nucleotide sequence encoding humanized antibody heavy chain hH1
SEQ ID NO: 54 - Amino acid sequence of variable region of humanized antibody heavy chain hH1
SEQ ID NO: 55 - Nucleotide sequence encoding variable region of humanized antibody heavy chain hH1
SEQ ID NO: 56 - Amino acid sequence of humanized antibody heavy chain hH2
SEQ ID NO: 57 - Nucleotide sequence encoding humanized antibody heavy chain hH2
SEQ ID NO: 58 - Amino acid sequence of variable region of humanized antibody heavy chain hH2
SEQ ID NO: 59 - Nucleotide sequence encoding variable region of humanized antibody heavy chain hH2
SEQ ID NO: 60 - Amino acid sequence of humanized antibody heavy chain hH3
SEQ ID NO: 61 - Nucleotide sequence encoding humanized antibody heavy chain hH3

SEQ ID NO: 62 - Amino acid sequence of variable region of humanized antibody heavy chain hH3
SEQ ID NO: 63 - Nucleotide sequence encoding variable region of humanized antibody heavy chain hH3
SEQ ID NO: 64 - Amino acid sequence of Trastuzumab light chain
SEQ ID NO: 65 - Amino acid sequence of Trastuzumab heavy chain
SEQ ID NO: 66 - Amino acid sequence of anti-LPS antibody (h#1G5-H1L1) light chain
SEQ ID NO: 67 - Amino acid sequence of anti-LPS antibody (h#1G5-H1L1) heavy chain
SEQ ID NO: 68 - Amino acid sequence of anti-TROP2 antibody (hRS7) light chain
SEQ ID NO: 69 - Amino acid sequence of anti-TROP2 antibody (hRS7) heavy chain
SEQ ID NO: 70 - Amino acid sequence of anti-CD98 antibody (hM23-H1L1) light chain
SEQ ID NO: 71 - Amino acid sequence of anti-CD98 antibody (hM23-H1L1) heavy chain
SEQ ID NO: 72 - Nucleotide sequence encoding Trastuzumab variant light chain
SEQ ID NO: 73 - Amino acid sequence of Trastuzumab variant light chain
SEQ ID NO: 74 - Nucleotide sequence encoding Trastuzumab variant heavy chain
SEQ ID NO: 75 - Amino acid sequence of Trastuzumab variant heavy chain
SEQ ID NO: 76 - Amino acid sequence of Trastuzumab variant 2 light chain
SEQ ID NO: 77 - Amino acid sequence of Trastuzumab variant 2 heavy chain

## Claims

**1.** An antibody-drug conjugate represented by the following formula:

[Formula 1]

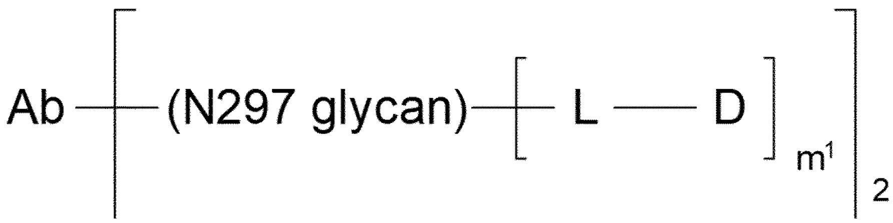

wherein

m1 represents an integer of 1 or 2;
D is any one selected from the following group:

[Formula 2]

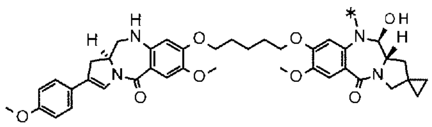

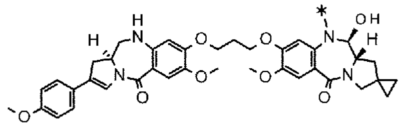

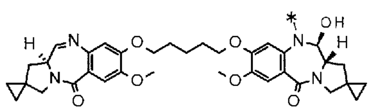

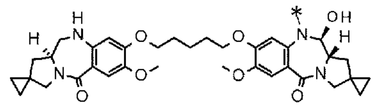

wherein

each asterisk * represents bonding to L;
L is a linker linking the glycan and D, the glycan bonding to Asn297 of Ab (N297 glycan);
the N297 glycan is optionally remodeled; and
Ab represents an antibody or a functional fragment of the antibody.

**2.** The antibody-drug conjugate according to claim 1, wherein

L is represented by -Lb-La-Lp-NH-B-$CH_2$-O(C=O)-*, the asterisk * representing bonding to D;
B is a 1,4-phenyl group, a 2,5-pyridyl group, a 3,6-pyridyl group, a 2,5-pyrimidyl group, or a 2,5-thienyl group;
Lp represents any one selected from the following group:

- GGVA-, -GG-(D-)VA-, -VA-, -GGFG-, -GGPI-, -GGVCit-, -GGVK-, and - GGPL-;

La represents any one selected from the following group:

- C(=O)-$CH_2CH_2$-C(=O)-,-C(=O)-$(CH_2CH_2)_2$-C(=O)-,
- C(=O)-$CH_2CH_2$-C(=O)-NH-$(CH_2CH_2)_2$-C(=O)-,
- C(=O)-$CH_2CH_2$-C(=O)-NH-$(CH_2CH_2O)_2$-$CH_2$-C(=O)-,
- C(=O)-$CH_2CH_2$-NH-C(=O)-$(CH_2CH_2O)_4$-$CH_2CH_2$-C(=O)-, -$CH_2$-OC(=O)-, and -OC(=O)-; and

Lb is represented by the following formula:

[Formula 3]

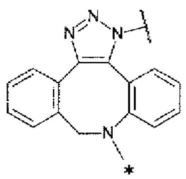

or

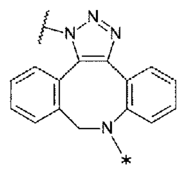

[Formula 4]

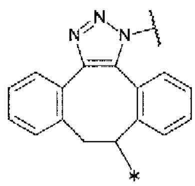

or

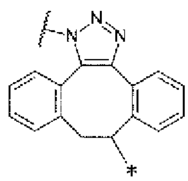

, or

[Formula 5]

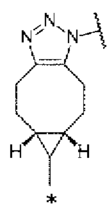

or

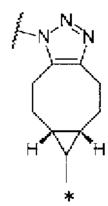

wherein, in each structural formula for Lb shown above,
each asterisk * represents bonding to La, and each wavy line represents bonding to N297 glycan or remodeled N297 glycan.

**3.** The antibody-drug conjugate according to claim 1 or 2, wherein
L represents any one selected from the following group:

- $Z^1$C(=O)-$CH_2CH_2$-C(=O)-GGVA-NH-B-$CH_2$-OC(=O)-,
- $Z^1$C(=O)-$CH_2CH_2$-C(=O)-GG-(D-)VA-NH-B-$CH_2$-OC(=O)-,

- $Z^1C(=O)-CH_2CH_2-C(=O)-VA-NH-B-CH_2-OC(=O)-$,
- $Z^1C(=O)-(CH_2CH_2)_2-C(=O)-VA-NH-B-CH_2-OC(=O)-$,
- $Z^1C(=O)-CH_2CH_2-C(=O)-GGPI-NH-B-CH_2-OC(=O)-$,
- $Z^1C(=O)-CH_2CH_2-C(=O)-GGFG-NH-B-CH_2-OC(=O)-$,
- $Z^1-C(=O)-CH_2CH_2-C(=O)-GGVCit-NH-B-CH_2-OC(=O)-$,
- $Z^1C(=O)-CH_2CH_2-C(=O)-GGVK-NH-B-CH_2-OC(=O)-$,
- $Z^1C(=O)-CH_2CH_2-C(=O)-GGPL-NH-B-CH_2-OC(=O)-$,
- $Z^1C(=O)-CH_2CH_2-C(=O)-NH-(CH_2CH_2)_2-C(=O)-VA-NH-B-CH_2-OC(=O)-$,
- $Z^1C(=O)-CH_2CH_2-C(=O)-NH-(CH_2CH_2O)_2-CH_2-C(=O)-VA-NH-B-CH_2-OC(=O)-$,
- $Z^1C(=O)-CH_2CH_2-NH-C(=O)-(CH_2CH_2O)_4-CH_2CH_2-C(=O)-VA-NH-B-CH_2-OC(=O)-$,
- $Z^2-OC(=O)-GGVA-NH-B-CH_2-OC(=O)-$, and
- $Z^3-CH_2-OC(=O)-GGVA-NH-B-CH_2-OC(=O)-$, wherein

B represents a 1,4-phenyl group,
$Z^1$ represents the following structural formula:

[Formula 6]

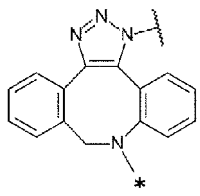

or

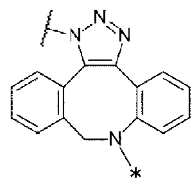

$Z^2$ represents the following structural formula:

[Formula 7]

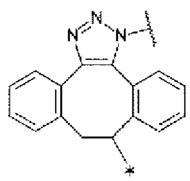

or

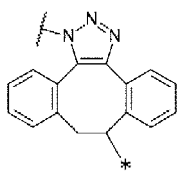

$Z^3$ represents the following structural formula:

[Formula 8]

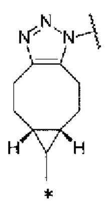

or

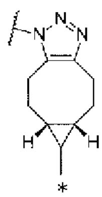

wherein, in each structural formula for $Z^1$, $Z^2$, and $Z^3$,
each asterisk * represents bonding to neighboring C(=O), OC(=O), or $CH_2$, and each wavy line represents bonding to N297 glycan or remodeled N297 glycan.

**4.** The antibody-drug conjugate according to claim 3, wherein
L represents any one selected from the following group:

- $Z^1C(=O)-CH_2CH_2-C(=O)-GGVA-NH-B-CH_2-OC(=O)-$,
- $Z^1C(=O)-CH_2CH_2-C(=O)-VA-NH-B-CH_2-OC(=O)-$,

- $Z^1C(=O)-(CH_2CH_2)_2-C(=O)-VA-NH-B-CH_2-OC(=O)-$,
- $Z^1-C(=O)-CH_2CH_2-C(=O)-GGVCit-NH-B-CH_2-OC(=O)-$,
- $Z^1C(=O)-CH_2CH_2-C(=O)-NH-(CH_2CH_2)_2-C(=O)-VA-NH-B-CH_2-OC(=O)-$,
- $Z^1C(=O)-CH_2CH_2-C(=O)-NH-(CH_2CH_2O)_2-CH_2-C(=O)-VA-NH-B-CH_2-OC(=O)-$, and
- $Z^1C(=O)-CH_2CH_2-NH-C(=O)-(CH_2CH_2O)_4-CH_2CH_2-C(=O)-VA-NH-B-CH_2-OC(=O)-$, wherein

B is a 1,4-phenyl group,
$Z^1$ represents the following structural formula:

[Formula 9]

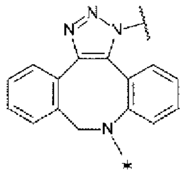

or

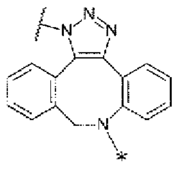

wherein, in the structural formula for $Z^1$, each asterisk * represents bonding to C(=O) neighboring to $Z^1$, and each wavy line represents bonding to N297 glycan or remodeled N297 glycan.

**5.** The antibody-drug conjugate according to any one of claims 1 to 4, wherein the N297 glycan is a remodeled glycan.

**6.** The antibody-drug conjugate according to any one of claims 1 to 5, wherein the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 10]

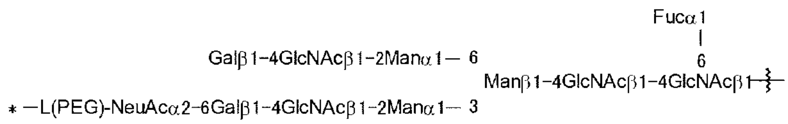

[N297-(Fuc)MSG1]

[Formula 11]

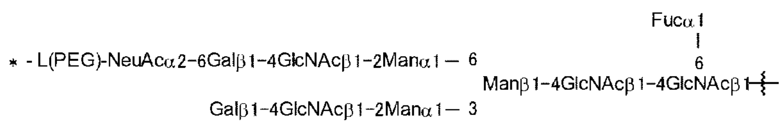

[N297-(Fuc)MSG2]

[Formula 12]

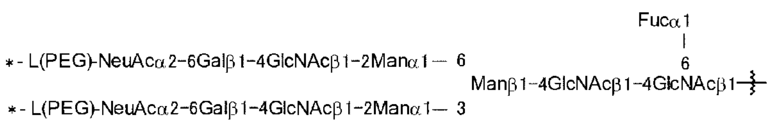

[N297-(Fuc)SG]

wherein

each wavy line represents bonding to Asn297 of the antibody,
L(PEG) in the N297 glycan represents $*-(CH_2CH_2-O)n^5-CH_2CH_2-NH-$, wherein $n^5$ represents an integer of 2 to 5, the amino group at the right end is bound via an amide bond to carboxylic acid at the 2-position of a sialic

acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring of $Z^1$ in L.

**7.** The antibody-drug conjugate according to claim 6, wherein $n^5$ is 3.

**8.** An antibody-drug conjugate represented by the following formula:

[Formula 13]

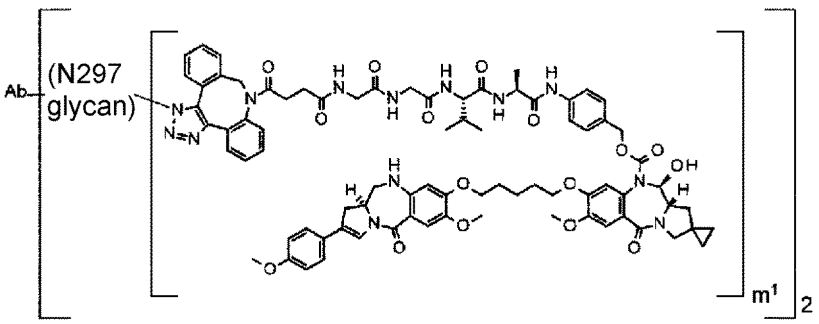

or

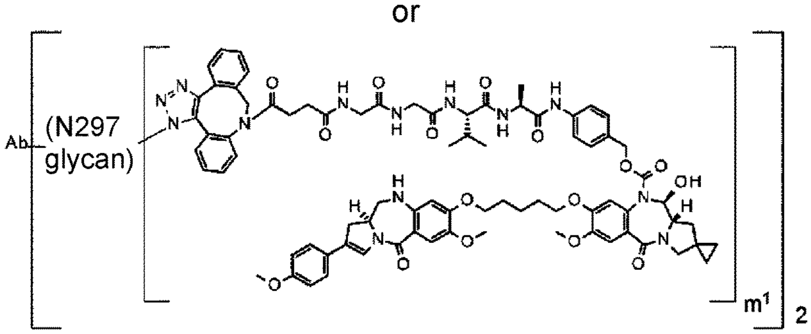

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 14]

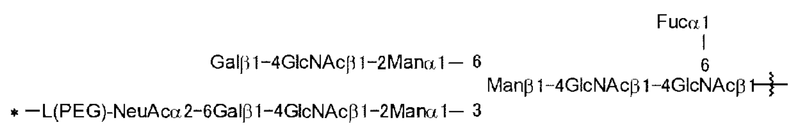

[N297-(Fuc)MSG1]

[Formula 15]

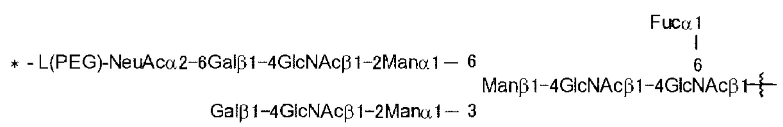

[N297-(Fuc)MSG2]

[Formula 16]

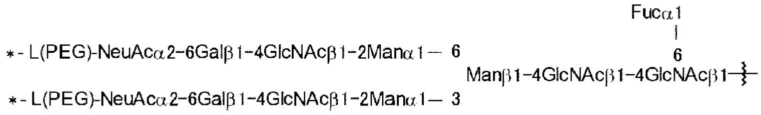

[N297-(Fuc)SG]

wherein

each wavy line represents bonding to Asn297 of the antibody,
L(PEG) in the N297 glycan represents *-$(CH_2CH_2\text{-}O)_3\text{-}CH_2CH_2\text{-}NH$-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

**9.** An antibody-drug conjugate represented by the following formula:

[Formula 17]

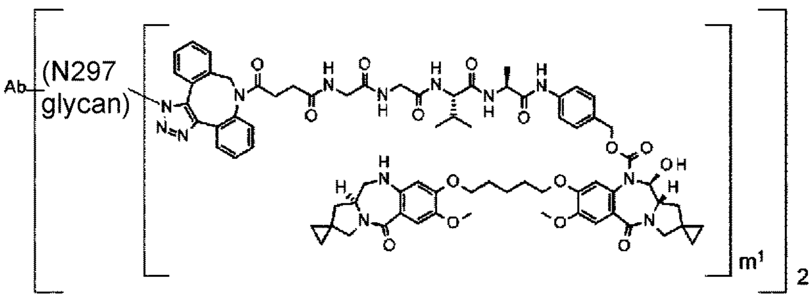

or

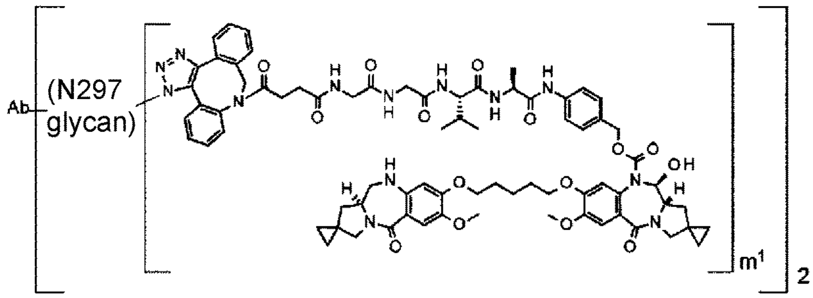

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 18]

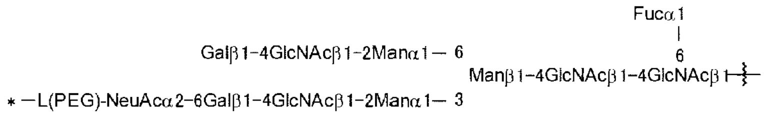

[N297-(Fuc)MSG1]

[Formula 19]

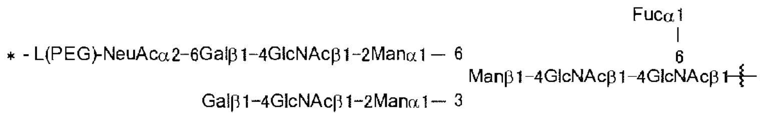

[N297-(Fuc)MSG2]

[Formula 20]

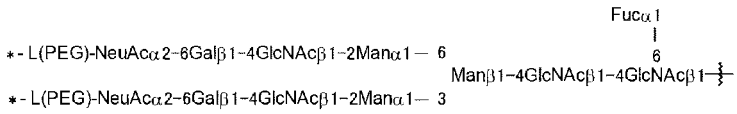

[N297-(Fuc)SG]

wherein

each wavy line represents bonding to Asn297 of the antibody,
L(PEG) in the N297 glycan represents *-$(CH_2CH_2\text{-}O)_3\text{-}CH_2CH_2$-NH-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

**10.** An antibody-drug conjugate represented by the following formula:

[Formula 21]

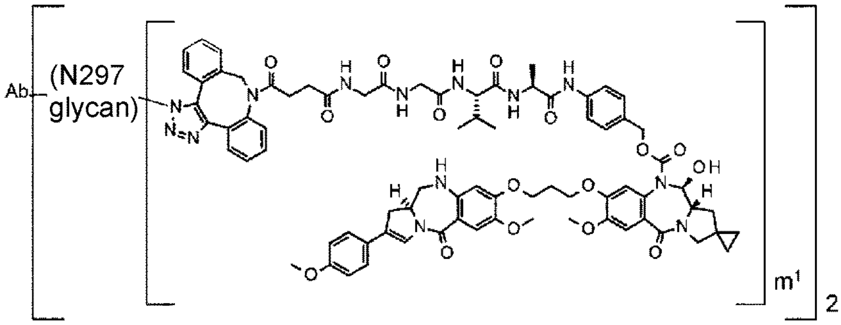

or

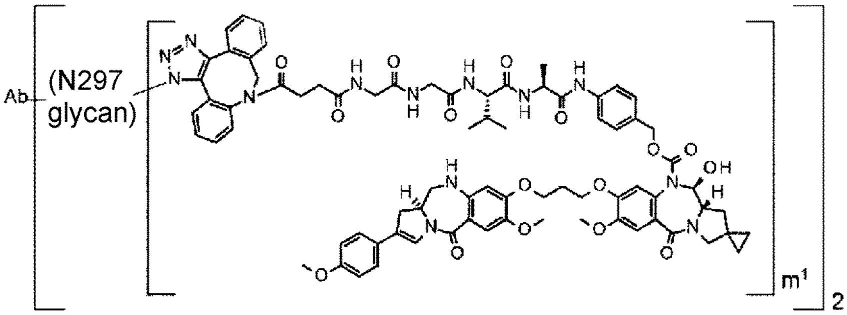

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 22]

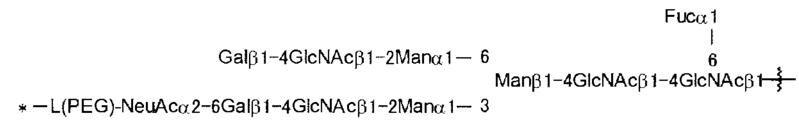

[N297-(Fuc)MSG1]

[Formula 23]

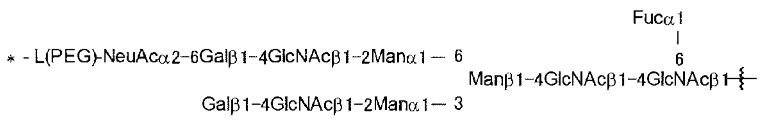

[N297-(Fuc)MSG2]

[Formula 24]

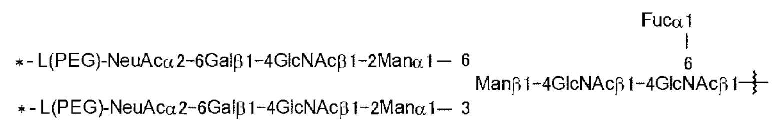

[N297-(Fuc)SG]

wherein

each wavy line represents bonding to Asn297 of the antibody,
L(PEG) in the N297 glycan represents *-$(CH_2CH_2\text{-}O)_3\text{-}CH_2CH_2\text{-}NH$-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

**11.** An antibody-drug conjugate represented by the following formula:

[Formula 25]

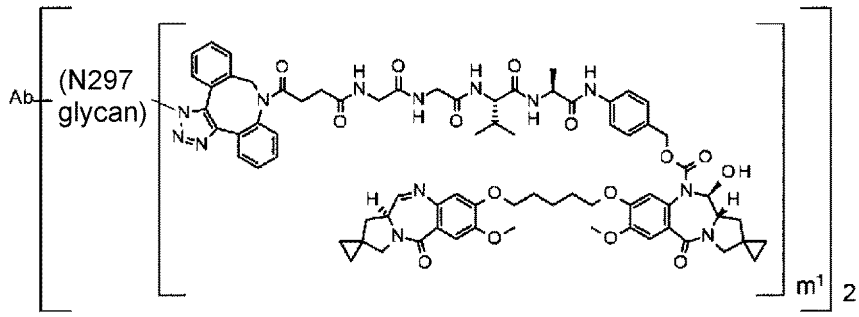

or

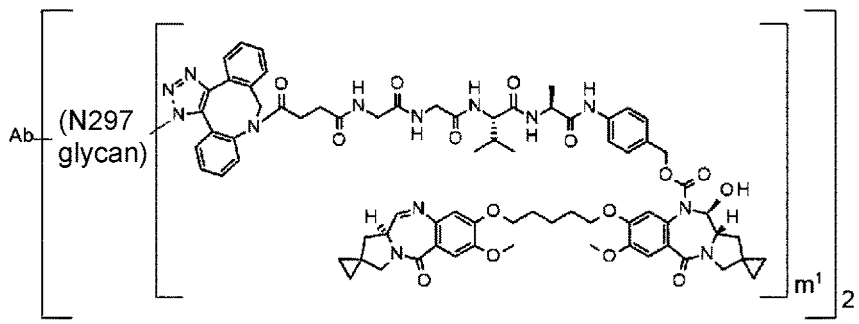

wherein, in each structural formula shown above,

$m^1$ represents an integer of 1 or 2;
Ab is an antibody or a functional fragment of the antibody;
the N297 glycan is any one of N297-(Fuc)MSG1, N297-(Fuc)MSG2, and a mixture thereof, and N297-(Fuc)SG, with N297-(Fuc)MSG1, N297-(Fuc)MSG2, and N297-(Fuc)SG having structures represented by the following formulas:

[Formula 26]

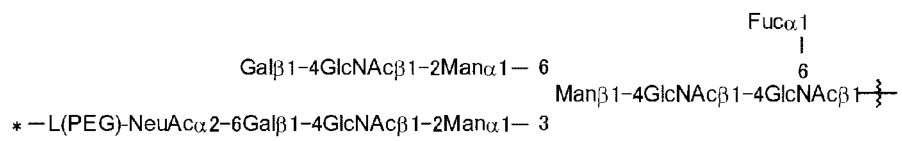

[N297-(Fuc)MSG1]

[Formula 27]

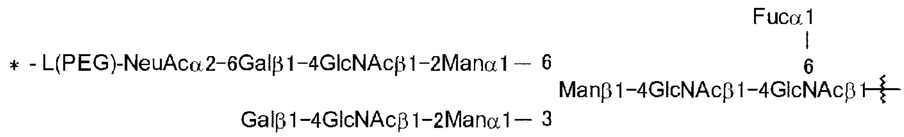

[N297-(Fuc)MSG2]

[Formula 28]

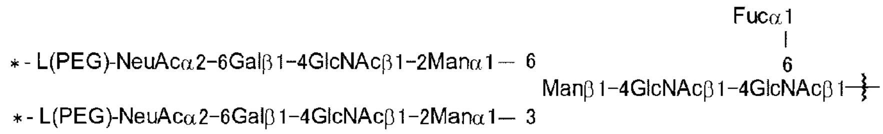

[N297-(Fuc)SG]

wherein

each wavy line represents bonding to Asn297 of the antibody,
L(PEG) in the N297 glycan represents *-$(CH_2CH_2\text{-}O)_3$-$CH_2CH_2$-NH-,
wherein the amino group at the right end is bound via an amide bond to carboxylic acid at the 2-position of a sialic acid at the non-reducing terminal in each or either one of the 1-3 and 1-6 branched chains of β-Man in the N297 glycan, and the asterisk * at the left end represents bonding to a nitrogen atom at the 1- or 3-position of the triazole ring in the corresponding structural formula.

**12.** The antibody-drug conjugate according to any one of claims 1 to 11, comprising an antibody having internalization activity.

**13.** The antibody-drug conjugate according to any one of claims 1 to 12, being an antibody having a characteristic of binding to an antigen expressed on a tumor cell and being incorporated and internalizing in the tumor cell.

**14.** The antibody-drug conjugate according to any one of claims 1 to 13, wherein the antibody has antitumor effect.

**15.** The antibody-drug conjugate according to any one of claims 1 to 14, wherein the antibody is an anti-CLDN6 antibody, an anti-CLDN9 antibody, an anti-CLDN6/CLDN9 antibody, an anti-HER2 antibody, an anti-HER3 antibody, an anti-DLL3 antibody, an anti-FAP antibody, an anti-CDH11 antibody, an anti-A33 antibody, an anti-CanAg antibody, an anti-CD 19 antibody, an anti-CD20 antibody, an anti-CD22 antibody, an anti-CD25 antibody, an anti-CD30 antibody, an anti-CD33 antibody, an anti-CD37 antibody, an anti-CD56 antibody, an anti-CD70 antibody, an anti-CD98 antibody, an anti-B7-H3 antibody, an anti-TROP2 antibody, an anti-CEA antibody, an anti-Cripto antibody, an anti-EphA2 antibody, an anti-FGFR2 antibody, an anti-G250 antibody, an anti-MUCl antibody, an anti-GPNMB antibody, an anti-Integrin antibody, an anti-PSMA antibody, an anti-Tenascin-C antibody, an anti-SLC44A4 antibody, an anti-Mesothelin antibody, an anti-EGFR antibody, an anti-5T4 antibody, an anti-LRRC15 antibody, an anti-DR5 antibody, an anti-CDH3 antibody, an anti-PDPN antibody, or an anti-CD123 antibody.

**16.** The antibody-drug conjugate according to any one of claims 1 to 15, wherein the antibody specifically binds to CLDN6 and/or CLDN9.

**17.** The antibody-drug conjugate according to claim 16, the antibody comprising a heavy chain comprising CDRH1, CDRH2, and CDRH3 and a light chain comprising CDRL1, CDRL2, and CDRL3 as described in any one of the following (a) and (b):

(a) CDRH1 consisting of an amino acid sequence represented by SEQ ID NO: 9, CDRH2 consisting of an amino acid sequence represented by SEQ ID NO: 10, and CDRH3 consisting of an amino acid sequence represented by SEQ ID NO: 11, and CDRL1 consisting of an amino acid sequence represented by SEQ ID NO: 5, CDRL2 consisting of an amino acid sequence represented by SEQ ID NO: 6, and CDRL3 consisting of an amino acid sequence represented by SEQ ID NO: 7 or an amino acid sequence having one or two amino acid substitutions

in the amino acid sequence represented by SEQ ID NO: 7; and
(b) CDRH1 consisting of an amino acid sequence represented by SEQ ID NO: 15, CDRH2 consisting of an amino acid sequence represented by SEQ ID NO: 16, and CDRH3 consisting of an amino acid sequence represented by SEQ ID NO: 17, and CDRL1 consisting of an amino acid sequence represented by SEQ ID NO: 12, CDRL2 consisting of an amino acid sequence represented by SEQ ID NO: 13, and CDRL3 consisting of an amino acid sequence represented by SEQ ID NO: 14.

**18.** The antibody-drug conjugate according to claim 17, the antibody comprising a heavy chain comprising CDRH1, CDRH2, and CDRH3 and a light chain comprising CDRL1, CDRL2, and CDRL3 as described in any one of the following (a) and (b):

(a) CDRH1 consisting of an amino acid sequence represented by SEQ ID NO: 9, CDRH2 consisting of an amino acid sequence represented by SEQ ID NO: 10, and CDRH3 consisting of an amino acid sequence represented by SEQ ID NO: 11, and CDRL1 consisting of an amino acid sequence represented by SEQ ID NO: 5, CDRL2 consisting of an amino acid sequence represented by SEQ ID NO: 6, and CDRL3 consisting of an amino acid sequence represented by SEQ ID NO: 7 or an amino acid sequence represented by SEQ ID NO: 8; and
(b) CDRH1 consisting of an amino acid sequence represented by SEQ ID NO: 15, CDRH2 consisting of an amino acid sequence represented by SEQ ID NO: 16, and CDRH3 consisting of an amino acid sequence represented by SEQ ID NO: 17, and CDRL1 consisting of an amino acid sequence represented by SEQ ID NO: 12, CDRL2 consisting of an amino acid sequence represented by SEQ ID NO: 13, and CDRL3 consisting of an amino acid sequence represented by SEQ ID NO: 14.

**19.** The antibody-drug conjugate according to any one of claims 16 to 18, the antibody comprising a heavy chain variable region and a light chain variable region as described in any one of the following (a) and (b):

(a) a heavy chain variable region consisting of an amino acid sequence represented by SEQ ID NO: 21 and a light chain variable region consisting of an amino acid sequence represented by SEQ ID NO: 19; and
(b) a heavy chain variable region consisting of an amino acid sequence represented by SEQ ID NO: 25 and a light chain variable region consisting of an amino acid sequence represented by SEQ ID NO: 23.

**20.** The antibody-drug conjugate according to any one of claims 16 to 19, comprising a heavy chain variable region consisting of an amino acid sequence selected from the group consisting of the following (a) to (e) and a light chain variable region consisting of an amino acid sequence selected from the group consisting of the following (f) to (k):

(a) an amino acid sequence represented by SEQ ID NO: 54;
(b) an amino acid sequence represented by SEQ ID NO: 58;
(c) an amino acid sequence represented by SEQ ID NO: 62;
(d) an amino acid sequence with a homology of at least 95% or higher to a sequence of a framework region excluding CDR sequences in any of the sequences (a) to (c);
(e) an amino acid sequence having one to several amino acid deletions, substitutions, or additions in a sequence of a framework region excluding CDR sequences in any of the sequences (a) to (c);
(f) an amino acid sequence represented by SEQ ID NO: 38;
(g) an amino acid sequence represented by SEQ ID NO: 42;
(h) an amino acid sequence represented by SEQ ID NO: 46;
(i) an amino acid sequence represented by SEQ ID NO: 50;
(j) an amino acid sequence with a homology of at least 95% or higher to a sequence of a framework region excluding CDR sequences in any of the sequences (f) to (i); and
(k) an amino acid sequence having one to several amino acid deletions, substitutions, or additions in a sequence of a framework region excluding CDR sequences in any of the sequences (f) to (i).

**21.** The antibody-drug conjugate according to claim 20, the antibody comprising a heavy chain variable region and a light chain variable region selected from the group consisting of the following (a) to (e):

(a) a heavy chain variable region consisting of an amino acid sequence represented by SEQ ID NO: 54 and a light chain variable region consisting of an amino acid sequence represented by SEQ ID NO: 38;
(b) a heavy chain variable region consisting of an amino acid sequence represented by SEQ ID NO: 58 and a light chain variable region consisting of an amino acid sequence represented by SEQ ID NO: 42;
(c) a heavy chain variable region consisting of an amino acid sequence represented by SEQ ID NO: 54 and a

light chain variable region consisting of an amino acid sequence represented by SEQ ID NO: 46;
(d) a heavy chain variable region consisting of an amino acid sequence represented by SEQ ID NO: 58 and a light chain variable region consisting of a light chain variable region consisting of an amino acid sequence represented by SEQ ID NO: 50; and
(e) a heavy chain variable region consisting of an amino acid sequence represented by SEQ ID NO: 62 and a light chain variable region consisting of an amino acid sequence represented by SEQ ID NO: 46.

**22.** The antibody-drug conjugate according to any one of claims 16 to 21, wherein the antibody is a chimeric antibody.

**23.** The antibody-drug conjugate according to any one of claims 16 to 21, wherein the antibody is a humanized antibody.

**24.** The antibody-drug conjugate according to any one of claims 16 to 23, wherein the antibody comprises a heavy chain constant region of human IgG1, human IgG2, or human IgG4.

**25.** The antibody-drug conjugate according to claim 23 or 24, comprising a heavy chain and a light chain selected from the group consisting of the following (a) to (e):

(a) a heavy chain consisting of an amino acid sequence consisting of amino acid residues 20 to 471 of SEQ ID NO: 52 and a light chain consisting of an amino acid sequence consisting of amino acid residues 21 to 234 of SEQ ID NO: 36;
(b) a heavy chain consisting of an amino acid sequence consisting of amino acid residues 20 to 471 of SEQ ID NO: 56 and a light chain consisting of an amino acid sequence consisting of amino acid residues 21 to 234 of SEQ ID NO: 40;
(c) a heavy chain consisting of an amino acid sequence consisting of amino acid residues 20 to 471 of SEQ ID NO: 52 and a light chain consisting of an amino acid sequence consisting of amino acid residues 21 to 234 of SEQ ID NO: 44;
(d) a heavy chain consisting of an amino acid sequence consisting of amino acid residues 20 to 471 of SEQ ID NO: 56 and a light chain consisting of an amino acid sequence consisting of amino acid residues 21 to 234 of SEQ ID NO: 48; and
(e) a heavy chain consisting of an amino acid sequence consisting of amino acid residues 20 to 471 of SEQ ID NO: 60 and a light chain consisting of an amino acid sequence consisting of amino acid residues 21 to 234 of SEQ ID NO: 44.

**26.** The antibody-drug conjugate according to claim 16, wherein the antibody competes with the antibody according to any one of claims 17 to 21 and 25 for binding to CLDN6 and/or CLDN9, or binds to a site of CLDN6 and/or CLDN9 recognizable to the antibody according to any one of claims 17 to 21 and 25.

**27.** The antibody-drug conjugate according to any one of claims 1 to 15, wherein the antibody specifically binds to HER2.

**28.** The antibody-drug conjugate according to claim 27, having activities or activity of antibody-dependent cellular cytotoxicity (ADCC) and/or complement-dependent cytotoxicity (CDC).

**29.** The antibody-drug conjugate according to claim 27, wherein the heavy chain constant region of the antibody is a heavy chain constant region of human IgG1, and comprises a mutation that causes lowering of activities or activity of ADCC and/or CDC.

**30.** The antibody-drug conjugate according to claim 29, wherein the heavy chain constant region of the antibody is a heavy chain constant region of human IgG1, and leucine at the 234- and 235-positions specified by EU Index numbering in the heavy chain constant region is substituted with alanine.

**31.** The antibody-drug conjugate according to claim 27 or 28, being an antibody comprising a heavy chain consisting of an amino acid sequence represented by SEQ ID NO: 65 and a light chain consisting of an amino acid sequence represented by SEQ ID NO: 64.

**32.** The antibody-drug conjugate according to any one of claims 27, 29, and 30, being an antibody comprising a heavy chain variable region consisting of an amino acid sequence consisting of amino acid residues 20 to 139 of SEQ ID NO: 75 and a light chain variable region consisting of an amino acid sequence consisting of amino acid residues 21 to 127 of SEQ ID NO: 73.

**33.** The antibody-drug conjugate according to any one of claims 27, 29, 30, and 32, being an antibody comprising a heavy chain consisting of an amino acid sequence consisting of amino acid residues 20 to 469 of SEQ ID NO: 75 and a light chain consisting of an amino acid sequence consisting of amino acid residues 21 to 234 of SEQ ID NO: 73.

**34.** The antibody-drug conjugate according to any one of claims 16 to 33, wherein the antibody comprises one or two or more modifications selected from the group consisting of N-linked glycosylation, O-linked glycosylation, N-terminal processing, C-terminal processing, deamidation, isomerization of aspartic acid, oxidation of methionine, addition of a methionine residue at an N terminus, amidation of a proline residue, and deletion of one or two amino acid residues at the carboxyl terminus of a heavy chain.

**35.** The antibody-drug conjugate according to claim 34, wherein one or several amino acid residues are deleted at the carboxyl terminus of a heavy chain of the antibody.

**36.** The antibody-drug conjugate according to claim 34 or 35, wherein one amino acid residue is deleted at the carboxyl terminus of each of the two heavy chains of the antibody.

**37.** The antibody-drug conjugate according to any one of claims 34 to 36, wherein a proline residue at the carboxyl terminus of a heavy chain of the antibody is further amidated.

**38.** The antibody-drug conjugate according to any one of claims 1 to 37, wherein the antibody is a glycan-remodeled antibody obtained through the following steps of:

i) culturing a host cell containing a polynucleotide encoding the antibody according to any one of claims 12 to 37 and collecting a targeted antibody from the culture obtained;
ii) treating the antibody obtained in step i) with hydrolase to produce a (Fucα1,6)GlcNAc-antibody; and
iii) reacting the (Fucα1,6)GlcNAc-antibody with a glycan donor molecule in the presence of transglycosidase, the glycan donor molecule obtained by introducing a PEG linker having an azide group to the carbonyl group of carboxylic acid at the 2-position of a sialic acid in MSG (9) or SG (10) and oxazolinating the reducing terminal.

**39.** The antibody-drug conjugate according to claim 38, further comprising the step of purifying the (Fucα1,6)GlcNAc-antibody through purification of a reaction solution in step ii) with a hydroxyapatite column.

**40.** A compound, a salt of the compound, or a hydrate of the compound or the salt, wherein the compound is any one represented by the following formula:

[Formula 29]

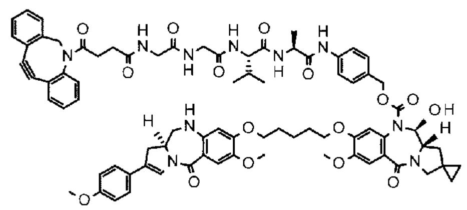

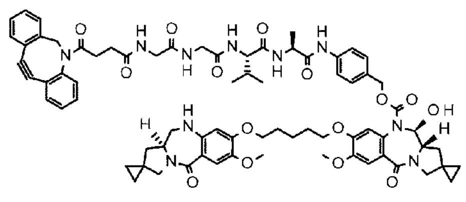

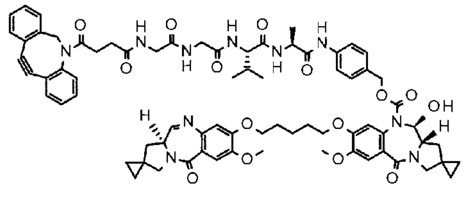

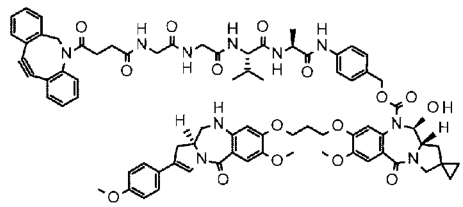

**41.** A method for producing the antibody-drug conjugate according to any one of claims 1 to 39, the method comprising the step of reacting the glycan-remodeled antibody according to claim 38 or 39 and the compound according to claim 40, a salt of the compound, or a hydrate of the compound or the salt.

**42.** An antibody-drug conjugate obtained by using the method according to claim 41.

**43.** The antibody-drug conjugate according to any one of claims 1 to 39 and 42, wherein the N297 glycan is N297-(Fuc)MSG1.

**44.** The antibody-drug conjugate according to any one of claims 1 to 39, 42, and 43, wherein $m^1$ is an integer of 1.

**45.** The antibody-drug conjugate according to any one of claims 1 to 39 and 42 to 44, wherein the average number of conjugated drug molecules per antibody molecule in the antibody-drug conjugate is 1 to 3 or 3 to 5.

**46.** A pharmaceutical composition comprising the antibody-drug conjugate according to any one of claims 1 to 39 and 42 to 45, a salt of the antibody-drug conjugate, or a hydrate of the antibody-drug conjugate or the salt.

**47.** The pharmaceutical composition according to claim 46, being an antitumor drug.

**48.** A method for treating a tumor, wherein the antibody-drug conjugate according to any one of claims 1 to 39 and 42 to 45, a salt of the antibody-drug conjugate, or a hydrate of the antibody-drug conjugate or the salt is administered to an individual.

**49.** A method for treating a tumor, wherein a pharmaceutical composition comprising the antibody-drug conjugate according to any one of claims 1 to 39 and 42 to 45, a salt of the antibody-drug conjugate, or a hydrate of the antibody-drug conjugate or the salt, and at least one antitumor drug are administered to an individual simultaneously, separately, or consecutively.

[Figure 1]

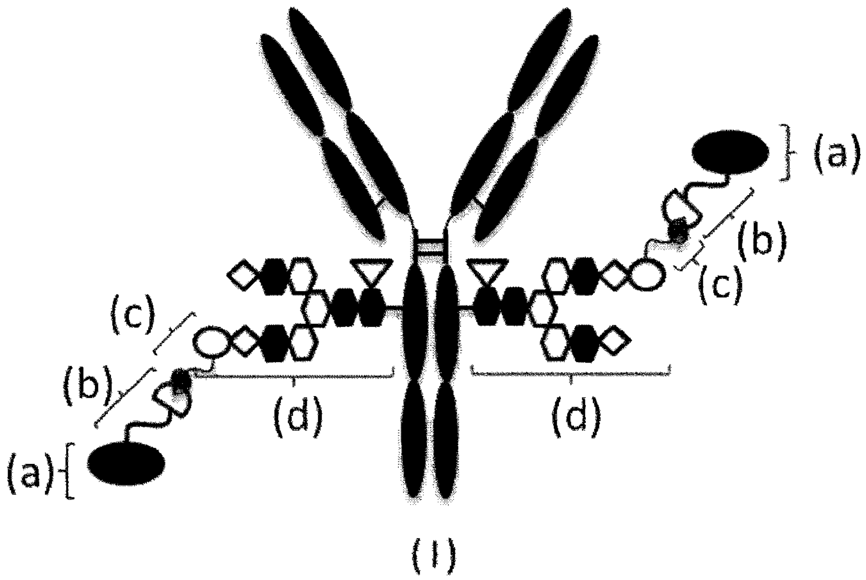


[Figure 2]

A

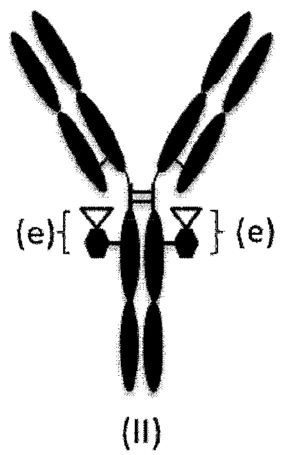


B

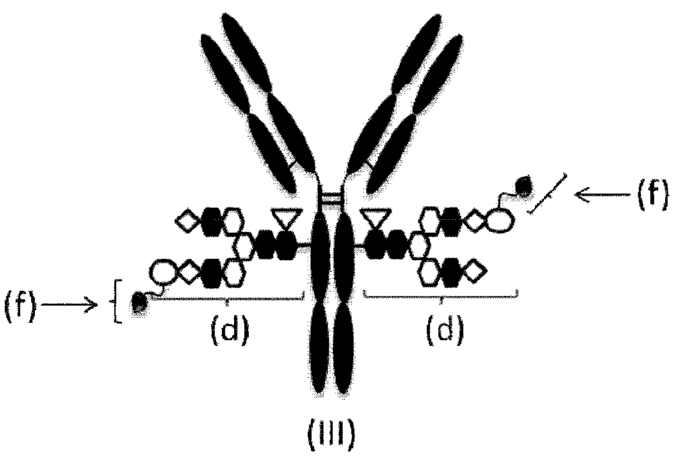

[Figure 3]

A

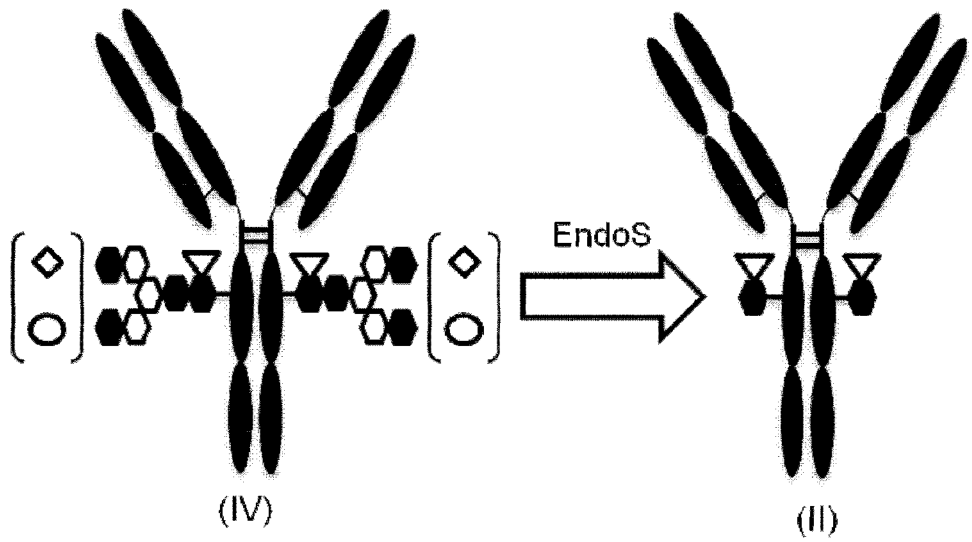


B

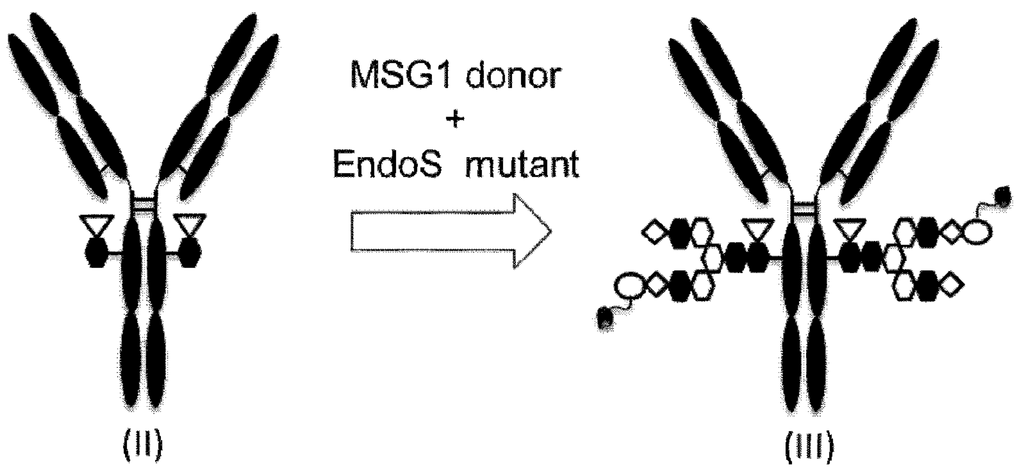

[Figure 4]

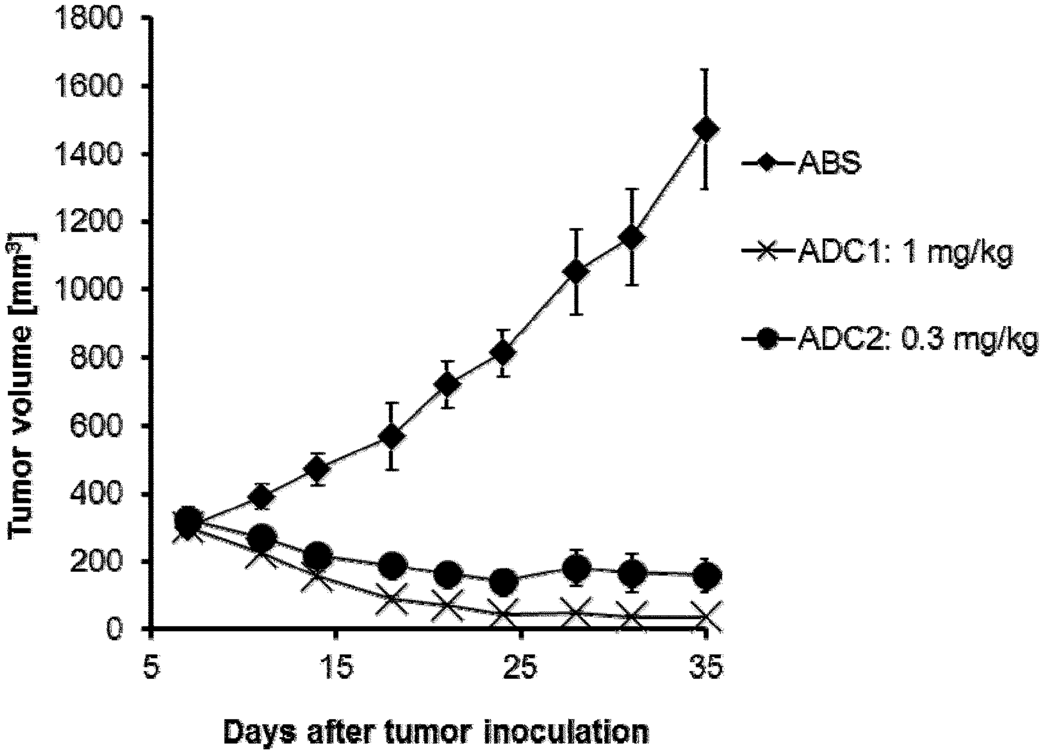


[Figure 5]

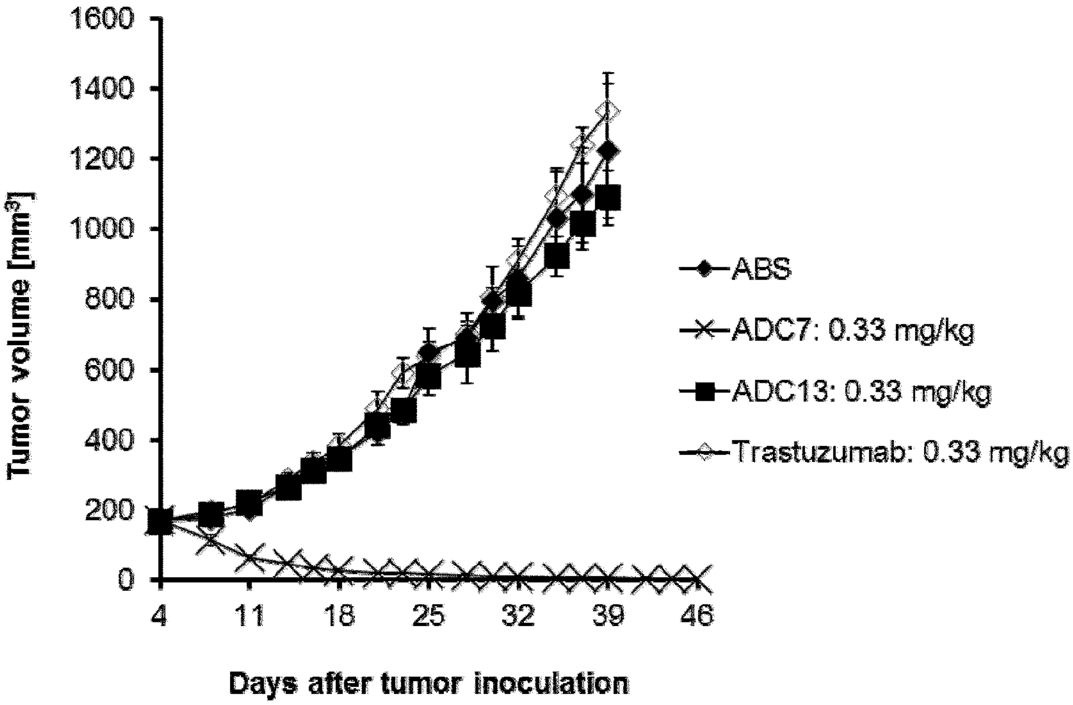

[Figure 6]

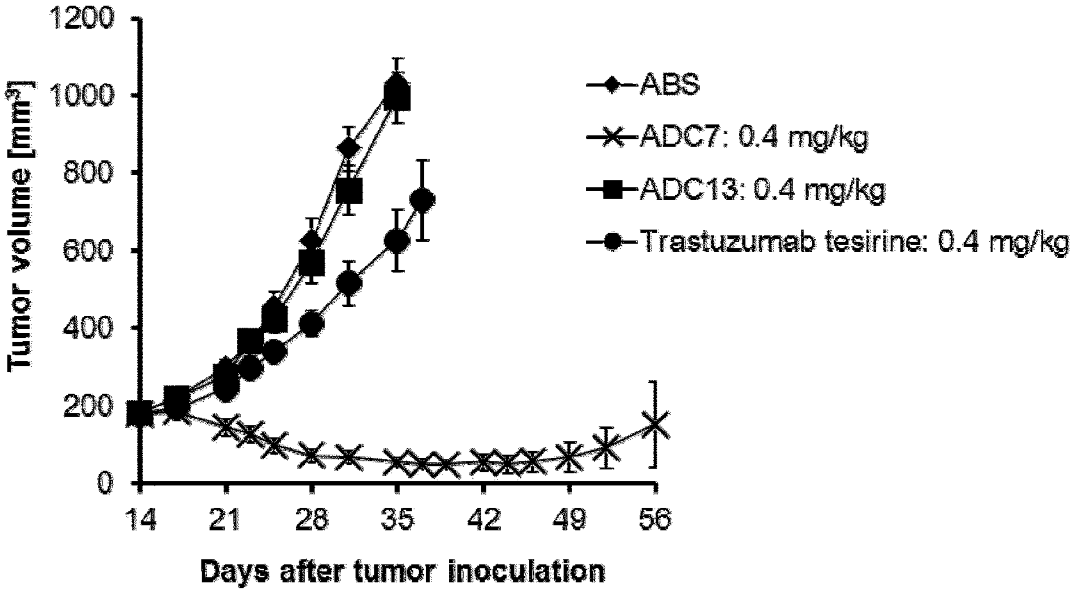


[Figure 7]

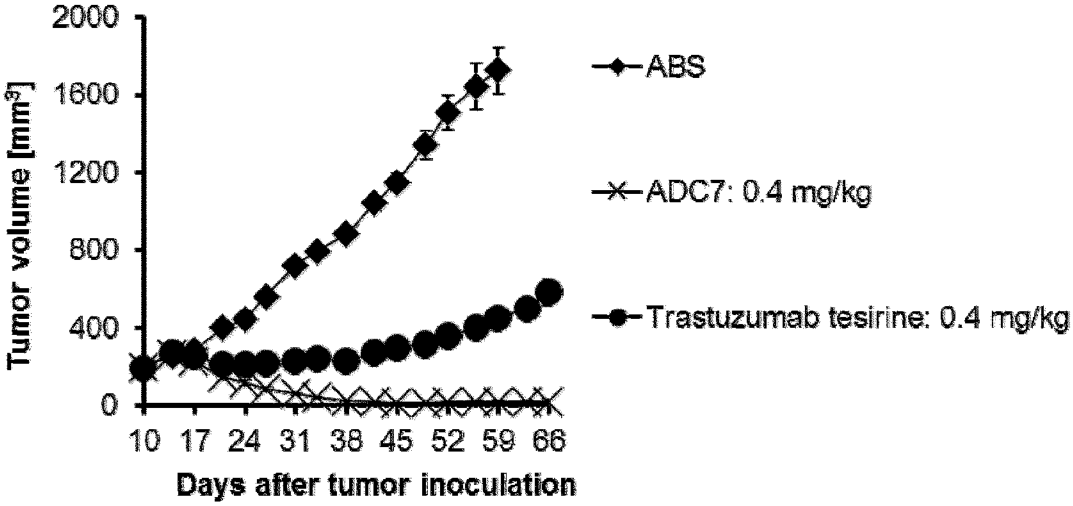

[Figure 8]

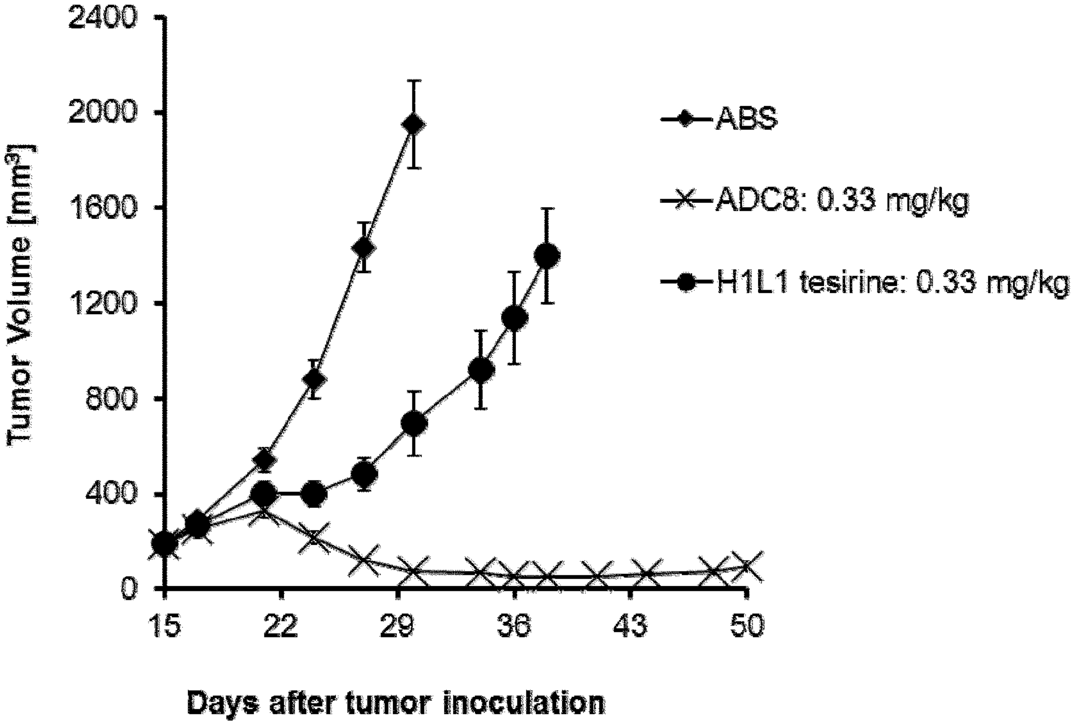


[Figure 9]

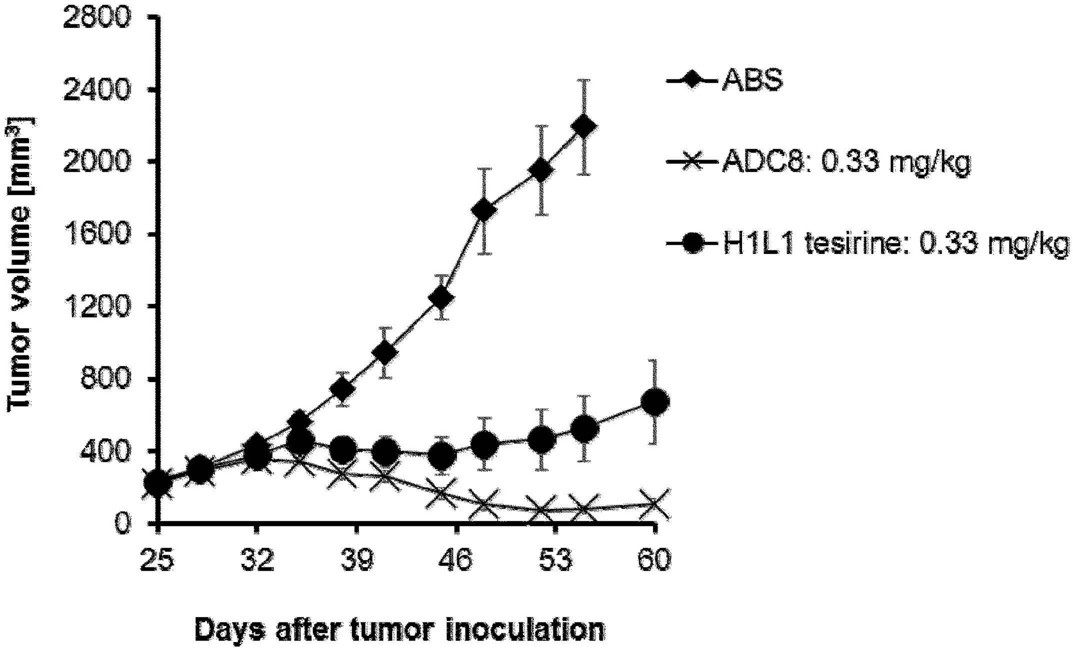

[Figure 10]

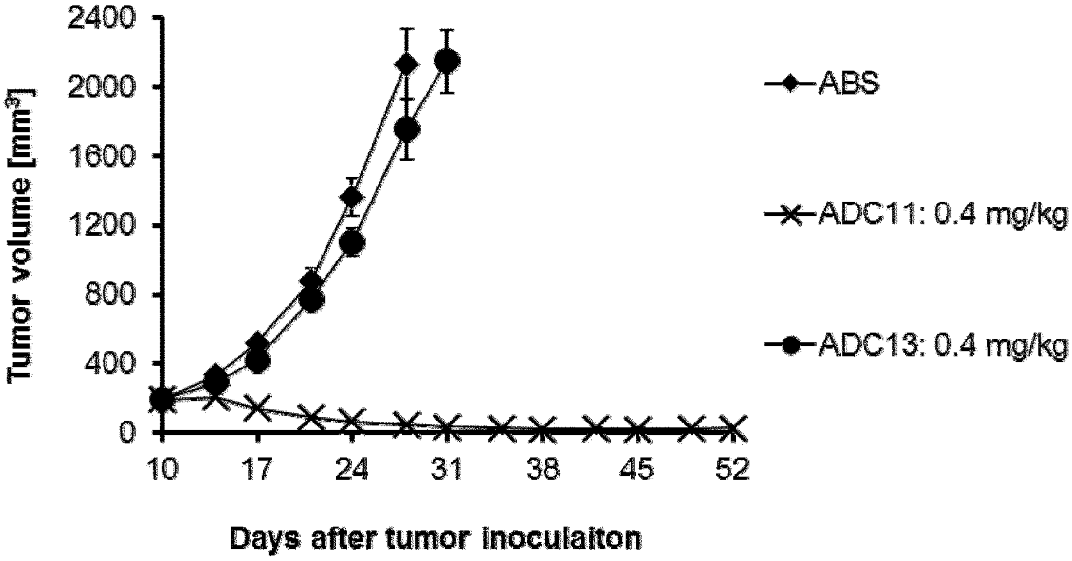


[Figure 11]

**Full-length amino acid sequence of human CLDN6 (SEQ ID NO: 1)**

MASAGMQILGVVLTLLGWVNGLVSCALPMWKVTAFIGNSIVVAQVVWEGLWMSCVVQSTGQMQCKVYDSL
LALPQDLQAARALCVIALLVALFGLLVYLAGAKCTTCVEEKDSKARLVLTSGIVFVISGVLTLIPVCWTA
HAIIRDFYNPLVAEAQKRELGASLYLGWAASGLLLLGGGLLCCTCPSGGSQGPSHYMARYSTSAPAISRG
PSEYPTKNYV

**Nucleotide sequence of full-length cDNA for human CLDN6 (SEQ ID NO: 2)**

atggcctctgccggaatgcagatcctgggagtcgtcctgacactgctgggctgggtgaatggcctggtct
cctgtgccctgcccatgtggaaggtgaccgctttcatcggcaacagcatcgtggtggcccaggtggtgtg
ggagggcctgtggatgtcctgcgtggtgcagagcaccggccagatgcagtgcaaggtgtacgactcactg
ctggcgctgccacaggacctgcaggctgcacgtgccctctgtgtcatcgccctccttgtggccctgttcg
gcttgctggtctaccttgctggggccaagtgtaccacctgtgtggaggagaaggattccaaggcccgcct
ggtgctcacctctgggattgtctttgtcatctcaggggtcctgacgctaatccccgtgtgctggacggcg
catgccatcatccgggacttctataacccccctggtggctgaggcccaaaagcgggagctgggggcctccc
tctacttgggctgggcggcctcaggccttttgttgctgggtggggggttgctgtgctgcacttgcccctc
gggggggtcccagggccccagccattacatggcccgctactcaacatctgcccctgccatctctcggggg
ccctctgagtaccctaccaagaattacgtctga

[Figure 12]

**Full-length amino acid sequence of human CLDN9 (SEQ ID NO: 3)**

MASTGLELLGMTLAVLGWLGTLVSCALPLWKVTAFIGNSIVVAQVVWEGLWMSCVVQSTGQMQCKVY
DSLLALPQDLQAARALCVIALLLALLGLLVAITGAQCTTCVEDEGAKARIVLTAGVILLLAGILVLI
PVCWTAHAIIQDFYNPLVAEALKRELGASLYLGWAAAALLMLGGGLLCCTCPPPQVERPRGPRLGYS
IPSRSGASGLDKRDYV

**Nucleotide sequence of full-length cDNA for human CLDN9 (SEQ ID NO: 4)**

atggcttcgaccggcttagaactgctgggcatgaccctggctgtgctgggctggctggggaccctgg
tgtcctgcgccctgcccctgtggaaggtgaccgccttcatcggcaacagcatcgtggtggcccaggt
ggtgtgggagggcctgtggatgtcctgcgtggtgcagagcacgggccagatgcagtgcaaggtgtac
gactcactgctggctctgccgcaggacctgcaggccgcacgtgccctctgtgtcattgccctcctgc
tggccctgcttggcctcctggtggccatcacaggtgcccagtgtaccacgtgtgtggaggacgaagg
tgccaaggcccgtatcgtgctcaccgcgggggtcatcctcctcctcgccggcatcctggtgctcatc
cctgtgtgctggacggcgcacgccatcatccaggacttctacaaccccctggtggctgaggccctca
agcgggagctgggggcctccctctacctgggctgggcggcggctgcactgcttatgctgggcggggg
gctcctctgctgcacgtgcccccccgccccaggtcgagcggccccgcggacctcggctgggctactcc
atcccctcccgctcgggtgcatctggactggacaagagggactacgtgtga

[Figure 13]

**Amino acid sequence of CDRL1 of B1 antibody light chain (SEQ ID NO: 5)**

RASQDINNYLN

**Amino acid sequence of CDRL2 of B1 antibody light chain (SEQ ID NO: 6)**

FTSRLHS

**Amino acid sequence of CDRL3 of B1 antibody light chain (SEQ ID NO: 7)**

QQGYPLPWT

[Figure 14]

**Amino acid sequence of CDRL3 of humanized B1 antibody light chain L4 (SEQ ID NO: 8)**

QQGNTLPWT

[Figure 15]

**Amino acid sequence of CDRH1 of B1 antibody heavy chain (SEQ ID NO: 9)**

GYTFTEYTMH

**Amino acid sequence of CDRH2 of B1 antibody heavy chain (SEQ ID NO: 10)**

GVNPNSGDTS

**Amino acid sequence of CDRH3 of B1 antibody heavy chain (SEQ ID NO: 11)**

PGGYDVGYYAMDY

[Figure 16]

**Amino acid sequence of CDRL1 of C7 antibody light chain (SEQ ID NO: 12)**

RASQDINNYLN

**Amino acid sequence of CDRL2 of C7 antibody light chain (SEQ ID NO: 13)**

STSRLHS

**Amino acid sequence of CDRL3 of C7 antibody light chain (SEQ ID NO: 14)**

QQGYPLPWT

[Figure 17]

**Amino acid sequence of CDRH1 of C7 antibody heavy chain (SEQ ID NO: 15)**

GYTFTEYTMH

**Amino acid sequence of CDRH2 of C7 antibody heavy chain (SEQ ID NO: 16)**

GVNPNSGDTS

**Amino acid sequence of CDRH3 of C7 antibody heavy chain (SEQ ID NO: 17)**

PGGYDVGYYAMDY

[Figure 18]

**Nucleotide sequence of cDNA encoding variable region of B1 antibody light chain (SEQ ID NO: 18)**

GATATCCAGATGACACAGACTGCATCCTCCCTGTCTGCCTCTCTTGGAGACAGAGTCACCATCAGTT
GCAGGGCAAGTCAGGACATTAACAATTATTTAAACTGGTATCAGCAGAAACCAGATGGAACTGTTAA
ACTCCTGATCTACTTCACATCAAGATTACACTCAGGAGTCCCATCAAGGTTCAGTGGCAGTGGGTCT
GGAACACATTATTCTCTCACCATTACTAACCTGGAACAAGAAGATATTGCCACTTACTTTTGCCAAC
AGGGTTATCCGCTTCCGTGGACGTTCGGTGGAGGCACCAAACTGGAAATCAAA

**Amino acid sequence of variable region of B1 antibody light chain (SEQ ID NO: 19)**

DIQMTQTASSLSASLGDRVTISCRASQDINNYLNWYQQKPDGTVKLLIYFTSRLHSGVPSRFSGSGS
GTHYSLTITNLEQEDIATYFCQQGYPLPWTFGGGTKLEIK

[Figure 19]

**Nucleotide sequence of cDNA encoding variable region of B1 antibody heavy chain (SEQ ID NO: 20)**

GAGGTCCAGCTTCAACAGTCTGGACCTGAACTGGTGAAGCCTGGGGCTTCAGTGAAGATATCCTGCA
AGACTTCTGGATACACATTCACTGAATACACCATGCACTGGGTGCAGCAGAGCCATGGAAAGAGCCT
TGAGTGGATTGGAGGTGTTAATCCTAATAGTGGTGATACTAGCTACAACCAGAAGTTCAAGGGCAAG
GCCACATTGACTGTTGACAAGTCCTCCAGCACAGCCTACATGGAGCTCCGCAGCCTGACATCTGAGG
ATTCTGCAGTCTATTACTGTGCAAGACCCGGGGGGTACGACGTGGGTTACTATGCTATGGACTACTG
GGGTCAAGGAACCTCAGTCACCGTCTCCTCA

**Amino acid sequence of variable region of B1 antibody heavy chain (SEQ ID NO: 21)**

EVQLQQSGPELVKPGASVKISCKTSGYTFTEYTMHWVQQSHGKSLEWIGGVNPNSGDTSYNQKFKGK
ATLTVDKSSSTAYMELRSLTSEDSAVYYCARPGGYDVGYYAMDYWGQGTSVTVSS

[Figure 20]

**Nucleotide sequence of cDNA encoding variable region of C7 antibody light chain (SEQ ID NO: 22)**

GATATCCAGATGACACAGACTGCATCCTCCCTGTCTGCCTCTCTTGGAGACAGAGTCACCATCAGTT
GCAGGGCAAGTCAGGACATTAACAATTATTTAAACTGGTATCAGCAGAAACCAGATGGAACTGTTAA
ACTCCTGATCTACTCCACATCAAGATTACACTCAGGAGTCCCATCAAGGTTCAGTGGCAGTGGGTCT
GGAACACATTATTCTCTCACCATTACTCACCTGGAACAAGAAGATATTGCCACTTACTTTTGCCAAC
AGGGTTATCCGCTTCCGTGGACGTTCGGTGGAGGCACCAAACTGGAAATCAAA

**Amino acid sequence of variable region of C7 antibody light chain (SEQ ID NO: 23)**

DIQMTQTASSLSASLGDRVTISCRASQDINNYLNWYQQKPDGTVKLLIYSTSRLHSGVPSRFSGSGS
GTHYSLTITHLEQEDIATYFCQQGYPLPWTFGGGTKLEIK

[Figure 21]

**Nucleotide sequence of cDNA encoding variable region of C7 antibody heavy chain (SEQ ID NO: 24)**

GAGGTCCAGCTTCAACAGTCTGGACCTGAACTGGTGAAGCCTGGGGCTTCAGTGAAGATATCCTGCA
AGACTTCTGGATACACATTCACTGAATACACCATGCACTGGGTGCAGCAGAGCCATGGAAAGAGCCT
TGAGTGGATTGGAGGTGTTAATCCTAATAGTGGTGATACTAGCTACAACCAGAAGTTCAAGGGCAAG
GCCACATTGACTGTTGACAAGTCCTCCAGCACAGCCTACATGGAGCTCCGCAGCCTGACATCTGAGG
ATTCTGCAGTCTATTACTGTGCAAGACCCGGGGGGTACGACGTGGGTTACTATGCTATGGACTACTG
GGGTCAAGGAACCTCAGTCACCGTCTCCTCA

**Amino acid sequence of variable region of C7 antibody heavy chain (SEQ ID NO: 25)**

EVQLQQSGPELVKPGASVKISCKTSGYTFTEYTMHWVQQSHGKSLEWIGGVNPNSGDTSYNQKFKGK
ATLTVDKSSSTAYMELRSLTSEDSAVYYCARPGGYDVGYYAMDYWGQGTSVTVSS

[Figure 22]

**Amino acid sequence of chB1 light chain (SEQ ID NO: 28)**

MVLQTQVFISLLLWISGAYGDIQMTQTASSLSASLGDRVTISCRASQDINNYLNWYQQKPDGTVKLL
IYFTSRLHSGVPSRFSGSGSGTHYSLTITNLEQEDIATYFCQQGYPLPWTFGGGTKLEIKRAVAAPS
VFIFPPSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**Signal sequence (1 - 20), Light chain variable region (21 - 127), Light chain constant region (128 - 234)**

DNA fragment including DNA sequence encoding amino acid sequence of chB1 light chain (SEQ ID NO: 29)

ccagcctccggactctagagccaccATGGTGCTGCAGACCCAGGTGTTCATCAGCCTGCTGCTGTGG
ATCAGCGGCGCCTACGGCGACATCCAGATGACCCAGACAGCCAGCAGCCTGAGCGCCAGCCTGGGCG
ATAGAGTGACCATCAGCTGCAGAGCCAGCCAGGACATCAACAACTACCTGAACTGGTATCAGCAGAA
ACCCGACGGCACCGTGAAGCTGCTGATCTACTTCACCAGCAGACTGCACAGCGGCGTGCCCAGCAGA
TTTTCTGGCAGCGGCTCTGGCACCCACTACAGCCTGACCATCACCAACCTGGAACAGGAAGATATCG
CTACCTACTTCTGTCAGCAAGGCTACCCCCTGCCCTGGACCTTTGGCGGCGGAACAAAGCTGGAAAT
CAAGCGGGCCGTGGCCGCTCCCTCCGTGTTCATCTTTCCACCCAGCGACGAGCAGCTGAAGTCCGGC
ACAGCTAGCGTCGTGTGCCTGCTGAACAACTTCTACCCCCGCGAGGCCAAGGTGCAGTGGAAGGTGG
ACAATGCCCTGCAGAGCGGCAACAGCCAGGAAAGCGTGACCGAGCAGGACAGCAAGGACTCCACCTA
CTCCCTGAGCAGCACCCTGACCCTGAGCAAGGCCGACTACGAGAAGCACAAGGTGTACGCCTGCGAA
GTGACCCACCAGGGCCTGTCTAGCCCCGTGACCAAGAGCTTCAACCGGGGCGAGTGTtgagtttaaa
cgggggaggctaact

**Signal sequence (26 - 85), Light chain variable region (86 - 406), Light chain constant region (407 - 727)**

[Figure 23]

**Amino acid sequence of variable region of chB1 light chain (SEQ ID NO: 30)**

DIQMTQTASSLSASLGDRVTISCRASQDINNYLNWYQQKPDGTVKLLIYFTSRLHSGVPSRFSGSGS
GTHYSLTITNLEQEDIATYFCQQGYPLPWTFGGGTKLEIK

**Nucleotide sequence encoding chB1 light chain variable region (SEQ ID NO: 31)**

GACATCCAGATGACCCAGACAGCCAGCAGCCTGAGCGCCAGCCTGGGCGATAGAGTGACCATCAGCT
GCAGAGCCAGCCAGGACATCAACAACTACCTGAACTGGTATCAGCAGAAACCCGACGGCACCGTGAA
GCTGCTGATCTACTTCACCAGCAGACTGCACAGCGGCGTGCCCAGCAGATTTTCTGGCAGCGGCTCT
GGCACCCACTACAGCCTGACCATCACCAACCTGGAACAGGAAGATATCGCTACCTACTTCTGTCAGC
AAGGCTACCCCCTGCCCTGGACCTTTGGCGGCGGAACAAAGCTGGAAATCAAG

[Figure 24]

**Amino acid sequence of chB1 heavy chain (SEQ ID NO: 32)**

MKHLWFFLLLVAAPRWVLSEVQLQQSGPELVKPGASVKISCKTSGYTFTEYTMHWVQQSHGKSLEWI
GGVNPNSGDTSYNQKFKGKATLTVDKSSSTAYMELRSLTSEDSAVYYCARPGGYDVGYYAMDYWGQG
TSVTVSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPSVFLFP
PKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEALHNHYTQKSLSLSP
GK

**Signal sequence (1 - 19), Heavy chain variable region (20 - 141), Heavy chain constant region (142 - 471)**

Nucleotide sequence encoding chB1 heavy chain (SEQ ID NO: 33)

ATGAAACACCTGTGGTTCTTCCTCCTGCTGGTGGCAGCTCCCAGATGGGTGCTGAGCGAAGTGCAGC
TGCAGCAGTCTGGCCCCGAGCTCGTGAAACCTGGCGCCTCCGTGAAGATCAGCTGCAAGACCAGCGG
CTACACCTTCACCGAGTACACCATGCACTGGGTGCAGCAGAGCCACGGCAAGAGCCTGGAATGGATC
GGCGGCGTGAACCCCAACAGCGGCGACACCAGCTACAACCAGAAGTTCAAGGGCAAGGCCACCCTGA
CCGTGGACAAGAGCAGCAGCACCGCCTACATGGAACTGCGGAGCCTGACCAGCGAGGACAGCGCCGT
GTACTACTGTGCCAGACCTGGCGGCTACGACGTGGGCTACTACGCCATGGATTACTGGGGCCAGGGC
ACCAGCGTGACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAACCCGTGAC
CGTGAGCTGGAACTCAGGCGCCCTGACCAGCGGCGTGCACACCTTCCCCGCTGTCCTGCAGTCCTCA
GGACTCTACTCCCTCAGCAGCGTGGTGACCGTGCCCTCCAGCAGCTTGGGCACCCAGACCTACATCT
GCAACGTGAATCACAAGCCCAGCAACACCAAGGTGGACAAGAGAGTTGAGCCCAAATCTTGTGACAA
AACTCACACATGCCCACCCTGCCCAGCACCTGAAGCCGCGGGGGGACCCTCAGTCTTCCTCTTCCCC
CCAAAACCCAAGGACACCCTCATGATCTCCCGGACCCCTGAGGTCACATGCGTGGTGGTGGACGTGA
GCCACGAAGACCCTGAGGTCAAGTTCAACTGGTACGTGGACGGCGTGGAGGTGCATAATGCCAAGAC
AAAGCCCCGGGAGGAGCAGTACAACAGCACGTACCGGGTGGTCAGCGTCCTCACCGTCCTGCACCAG
GACTGGCTGAATGGCAAGGAGTACAAGTGCAAGGTCTCCAACAAAGCCCTCCCAGCCCCCATCGAGA
AAACCATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACATCGCC
GTGGAGTGGGAGAGCAATGGCCAGCCCGAGAACAACTACAAGACCACCCCTCCCGTGCTGGACTCCG
ACGGCTCCTTCTTCCTCTACAGCAAGCTCACCGTGGACAAGAGCAGGTGGCAGCAGGGCAACGTCTT
CTCATGCTCCGTGATGCATGAGGCTCTGCACAACCACTACACCCAGAAGAGCCTCTCCCTGTCTCCC
GGCAAA

**Signal sequence (1 - 57), Heavy chain variable region (58 - 423), Heavy chain constant region (424 - 1413)**

[Figure 25]

**Amino acid sequence of variable region of chB1 heavy chain (SEQ ID NO: 34)**

EVQLQQSGPELVKPGASVKISCKTSGYTFTEYTMHWVQQSHGKSLEWIGGVNPNSGDTSYNQKFKGK

ATLTVDKSSSTAYMELRSLTSEDSAVYYCARPGGYDVGYYAMDYWGQGTSVTVSS

**Nucleotide sequence encoding variable region of chB1 heavy chain (SEQ ID NO: 35)**

GAAGTGCAGCTGCAGCAGTCTGGCCCCGAGCTCGTGAAACCTGGCGCCTCCGTGAAGATCAGCTGCA

AGACCAGCGGCTACACCTTCACCGAGTACACCATGCACTGGGTGCAGCAGAGCCACGGCAAGAGCCT

GGAATGGATCGGCGGCGTGAACCCCAACAGCGGCGACACCAGCTACAACCAGAAGTTCAAGGGCAAG

GCCACCCTGACCGTGGACAAGAGCAGCAGCACCGCCTACATGGAACTGCGGAGCCTGACCAGCGAGG

ACAGCGCCGTGTACTACTGTGCCAGACCTGGCGGCTACGACGTGGGCTACTACGCCATGGATTACTG

GGGCCAGGGCACCAGCGTGACCGTCAGCTCA

[Figure 26]

**Amino acid sequence of humanized antibody light chain hL1 (SEQ ID NO: 36)**

MVLQTQVFISLLLWISGAYGDIQMTQSPSSLSASVGDRVTITCRASQDINNYLNWYQQKPGKAPKLL

IYFTSRLHSGVPSRFSGSGSGTDYTLTISSLQPEDFATYYCQQGYPLPWTFGQGTKVEIKRTVAAPS

VFIFPPSDEQLKSGTASVVCLLNNFYPREAKVQWKVDNALQSGNSQESVTEQDSKDSTYSLSSTLTL

SKADYEKHKVYACEVTHQGLSSPVTKSFNRGEC

**Signal sequence (1 - 20), Light chain variable region (21 - 127), Light chain constant region (128 - 234)**

**Nucleotide sequence encoding humanized antibody light chain hL1 (SEQ ID NO: 37)**

ATGGTGCTGCAGACCCAGGTGTTCATCTCCCTGCTGCTGTGGATCTCCGGCGCGTACGGCGACATCC

AGATGACCCAGAGCCCTAGCAGCCTGAGCGCCAGCGTGGGCGACAGAGTGACCATCACCTGTAGAGC

CAGCCAGGACATCAACAACTACCTGAACTGGTATCAGCAGAAGCCCGGCAAGGCCCCCAAGCTGCTG

ATCTACTTCACCAGCAGACTGCACAGCGGCGTGCCCAGCAGATTTTCTGGCAGCGGCTCCGGCACCG

ACTACACCCTGACAATCAGCAGCCTGCAGCCCGAGGACTTCGCCACCTACTACTGCCAGCAGGGCTA

CCCCCTGCCTTGGACATTTGGCCAGGGCACCAAGGTGGAAATCAAGCGTACGGTGGCCGCCCCCTCC

GTGTTCATCTTCCCCCCCTCCGACGAGCAGCTGAAGTCCGGCACCGCCTCCGTGGTGTGCCTGCTGA

ATAACTTCTACCCCAGAGAGGCCAAGGTGCAGTGGAAGGTGGACAACGCCCTGCAGTCCGGGAACTC

CCAGGAGAGCGTGACCGAGCAGGACAGCAAGGACAGCACCTACAGCCTGAGCAGCACCCTGACCCTG

AGCAAAGCCGACTACGAGAAGCACAAGGTGTACGCCTGCGAGGTGACCCACCAGGGCCTGAGCTCCC

CCGTCACCAAGAGCTTCAACAGGGGGGAGTGT

**Signal sequence (1 - 60), Light chain variable region (61 - 381), Light chain constant region (382 - 702)**

[Figure 27]

**Amino acid sequence of variable region of humanized antibody light chain hL1 (SEQ ID NO: 38)**

DIQMTQSPSSLSASVGDRVTITCRASQDINNYLNWYQQKPGKAPKLLIYFTSRLHSGVPSRFSGSGS
GTDYTLTISSLQPEDFATYYCQQGYPLPWTFGQGTKVEIK

**Nucleotide sequence encoding variable region of humanized antibody light chain hL1 (SEQ ID NO: 39)**

GACATCCAGATGACCCAGAGCCCTAGCAGCCTGAGCGCCAGCGTGGGCGACAGAGTGACCATCACCT
GTAGAGCCAGCCAGGACATCAACAACTACCTGAACTGGTATCAGCAGAAGCCCGGCAAGGCCCCCAA
GCTGCTGATCTACTTCACCAGCAGACTGCACAGCGGCGTGCCCAGCAGATTTTCTGGCAGCGGCTCC
GGCACCGACTACACCCTGACAATCAGCAGCCTGCAGCCCGAGGACTTCGCCACCTACTACTGCCAGC
AGGGCTACCCCCTGCCTTGGACATTTGGCCAGGGCACCAAGGTGGAAATCAAG

[Figure 28]

**Amino acid sequence of humanized antibody light chain hL2 (SEQ ID NO: 40)**

MVLQTQVFISLLLWISGAYGDIQMTQSPSSLSASVGDRVTITCRASQDINNYLNWYQQKPGKAVKLL
IYFTSRLHSGVPSRFSGSGSGTHYTLTISSLQPEDFATYYCQQGYPLPWTFGQGTKVEIKRTVAAPS
VFIFPPSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**Signal sequence (1 - 20), Light chain variable region (21 - 127), Light chain constant region (128 - 234)**

**Nucleotide sequence encoding humanized antibody light chain hL2 (SEQ ID NO: 41)**

ATGGTGCTGCAGACCCAGGTGTTCATCTCCCTGCTGCTGTGGATCTCCGGCGCGTACGGCGACATCC
AGATGACCCAGAGCCCTAGCAGCCTGAGCGCCAGCGTGGGCGACAGAGTGACCATCACCTGTAGAGC
CAGCCAGGACATCAACAACTACCTGAACTGGTATCAGCAGAAACCCGGCAAGGCCGTGAAGCTGCTG
ATCTACTTCACCAGCAGACTGCACAGCGGCGTGCCCAGCAGATTTTCTGGCAGCGGCTCTGGCACCC
ACTACACCCTGACAATCAGCAGCCTGCAGCCCGAGGACTTCGCCACCTACTACTGCCAGCAGGGCTA
CCCCCTGCCTTGGACATTTGGCCAGGGCACCAAGGTGGAAATCAAGCGTACGGTGGCCGCCCCCTCC
GTGTTCATCTTCCCCCCCTCCGACGAGCAGCTGAAGTCCGGCACCGCCTCCGTGGTGTGCCTGCTGA
ATAACTTCTACCCCAGAGAGGCCAAGGTGCAGTGGAAGGTGGACAACGCCCTGCAGTCCGGGAACTC
CCAGGAGAGCGTGACCGAGCAGGACAGCAAGGACAGCACCTACAGCCTGAGCAGCACCCTGACCCTG
AGCAAAGCCGACTACGAGAAGCACAAGGTGTACGCCTGCGAGGTGACCCACCAGGGCCTGAGCTCCC
CCGTCACCAAGAGCTTCAACAGGGGGGAGTGT

**Signal sequence (1 - 60), Light chain variable region (61 - 381), Light chain constant region (382 - 702)**

[Figure 29]

**Amino acid sequence of variable region of humanized antibody light chain hL2 (SEQ ID NO: 42)**

DIQMTQSPSSLSASVGDRVTITC<u>RASQDINNYLN</u>WYQQKPGKAVKLLIY<u>FTSRLHS</u>GVPSRFSGSGS

GTHYTLTISSLQPEDFATYYC<u>QQGYPLPWT</u>FGQGTKVEIK

**Nucleotide sequence encoding variable region of humanized antibody light chain hL2 (SEQ ID NO: 43)**

GACATCCAGATGACCCAGAGCCCTAGCAGCCTGAGCGCCAGCGTGGGCGACAGAGTGACCATCACCT

GTAGAGCCAGCCAGGACATCAACAACTACCTGAACTGGTATCAGCAGAAACCCGGCAAGGCCGTGAA

GCTGCTGATCTACTTCACCAGCAGACTGCACAGCGGCGTGCCCAGCAGATTTTCTGGCAGCGGCTCT

GGCACCCACTACACCCTGACAATCAGCAGCCTGCAGCCCGAGGACTTCGCCACCTACTACTGCCAGC

AGGGCTACCCCCTGCCTTGGACATTTGGCCAGGGCACCAAGGTGGAAATCAAG

[Figure 30]

**Amino acid sequence of humanized antibody light chain hL3 (SEQ ID NO: 44)**

MVLQTQVFISLLLWISGAYGDIQMTQSPSSLSASVGDRVTITC<u>RASQDINNYLN</u>WYQQKPGGAVKLL

IY<u>FTSRLHS</u>GVPSRFSGSGSGTDYTLTISSLQPEDFATYYC<u>QQGYPLPWT</u>FGGGTKVEIKRTVAAPS

VFIFPPSDEQLKSGTASVVCLLNNFYPREAKVQWKVDNALQSGNSQESVTEQDSKDSTYSLSSTLTL

SKADYEKHKVYACEVTHQGLSSPVTKSFNRGEC

**Signal sequence (1 - 20), Light chain variable region (21 - 127), Light chain constant region (128 - 234)**

**Nucleotide sequence encoding humanized antibody light chain hL3 (SEQ ID NO: 45)**

ATGGTGCTGCAGACCCAGGTGTTCATCTCCCTGCTGCTGTGGATCTCCGGCGCGTACGGCGACATCC

AGATGACCCAGAGCCCTAGCAGCCTGAGCGCCAGCGTGGGCGACAGAGTGACCATCACCTGTAGAGC

CAGCCAGGACATCAACAACTACCTGAACTGGTATCAGCAGAAACCCGGCGGAGCCGTGAAGCTGCTG

ATCTACTTCACCAGCAGACTGCACAGCGGCGTGCCCAGCAGATTTTCTGGCAGCGGCTCCGGCACCG

ACTACACCCTGACAATCAGCAGCCTGCAGCCCGAGGACTTCGCCACCTACTACTGCCAGCAGGGCTA

CCCCCTGCCCTGGACATTTGGCGGCGGAACAAAGGTGGAAATCAAGCGTACGGTGGCCGCCCCCTCC

GTGTTCATCTTCCCCCCCTCCGACGAGCAGCTGAAGTCCGGCACCGCCTCCGTGGTGTGCCTGCTGA

ATAACTTCTACCCCAGAGAGGCCAAGGTGCAGTGGAAGGTGGACAACGCCCTGCAGTCCGGGAACTC

CCAGGAGAGCGTGACCGAGCAGGACAGCAAGGACAGCACCTACAGCCTGAGCAGCACCCTGACCCTG

AGCAAAGCCGACTACGAGAAGCACAAGGTGTACGCCTGCGAGGTGACCCACCAGGGCCTGAGCTCCC

CCGTCACCAAGAGCTTCAACAGGGGGGAGTGT

**Signal sequence (1 - 60), Light chain variable region (61 - 381), Light chain constant region (382 - 702)**

[Figure 31]

**Amino acid sequence of variable region of humanized antibody light chain hL3 (SEQ ID NO: 46)**

DIQMTQSPSSLSASVGDRVTITCRASQDINNYLNWYQQKPGGAVKLLIYFTSRLHSGVPSRFSGSGS
GTDYTLTISSLQPEDFATYYCQQGYPLPWTFGGGTKVEIK

**Nucleotide sequence encoding variable region of humanized antibody light chain hL3 (SEQ ID NO: 47)**

GACATCCAGATGACCCAGAGCCCTAGCAGCCTGAGCGCCAGCGTGGGCGACAGAGTGACCATCACCT
GTAGAGCCAGCCAGGACATCAACAACTACCTGAACTGGTATCAGCAGAAACCCGGCGGAGCCGTGAA
GCTGCTGATCTACTTCACCAGCAGACTGCACAGCGGCGTGCCCAGCAGATTTTCTGGCAGCGGCTCC
GGCACCGACTACACCCTGACAATCAGCAGCCTGCAGCCCGAGGACTTCGCCACCTACTACTGCCAGC
AGGGCTACCCCCTGCCCTGGACATTTGGCGGCGGAACAAAGGTGGAAATCAAG

[Figure 32]

**Amino acid sequence of humanized antibody light chain hL4 (SEQ ID NO: 48)**

MVLQTQVFISLLLWISGAYGDIQMTQSPSSLSASVGDRVTITCRASQDINNYLNWYQQKPGGAVKLL
IYFTSRLHSGVPSRFSGSGSGTDYTLTISSLQPEDFATYYCQQGNTLPWTFGGGTKVEIKRTVAAPS
VFIFPPSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**Signal sequence (1 - 20), Light chain variable region (21 - 127), Light chain constant region (128 - 234)**

**Nucleotide sequence encoding humanized antibody light chain hL4 (SEQ ID NO: 49)**

ATGGTGCTGCAGACCCAGGTGTTCATCTCCCTGCTGCTGTGGATCTCCGGCGCGTACGGCGACATCC
AGATGACCCAGAGCCCTAGCAGCCTGAGCGCCAGCGTGGGCGACAGAGTGACCATCACCTGTAGAGC
CAGCCAGGACATCAACAACTACCTGAACTGGTATCAGCAGAAACCCGGCGGAGCCGTGAAGCTGCTG
ATCTACTTCACCAGCAGACTGCACAGCGGCGTGCCCAGCAGATTTTCTGGCAGCGGCTCCGGCACCG
ACTACACCCTGACAATCAGCAGCCTGCAGCCCGAGGACTTCGCCACCTACTACTGCCAGCAGGGCAA
CACCCTGCCCTGGACATTTGGCGGAGGCACCAAGGTGGAAATCAAGCGTACGGTGGCCGCCCCCTCC
GTGTTCATCTTCCCCCCCTCCGACGAGCAGCTGAAGTCCGGCACCGCCTCCGTGGTGTGCCTGCTGA
ATAACTTCTACCCCAGAGAGGCCAAGGTGCAGTGGAAGGTGGACAACGCCCTGCAGTCCGGGAACTC
CCAGGAGAGCGTGACCGAGCAGGACAGCAAGGACAGCACCTACAGCCTGAGCAGCACCCTGACCCTG
AGCAAAGCCGACTACGAGAAGCACAAGGTGTACGCCTGCGAGGTGACCCACCAGGGCCTGAGCTCCC
CCGTCACCAAGAGCTTCAACAGGGGGGAGTGT

**Signal sequence (1 - 60), Light chain variable region (61 - 381), Light chain constant region (382 - 702)**

[Figure 33]

**Amino acid sequence of variable region of humanized antibody light chain hL4 (SEQ ID NO: 50)**

DIQMTQSPSSLSASVGDRVTITCRASQDINNYLNWYQQKPGGAVKLLIYFTSRLHSGVPSRFSGSGS

GTDYTLTISSLQPEDFATYYCQQGNTLPWTFGGGTKVEIK

**Nucleotide sequence encoding variable region of humanized antibody light chain hL4 (SEQ ID NO: 51)**

GACATCCAGATGACCCAGAGCCCTAGCAGCCTGAGCGCCAGCGTGGGCGACAGAGTGACCATCACCT

GTAGAGCCAGCCAGGACATCAACAACTACCTGAACTGGTATCAGCAGAAACCCGGCGGAGCCGTGAA

GCTGCTGATCTACTTCACCAGCAGACTGCACAGCGGCGTGCCCAGCAGATTTTCTGGCAGCGGCTCC

GGCACCGACTACACCCTGACAATCAGCAGCCTGCAGCCCGAGGACTTCGCCACCTACTACTGCCAGC

AGGGCAACACCCTGCCCTGGACATTTGGCGGAGGCACCAAGGTGGAAATCAAG

[Figure 34]

**Amino acid sequence of humanized antibody heavy chain hH1 (SEQ ID NO: 52)**

MKHLWFFLLLVAAPRWVLSQVQLVQSGAEVKKPGASVKVSCKASGYTFTEYTMHWVRQAPGQGLEWM
GGVNPNSGDTSYAQKFQGRVTITADTSTSTAYMELSSLRSEDTAVYYCARPGGYDVGYYAMDYWGQG
TLVTVSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPSVFLFP
PKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEALHNHYTQKSLSLSP
GK

**Signal sequence (1 - 19), Heavy chain variable region (20 - 141), Heavy chain constant region (142 - 471)**

Nucleotide sequence encoding humanized antibody heavy chain hH1 (SEQ ID NO: 53)

ATGAAACACCTGTGGTTCTTCCTCCTGCTGGTGGCAGCTCCCAGATGGGTGCTGAGCCAGGTGCAGC
TGGTGCAGTCTGGCGCCGAAGTGAAGAAACCAGGCGCCAGCGTGAAGGTGTCCTGCAAGGCCAGCGG
CTACACCTTTACCGAGTACACCATGCACTGGGTGCGCCAGGCTCCAGGCCAGGGACTGGAATGGATG
GGCGGCGTGAACCCCAACAGCGGCGATACAAGCTACGCCCAGAAATTCCAGGGCAGAGTGACCATCA
CCGCCGACACCAGCACCTCCACCGCCTACATGGAACTGAGCAGCCTGCGGAGCGAGGACACCGCCGT
GTACTACTGTGCTAGACCTGGCGGCTACGACGTGGGCTACTACGCCATGGATTACTGGGGCCAGGGC
ACCCTCGTGACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAACCCGTGAC
CGTGAGCTGGAACTCAGGCGCCCTGACCAGCGGCGTGCACACCTTCCCCGCTGTCCTGCAGTCCTCA
GGACTCTACTCCCTCAGCAGCGTGGTGACCGTGCCCTCCAGCAGCTTGGGCACCCAGACCTACATCT
GCAACGTGAATCACAAGCCCAGCAACACCAAGGTGGACAAGAGAGTTGAGCCCAAATCTTGTGACAA
AACTCACACATGCCCACCCTGCCCAGCACCTGAAGCCGCGGGGGGACCCTCAGTCTTCCTCTTCCCC
CCAAAACCCAAGGACACCCTCATGATCTCCCGGACCCCTGAGGTCACATGCGTGGTGGTGGACGTGA
GCCACGAAGACCCTGAGGTCAAGTTCAACTGGTACGTGGACGGCGTGGAGGTGCATAATGCCAAGAC
AAAGCCCCGGGAGGAGCAGTACAACAGCACGTACCGGGTGGTCAGCGTCCTCACCGTCCTGCACCAG
GACTGGCTGAATGGCAAGGAGTACAAGTGCAAGGTCTCCAACAAAGCCCTCCCAGCCCCCATCGAGA
AAACCATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACATCGCC
GTGGAGTGGGAGAGCAATGGCCAGCCCGAGAACAACTACAAGACCACCCCTCCCGTGCTGGACTCCG
ACGGCTCCTTCTTCCTCTACAGCAAGCTCACCGTGGACAAGAGCAGGTGGCAGCAGGGCAACGTCTT
CTCATGCTCCGTGATGCATGAGGCTCTGCACAACCACTACACCCAGAAGAGCCTCTCCCTGTCTCCC
GGCAAA

**Signal sequence (1 - 57), Heavy chain variable region (58 - 423), Heavy chain constant region (424 - 1413)**

[Figure 35]

**Amino acid sequence of variable region of humanized antibody heavy chain hH1 (SEQ ID NO: 54)**

QVQLVQSGAEVKKPGASVKVSCKASGYTFTEYTMHWVRQAPGQGLEWMGGVNPNSGDTSYAQKFQGR

VTITADTSTSTAYMELSSLRSEDTAVYYCARPGGYDVGYYAMDYWGQGTLVTVSS

**Nucleotide sequence encoding variable region of humanized antibody heavy chain hH1 (SEQ ID NO: 55)**

CAGGTGCAGCTGGTGCAGTCTGGCGCCGAAGTGAAGAAACCAGGCGCCAGCGTGAAGGTGTCCTGCA

AGGCCAGCGGCTACACCTTTACCGAGTACACCATGCACTGGGTGCGCCAGGCTCCAGGCCAGGGACT

GGAATGGATGGGCGGCGTGAACCCCAACAGCGGCGATACAAGCTACGCCCAGAAATTCCAGGGCAGA

GTGACCATCACCGCCGACACCAGCACCTCCACCGCCTACATGGAACTGAGCAGCCTGCGGAGCGAGG

ACACCGCCGTGTACTACTGTGCTAGACCTGGCGGCTACGACGTGGGCTACTACGCCATGGATTACTG

GGGCCAGGGCACCCTCGTGACCGTCAGCTCA

[Figure 36]

**Amino acid sequence of humanized antibody heavy chain hH2 (SEQ ID NO: 56)**

MKHLWFFLLLVAAPRWVLSEVQLVQSGAEVKKPGASVKVSCKTSGYTFTEYTMHWVRQAPGKSLEWM

GGVNPNSGDTSYAQKFQGRVTITADTSTSTAYMELSSLRSEDTAVYYCARPGGYDVGYYAMDYWGQG

TLVTVSSASTKGPSVFPLAPSSKSTSGGTAALGCLVKDYFPEPVTVSWNSGALTSGVHTFPAVLQSS

GLYSLSSVVTVPSSSLGTQTYICNVNHKPSNTKVDKRVEPKSCDKTHTCPPCPAPEAAGGPSVFLFP

PKPKDTLMISRTPEVTCVVVDVSHEDPEVKFNWYVDGVEVHNAKTKPREEQYNSTYRVVSVLTVLHQ

DWLNGKEYKCKVSNKALPAPIEKTISKAKGQPREPQVYTLPPSREEMTKNQVSLTCLVKGFYPSDIA

VEWESNGQPENNYKTTPPVLDSDGSFFLYSKLTVDKSRWQQGNVFSCSVMHEALHNHYTQKSLSLSP

GK

**Signal sequence (1 - 19), Heavy chain variable region (20 - 141), Heavy chain constant region (142 - 471)**

**Nucleotide sequence encoding humanized antibody heavy chain hH2 (SEQ ID NO: 57)**

ATGAAACACCTGTGGTTCTTCCTCCTGCTGGTGGCAGCTCCCAGATGGGTGCTGAGCGAAGTGCAGC

TGGTGCAGTCTGGCGCCGAAGTGAAGAAACCAGGCGCCAGCGTGAAGGTGTCCTGCAAGACCAGCGG

CTACACCTTTACCGAGTACACCATGCACTGGGTGCGCCAGGCCCCTGGCAAGAGCCTGGAATGGATG

GGCGGCGTGAACCCCAACAGCGGCGATACAAGCTACGCCCAGAAATTCCAGGGCAGAGTGACCATCA

CCGCCGACACCAGCACCTCCACCGCCTACATGGAACTGAGCAGCCTGCGGAGCGAGGACACCGCCGT

GTACTACTGTGCTAGACCTGGCGGCTACGACGTGGGCTACTACGCCATGGATTACTGGGGCCAGGGC

ACCCTCGTGACCGTCAGCTCAGCCTCCACCAAGGGCCCAAGCGTCTTCCCCCTGGCACCCTCCTCCA

AGAGCACCTCTGGCGGCACAGCCGCCCTGGGCTGCCTGGTCAAGGACTACTTCCCCGAACCCGTGAC

CGTGAGCTGGAACTCAGGCGCCCTGACCAGCGGCGTGCACACCTTCCCCGCTGTCCTGCAGTCCTCA

GGACTCTACTCCCTCAGCAGCGTGGTGACCGTGCCCTCCAGCAGCTTGGGCACCCAGACCTACATCT

GCAACGTGAATCACAAGCCCAGCAACACCAAGGTGGACAAGAGAGTTGAGCCCAAATCTTGTGACAA

AACTCACACATGCCCACCCTGCCCAGCACCTGAAGCCGCGGGGGGACCCTCAGTCTTCCTCTTCCCC

CCAAAACCCAAGGACACCCTCATGATCTCCCGGACCCCTGAGGTCACATGCGTGGTGGTGGACGTGA

GCCACGAAGACCCTGAGGTCAAGTTCAACTGGTACGTGGACGGCGTGGAGGTGCATAATGCCAAGAC

AAAGCCCCGGGAGGAGCAGTACAACAGCACGTACCGGGTGGTCAGCGTCCTCACCGTCCTGCACCAG

GACTGGCTGAATGGCAAGGAGTACAAGTGCAAGGTCTCCAACAAAGCCCTCCCAGCCCCCATCGAGA

AAACCATCTCCAAAGCCAAAGGCCAGCCCCGGGAACCACAGGTGTACACCCTGCCCCCATCCCGGGA

GGAGATGACCAAGAACCAGGTCAGCCTGACCTGCCTGGTCAAAGGCTTCTATCCCAGCGACATCGCC

GTGGAGTGGGAGAGCAATGGCCAGCCCGAGAACAACTACAAGACCACCCCTCCCGTGCTGGACTCCG

ACGGCTCCTTCTTCCTCTACAGCAAGCTCACCGTGGACAAGAGCAGGTGGCAGCAGGGCAACGTCTT

CTCATGCTCCGTGATGCATGAGGCTCTGCACAACCACTACACCCAGAAGAGCCTCTCCCTGTCTCCC

GGCAAA

**Signal sequence (1 - 57), Heavy chain variable region (58 - 423), Heavy chain constant region (424 - 1413)**

[Figure 37]

**Amino acid sequence of variable region of humanized antibody heavy chain hH2 (SEQ ID NO: 58)**

EVQLVQSGAEVKKPGASVKVSCKTSGYTFTEYTMHWVRQAPGKSLEWMGGVNPNSGDTSYAQKFQGR

VTITADTSTSTAYMELSSLRSEDTAVYYCARPGGYDVGYYAMDYWGQGTLVTVSS

**Nucleotide sequence encoding variable region of humanized antibody heavy chain hH2 (SEQ ID NO: 59)**

GAAGTGCAGCTGGTGCAGTCTGGCGCCGAAGTGAAGAAACCAGGCGCCAGCGTGAAGGTGTCCTGCA

AGACCAGCGGCTACACCTTTACCGAGTACACCATGCACTGGGTGCGCCAGGCCCCTGGCAAGAGCCT

GGAATGGATGGGCGGCGTGAACCCCAACAGCGGCGATACAAGCTACGCCCAGAAATTCCAGGGCAGA

GTGACCATCACCGCCGACACCAGCACCTCCACCGCCTACATGGAACTGAGCAGCCTGCGGAGCGAGG

ACACCGCCGTGTACTACTGTGCTAGACCTGGCGGCTACGACGTGGGCTACTACGCCATGGATTACTG

GGGCCAGGGCACCCTCGTGACCGTCAGCTCA

[Figure 38]

**Amino acid sequence of humanized antibody heavy chain hH3 (SEQ ID NO: 60)**

MKHLWFFLLLVAAPRWVLSEVQLVQSGAEVKKPGASVKVSCKTSGYTFTEYTMHWVRQAPGQGLEWM
GGVNPNSGDTSYAQKFQGRVTLTVDKSTSTAYMELSSLRSEDTAVYYCARPGGYDVGYYAMDYWGQG
TLVTVSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPSVFLFP
PKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEALHNHYTQKSLSLSP
GK

**Signal sequence (1 - 19), Heavy chain variable region (20 - 141), Heavy chain constant region (142 - 471)**

**Nucleotide sequence encoding humanized antibody heavy chain hH3 (SEQ ID NO: 61)**

ATGAAACACCTGTGGTTCTTCCTCCTGCTGGTGGCAGCTCCCAGATGGGTGCTGAGCGAAGTGCAGC
TGGTGCAGTCTGGCGCCGAAGTGAAGAAACCAGGCGCCAGCGTGAAGGTGTCCTGCAAGACCAGCGG
CTACACCTTTACCGAGTACACCATGCACTGGGTGCGCCAGGCTCCAGGCCAGGGACTGGAATGGATG
GGCGGCGTGAACCCCAACAGCGGCGATACAAGCTACGCCCAGAAATTCCAGGGCAGAGTGACCCTGA
CCGTGGACAAGAGCACCAGCACCGCCTACATGGAACTGAGCAGCCTGCGGAGCGAGGACACCGCCGT
GTACTACTGTGCTAGACCTGGCGGCTACGACGTGGGCTACTACGCCATGGATTACTGGGGCCAGGGC
ACCCTCGTGACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAACCCGTGAC
CGTGAGCTGGAACTCAGGCGCCCTGACCAGCGGCGTGCACACCTTCCCCGCTGTCCTGCAGTCCTCA
GGACTCTACTCCCTCAGCAGCGTGGTGACCGTGCCCTCCAGCAGCTTGGGCACCCAGACCTACATCT
GCAACGTGAATCACAAGCCCAGCAACACCAAGGTGGACAAGAGAGTTGAGCCCAAATCTTGTGACAA
AACTCACACATGCCCACCCTGCCCAGCACCTGAAGCCGCGGGGGGACCCTCAGTCTTCCTCTTCCCC
CCAAAACCCAAGGACACCCTCATGATCTCCCGGACCCCTGAGGTCACATGCGTGGTGGTGGACGTGA
GCCACGAAGACCCTGAGGTCAAGTTCAACTGGTACGTGGACGGCGTGGAGGTGCATAATGCCAAGAC
AAAGCCCCGGGAGGAGCAGTACAACAGCACGTACCGGGTGGTCAGCGTCCTCACCGTCCTGCACCAG
GACTGGCTGAATGGCAAGGAGTACAAGTGCAAGGTCTCCAACAAAGCCCTCCCAGCCCCCATCGAGA
AAACCATCTCCAAAGCCAAAGGCCAGCCCCGGGAACCACAGGTGTACACCCTGCCCCCATCCCGGGA
GGAGATGACCAAGAACCAGGTCAGCCTGACCTGCCTGGTCAAAGGCTTCTATCCCAGCGACATCGCC
GTGGAGTGGGAGAGCAATGGCCAGCCCGAGAACAACTACAAGACCACCCCTCCCGTGCTGGACTCCG
ACGGCTCCTTCTTCCTCTACAGCAAGCTCACCGTGGACAAGAGCAGGTGGCAGCAGGGCAACGTCTT
CTCATGCTCCGTGATGCATGAGGCTCTGCACAACCACTACACCCAGAAGAGCCTCTCCCTGTCTCCC
GGCAAA

**Signal sequence (1 - 57), Heavy chain variable region (58 - 423), Heavy chain constant region (424 - 1413)**

[Figure 39]

**Amino acid sequence of variable region of humanized antibody heavy chain hH3 (SEQ ID NO: 62)**

EVQLVQSGAEVKKPGASVKVSCKTSGYTFTEYTMHWVRQAPGQGLEWMGGVNPNSGDTSYAQKFQGR

VTLTVDKSTSTAYMELSSLRSEDTAVYYCARPGGYDVGYYAMDYWGQGTLVTVSS

**Nucleotide sequence encoding variable region of humanized antibody heavy chain hH3 (SEQ ID NO: 63)**

GAAGTGCAGCTGGTGCAGTCTGGCGCCGAAGTGAAGAAACCAGGCGCCAGCGTGAAGGTGTCCTGCA

AGACCAGCGGCTACACCTTTACCGAGTACACCATGCACTGGGTGCGCCAGGCTCCAGGCCAGGGACT

GGAATGGATGGGCGGCGTGAACCCCAACAGCGGCGATACAAGCTACGCCCAGAAATTCCAGGGCAGA

GTGACCCTGACCGTGGACAAGAGCACCAGCACCGCCTACATGGAACTGAGCAGCCTGCGGAGCGAGG

ACACCGCCGTGTACTACTGTGCTAGACCTGGCGGCTACGACGTGGGCTACTACGCCATGGATTACTG

GGGCCAGGGCACCCTCGTGACCGTCAGCTCA

[Figure 40]

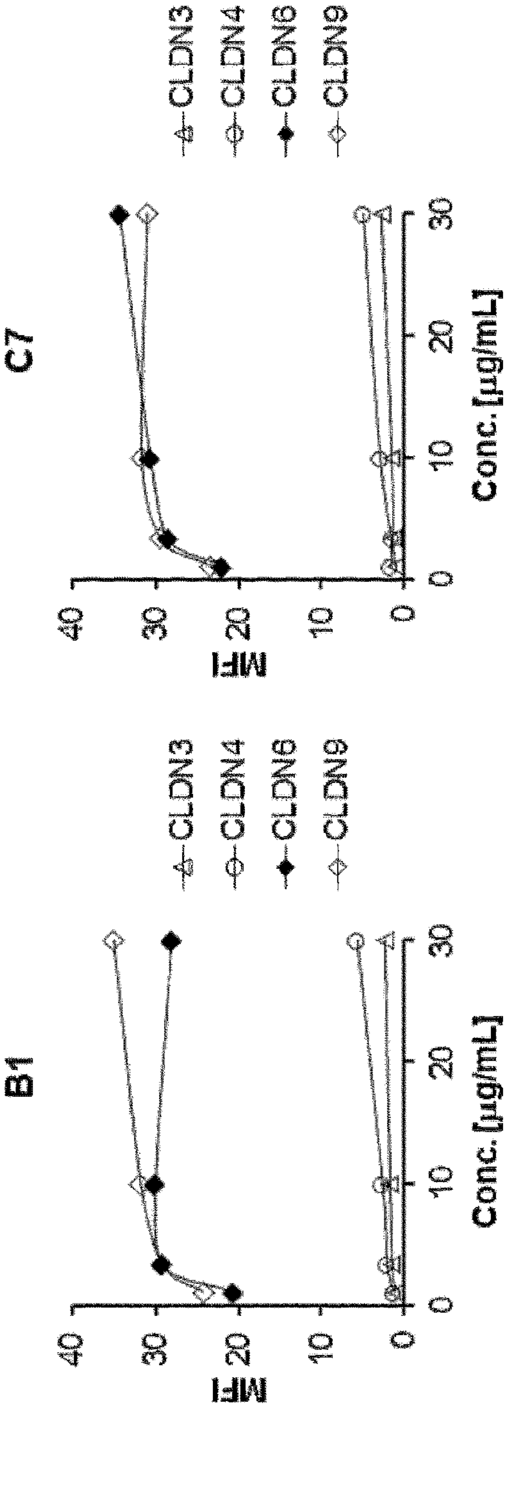

[Figure 41]

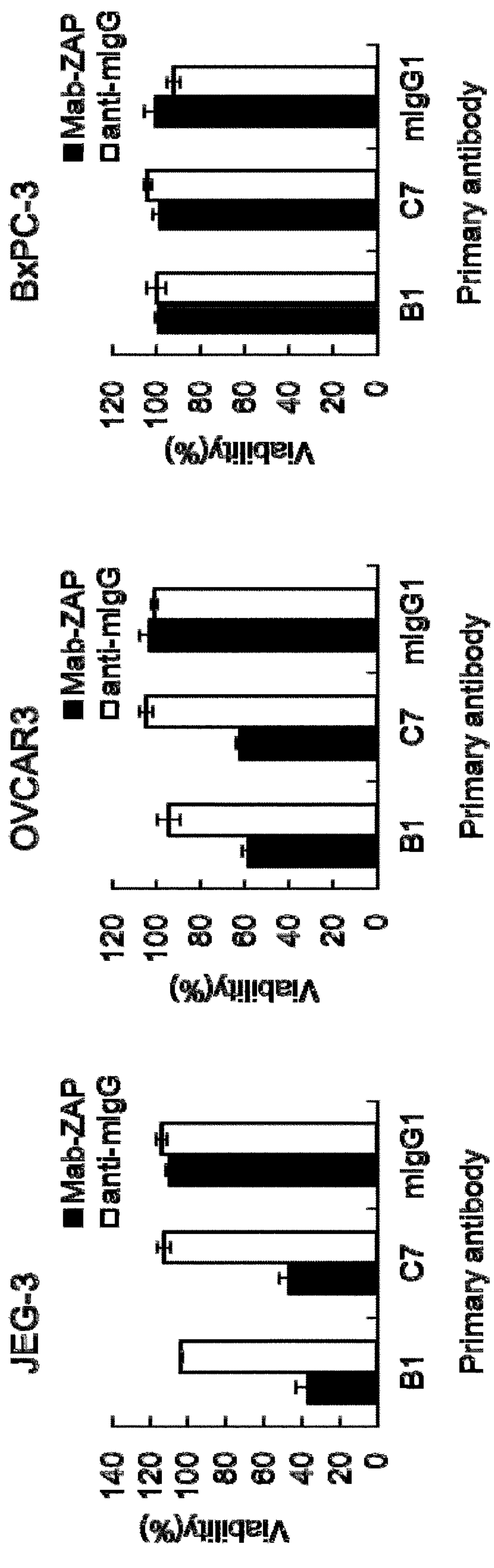
BxPC-3
OVCAR3
JEG-3
■ Mab-ZAP
□ anti-mIgG
Viability(%)
B1
C7
mIgG1
Primary antibody
0
20
40
60
80
100
120
140

[Figure 42]

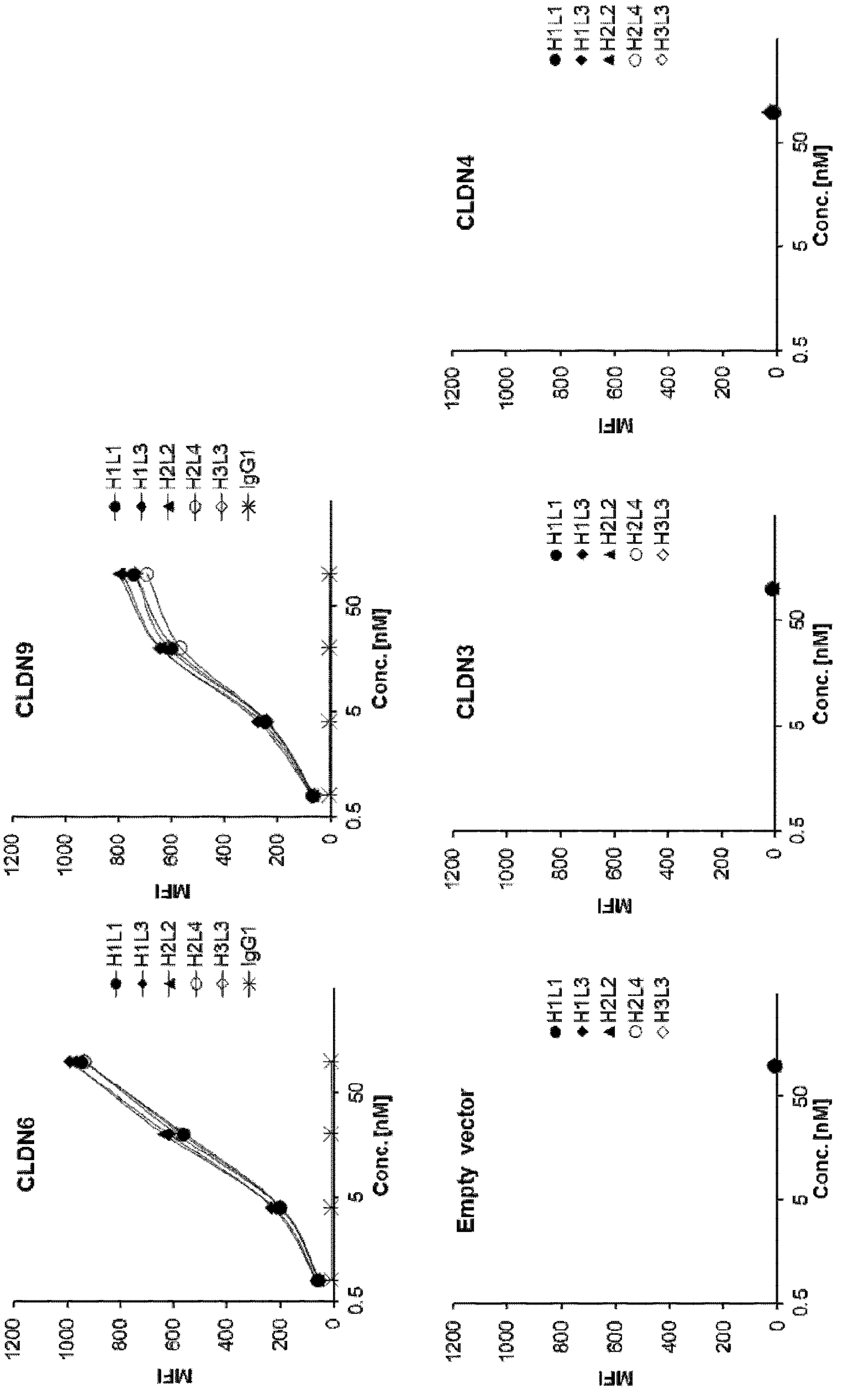
CLDN6
CLDN9
Empty vector
CLDN3
CLDN4
MFI
1200
1000
800
600
400
200
0
0.5
5
50
Conc. [nM]
H1L1
H1L3
H2L2
H2L4
H3L3
IgG1

[Figure 43]

**Amino acid sequence of trastuzumab light chain (SEQ ID NO: 64)**

DIQMTQSPSSLSASVGDRVTITCRASQDVNTAVAWYQQKPGKAPKLLIYSASFLYSGVPSRFSGSRS
GTDFTLTISSLQPEDFATYYCQQHYTTPPTFGQGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQGL
SSPVTKSFNRGEC

**Amino acid sequence of trastuzumab heavy chain (SEQ ID NO: 65)**

EVQLVESGGGLVQPGGSLRLSCAASGFNIKDTYIHWVRQAPGKGLEWVARIYPTNGYTRYADSVKGR
FTISADTSKNTAYLQMNSLRAEDTAVYYCSRWGGDGFYAMDYWGQGTLVTVSSASTKGPSVFPLAPS
SKSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPGK

[Figure 44]

**Amino acid sequence of trastuzumab variant light chain (SEQ ID NO: 73)**

MVLQTQVFISLLLWISGAYGDIQMTQSPSSLSASVGDRVTITCRASQDVNTAVAWYQQKPGKAPKLLIYSAS
FLYSGVPSRFSGSRSGTDFTLTISSLQPEDFATYYCQQHYTTPPTFGQGTKVEIKRTVAAPSVFIFPPSDEQ
LKSGTASVVCLLNNFYPREAKVQWKVDNALQSGNSQESVTEQDSKDSTYSLSSTLTLSKADYEKHKVYACEV
THQGLSSPVTKSFNRGEC

**Signal sequence (1 - 20), Light chain variable region (21 - 127), Light chain constant region (128 - 234)**

**Amino acid sequence of trastuzumab variant heavy chain (SEQ ID NO: 75)**

MKHLWFFLLLVAAPRWVLSEVQLVESGGGLVQPGGSLRLSCAASGFNIKDTYIHWVRQAPGKGLEWVARIYP
TNGYTRYADSVKGRFTISADTSKNTAYLQMNSLRAEDTAVYYCSRWGGDGFYAMDYWGQGTLVTVSSASTKG
PSVFPLAPSSKSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK

**Signal sequence (1 - 19), Heavy chain variable region (20 - 139), Heavy chain constant region (140 - 469)**

[Figure 45]

```
chB1_H   1 EVQLQQSGPELVKPGASVKISCKTSGYTFTEYTMHWVQQSHGKSLEWIGGVNPNSGDTSYNQKFKGKATL 70
hH1      1 Q...V...A.VK.......V...A.............R.AP.QG...M............A...Q.RV.I 70
hH2      1 ....V...A.VK.......V.................R.AP......M............A...Q.RV.I 70
hH3      1 ....V...A.VK.......V.................R.AP.QG...M............A...Q.RV.. 70

chB1_H  71 TVDKSSSTAYMELRSLTSEDSAVYYCARPGGYDVGYYAMDYWGQGTSVTVSS                   122
hH1     71 .A.T.T.......S..R...T.........................L.....                   122
hH2     71 .A.T.T.......S..R...T.........................L.....                   122
hH3     71 .....T.......S..R...T.........................L.....                   122
```

[Figure 46]

```
chB1_L  1 DIQMTQTASSLSASLGDRVTISCRASQDINNYLNWYQQKPDGTVKLLIYFTSRLHSGVPS 60
hL1     1 ......SP......V......T..................GKAP................ 60
hL2     1 ......SP......V......T..................GKA................. 60
hL3     1 ......SP......V......T..................G.A................. 60
hL4     1 ......SP......V......T..................G.A................. 60

chB1_L 61 RFSGSGSGTHYSLTITNLEQEDIATYFCQQGYPLPWTFGGGTKLEIK              107
hL1    61 .........D.T...SS.QP..F...Y............Q...V...              107
hL2    61 ...........T...SS.QP..F...Y............Q...V...              107
hL3    61 .........D.T...SS.QP..F...Y................V...              107
hL4    61 .........D.T...SS.QP..F...Y....NT..........V...              107
```

[Figure 47]

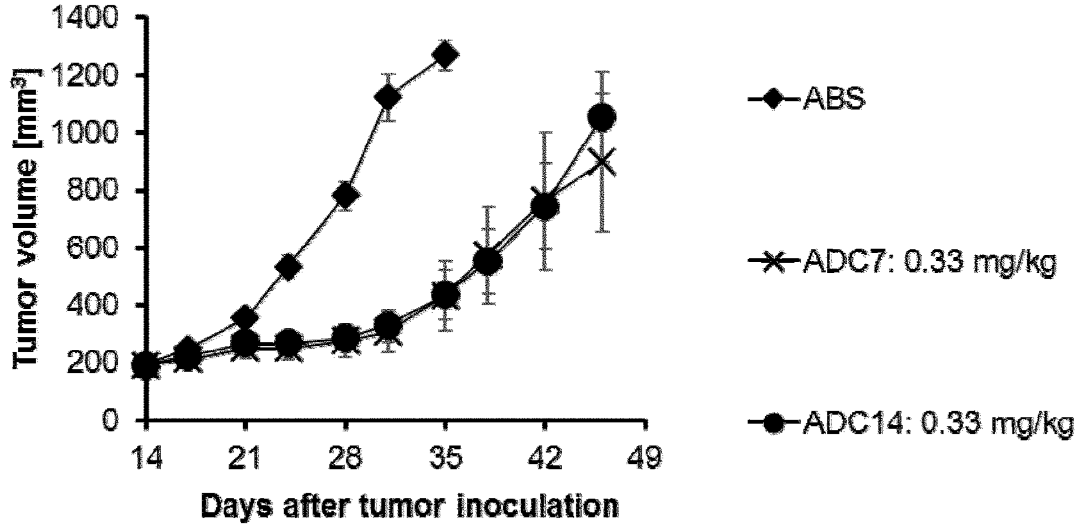


[Figure 48]

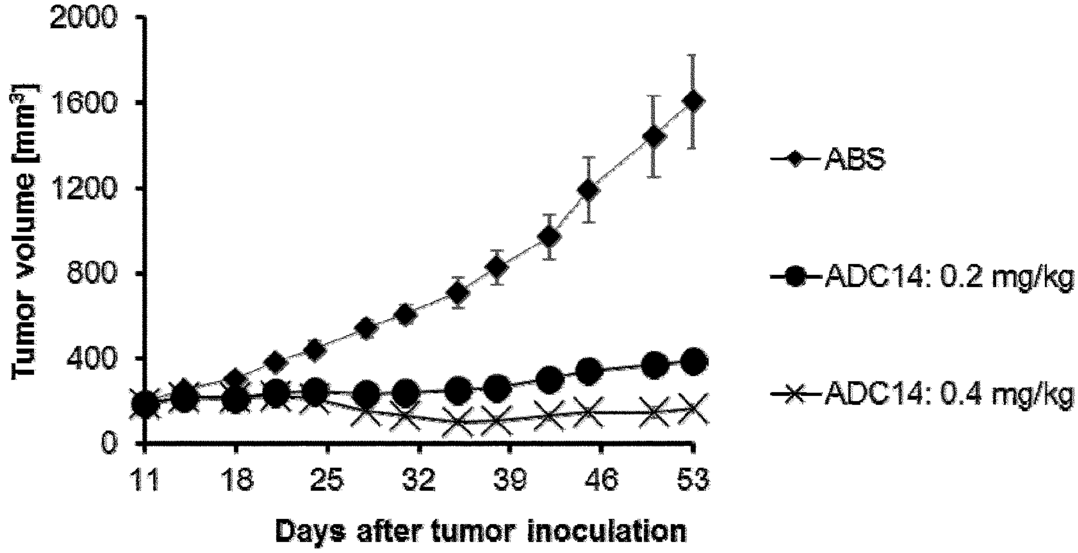

[Figure 49]

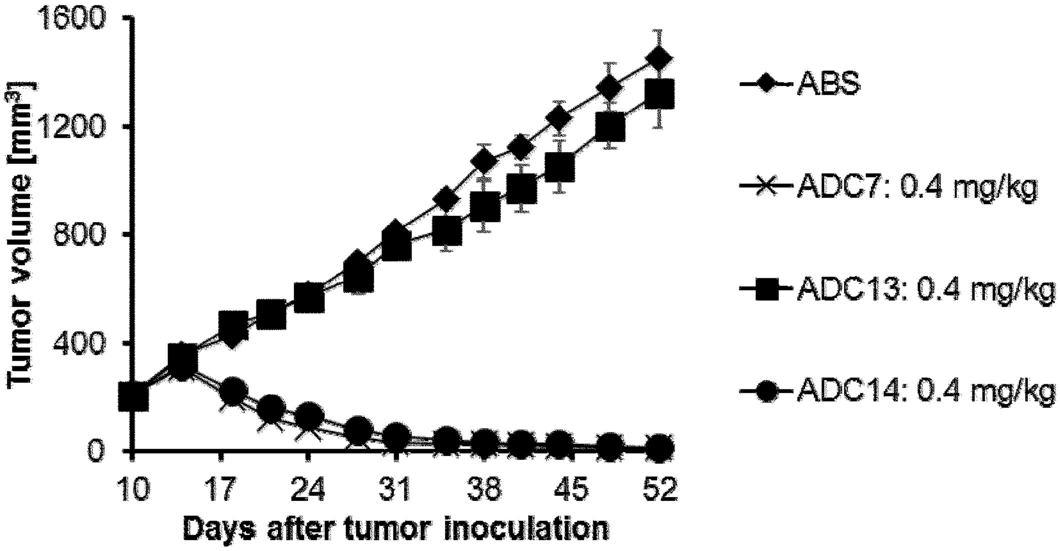
1600
1200
800
400
0
Tumor volume [mm³]
10
17
24
31
38
45
52
Days after tumor inoculation
ABS
ADC7: 0.4 mg/kg
ADC13: 0.4 mg/kg
ADC14: 0.4 mg/kg

**INTERNATIONAL SEARCH REPORT**

International application No. PCT/JP2020/012883

A. CLASSIFICATION OF SUBJECT MATTER
A61P 35/00(2006.01)i; C07K 16/30(2006.01)i; A61K 47/68(2017.01)i; C12N 15/13(2006.01)i; A61K 31/551(2006.01)i
FI: A61K47/68; A61P35/00; C07K16/30; C12N15/13 ZNA; A61K31/551
According to International Patent Classification (IPC) or to both national classification and IPC

B. FIELDS SEARCHED
Minimum documentation searched (classification system followed by classification symbols)
A61P35/00; C07K16/30; A61K47/68; C12N15/13; A61K31/551

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| P, X | WO 2019/065964 A1 (DAIICHI SANKYO COMPANY, LIMITED) 04.04.2019 (2019-04-04) claims, examples 1-154 | 1-49 |
| A | WO 2017/137556 A1 (ADC THERAPEUTICS SA) 17.08.2017 (2017-08-17) claims, examples 1-7 | 1-49 |
| A | JP 2016-512540 A (SEATTLE GENETICS, INC.) 28.04.2016 (2016-04-28) claims, examples 1-10 | 1-49 |
| A | US 2009/0149449 A1 (THE UNITED STATES OF AMERICA, AS REPRESENTED BY THE SECRETARY, DEPT. OF HEALTH AND HUMAN SERVICE) 11.06.2009 (2009-06-11) claims, examples 1-11 | 1-49 |
| A | JP 2015-534996 A (SYNAFFIX B.V.) 07.12.2015 (2015-12-07) claims, examples 1-41 | 1-49 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 June 2020 (03.06.2020) | 23 June 2020 (23.06.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/012883

| Patent Documents referred in the Report | Publication Date | Patent Family | Publication Date |
|---|---|---|---|
| WO 2019/065964 A1 | 04 Apr. 2019 | TW 201922788 A | |
| WO 2017/137556 A1 | 17 Aug. 2017 | GB 201602363 A | |
| JP 2016-512540 A | 28 Apr. 2016 | US 2016/0045615 A1<br>claims, examples 1-10<br>WO 2014/165119 A1<br>EP 2970444 A1<br>CA 2901575 A<br>AU 2014248640 A<br>KR 10-2015-0129697 A<br>CN 105189546 A<br>MX 2015011367 A | |
| US 2009/0149449 A1 | 11 Jun. 2009 | GB 416511 A<br>WO 2005/040170 A2<br>EP 1675857 A1<br>CA 2543318 A<br>AU 2004284075 A | |
| JP 2015-534996 A | 07 Dec. 2015 | US 2015/0258210 A1<br>claims, examples 1-41<br>WO 2014/065661 A1<br>EP 2911699 A1<br>CN 105142672 A<br>RS 56953 B | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013173496 A
- WO 2014130879 A
- WO 2017004330 A
- WO 2017004025 A
- WO 2017020972 A
- WO 2016036804 A
- WO 2015095124 A
- WO 2015052322 A
- WO 2015052534 A
- WO 2016115191 A
- WO 2015052321 A
- WO 2015031693 A
- WO 2011130613 A
- WO 2005040170 A
- WO 2017137556 A
- WO 2009087978 A
- WO 2011057788 A
- WO 2015069794 A
- WO 2017096163 A
- WO 8807089 A
- WO 9428027 A
- WO 9429351 A
- WO 201315206 A
- WO 2011012309 A
- WO 9007861 A
- WO 2012075581 A
- WO 2011084496 A
- US 20180501692 A
- WO 2013154206 A
- WO 9201047 A
- WO 9220791 A
- WO 9306213 A
- WO 9311236 A
- WO 9319172 A
- WO 9501438 A
- WO 9515388 A
- WO 199954342 A
- WO 200061739 A
- WO 200231140 A
- WO 2007133855 A
- WO 2013120066 A
- US 2016361436 A
- WO 20110278681 A
- WO 2017010559 A
- WO 2003038043 A
- US 5821337 A
- WO 2015046505 A
- WO 2003074566 A
- WO 2015098099 A
- WO 2015146132 A
- WO 2012087596 A
- US 20150283262 A
- WO 2011130598 A
- WO 2016149546 A


### Non-patent literature cited in the description


- **JULIA MANTAJ.** *Angewandte Chemie Internationl Edition,* 2016, vol. 55, 2-29
- **DYEISON ANTONOW.** *Chemical Reviews,* 2010, vol. 111, 2815-2864
- Antibiotics. Springer Verlag, vol. III, 3-11
- *Accounts of Chemical Research,* 1986, vol. 19, 230
- *Journal of the American Chemical Society,* 1992, vol. 114, 4939
- *Journal of Organic Chemistry,* 1996, vol. 61, 8141
- *BMC Cancer,* 2006, vol. 6, 186
- *Histopatholody,* 2012, vol. 61, 1043-1056
- *Int J Cancer,* 2014, vol. 135, 2206-2214
- *J Membrane Biol,* 2004, vol. 199, 29-38
- *14th Annu Meet Cancer Immunother (CIMT),* 10 May 2016
- *Proc. Natl. Acad. Sci. U.S.A.,* 15 May 1969, vol. 63 (1), 78-85
- *Nature,* 1986, vol. 321, 522-525
- **ALTSCHUL, STEPHEN F. ; THOMAS L.MADDEN ; ALEJANDRO A. SCHAAFFER ; JINGHUI ZHANG ; ZHENG ZHANG ; WEBB MILLER ; DAVID J.LIPMAN.** Gapped BLAST and PSI-BLAST: a new generation of protein database search programs. *Nucleic Acids Res,* 1997, vol. 25, 3389-3402
- *Nature Genetics,* 1997, vol. 16, 133-143
- *Nucl. Acids Res.,* 1998, vol. 26, 3447-3448
- Animal Cell Technology: Basic and Applied Aspects. Kluwer Academic Publishers, 1999, vol. 10, 69-73
- *Proc. Natl. Acad. Sci. USA,* 2000, vol. 97, 722-727
- *Investigative Ophthalmology & Visual Science,* 2002, vol. 43 (7), 2301-2308

- *Briefings in Functional Genomics and Proteomics,* 2002, vol. 1 (2), 189-203
- *Ophthalmology,* 2002, vol. 109 (3), 427-431
- *Annu. Rev. Immunol,* 1994, vol. 12, 433-455
- *Nature Biotechnology,* 2005, vol. 23 (9), 1105-1116
- *Cell,* 1981, vol. 23, 175-182
- *Proc. Natl. Acad. Sci. U.S.A.,* 1980, vol. 77, 4126-4220
- *Journal of Chromatography A,* 1995, vol. 705, 129-134
- *Analytical Biochemistry,* 2007, vol. 360, 75-83
- Strategies for Protein Purification and Characterization: A Laboratory Course Manual. Cold Spring Harbor Laboratory Press, 1996
- Antibodies: A Laboratory Manual. Cold Spring Harbor Laboratory, 1988
- *ACS Chemical Biology,* 2012, vol. 7, 110
- *ACS Medicinal Chemistry Letters,* 2016, vol. 7, 1005
- *Bioconjugate Chemistry,* 2015, vol. 26, 2233
- *Angew. Chem. Int. Ed.,* 2016, vol. 55, 2361-2367
- *Biotechnol. Prog.,* 2012, vol. 28, 608-622
- *Anal. Chem.,* 2013, vol. 85, 715-736
- **EDELMAN et al.** *Proc. Natl. Acad. Sci. U.S.A.,* 15 May 1969, vol. 63, 78-85
- *J.Org.Chem.,* 2009, vol. 74 (5), 2210-2212
- *JACS,* 2012, vol. 134, 12308-12318
- *JACS,* 2004, vol. 126, 15046-15047
- *Protein Science,* 1995, vol. 4, 2411-2423
- *Med. Chem. Lett.,* 2016, vol. 7, 983-987
- *Journal of Medicinal Chemistry,* 2004, vol. 47, 1161
- *Tetrahedron,* 1995, vol. 51, 5617
- *Tetrahedron Letters,* 2012, vol. 53, 3847
- **YAMAGUCHI, M. et al.** *Biochemical and Biophysical Research Communications,* 2014, vol. 454, 600-603
- *Methods in Enzymology,* 1991, vol. 203, 121-153
- *Proc. Natl. Acad. Sci. USA,* 1989, vol. 86, 10029-10033
- **KABAT et al.** Sequences of Proteins of Immunological Interest. Public Health Service National Institutes of Health, 1991
- **QUEEN et al.** *Proc. Natl. Acad. Sci. USA,* 1989, vol. 86, 10029-10033
- **DR. JUNICHI KUREBAYASHI.** *British Journal of Cancer,* 1999, vol. 79 (5-6), 707-717

SEQUENCE LISTING

<110> DAIICHI SANKYO COMPANY, LIMITED

<120> Antibody-Pyrrolobenzodiazepine Derivative Conjugate

<130> SAP-859-PCT

<141> 2020-03-24

<150> JP 2019057041
<151> 2019-03-25

<160> 77

<170> PatentIn version 3.5

<210> 1
<211> 220
<212> PRT
<213> Homo sapiens

<220>
<221> MISC_FEATURE
<223> amino acid sequence of human CLDN6

<400> 1

```
Met Ala Ser Ala Gly Met Gln Ile Leu Gly Val Val Leu Thr Leu Leu
1               5                   10                  15

Gly Trp Val Asn Gly Leu Val Ser Cys Ala Leu Pro Met Trp Lys Val
            20                  25                  30

Thr Ala Phe Ile Gly Asn Ser Ile Val Val Ala Gln Val Val Trp Glu
        35                  40                  45

Gly Leu Trp Met Ser Cys Val Val Gln Ser Thr Gly Gln Met Gln Cys
    50                  55                  60

Lys Val Tyr Asp Ser Leu Leu Ala Leu Pro Gln Asp Leu Gln Ala Ala
65                  70                  75                  80

Arg Ala Leu Cys Val Ile Ala Leu Leu Val Ala Leu Phe Gly Leu Leu
                85                  90                  95

Val Tyr Leu Ala Gly Ala Lys Cys Thr Thr Cys Val Glu Glu Lys Asp
            100                 105                 110

Ser Lys Ala Arg Leu Val Leu Thr Ser Gly Ile Val Phe Val Ile Ser
        115                 120                 125

Gly Val Leu Thr Leu Ile Pro Val Cys Trp Thr Ala His Ala Ile Ile
    130                 135                 140
```

```
Arg Asp Phe Tyr Asn Pro Leu Val Ala Glu Ala Gln Lys Arg Glu Leu
145                 150                 155                 160

Gly Ala Ser Leu Tyr Leu Gly Trp Ala Ala Ser Gly Leu Leu Leu Leu
                165                 170                 175

Gly Gly Gly Leu Leu Cys Cys Thr Cys Pro Ser Gly Gly Ser Gln Gly
            180                 185                 190

Pro Ser His Tyr Met Ala Arg Tyr Ser Thr Ser Ala Pro Ala Ile Ser
        195                 200                 205

Arg Gly Pro Ser Glu Tyr Pro Thr Lys Asn Tyr Val
    210                 215                 220
```

<210> 2
<211> 663
<212> DNA
<213> Homo sapiens

<220>
<221> misc_feature
<223> nucleotide sequence of cDNA encoding amino acid sequence of human CLDN6

<400> 2

```
atggcctctg ccggaatgca gatcctggga gtcgtcctga cactgctggg ctgggtgaat       60

ggcctggtct cctgtgccct gcccatgtgg aaggtgaccg ctttcatcgg caacagcatc      120

gtggtggccc aggtggtgtg ggagggcctg tggatgtcct gcgtggtgca gagcaccggc      180

cagatgcagt gcaaggtgta cgactcactg ctggcgctgc cacaggacct gcaggctgca      240

cgtgccctct gtgtcatcgc cctccttgtg gccctgttcg gcttgctggt ctaccttgct      300

ggggccaagt gtaccacctg tgtggaggag aaggattcca aggcccgcct ggtgctcacc      360

tctgggattg tctttgtcat ctcaggggtc ctgacgctaa tccccgtgtg ctggacggcg      420

catgccatca tccgggactt ctataacccc ctggtggctg aggcccaaaa gcgggagctg      480

ggggcctccc tctacttggg ctgggcggcc tcaggccttt tgttgctggg tggggggttg      540

ctgtgctgca cttgcccctc gggggggtcc cagggcccca gccattacat ggcccgctac      600

tcaacatctg cccctgccat ctctcggggg ccctctgagt accctaccaa gaattacgtc      660

tga                                                                    663
```

<210> 3
<211> 217
<212> PRT
<213> Homo sapiens

<220>
<221> MISC_FEATURE
<223> amino acid sequence of human CLDN9

<400> 3

Met Ala Ser Thr Gly Leu Glu Leu Leu Gly Met Thr Leu Ala Val Leu
1 5 10 15

Gly Trp Leu Gly Thr Leu Val Ser Cys Ala Leu Pro Leu Trp Lys Val
20 25 30

Thr Ala Phe Ile Gly Asn Ser Ile Val Val Ala Gln Val Val Trp Glu
35 40 45

Gly Leu Trp Met Ser Cys Val Val Gln Ser Thr Gly Gln Met Gln Cys
50 55 60

Lys Val Tyr Asp Ser Leu Leu Ala Leu Pro Gln Asp Leu Gln Ala Ala
65 70 75 80

Arg Ala Leu Cys Val Ile Ala Leu Leu Leu Ala Leu Leu Gly Leu Leu
85 90 95

Val Ala Ile Thr Gly Ala Gln Cys Thr Thr Cys Val Glu Asp Glu Gly
100 105 110

Ala Lys Ala Arg Ile Val Leu Thr Ala Gly Val Ile Leu Leu Leu Ala
115 120 125

Gly Ile Leu Val Leu Ile Pro Val Cys Trp Thr Ala His Ala Ile Ile
130 135 140

Gln Asp Phe Tyr Asn Pro Leu Val Ala Glu Ala Leu Lys Arg Glu Leu
145 150 155 160

Gly Ala Ser Leu Tyr Leu Gly Trp Ala Ala Ala Ala Leu Leu Met Leu
165 170 175

Gly Gly Gly Leu Leu Cys Cys Thr Cys Pro Pro Pro Gln Val Glu Arg
180 185 190

Pro Arg Gly Pro Arg Leu Gly Tyr Ser Ile Pro Ser Arg Ser Gly Ala
195 200 205

Ser Gly Leu Asp Lys Arg Asp Tyr Val
210 215

<210> 4
<211> 654
<212> DNA
<213> Homo sapiens

<220>
<221> misc_feature
<223> nucleotide sequence of cDNA encoding amino acid sequence of human CLDN9

<400> 4
atggcttcga ccggcttaga actgctgggc atgaccctgg ctgtgctggg ctggctgggg 60

accctggtgt cctgcgccct gcccctgtgg aaggtgaccg ccttcatcgg caacagcatc 120

gtggtggccc aggtggtgtg ggagggcctg tggatgtcct gcgtggtgca gagcacgggc 180

cagatgcagt gcaaggtgta cgactcactg ctggctctgc cgcaggacct gcaggccgca 240

cgtgccctct gtgtcattgc cctcctgctg gccctgcttg gcctcctggt ggccatcaca 300

ggtgcccagt gtaccacgtg tgtggaggac gaaggtgcca aggcccgtat cgtgctcacc 360

gcgggggtca tcctcctcct cgccggcatc ctggtgctca tccctgtgtg ctggacggcg 420

cacgccatca tccaggactt ctacaacccc ctggtggctg aggccctcaa gcgggagctg 480

ggggcctccc tctacctggg ctgggcggcg gctgcactgc ttatgctggg cggggggctc 540

ctctgctgca cgtgcccccc gccccaggtc gagcggcccc gcggacctcg gctgggctac 600

tccatcccct cccgctcggg tgcatctgga ctggacaaga gggactacgt gtga 654

<210> 5
<211> 11
<212> PRT
<213> Mus musculus

<220>
<221> MISC_FEATURE
<223> amino acid sequence of CDRL1 of B1 antibody light chain

<400> 5

Arg Ala Ser Gln Asp Ile Asn Asn Tyr Leu Asn
1 5 10

<210> 6
<211> 7
<212> PRT
<213> Mus musculus

<220>
<221> MISC_FEATURE
<223> amino acid sequence of CDRL2 of B1 antibody light chain

<400> 6

Phe Thr Ser Arg Leu His Ser
1 5

<210> 7
<211> 9
<212> PRT
<213> Mus musculus

<220>
<221> MISC_FEATURE
<223> amino acid sequence of CDRL3 of B1 antibody light chain

<400> 7

Gln Gln Gly Tyr Pro Leu Pro Trp Thr
1 5

<210> 8
<211> 9
<212> PRT
<213> Artificial Sequence

<220>
<223> humanized antibody

<220>
<221> MISC_FEATURE
<223> amino acid sequence of CDRL3 of humanized B1 antibody light chain L4

<400> 8

Gln Gln Gly Asn Thr Leu Pro Trp Thr
1 5

<210> 9
<211> 10
<212> PRT
<213> Mus musculus

<220>
<221> MISC_FEATURE
<223> amino acid sequence of CDRH1 of B1 antibody heavy chain

<400> 9

Gly Tyr Thr Phe Thr Glu Tyr Thr Met His
1 5 10

<210> 10
<211> 10
<212> PRT
<213> Mus musculus

<220>
<221> MISC_FEATURE
<223> amino acid sequence of CDRH2 of B1 antibody heavy chain

<400> 10

Gly Val Asn Pro Asn Ser Gly Asp Thr Ser
1 5 10

<210> 11
<211> 13
<212> PRT
<213> Mus musculus

<220>
<221> MISC_FEATURE
<223> amino acid sequence of CDRH3 of B1 antibody heavy chain

<400> 11

Pro Gly Gly Tyr Asp Val Gly Tyr Tyr Ala Met Asp Tyr
1 5 10

<210> 12
<211> 11
<212> PRT
<213> Mus musculus

<220>
<221> MISC_FEATURE
<223> amino acid sequence of CDRL1 of C7 antibody light chain

<400> 12

Arg Ala Ser Gln Asp Ile Asn Asn Tyr Leu Asn
1 5 10

<210> 13
<211> 7
<212> PRT
<213> Mus musculus

<220>
<221> MISC_FEATURE
<223> amino acid sequence of CDRL2 of C7 antibody light chain

<400> 13

Ser Thr Ser Arg Leu His Ser
1 5

<210> 14
<211> 9
<212> PRT
<213> Mus musculus

<220>
<221> MISC_FEATURE
<223> amino acid sequence of CDRL3 of C7 antibody light chain

<400> 14

Gln Gln Gly Tyr Pro Leu Pro Trp Thr
1 5

<210> 15
<211> 10
<212> PRT
<213> Mus musculus

<220>
<221> MISC_FEATURE
<223> amino acid sequence of CDRH1 of C7 antibody heavy chain

<400> 15

Gly Tyr Thr Phe Thr Glu Tyr Thr Met His
1 5 10

<210> 16
<211> 10
<212> PRT
<213> Mus musculus

<220>
<221> MISC_FEATURE
<223> amino acid sequence of CDRH2 of C7 antibody heavy chain

<400> 16

Gly Val Asn Pro Asn Ser Gly Asp Thr Ser
1 5 10

<210> 17
<211> 13
<212> PRT
<213> Mus musculus

<220>
<221> MISC_FEATURE
<223> amino acid sequence of CDRH3 of C7 antibody heavy chain

<400> 17

Pro Gly Gly Tyr Asp Val Gly Tyr Tyr Ala Met Asp Tyr
1 5 10

<210> 18
<211> 321
<212> DNA
<213> Mus musculus

<220>
<221> misc_feature
<223> nucleotide sequence of cDNA encoding variable region of B1 antibody light chain

<400> 18
gatatccaga tgacacagac tgcatcctcc ctgtctgcct ctcttggaga cagagtcacc 60

atcagttgca gggcaagtca ggacattaac aattatttaa actggtatca gcagaaacca 120

gatggaactg ttaaactcct gatctacttc acatcaagat tacactcagg agtcccatca 180

aggttcagtg gcagtgggtc tggaacacat tattctctca ccattactaa cctggaacaa 240

gaagatattg ccacttactt ttgccaacag ggttatccgc ttccgtggac gttcggtgga 300

ggcaccaaac tggaaatcaa a 321

<210> 19
<211> 107
<212> PRT
<213> Mus musculus

<220>
<221> MISC_FEATURE
<223> amino acid sequence of variable region of B1 antibody light chain

<400> 19

Asp Ile Gln Met Thr Gln Thr Ala Ser Ser Leu Ser Ala Ser Leu Gly
1 5 10 15

Asp Arg Val Thr Ile Ser Cys Arg Ala Ser Gln Asp Ile Asn Asn Tyr
20 25 30

Leu Asn Trp Tyr Gln Gln Lys Pro Asp Gly Thr Val Lys Leu Leu Ile
35 40 45

Tyr Phe Thr Ser Arg Leu His Ser Gly Val Pro Ser Arg Phe Ser Gly
50 55 60

Ser Gly Ser Gly Thr His Tyr Ser Leu Thr Ile Thr Asn Leu Glu Gln
65 70 75 80

Glu Asp Ile Ala Thr Tyr Phe Cys Gln Gln Gly Tyr Pro Leu Pro Trp
85 90 95

Thr Phe Gly Gly Gly Thr Lys Leu Glu Ile Lys
100 105

<210> 20
<211> 366

<212> DNA
<213> Mus musculus

<220>
<221> misc_feature
<223> nucleotide sequence of cDNA encoding variable region of B1 antibody heavy chain

<400> 20
gaggtccagc ttcaacagtc tggacctgaa ctggtgaagc ctggggcttc agtgaagata 60

tcctgcaaga cttctggata cacattcact gaatacacca tgcactgggt gcagcagagc 120

catggaaaga gccttgagtg gattggaggt gttaatccta atagtggtga tactagctac 180

aaccagaagt tcaagggcaa ggccacattg actgttgaca agtcctccag cacagcctac 240

atggagctcc gcagcctgac atctgaggat tctgcagtct attactgtgc aagacccggg 300

gggtacgacg tgggttacta tgctatggac tactggggtc aaggaacctc agtcaccgtc 360

tcctca 366

<210> 21
<211> 122
<212> PRT
<213> Mus musculus

<220>
<221> MISC_FEATURE
<223> amino acid sequence of variable region of B1 antibody heavy chain

<400> 21

Glu Val Gln Leu Gln Gln Ser Gly Pro Glu Leu Val Lys Pro Gly Ala
1 5 10 15

Ser Val Lys Ile Ser Cys Lys Thr Ser Gly Tyr Thr Phe Thr Glu Tyr
20 25 30

Thr Met His Trp Val Gln Gln Ser His Gly Lys Ser Leu Glu Trp Ile
35 40 45

Gly Gly Val Asn Pro Asn Ser Gly Asp Thr Ser Tyr Asn Gln Lys Phe
50 55 60

Lys Gly Lys Ala Thr Leu Thr Val Asp Lys Ser Ser Ser Thr Ala Tyr
65 70 75 80

Met Glu Leu Arg Ser Leu Thr Ser Glu Asp Ser Ala Val Tyr Tyr Cys
85 90 95

Ala Arg Pro Gly Gly Tyr Asp Val Gly Tyr Tyr Ala Met Asp Tyr Trp
100 105 110

```
Gly Gln Gly Thr Ser Val Thr Val Ser Ser
        115                 120


<210>  22
<211>  321
<212>  DNA
<213>  Mus musculus


<220>
<221>  misc_feature
<223>  nucleotide sequence of cDNA encoding variable region of C7
       antibody light chain

<400>  22
gatatccaga tgacacagac tgcatcctcc ctgtctgcct ctcttggaga cagagtcacc      60

atcagttgca gggcaagtca ggacattaac aattatttaa actggtatca gcagaaacca     120

gatggaactg ttaaactcct gatctactcc acatcaagat tacactcagg agtcccatca     180

aggttcagtg gcagtgggtc tggaacacat tattctctca ccattactca cctggaacaa     240

gaagatattg ccacttactt ttgccaacag ggttatccgc ttccgtggac gttcggtgga     300

ggcaccaaac tggaaatcaa a                                               321


<210>  23
<211>  107
<212>  PRT
<213>  Mus musculus


<220>
<221>  MISC_FEATURE
<223>  amino acid sequence of variable region of C7 antibody light chain

<400>  23

Asp Ile Gln Met Thr Gln Thr Ala Ser Ser Leu Ser Ala Ser Leu Gly
1               5                   10                  15


Asp Arg Val Thr Ile Ser Cys Arg Ala Ser Gln Asp Ile Asn Asn Tyr
            20                  25                  30


Leu Asn Trp Tyr Gln Gln Lys Pro Asp Gly Thr Val Lys Leu Leu Ile
        35                  40                  45


Tyr Ser Thr Ser Arg Leu His Ser Gly Val Pro Ser Arg Phe Ser Gly
    50                  55                  60


Ser Gly Ser Gly Thr His Tyr Ser Leu Thr Ile Thr His Leu Glu Gln
65                  70                  75                  80
```

Glu Asp Ile Ala Thr Tyr Phe Cys Gln Gln Gly Tyr Pro Leu Pro Trp
85 90 95

Thr Phe Gly Gly Gly Thr Lys Leu Glu Ile Lys
100 105

<210> 24
<211> 366
<212> DNA
<213> Mus musculus

<220>
<221> misc_feature
<223> nucleotide sequence of cDNA encoding variable region of C7 antibody heavy chain

<400> 24
gaggtccagc ttcaacagtc tggacctgaa ctggtgaagc ctggggcttc agtgaagata 60

tcctgcaaga cttctggata cacattcact gaatacacca tgcactgggt gcagcagagc 120

catggaaaga gccttgagtg gattggaggt gttaatccta atagtggtga tactagctac 180

aaccagaagt tcaagggcaa ggccacattg actgttgaca agtcctccag cacagcctac 240

atggagctcc gcagcctgac atctgaggat tctgcagtct attactgtgc aagacccggg 300

gggtacgacg tgggttacta tgctatggac tactggggtc aaggaacctc agtcaccgtc 360

tcctca 366

<210> 25
<211> 122
<212> PRT
<213> Mus musculus

<220>
<221> MISC_FEATURE
<223> amino acid sequence of variable region of C7 antibody heavy chain

<400> 25

Glu Val Gln Leu Gln Gln Ser Gly Pro Glu Leu Val Lys Pro Gly Ala
1 5 10 15

Ser Val Lys Ile Ser Cys Lys Thr Ser Gly Tyr Thr Phe Thr Glu Tyr
20 25 30

Thr Met His Trp Val Gln Gln Ser His Gly Lys Ser Leu Glu Trp Ile
35 40 45

Gly Gly Val Asn Pro Asn Ser Gly Asp Thr Ser Tyr Asn Gln Lys Phe
50 55 60

Lys Gly Lys Ala Thr Leu Thr Val Asp Lys Ser Ser Ser Thr Ala Tyr
65 70 75 80

Met Glu Leu Arg Ser Leu Thr Ser Glu Asp Ser Ala Val Tyr Tyr Cys
85 90 95

Ala Arg Pro Gly Gly Tyr Asp Val Gly Tyr Tyr Ala Met Asp Tyr Trp
100 105 110

Gly Gln Gly Thr Ser Val Thr Val Ser Ser
115 120

<210> 26
<211> 449
<212> DNA
<213> Homo sapiens

<220>
<221> misc_feature
<223> DNA fragment comprising DNA sequence encoding human light chain signal sequence and human \203È chain constant region

<400> 26
gcctccggac tctagagcca ccatggtgct gcagacccag gtgttcatct ccctgctgct 60

gtggatctcc ggcgcgtacg gcgatatcgt gatgattaaa cgtacggtgg ccgccccctc 120

cgtgttcatc ttcccccct ccgacgagca gctgaagtcc ggcaccgcct ccgtggtgtg 180

cctgctgaat aacttctacc ccagagaggc caaggtgcag tggaaggtgg acaacgccct 240

gcagtccggg aactcccagg agagcgtgac cgagcaggac agcaaggaca gcacctacag 300

cctgagcagc accctgaccc tgagcaaagc cgactacgag aagcacaagg tgtacgcctg 360

cgaggtgacc caccagggcc tgagctcccc cgtcaccaag agcttcaaca gggggagtg 420

ttaggggccc gtttaaacgg gggaggcta 449

<210> 27
<211> 1137
<212> DNA
<213> Homo sapiens

<220>
<221> misc_feature
<223> DNA fragment comprising DNA sequence encoding human heavy chain signal sequence and human IgG1 LALA constant region

<400> 27
ccagcctccg gactctagag ccaccatgaa acacctgtgg ttcttcctcc tgctggtggc 60

agctcccaga tgggtgctga gccaggtgca attgtgcagg cggttagctc agcctccacc 120

aagggcccaa gcgtcttccc cctggcaccc tcctccaaga gcacctctgg cggcacagcc 180

gccctgggct gcctggtcaa ggactacttc cccgaacccg tgaccgtgag ctggaactca 240

ggcgccctga ccagcggcgt gcacaccttc cccgctgtcc tgcagtcctc aggactctac 300

tccctcagca gcgtggtgac cgtgccctcc agcagcttgg gcacccagac ctacatctgc 360

aacgtgaatc acaagcccag caacaccaag gtggacaaga gagttgagcc caaatcttgt 420

gacaaaactc acacatgccc accctgccca gcacctgaag ccgcgggggg accctcagtc 480

ttcctcttcc ccccaaaacc caaggacacc ctcatgatct cccggacccc tgaggtcaca 540

tgcgtggtgg tggacgtgag ccacgaagac cctgaggtca agttcaactg gtacgtggac 600

ggcgtggagg tgcataatgc caagacaaag ccccgggagg agcagtacaa cagcacgtac 660

cgggtggtca gcgtcctcac cgtcctgcac caggactggc tgaatggcaa ggagtacaag 720

tgcaaggtct ccaacaaagc cctcccagcc cccatcgaga aaaccatctc caaagccaaa 780

ggccagcccc gggaaccaca ggtgtacacc ctgcccccat cccgggagga gatgaccaag 840

aaccaggtca gcctgacctg cctggtcaaa ggcttctatc ccagcgacat cgccgtggag 900

tgggagagca atggccagcc cgagaacaac tacaagacca cccctcccgt gctggactcc 960

gacggctcct tcttcctcta cagcaagctc accgtggaca agagcaggtg gcagcagggc 1020

aacgtcttct catgctccgt gatgcatgag gctctgcaca accactacac ccagaagagc 1080

ctctccctgt ctcccggcaa atgagatatc gggcccgttt aaacggggga ggctaac 1137

<210> 28
<211> 234
<212> PRT
<213> Artificial Sequence

<220>
<223> chimeric antibody

<220>
<221> MISC_FEATURE
<223> amino acid sequence of chB1 light chain

<400> 28

Met Val Leu Gln Thr Gln Val Phe Ile Ser Leu Leu Leu Trp Ile Ser
1 5 10 15

Gly Ala Tyr Gly Asp Ile Gln Met Thr Gln Thr Ala Ser Ser Leu Ser
20 25 30

Ala Ser Leu Gly Asp Arg Val Thr Ile Ser Cys Arg Ala Ser Gln Asp
35 40 45

Ile Asn Asn Tyr Leu Asn Trp Tyr Gln Gln Lys Pro Asp Gly Thr Val
50 55 60

```
Lys Leu Leu Ile Tyr Phe Thr Ser Arg Leu His Ser Gly Val Pro Ser
65                  70                  75                  80

Arg Phe Ser Gly Ser Gly Ser Gly Thr His Tyr Ser Leu Thr Ile Thr
                85                  90                  95

Asn Leu Glu Gln Glu Asp Ile Ala Thr Tyr Phe Cys Gln Gln Gly Tyr
            100                 105                 110

Pro Leu Pro Trp Thr Phe Gly Gly Gly Thr Lys Leu Glu Ile Lys Arg
        115                 120                 125

Ala Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230


<210>  29
<211>  752
<212>  DNA
<213>  Artificial Sequence

<220>
<223>  chimeric antibody


<220>
<221>  misc_feature
<223>  DNA fragment comprising DNA sequence encoding amino acid sequence
       of chB1 light chain

<400>  29
ccagcctccg gactctagag ccaccatggt gctgcagacc caggtgttca tcagcctgct       60

gctgtggatc agcggcgcct acggcgacat ccagatgacc cagacagcca gcagcctgag      120
```

cgccagcctg ggcgatagag tgaccatcag ctgcagagcc agccaggaca tcaacaacta 180

cctgaactgg tatcagcaga aacccgacgg caccgtgaag ctgctgatct acttcaccag 240

cagactgcac agcggcgtgc ccagcagatt ttctggcagc ggctctggca cccactacag 300

cctgaccatc accaacctgg aacaggaaga tatcgctacc tacttctgtc agcaaggcta 360

ccccctgccc tggacctttg gcggcggaac aaagctggaa atcaagcggg ccgtggccgc 420

tccctccgtg ttcatctttc cacccagcga cgagcagctg aagtccggca cagctagcgt 480

cgtgtgcctg ctgaacaact tctacccccg cgaggccaag gtgcagtgga aggtggacaa 540

tgccctgcag agcggcaaca gccaggaaag cgtgaccgag caggacagca aggactccac 600

ctactccctg agcagcaccc tgaccctgag caaggccgac tacgagaagc acaaggtgta 660

cgcctgcgaa gtgacccacc agggcctgtc tagccccgtg accaagagct tcaaccgggg 720

cgagtgttga gtttaaacgg gggaggctaa ct 752

<210> 30
<211> 107
<212> PRT
<213> Artificial Sequence

<220>
<223> chimeric antibody

<220>
<221> MISC_FEATURE
<223> amino acid sequence of variable region of chB1 light chain

<400> 30

```
Asp Ile Gln Met Thr Gln Thr Ala Ser Ser Leu Ser Ala Ser Leu Gly
1               5                   10                  15

Asp Arg Val Thr Ile Ser Cys Arg Ala Ser Gln Asp Ile Asn Asn Tyr
            20                  25                  30

Leu Asn Trp Tyr Gln Gln Lys Pro Asp Gly Thr Val Lys Leu Leu Ile
        35                  40                  45

Tyr Phe Thr Ser Arg Leu His Ser Gly Val Pro Ser Arg Phe Ser Gly
    50                  55                  60

Ser Gly Ser Gly Thr His Tyr Ser Leu Thr Ile Thr Asn Leu Glu Gln
65                  70                  75                  80

Glu Asp Ile Ala Thr Tyr Phe Cys Gln Gln Gly Tyr Pro Leu Pro Trp
                85                  90                  95
```

```
Thr Phe Gly Gly Gly Thr Lys Leu Glu Ile Lys
            100                 105


<210>  31
<211>  321
<212>  DNA
<213>  Artificial Sequence

<220>
<223>  chimeric antibody


<220>
<221>  misc_feature
<223>  nucleotide sequence encoding amino acid sequence of variable
       region of chB1 light chain

<400>  31
gacatccaga tgacccagac agccagcagc ctgagcgcca gcctgggcga tagagtgacc        60

atcagctgca gagccagcca ggacatcaac aactacctga actggtatca gcagaaaccc       120

gacggcaccg tgaagctgct gatctacttc accagcagac tgcacagcgg cgtgcccagc       180

agattttctg gcagcggctc tggcacccac tacagcctga ccatcaccaa cctggaacag       240

gaagatatcg ctacctactt ctgtcagcaa ggctaccccc tgccctggac ctttggcggc       300

ggaacaaagc tggaaatcaa g                                                  321


<210>  32
<211>  471
<212>  PRT
<213>  Artificial Sequence

<220>
<223>  chimeric antibody


<220>
<221>  MISC_FEATURE
<223>  amino acid sequence of chB1 heavy chain

<400>  32

Met Lys His Leu Trp Phe Phe Leu Leu Leu Val Ala Ala Pro Arg Trp
1               5                   10                  15


Val Leu Ser Glu Val Gln Leu Gln Gln Ser Gly Pro Glu Leu Val Lys
            20                  25                  30


Pro Gly Ala Ser Val Lys Ile Ser Cys Lys Thr Ser Gly Tyr Thr Phe
        35                  40                  45


Thr Glu Tyr Thr Met His Trp Val Gln Gln Ser His Gly Lys Ser Leu
    50                  55                  60
```

```
Glu Trp Ile Gly Gly Val Asn Pro Asn Ser Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Glu Leu Arg Ser Leu Thr Ser Glu Asp Ser Ala Val
            100                 105                 110

Tyr Tyr Cys Ala Arg Pro Gly Gly Tyr Asp Val Gly Tyr Tyr Ala Met
        115                 120                 125

Asp Tyr Trp Gly Gln Gly Thr Ser Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Ala Ala Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320
```

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 33
<211> 1413
<212> DNA
<213> Artificial Sequence

<220>
<223> chimeric antibody

<220>
<221> misc_feature
<223> nucleotide sequence encoding chB1 heavy chain

<400> 33
atgaaacacc tgtggttctt cctcctgctg gtggcagctc ccagatgggt gctgagcgaa 60

gtgcagctgc agcagtctgg ccccgagctc gtgaaacctg gcgcctccgt gaagatcagc 120

tgcaagacca gcggctacac cttcaccgag tacaccatgc actgggtgca gcagagccac 180

ggcaagagcc tggaatggat cggcggcgtg aaccccaaca gcggcgacac cagctacaac 240

cagaagttca agggcaaggc caccctgacc gtggacaaga gcagcagcac cgcctacatg 300

gaactgcgga gcctgaccag cgaggacagc gccgtgtact actgtgccag acctggcggc 360

tacgacgtgg gctactacgc catggattac tggggccagg gcaccagcgt gaccgtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga agccgcgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 34
<211> 122
<212> PRT
<213> Artificial Sequence

<220>
<223> chimeric antibody

<220>
<221> MISC_FEATURE
<223> amino acid sequence of variable region of chB1 heavy chain

<400> 34

Glu Val Gln Leu Gln Gln Ser Gly Pro Glu Leu Val Lys Pro Gly Ala
1 5 10 15

Ser Val Lys Ile Ser Cys Lys Thr Ser Gly Tyr Thr Phe Thr Glu Tyr
20 25 30

Thr Met His Trp Val Gln Gln Ser His Gly Lys Ser Leu Glu Trp Ile
35 40 45

Gly Gly Val Asn Pro Asn Ser Gly Asp Thr Ser Tyr Asn Gln Lys Phe
50 55 60

Lys Gly Lys Ala Thr Leu Thr Val Asp Lys Ser Ser Ser Thr Ala Tyr
65 70 75 80

Met Glu Leu Arg Ser Leu Thr Ser Glu Asp Ser Ala Val Tyr Tyr Cys
85 90 95

Ala Arg Pro Gly Gly Tyr Asp Val Gly Tyr Tyr Ala Met Asp Tyr Trp
100 105 110

Gly Gln Gly Thr Ser Val Thr Val Ser Ser
115 120

<210> 35
<211> 366
<212> DNA
<213> Artificial Sequence

<220>
<223> chimeric antibody

<220>
<221> misc_feature
<223> nucleotide sequence encoding variable region of chB1 heavy chain

<400> 35
gaagtgcagc tgcagcagtc tggccccgag ctcgtgaaac ctggcgcctc cgtgaagatc 60

agctgcaaga ccagcggcta caccttcacc gagtacacca tgcactgggt gcagcagagc 120

cacggcaaga gcctggaatg gatcggcggc gtgaacccca acagcggcga caccagctac 180

aaccagaagt tcaagggcaa ggccaccctg accgtggaca agagcagcag caccgcctac 240

atggaactgc ggagcctgac cagcgaggac agcgccgtgt actactgtgc cagacctggc 300

ggctacgacg tgggctacta cgccatggat tactggggcc agggcaccag cgtgaccgtc 360

agctca 366

<210> 36
<211> 234
<212> PRT
<213> Artificial Sequence

<220>
<223> humanized antibody

<220>
<221> MISC_FEATURE
<223> amino acid sequence of humanized antibody light chain hL1

<400> 36

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Arg Ala Ser Gln Asp
35 40 45

Ile Asn Asn Tyr Leu Asn Trp Tyr Gln Gln Lys Pro Gly Lys Ala Pro
50 55 60

Lys Leu Leu Ile Tyr Phe Thr Ser Arg Leu His Ser Gly Val Pro Ser
65 70 75 80

Arg Phe Ser Gly Ser Gly Ser Gly Thr Asp Tyr Thr Leu Thr Ile Ser
85 90 95

Ser Leu Gln Pro Glu Asp Phe Ala Thr Tyr Tyr Cys Gln Gln Gly Tyr
100 105 110

Pro Leu Pro Trp Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys

225                 230

<210>  37
<211>  702
<212>  DNA
<213>  Artificial Sequence

<220>
<223>  humanized antibody

<220>
<221>  misc_feature
<223>  nucleotide sequence encoding humanized antibody light chain hL1

<400>  37
atggtgctgc agacccaggt gttcatctcc ctgctgctgt ggatctccgg cgcgtacggc     60

gacatccaga tgacccagag ccctagcagc ctgagcgcca gcgtgggcga cagagtgacc    120

atcacctgta gagccagcca ggacatcaac aactacctga actggtatca gcagaagccc    180

ggcaaggccc ccaagctgct gatctacttc accagcagac tgcacagcgg cgtgcccagc    240

agattttctg gcagcggctc cggcaccgac tacaccctga caatcagcag cctgcagccc    300

gaggacttcg ccacctacta ctgccagcag ggctaccccc tgccttggac atttggccag    360

ggcaccaagg tggaaatcaa gcgtacggtg gccgccccct ccgtgttcat cttccccccc    420

tccgacgagc agctgaagtc cggcaccgcc tccgtggtgt gcctgctgaa taacttctac    480

cccagagagg ccaaggtgca gtggaaggtg gacaacgccc tgcagtccgg gaactcccag    540

gagagcgtga ccgagcagga cagcaaggac agcacctaca gcctgagcag caccctgacc    600

ctgagcaaag ccgactacga gaagcacaag gtgtacgcct gcgaggtgac ccaccagggc    660

ctgagctccc ccgtcaccaa gagcttcaac aggggggagt gt                        702

<210>  38
<211>  107
<212>  PRT
<213>  Artificial Sequence

<220>
<223>  humanized antibody

<220>
<221>  MISC_FEATURE
<223>  amino acid sequence of variable region of humanized antibody light chain hL1

<400>  38

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asn Asn Tyr

```
            20                  25                  30

Leu Asn Trp Tyr Gln Gln Lys Pro Gly Lys Ala Pro Lys Leu Leu Ile
        35                  40                  45

Tyr Phe Thr Ser Arg Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Tyr Pro Leu Pro Trp
                85                  90                  95

Thr Phe Gly Gln Gly Thr Lys Val Glu Ile Lys
            100                 105


<210>  39
<211>  321
<212>  DNA
<213>  Artificial Sequence

<220>
<223>  humanized antibody


<220>
<221>  misc_feature
<223>  nucleotide sequence encoding variable region of humanized
       antibody light chain hL1

<400>  39
gacatccaga tgacccagag ccctagcagc ctgagcgcca gcgtgggcga cagagtgacc        60

atcacctgta gagccagcca ggacatcaac aactacctga actggtatca gcagaagccc       120

ggcaaggccc ccaagctgct gatctacttc accagcagac tgcacagcgg cgtgcccagc       180

agatttctg gcagcggctc cggcaccgac tacaccctga caatcagcag cctgcagccc        240

gaggacttcg ccacctacta ctgccagcag ggctaccccc tgccttggac atttggccag       300

ggcaccaagg tggaaatcaa g                                                 321


<210>  40
<211>  234
<212>  PRT
<213>  Artificial Sequence

<220>
<223>  humanized antibody


<220>
<221>  MISC_FEATURE
<223>  amino acid sequence of humanized antibody light chain hL2
```

<400> 40

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Arg Ala Ser Gln Asp
35 40 45

Ile Asn Asn Tyr Leu Asn Trp Tyr Gln Gln Lys Pro Gly Lys Ala Val
50 55 60

Lys Leu Leu Ile Tyr Phe Thr Ser Arg Leu His Ser Gly Val Pro Ser
65 70 75 80

Arg Phe Ser Gly Ser Gly Ser Gly Thr His Tyr Thr Leu Thr Ile Ser
85 90 95

Ser Leu Gln Pro Glu Asp Phe Ala Thr Tyr Tyr Cys Gln Gln Gly Tyr
100 105 110

Pro Leu Pro Trp Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 41
<211> 702
<212> DNA
<213> Artificial Sequence

<220>
<223> humanized antibody

<220>
<221> misc_feature
<223> nucleotide sequence encoding humanized antibody light chain hL2

<400> 41
atggtgctgc agacccaggt gttcatctcc ctgctgctgt ggatctccgg cgcgtacggc 60

gacatccaga tgacccagag ccctagcagc ctgagcgcca gcgtgggcga cagagtgacc 120

atcacctgta gagccagcca ggacatcaac aactacctga actggtatca gcagaaaccc 180

ggcaaggccg tgaagctgct gatctacttc accagcagac tgcacagcgg cgtgcccagc 240

agattttctg gcagcggctc tggcacccac tacaccctga caatcagcag cctgcagccc 300

gaggacttcg ccacctacta ctgccagcag ggctaccccc tgccttggac atttggccag 360

ggcaccaagg tggaaatcaa gcgtacggtg gccgcccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 42
<211> 107
<212> PRT
<213> Artificial Sequence

<220>
<223> humanized antibody

<220>
<221> MISC_FEATURE
<223> amino acid sequence of variable region of humanized antibody light chain hL2

<400> 42

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Asn Asn Tyr
20 25 30

Leu Asn Trp Tyr Gln Gln Lys Pro Gly Lys Ala Val Lys Leu Leu Ile
35 40 45

Tyr Phe Thr Ser Arg Leu His Ser Gly Val Pro Ser Arg Phe Ser Gly
50 55 60

Ser Gly Ser Gly Thr His Tyr Thr Leu Thr Ile Ser Ser Leu Gln Pro
65 70 75 80

Glu Asp Phe Ala Thr Tyr Tyr Cys Gln Gln Gly Tyr Pro Leu Pro Trp
85 90 95

Thr Phe Gly Gln Gly Thr Lys Val Glu Ile Lys
100 105

<210> 43
<211> 321
<212> DNA
<213> Artificial Sequence

<220>
<223> humanized antibody

<220>
<221> misc_feature
<223> nucleotide sequence encoding variable region of humanized antibody light chain hL2

<400> 43
gacatccaga tgacccagag ccctagcagc ctgagcgcca gcgtgggcga cagagtgacc 60

atcacctgta gagccagcca ggacatcaac aactacctga actggtatca gcagaaaccc 120

ggcaaggccg tgaagctgct gatctacttc accagcagac tgcacagcgg cgtgcccagc 180

agattttctg gcagcggctc tggcacccac tacaccctga caatcagcag cctgcagccc 240

gaggacttcg ccacctacta ctgccagcag ggctaccccc tgccttggac atttggccag 300

ggcaccaagg tggaaatcaa g 321

<210> 44
<211> 234
<212> PRT
<213> Artificial Sequence

<220>
<223> humanized antibody

<220>
<221> MISC_FEATURE
<223> amino acid sequence of humanized antibody light chain hL3

<400> 44

```
Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Arg Ala Ser Gln Asp
        35                  40                  45

Ile Asn Asn Tyr Leu Asn Trp Tyr Gln Gln Lys Pro Gly Gly Ala Val
    50                  55                  60

Lys Leu Leu Ile Tyr Phe Thr Ser Arg Leu His Ser Gly Val Pro Ser
65                  70                  75                  80

Arg Phe Ser Gly Ser Gly Ser Gly Thr Asp Tyr Thr Leu Thr Ile Ser
                85                  90                  95

Ser Leu Gln Pro Glu Asp Phe Ala Thr Tyr Tyr Cys Gln Gln Gly Tyr
            100                 105                 110

Pro Leu Pro Trp Thr Phe Gly Gly Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 45
<211> 702
<212> DNA

<213> Artificial Sequence

<220>
<223> humanized antibody

<220>
<221> misc_feature
<223> nucleotide sequence encoding humanized antibody light chain hL3

<400> 45
atggtgctgc agacccaggt gttcatctcc ctgctgctgt ggatctccgg cgcgtacggc 60

gacatccaga tgacccagag ccctagcagc ctgagcgcca gcgtgggcga cagagtgacc 120

atcacctgta gagccagcca ggacatcaac aactacctga actggtatca gcagaaaccc 180

ggcggagccg tgaagctgct gatctacttc accagcagac tgcacagcgg cgtgcccagc 240

agattttctg gcagcggctc cggcaccgac tacaccctga caatcagcag cctgcagccc 300

gaggacttcg ccacctacta ctgccagcag ggctaccccc tgccctggac atttggcggc 360

ggaacaaagg tggaaatcaa gcgtacggtg gccgcccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 46
<211> 107
<212> PRT
<213> Artificial Sequence

<220>
<223> humanized antibody

<220>
<221> MISC_FEATURE
<223> amino acid sequence of variable region of humanized antibody light chain hL3

<400> 46

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asn Asn Tyr
            20                  25                  30

Leu Asn Trp Tyr Gln Gln Lys Pro Gly Gly Ala Val Lys Leu Leu Ile
        35                  40                  45

Tyr Phe Thr Ser Arg Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Tyr Pro Leu Pro Trp
                85                  90                  95

Thr Phe Gly Gly Gly Thr Lys Val Glu Ile Lys
            100                 105

<210> 47
<211> 321
<212> DNA
<213> Artificial Sequence

<220>
<223> humanized antibody

<220>
<221> misc_feature
<223> nucleotide sequence encoding variable region of humanized antibody light chain hL3

<400> 47
gacatccaga tgacccagag ccctagcagc ctgagcgcca gcgtgggcga cagagtgacc 60

atcacctgta gagccagcca ggacatcaac aactacctga actggtatca gcagaaaccc 120

ggcggagccg tgaagctgct gatctacttc accagcagac tgcacagcgg cgtgcccagc 180

agattttctg gcagcggctc cggcaccgac tacaccctga caatcagcag cctgcagccc 240

gaggacttcg ccacctacta ctgccagcag ggctaccccc tgccctggac atttggcggc 300

ggaacaaagg tggaaatcaa g 321

<210> 48
<211> 234
<212> PRT
<213> Artificial Sequence

<220>
<223> humanized antibody

<220>
<221> MISC_FEATURE
<223> amino acid sequence of humanized antibody light chain hL4

<400> 48

Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
20 25 30

Ala Ser Val Gly Asp Arg Val Thr Ile Thr Cys Arg Ala Ser Gln Asp
35 40 45

Ile Asn Asn Tyr Leu Asn Trp Tyr Gln Gln Lys Pro Gly Gly Ala Val
50 55 60

Lys Leu Leu Ile Tyr Phe Thr Ser Arg Leu His Ser Gly Val Pro Ser
65 70 75 80

Arg Phe Ser Gly Ser Gly Ser Gly Thr Asp Tyr Thr Leu Thr Ile Ser
85 90 95

Ser Leu Gln Pro Glu Asp Phe Ala Thr Tyr Tyr Cys Gln Gln Gly Asn
100 105 110

Thr Leu Pro Trp Thr Phe Gly Gly Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 49
<211> 702
<212> DNA
<213> Artificial Sequence

<220>

<223>  humanized antibody

<220>
<221>  misc_feature
<223>  nucleotide sequence encoding humanized antibody light chain hL4

<400>  49
atggtgctgc agacccaggt gttcatctcc ctgctgctgt ggatctccgg cgcgtacggc      60

gacatccaga tgacccagag ccctagcagc ctgagcgcca gcgtgggcga cagagtgacc     120

atcacctgta gagccagcca ggacatcaac aactacctga actggtatca gcagaaaccc     180

ggcggagccg tgaagctgct gatctacttc accagcagac tgcacagcgg cgtgcccagc     240

agattttctg gcagcggctc cggcaccgac tacaccctga caatcagcag cctgcagccc     300

gaggacttcg ccacctacta ctgccagcag ggcaacaccc tgccctggac atttggcgga     360

ggcaccaagg tggaaatcaa gcgtacggtg gccgccccct ccgtgttcat cttccccccc     420

tccgacgagc agctgaagtc cggcaccgcc tccgtggtgt gcctgctgaa taacttctac     480

cccagagagg ccaaggtgca gtggaaggtg gacaacgccc tgcagtccgg gaactcccag     540

gagagcgtga ccgagcagga cagcaaggac agcacctaca gcctgagcag caccctgacc     600

ctgagcaaag ccgactacga gaagcacaag gtgtacgcct gcgaggtgac ccaccagggc     660

ctgagctccc ccgtcaccaa gagcttcaac aggggggagt gt                        702

<210>  50
<211>  107
<212>  PRT
<213>  Artificial Sequence

<220>
<223>  humanized antibody

<220>
<221>  MISC_FEATURE
<223>  amino acid sequence of variable region of humanized antibody
       light chain hL4

<400>  50

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asn Asn Tyr
            20                  25                  30

Leu Asn Trp Tyr Gln Gln Lys Pro Gly Gly Ala Val Lys Leu Leu Ile
        35                  40                  45

Tyr Phe Thr Ser Arg Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Gly Asn Thr Leu Pro Trp
                85                  90                  95

Thr Phe Gly Gly Gly Thr Lys Val Glu Ile Lys
            100                 105

<210> 51
<211> 321
<212> DNA
<213> Artificial Sequence

<220>
<223> humanized antibody

<220>
<221> misc_feature
<223> nucleotide sequence encoding variable region of humanized antibody light chain hL4

<400> 51
gacatccaga tgacccagag ccctagcagc ctgagcgcca gcgtgggcga cagagtgacc 60

atcacctgta gagccagcca ggacatcaac aactacctga actggtatca gcagaaaccc 120

ggcggagccg tgaagctgct gatctacttc accagcagac tgcacagcgg cgtgcccagc 180

agattttctg gcagcggctc cggcaccgac tacaccctga caatcagcag cctgcagccc 240

gaggacttcg ccacctacta ctgccagcag ggcaacaccc tgccctggac atttggcgga 300

ggcaccaagg tggaaatcaa g 321

<210> 52
<211> 471
<212> PRT
<213> Artificial Sequence

<220>
<223> humanized antibody

<220>
<221> MISC_FEATURE
<223> amino acid sequence of humanized antibody heavy chain hH1

<400> 52

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
35 40 45

Thr Glu Tyr Thr Met His Trp Val Arg Gln Ala Pro Gly Gln Gly Leu
50 55 60

Glu Trp Met Gly Gly Val Asn Pro Asn Ser Gly Asp Thr Ser Tyr Ala
65 70 75 80

Gln Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Pro Gly Gly Tyr Asp Val Gly Tyr Tyr Ala Met
115 120 125

Asp Tyr Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Ala Ala Gly Gly Pro Ser Val Phe Leu Phe Pro Pro Lys Pro Lys
260 265 270

Asp Thr Leu Met Ile Ser Arg Thr Pro Glu Val Thr Cys Val Val Val

275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 53
<211> 1413
<212> DNA
<213> Artificial Sequence

<220>
<223> humanized antibody

<220>
<221> misc_feature
<223> nucleotide sequence encoding humanized antibody heavy chain hH1

<400> 53
atgaaacacc tgtggttctt cctcctgctg gtggcagctc ccagatgggt gctgagccag 60

gtgcagctgg tgcagtctgg cgccgaagtg aagaaaccag gcgccagcgt gaaggtgtcc 120

tgcaaggcca gcggctacac ctttaccgag tacaccatgc actgggtgcg ccaggctcca 180

ggccagggac tggaatggat gggcggcgtg aaccccaaca gcggcgatac aagctacgcc 240

cagaaattcc agggcagagt gaccatcacc gccgacacca gcacctccac cgcctacatg 300

gaactgagca gcctgcggag cgaggacacc gccgtgtact actgtgctag acctggcggc 360

tacgacgtgg gctactacgc catggattac tggggccagg gcaccctcgt gaccgtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga agccgcgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 54
<211> 122
<212> PRT
<213> Artificial Sequence

<220>
<223> humanized antibody

<220>
<221> MISC_FEATURE
<223> amino acid sequence of variable region of humanized antibody

heavy chain hH1

<400> 54

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Glu Tyr
20 25 30

Thr Met His Trp Val Arg Gln Ala Pro Gly Gln Gly Leu Glu Trp Met
35 40 45

Gly Gly Val Asn Pro Asn Ser Gly Asp Thr Ser Tyr Ala Gln Lys Phe
50 55 60

Gln Gly Arg Val Thr Ile Thr Ala Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Pro Gly Gly Tyr Asp Val Gly Tyr Tyr Ala Met Asp Tyr Trp
100 105 110

Gly Gln Gly Thr Leu Val Thr Val Ser Ser
115 120

<210> 55
<211> 366
<212> DNA
<213> Artificial Sequence

<220>
<223> humanized antibody

<220>
<221> misc_feature
<223> nucleotide sequence encoding variable region of humanized antibody heavy chain hH1

<400> 55
caggtgcagc tggtgcagtc tggcgccgaa gtgaagaaac caggcgccag cgtgaaggtg 60

tcctgcaagg ccagcggcta cacctttacc gagtacacca tgcactgggt gcgccaggct 120

ccaggccagg gactggaatg gatgggcggc gtgaacccca acagcggcga tacaagctac 180

gcccagaaat tccagggcag agtgaccatc accgccgaca ccagcacctc caccgcctac 240

atggaactga gcagcctgcg gagcgaggac accgccgtgt actactgtgc tagacctggc 300

ggctacgacg tgggctacta cgccatggat tactggggcc agggcaccct cgtgaccgtc 360

agctca 366

<210> 56
<211> 471
<212> PRT
<213> Artificial Sequence

<220>
<223> humanized antibody

<220>
<221> MISC_FEATURE
<223> amino acid sequence of humanized antibody heavy chain hH2

<400> 56

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Thr Ser Gly Tyr Thr Phe
35 40 45

Thr Glu Tyr Thr Met His Trp Val Arg Gln Ala Pro Gly Lys Ser Leu
50 55 60

Glu Trp Met Gly Gly Val Asn Pro Asn Ser Gly Asp Thr Ser Tyr Ala
65 70 75 80

Gln Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Pro Gly Gly Tyr Asp Val Gly Tyr Tyr Ala Met
115 120 125

Asp Tyr Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His

180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Ala Ala Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 57
<211> 1413
<212> DNA
<213> Artificial Sequence

<220>
<223> humanized antibody

<220>
<221> misc_feature
<223> nucleotide sequence encoding humanized antibody heavy chain hH2

<400> 57
atgaaacacc tgtggttctt cctcctgctg gtggcagctc ccagatgggt gctgagcgaa 60

gtgcagctgg tgcagtctgg cgccgaagtg aagaaaccag gcgccagcgt gaaggtgtcc 120

tgcaagacca gcggctacac ctttaccgag tacaccatgc actgggtgcg ccaggcccct 180

ggcaagagcc tggaatggat gggcggcgtg aaccccaaca gcggcgatac aagctacgcc 240

cagaaattcc agggcagagt gaccatcacc gccgacacca gcacctccac cgcctacatg 300

gaactgagca gcctgcggag cgaggacacc gccgtgtact actgtgctag acctggcggc 360

tacgacgtgg gctactacgc catggattac tggggccagg gcaccctcgt gaccgtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga agccgcgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 58
<211> 122
<212> PRT
<213> Artificial Sequence

<220>
<223> humanized antibody

<220>
<221> MISC_FEATURE
<223> amino acid sequence of variable region of humanized antibody heavy chain hH2

<400> 58

Glu Val Gln Leu Val Gln Ser Gly Ala Glu Val Lys Lys Pro Gly Ala
1 5 10 15

Ser Val Lys Val Ser Cys Lys Thr Ser Gly Tyr Thr Phe Thr Glu Tyr
20 25 30

Thr Met His Trp Val Arg Gln Ala Pro Gly Lys Ser Leu Glu Trp Met
35 40 45

Gly Gly Val Asn Pro Asn Ser Gly Asp Thr Ser Tyr Ala Gln Lys Phe
50 55 60

Gln Gly Arg Val Thr Ile Thr Ala Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Pro Gly Gly Tyr Asp Val Gly Tyr Tyr Ala Met Asp Tyr Trp
100 105 110

Gly Gln Gly Thr Leu Val Thr Val Ser Ser
115 120

<210> 59
<211> 366
<212> DNA

<213> Artificial Sequence

<220>
<223> humanized antibody

<220>
<221> misc_feature
<223> nucleotide sequence encoding variable region of humanized antibody heavy chain hH2

<400> 59
gaagtgcagc tggtgcagtc tggcgccgaa gtgaagaaac caggcgccag cgtgaaggtg 60

tcctgcaaga ccagcggcta cacctttacc gagtacacca tgcactgggt gcgccaggcc 120

cctggcaaga gcctggaatg gatgggcggc gtgaacccca acagcggcga tacaagctac 180

gcccagaaat tccagggcag agtgaccatc accgccgaca ccagcacctc caccgcctac 240

atggaactga gcagcctgcg gagcgaggac accgccgtgt actactgtgc tagacctggc 300

ggctacgacg tgggctacta cgccatggat tactggggcc agggcaccct cgtgaccgtc 360

agctca 366

<210> 60
<211> 471
<212> PRT
<213> Artificial Sequence

<220>
<223> humanized antibody

<220>
<221> MISC_FEATURE
<223> amino acid sequence of humanized antibody heavy chain hH3

<400> 60

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Thr Ser Gly Tyr Thr Phe
35 40 45

Thr Glu Tyr Thr Met His Trp Val Arg Gln Ala Pro Gly Gln Gly Leu
50 55 60

Glu Trp Met Gly Gly Val Asn Pro Asn Ser Gly Asp Thr Ser Tyr Ala
65 70 75 80

Gln Lys Phe Gln Gly Arg Val Thr Leu Thr Val Asp Lys Ser Thr Ser

85 90 95

Thr Ala Tyr Met Glu Leu Ser Ser Leu Arg Ser Glu Asp Thr Ala Val
100 105 110

Tyr Tyr Cys Ala Arg Pro Gly Gly Tyr Asp Val Gly Tyr Tyr Ala Met
115 120 125

Asp Tyr Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Ala Ala Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

```
Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Glu Glu Met Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470


<210>  61
<211>  1413
<212>  DNA
<213>  Artificial Sequence

<220>
<223>  humanized antibody


<220>
<221>  misc_feature
<223>  nucleotide sequence encoding humanized antibody heavy chain hH3

<400>  61
atgaaacacc tgtggttctt cctcctgctg gtggcagctc ccagatgggt gctgagcgaa      60

gtgcagctgg tgcagtctgg cgccgaagtg aagaaaccag gcgccagcgt gaaggtgtcc     120

tgcaagacca gcggctacac ctttaccgag tacaccatgc actgggtgcg ccaggctcca     180

ggccagggac tggaatggat gggcggcgtg aaccccaaca gcggcgatac aagctacgcc     240

cagaaattcc agggcagagt gaccctgacc gtggacaaga gcaccagcac cgcctacatg     300

gaactgagca gcctgcggag cgaggacacc gccgtgtact actgtgctag acctggcggc     360

tacgacgtgg gctactacgc catggattac tggggccagg gcaccctcgt gaccgtcagc     420
```

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga agccgcgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 62
<211> 122
<212> PRT
<213> Artificial Sequence

<220>
<223> humanized antibody

<220>
<221> MISC_FEATURE
<223> amino acid sequence of variable region of humanized antibody heavy chain hH3

<400> 62

Glu Val Gln Leu Val Gln Ser Gly Ala Glu Val Lys Lys Pro Gly Ala
1 5 10 15

Ser Val Lys Val Ser Cys Lys Thr Ser Gly Tyr Thr Phe Thr Glu Tyr
20 25 30

Thr Met His Trp Val Arg Gln Ala Pro Gly Gln Gly Leu Glu Trp Met
35 40 45

Gly Gly Val Asn Pro Asn Ser Gly Asp Thr Ser Tyr Ala Gln Lys Phe
    50                  55                  60

Gln Gly Arg Val Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Pro Gly Gly Tyr Asp Val Gly Tyr Tyr Ala Met Asp Tyr Trp
            100                 105                 110

Gly Gln Gly Thr Leu Val Thr Val Ser Ser
        115                 120

<210> 63
<211> 366
<212> DNA
<213> Artificial Sequence

<220>
<223> humanized antibody

<220>
<221> misc_feature
<223> nucleotide sequence encoding variable region of humanized antibody heavy chain hH3

<400> 63
gaagtgcagc tggtgcagtc tggcgccgaa gtgaagaaac caggcgccag cgtgaaggtg 60

tcctgcaaga ccagcggcta cacctttacc gagtacacca tgcactgggt gcgccaggct 120

ccaggccagg gactggaatg gatgggcggc gtgaacccca acagcggcga tacaagctac 180

gcccagaaat tccagggcag agtgaccctg accgtggaca agagcaccag caccgcctac 240

atggaactga gcagcctgcg gagcgaggac accgccgtgt actactgtgc tagacctggc 300

ggctacgacg tgggctacta cgccatggat tactggggcc agggcaccct cgtgaccgtc 360

agctca 366

<210> 64
<211> 214
<212> PRT
<213> Artificial Sequence

<220>
<223> humanized antibody

<220>
<221> MISC_FEATURE
<223> amino acid sequence of Trastuzumab light chain

<400> 64

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 65
<211> 450
<212> PRT
<213> Artificial Sequence

```
<220>
<223>  humanized antibody


<220>
<221>  MISC_FEATURE
<223>  amino acid sequence of Trastuzumab heavy chain

<400>  65

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Asn Ile Lys Asp Thr
            20                  25                  30


Tyr Ile His Trp Val Arg Gln Ala Pro Gly Lys Gly Leu Glu Trp Val
        35                  40                  45


Ala Arg Ile Tyr Pro Thr Asn Gly Tyr Thr Arg Tyr Ala Asp Ser Val
    50                  55                  60


Lys Gly Arg Phe Thr Ile Ser Ala Asp Thr Ser Lys Asn Thr Ala Tyr
65                  70                  75                  80


Leu Gln Met Asn Ser Leu Arg Ala Glu Asp Thr Ala Val Tyr Tyr Cys
                85                  90                  95


Ser Arg Trp Gly Gly Asp Gly Phe Tyr Ala Met Asp Tyr Trp Gly Gln
            100                 105                 110


Gly Thr Leu Val Thr Val Ser Ser Ala Ser Thr Lys Gly Pro Ser Val
        115                 120                 125


Phe Pro Leu Ala Pro Ser Ser Lys Ser Thr Ser Gly Gly Thr Ala Ala
    130                 135                 140


Leu Gly Cys Leu Val Lys Asp Tyr Phe Pro Glu Pro Val Thr Val Ser
145                 150                 155                 160


Trp Asn Ser Gly Ala Leu Thr Ser Gly Val His Thr Phe Pro Ala Val
                165                 170                 175


Leu Gln Ser Ser Gly Leu Tyr Ser Leu Ser Ser Val Val Thr Val Pro
            180                 185                 190


Ser Ser Ser Leu Gly Thr Gln Thr Tyr Ile Cys Asn Val Asn His Lys
        195                 200                 205
```

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 66
<211> 234
<212> PRT
<213> Artificial Sequence

<220>
<223> humanized antibody

<220>
<221> MISC_FEATURE
<223> amino acid sequence of anti-LPS antibody (h#1G5-H1L1) light chain

<400> 66

```
Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Asp Ser Leu Ala
            20                  25                  30

Val Ser Leu Gly Glu Arg Ala Thr Ile Asn Cys Lys Ala Ser Glu Asn
        35                  40                  45

Val Gly Asn Ser Val Ser Trp Tyr Gln Gln Lys Pro Gly Gln Pro Pro
    50                  55                  60

Lys Leu Leu Ile Tyr Gly Ala Ser Asn Arg Tyr Thr Gly Val Pro Asp
65                  70                  75                  80

Arg Phe Ser Gly Ser Gly Ser Gly Thr Asp Phe Thr Leu Thr Ile Ser
                85                  90                  95

Ser Leu Gln Ala Glu Asp Val Ala Val Tyr Tyr Cys Gly Gln Ser Tyr
            100                 105                 110

Ser Tyr Pro Tyr Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190
```

```
Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230


<210>  67
<211>  474
<212>  PRT
<213>  Artificial Sequence

<220>
<223>  humanized antibody


<220>
<221>  MISC_FEATURE
<223>  amino acid sequence of anti-LPS antibody (h#1G5-H1L1) heavy chain

<400>  67

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Trp Ile Asn Trp Val Arg Gln Ala Pro Gly Gln Gly Leu
    50                  55                  60

Glu Trp Met Gly Asn Ile Tyr Pro Gly Ser Ser Ser Ile Asn Tyr Asn
65                  70                  75                  80

Glu Lys Phe Lys Ser Arg Val Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Thr Ile Tyr Asn Tyr Gly Ser Ser Gly Tyr Asn
        115                 120                 125

Tyr Ala Met Asp Tyr Trp Gly Gln Gly Thr Leu Val Thr Val Ser Ser
    130                 135                 140
```

Ala Ser Thr Lys Gly Pro Ser Val Phe Pro Leu Ala Pro Ser Ser Lys
145 150 155 160

Ser Thr Ser Gly Gly Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Gln Thr
210 215 220

Tyr Ile Cys Asn Val Asn His Lys Pro Ser Asn Thr Lys Val Asp Lys
225 230 235 240

Arg Val Glu Pro Lys Ser Cys Asp Lys Thr His Thr Cys Pro Pro Cys
245 250 255

Pro Ala Pro Glu Leu Leu Gly Gly Pro Ser Val Phe Leu Phe Pro Pro
260 265 270

Lys Pro Lys Asp Thr Leu Met Ile Ser Arg Thr Pro Glu Val Thr Cys
275 280 285

Val Val Val Asp Val Ser His Glu Asp Pro Glu Val Lys Phe Asn Trp
290 295 300

Tyr Val Asp Gly Val Glu Val His Asn Ala Lys Thr Lys Pro Arg Glu
305 310 315 320

Glu Gln Tyr Asn Ser Thr Tyr Arg Val Val Ser Val Leu Thr Val Leu
325 330 335

His Gln Asp Trp Leu Asn Gly Lys Glu Tyr Lys Cys Lys Val Ser Asn
340 345 350

Lys Ala Leu Pro Ala Pro Ile Glu Lys Thr Ile Ser Lys Ala Lys Gly
355 360 365

Gln Pro Arg Glu Pro Gln Val Tyr Thr Leu Pro Pro Ser Arg Glu Glu
370 375 380

Met Thr Lys Asn Gln Val Ser Leu Thr Cys Leu Val Lys Gly Phe Tyr
385 390 395 400

Pro Ser Asp Ile Ala Val Glu Trp Glu Ser Asn Gly Gln Pro Glu Asn
405 410 415

Asn Tyr Lys Thr Thr Pro Pro Val Leu Asp Ser Asp Gly Ser Phe Phe
420 425 430

Leu Tyr Ser Lys Leu Thr Val Asp Lys Ser Arg Trp Gln Gln Gly Asn
435 440 445

Val Phe Ser Cys Ser Val Met His Glu Ala Leu His Asn His Tyr Thr
450 455 460

Gln Lys Ser Leu Ser Leu Ser Pro Gly Lys
465 470

<210> 68
<211> 234
<212> PRT
<213> Artificial Sequence

<220>
<223> humanized antibody

<220>
<221> MISC_FEATURE
<223> amino acid sequence of anti-TROP2 antibody (hRS7) light chain

<400> 68

Met Val Leu Gln Thr Gln Val Phe Ile Ser Leu Leu Leu Trp Ile Ser
1 5 10 15

Gly Ala Tyr Gly Asp Ile Gln Leu Thr Gln Ser Pro Ser Ser Leu Ser
20 25 30

Ala Ser Val Gly Asp Arg Val Ser Ile Thr Cys Lys Ala Ser Gln Asp
35 40 45

Val Ser Ile Ala Val Ala Trp Tyr Gln Gln Lys Pro Gly Lys Ala Pro
50 55 60

Lys Leu Leu Ile Tyr Ser Ala Ser Tyr Arg Tyr Thr Gly Val Pro Asp
65 70 75 80

Arg Phe Ser Gly Ser Gly Ser Gly Thr Asp Phe Thr Leu Thr Ile Ser
85 90 95

Ser Leu Gln Pro Glu Asp Phe Ala Val Tyr Tyr Cys Gln Gln His Tyr
100 105 110

```
Ile Thr Pro Leu Thr Phe Gly Ala Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230


<210>  69
<211>  470
<212>  PRT
<213>  Artificial Sequence

<220>
<223>  humanized antibody


<220>
<221>  MISC_FEATURE
<223>  amino acid sequence of anti-TROP2 antibody (hRS7) heavy chain

<400>  69

Met Lys His Leu Trp Phe Phe Leu Leu Leu Val Ala Ala Pro Arg Trp
1               5                   10                  15

Val Leu Ser Gln Val Gln Leu Gln Gln Ser Gly Ser Glu Leu Lys Lys
            20                  25                  30

Pro Gly Ala Ser Val Lys Val Ser Cys Lys Ala Ser Gly Tyr Thr Phe
        35                  40                  45

Thr Asn Tyr Gly Met Asn Trp Val Lys Gln Ala Pro Gly Gln Gly Leu
    50                  55                  60
```

```
Lys Trp Met Gly Trp Ile Asn Thr Tyr Thr Gly Glu Pro Thr Tyr Thr
65                  70                  75                  80

Asp Asp Phe Lys Gly Arg Phe Ala Phe Ser Leu Asp Thr Ser Val Ser
                85                  90                  95

Thr Ala Tyr Leu Gln Ile Ser Ser Leu Lys Ala Asp Asp Thr Ala Val
            100                 105                 110

Tyr Phe Cys Ala Arg Gly Gly Phe Gly Ser Ser Tyr Trp Tyr Phe Asp
        115                 120                 125

Val Trp Gly Gln Gly Ser Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Arg Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
```

305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Glu Glu Met Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys
465 470

<210> 70
<211> 240
<212> PRT
<213> Artificial Sequence

<220>
<223> humanized antibody

<220>
<221> MISC_FEATURE
<223> amino acid sequence of anti-CD98 antibody (hM23-H1L1) light chain

<400> 70

Met Val Leu Gln Thr Gln Val Phe Ile Ser Leu Leu Leu Trp Ile Ser
1 5 10 15

Gly Ala Tyr Gly Asp Ile Val Met Thr Gln Ser Pro Asp Ser Leu Ala

20 25 30

Val Ser Leu Gly Glu Arg Ala Thr Ile Asn Cys Lys Ser Ser Gln Ser
35 40 45

Leu Leu Tyr Ser Ser Asn Gln Lys Asn Tyr Leu Ala Trp Tyr Gln Gln
50 55 60

Lys Pro Gly Gln Pro Pro Lys Leu Leu Ile Tyr Trp Ala Ser Thr Arg
65 70 75 80

Glu Ser Gly Val Pro Asp Arg Phe Ser Gly Ser Gly Ser Gly Thr Asp
85 90 95

Phe Thr Leu Thr Ile Ser Ser Leu Gln Ala Glu Asp Val Ala Val Tyr
100 105 110

Tyr Cys Gln Arg Tyr Tyr Gly Tyr Pro Trp Thr Phe Gly Gln Gly Thr
115 120 125

Lys Val Glu Ile Lys Arg Thr Val Ala Ala Pro Ser Val Phe Ile Phe
130 135 140

Pro Pro Ser Asp Glu Gln Leu Lys Ser Gly Thr Ala Ser Val Val Cys
145 150 155 160

Leu Leu Asn Asn Phe Tyr Pro Arg Glu Ala Lys Val Gln Trp Lys Val
165 170 175

Asp Asn Ala Leu Gln Ser Gly Asn Ser Gln Glu Ser Val Thr Glu Gln
180 185 190

Asp Ser Lys Asp Ser Thr Tyr Ser Leu Ser Ser Thr Leu Thr Leu Ser
195 200 205

Lys Ala Asp Tyr Glu Lys His Lys Val Tyr Ala Cys Glu Val Thr His
210 215 220

Gln Gly Leu Ser Ser Pro Val Thr Lys Ser Phe Asn Arg Gly Glu Cys
225 230 235 240

<210> 71
<211> 465
<212> PRT
<213> Artificial Sequence

<220>
<223> humanized antibody

<220>
<221> MISC_FEATURE
<223> amino acid sequence of anti-CD98 antibody (hM23-H1L1) heavy chain

<400> 71

Met Lys His Leu Trp Phe Phe Leu Leu Leu Val Ala Ala Pro Arg Trp
1 5 10 15

Val Leu Ser Gln Val Gln Leu Val Gln Ser Gly Ala Glu Val Lys Lys
20 25 30

Pro Gly Ala Ser Val Lys Val Ser Cys Lys Ala Ser Gly Tyr Ala Phe
35 40 45

Ser Asn Tyr Leu Ile Glu Trp Val Arg Gln Ala Pro Gly Gln Gly Leu
50 55 60

Glu Trp Met Gly Val Ile Asn Pro Gly Ser Gly Val Thr Asn Tyr Asn
65 70 75 80

Glu Lys Phe Lys Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Ala Glu Ala Trp Phe Ala Tyr Trp Gly Gln Gly
115 120 125

Thr Leu Val Thr Val Ser Ser Ala Ser Thr Lys Gly Pro Ser Val Phe
130 135 140

Pro Leu Ala Pro Ser Ser Lys Ser Thr Ser Gly Gly Thr Ala Ala Leu
145 150 155 160

Gly Cys Leu Val Lys Asp Tyr Phe Pro Glu Pro Val Thr Val Ser Trp
165 170 175

Asn Ser Gly Ala Leu Thr Ser Gly Val His Thr Phe Pro Ala Val Leu
180 185 190

Gln Ser Ser Gly Leu Tyr Ser Leu Ser Ser Val Val Thr Val Pro Ser
195 200 205

Ser Ser Leu Gly Thr Gln Thr Tyr Ile Cys Asn Val Asn His Lys Pro
210 215 220

Ser Asn Thr Lys Val Asp Lys Arg Val Glu Pro Lys Ser Cys Asp Lys
225 230 235 240

Thr His Thr Cys Pro Pro Cys Pro Ala Pro Glu Leu Leu Gly Gly Pro
245 250 255

Ser Val Phe Leu Phe Pro Pro Lys Pro Lys Asp Thr Leu Met Ile Ser
260 265 270

Arg Thr Pro Glu Val Thr Cys Val Val Val Asp Val Ser His Glu Asp
275 280 285

Pro Glu Val Lys Phe Asn Trp Tyr Val Asp Gly Val Glu Val His Asn
290 295 300

Ala Lys Thr Lys Pro Arg Glu Glu Gln Tyr Asn Ser Thr Tyr Arg Val
305 310 315 320

Val Ser Val Leu Thr Val Leu His Gln Asp Trp Leu Asn Gly Lys Glu
325 330 335

Tyr Lys Cys Lys Val Ser Asn Lys Ala Leu Pro Ala Pro Ile Glu Lys
340 345 350

Thr Ile Ser Lys Ala Lys Gly Gln Pro Arg Glu Pro Gln Val Tyr Thr
355 360 365

Leu Pro Pro Ser Arg Glu Glu Met Thr Lys Asn Gln Val Ser Leu Thr
370 375 380

Cys Leu Val Lys Gly Phe Tyr Pro Ser Asp Ile Ala Val Glu Trp Glu
385 390 395 400

Ser Asn Gly Gln Pro Glu Asn Asn Tyr Lys Thr Thr Pro Pro Val Leu
405 410 415

Asp Ser Asp Gly Ser Phe Phe Leu Tyr Ser Lys Leu Thr Val Asp Lys
420 425 430

Ser Arg Trp Gln Gln Gly Asn Val Phe Ser Cys Ser Val Met His Glu
435 440 445

Ala Leu His Asn His Tyr Thr Gln Lys Ser Leu Ser Leu Ser Pro Gly
450 455 460

Lys
465

<210> 72
<211> 702
<212> DNA
<213> Artificial Sequence

<220>
<223> humanized antibody

<220>
<221> misc_feature
<223> nucleotide sequence encoding Trastuzumab mutant light chain

<400> 72
atggtgctgc agacccaggt gttcatctcc ctgctgctgt ggatctccgg cgcgtacggc 60

gacatccaga tgacacagag ccctagcagc ctgtctgcca gcgtgggaga cagagtgacc 120

atcacctgta gagccagcca ggacgtgaac acagccgtgg cttggtatca gcagaagcct 180

ggcaaggccc ctaagctgct gatctacagc gccagctttc tgtacagcgg cgtgcccagc 240

agattcagcg gctctagaag cggcaccgac ttcaccctga ccataagcag tctgcagccc 300

gaggacttcg ccacctacta ctgtcagcag cactacacca cacctccaac ctttggccag 360

ggcaccaagg tggaaatcaa gcgtacggtg gccgcccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 73
<211> 234
<212> PRT
<213> Artificial Sequence

<220>
<223> humanized antibody

<220>
<221> MISC_FEATURE
<223> amino acid sequence of Trastuzumab mutant light chain

<400> 73

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Arg Ala Ser Gln Asp
35 40 45

Val Asn Thr Ala Val Ala Trp Tyr Gln Gln Lys Pro Gly Lys Ala Pro
50 55 60

Lys Leu Leu Ile Tyr Ser Ala Ser Phe Leu Tyr Ser Gly Val Pro Ser
65 70 75 80

Arg Phe Ser Gly Ser Arg Ser Gly Thr Asp Phe Thr Leu Thr Ile Ser
85 90 95

Ser Leu Gln Pro Glu Asp Phe Ala Thr Tyr Tyr Cys Gln Gln His Tyr
100 105 110

Thr Thr Pro Pro Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 74
<211> 1407
<212> DNA
<213> Artificial Sequence

<220>
<223> humanized antibody

<220>
<221> misc_feature

<223> nucleotide sequence encoding Trastuzumab mutant heavy chain

<400> 74
atgaaacacc tgtggttctt cctcctgctg gtggcagctc ccagatgggt gctgagcgag 60

gtgcagctgg ttgaatctgg cggaggactg gttcagcctg gcggatctct gagactgtct 120

tgtgccgcca gcggcttcaa catcaaggac acctacatcc actgggtccg acaggcccct 180

ggcaaaggac ttgaatgggt cgccagaatc taccccacca acggctacac cagatacgcc 240

gactctgtga agggcagatt caccatcagc gccgacacca gcaagaacac cgcctacctg 300

cagatgaaca gcctgagagc cgaggacacc gccgtgtact actgttctag atggggaggc 360

gacggcttct acgccatgga ttattggggc cagggcaccc tggttaccgt tagctcagcc 420

tccaccaagg gcccaagcgt cttccccctg gcaccctcct ccaagagcac ctctggcggc 480

acagccgccc tgggctgcct ggtcaaggac tacttccccg aacccgtgac cgtgagctgg 540

aactcaggcg ccctgaccag cggcgtgcac accttccccg ctgtcctgca gtcctcagga 600

ctctactccc tcagcagcgt ggtgaccgtg ccctccagca gcttgggcac ccagacctac 660

atctgcaacg tgaatcacaa gcccagcaac accaaggtgg acaagagagt tgagcccaaa 720

tcttgtgaca aaactcacac atgcccaccc tgcccagcac ctgaagccgc gggggggaccc 780

tcagtcttcc tcttcccccc aaaacccaag gacaccctca tgatctcccg gacccctgag 840

gtcacatgcg tggtggtgga cgtgagccac gaagaccctg aggtcaagtt caactggtac 900

gtggacggcg tggaggtgca taatgccaag acaaagcccc gggaggagca gtacaacagc 960

acgtaccggg tggtcagcgt cctcaccgtc ctgcaccagg actggctgaa tggcaaggag 1020

tacaagtgca aggtctccaa caaagccctc ccagccccca tcgagaaaac catctccaaa 1080

gccaaaggcc agccccggga accacaggtg tacaccctgc ccccatcccg ggaggagatg 1140

accaagaacc aggtcagcct gacctgcctg gtcaaaggct tctatcccag cgacatcgcc 1200

gtggagtggg agagcaatgg ccagcccgag aacaactaca agaccacccc tcccgtgctg 1260

gactccgacg gctccttctt cctctacagc aagctcaccg tggacaagag caggtggcag 1320

cagggcaacg tcttctcatg ctccgtgatg catgaggctc tgcacaacca ctacacccag 1380

aagagcctct ccctgtctcc cggcaaa 1407

<210> 75
<211> 469
<212> PRT
<213> Artificial Sequence

<220>
<223> humanized antibody

<220>
<221> MISC_FEATURE

<223> amino acid sequence of Trastuzumab mutant heavy chain

<400> 75

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Asn Ile
        35                  40                  45

Lys Asp Thr Tyr Ile His Trp Val Arg Gln Ala Pro Gly Lys Gly Leu
    50                  55                  60

Glu Trp Val Ala Arg Ile Tyr Pro Thr Asn Gly Tyr Thr Arg Tyr Ala
65                  70                  75                  80

Asp Ser Val Lys Gly Arg Phe Thr Ile Ser Ala Asp Thr Ser Lys Asn
                85                  90                  95

Thr Ala Tyr Leu Gln Met Asn Ser Leu Arg Ala Glu Asp Thr Ala Val
            100                 105                 110

Tyr Tyr Cys Ser Arg Trp Gly Gly Asp Gly Phe Tyr Ala Met Asp Tyr
        115                 120                 125

Trp Gly Gln Gly Thr Leu Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Arg Val Glu Pro Lys
225                 230                 235                 240

Ser Cys Asp Lys Thr His Thr Cys Pro Pro Cys Pro Ala Pro Glu Ala
245 250 255

Ala Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Glu Glu Met Thr Lys Asn Gln
370 375 380

Val Ser Leu Thr Cys Leu Val Lys Gly Phe Tyr Pro Ser Asp Ile Ala
385 390 395 400

Val Glu Trp Glu Ser Asn Gly Gln Pro Glu Asn Asn Tyr Lys Thr Thr
405 410 415

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly Lys
465

<210> 76
<211> 234

<212> PRT
<213> Artificial Sequence

<220>
<223> humanized antibody

<220>
<221> MISC_FEATURE
<223> amino acid sequence of Trastuzumab mutant 2 light chain

<400> 76

```
Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Arg Ala Ser Gln Asp
        35                  40                  45

Val Asn Thr Ala Val Ala Trp Tyr Gln Gln Lys Pro Gly Lys Ala Pro
    50                  55                  60

Lys Leu Leu Ile Tyr Ser Ala Ser Phe Leu Tyr Ser Gly Val Pro Ser
65                  70                  75                  80

Arg Phe Ser Gly Ser Arg Ser Gly Thr Asp Phe Thr Leu Thr Ile Ser
                85                  90                  95

Ser Leu Gln Pro Glu Asp Phe Ala Thr Tyr Tyr Cys Gln Gln His Tyr
            100                 105                 110

Thr Thr Pro Pro Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205
```

```
His Lys Val Tyr Ala Cys Glu Val Thr His Gln Gly Leu Ser Ser Pro
    210                 215                 220


Val Thr Lys Ser Phe Asn Arg Gly Glu Cys
225                 230


<210>  77
<211>  469
<212>  PRT
<213>  Artificial Sequence

<220>
<223>  humanized antibody


<220>
<221>  MISC_FEATURE
<223>  amino acid sequence of Trastuzumab mutant 2 heavy chain

<400>  77

Met Lys His Leu Trp Phe Phe Leu Leu Leu Val Ala Ala Pro Arg Trp
1               5                   10                  15


Val Leu Ser Glu Val Gln Leu Val Glu Ser Gly Gly Gly Leu Val Gln
            20                  25                  30


Pro Gly Gly Ser Leu Arg Leu Ser Cys Ala Ala Ser Gly Phe Asn Ile
        35                  40                  45


Lys Asp Thr Tyr Ile His Trp Val Arg Gln Ala Pro Gly Lys Gly Leu
    50                  55                  60


Glu Trp Val Ala Arg Ile Tyr Pro Thr Asn Gly Tyr Thr Arg Tyr Ala
65                  70                  75                  80


Asp Ser Val Lys Gly Arg Phe Thr Ile Ser Ala Asp Thr Ser Lys Asn
                85                  90                  95


Thr Ala Tyr Leu Gln Met Asn Ser Leu Arg Ala Glu Asp Thr Ala Val
            100                 105                 110


Tyr Tyr Cys Ser Arg Trp Gly Gly Asp Gly Phe Tyr Ala Met Asp Tyr
        115                 120                 125


Trp Gly Gln Gly Thr Leu Val Thr Val Ser Ser Ala Ser Thr Lys Gly
    130                 135                 140


Pro Ser Val Phe Pro Leu Ala Pro Ser Ser Lys Ser Thr Ser Gly Gly
145                 150                 155                 160
```

Thr Ala Ala Leu Gly Cys Leu Val Lys Asp Tyr Phe Pro Glu Pro Val
165 170 175

Thr Val Ser Trp Asn Ser Gly Ala Leu Thr Ser Gly Val His Thr Phe
180 185 190

Pro Ala Val Leu Gln Ser Ser Gly Leu Tyr Ser Leu Ser Ser Val Val
195 200 205

Thr Val Pro Ser Ser Ser Leu Gly Thr Gln Thr Tyr Ile Cys Asn Val
210 215 220

Asn His Lys Pro Ser Asn Thr Lys Val Asp Lys Lys Val Glu Pro Lys
225 230 235 240

Ser Cys Asp Lys Thr His Thr Cys Pro Pro Cys Pro Ala Pro Glu Ala
245 250 255

Ala Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Glu Glu Met Thr Lys Asn Gln
370 375 380

Val Ser Leu Thr Cys Leu Val Lys Gly Phe Tyr Pro Ser Asp Ile Ala
385 390 395 400

Val Glu Trp Glu Ser Asn Gly Gln Pro Glu Asn Asn Tyr Lys Thr Thr

405 410 415

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly Lys